(19) Europäisches Patentamt
European Patent Office
Office européen des brevets

(11) EP 3 692 167 B1

(12) **EUROPEAN PATENT SPECIFICATION**

(45) Date of publication and mention of the grant of the patent:
**13.03.2024 Bulletin 2024/11**

(21) Application number: **18792781.9**

(22) Date of filing: **04.10.2018**

(51) International Patent Classification (IPC):
***C12Q 1/6809*** *(2018.01)*

(52) Cooperative Patent Classification (CPC):
(C-Sets available)
**C12Q 1/6809** (Cont.)

(86) International application number:
**PCT/US2018/054292**

(87) International publication number:
**WO 2019/070930 (11.04.2019 Gazette 2019/15)**

(54) **RNA QUALITY ASSAY**

RNA-QUALITÄTSASSAY

ESSAI POUR DETERMINER LA QUALITÉ DE L'ARN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2017 US 201762568896 P**

(43) Date of publication of application:
**12.08.2020 Bulletin 2020/33**

(73) Proprietor: **Life Technologies Corporation**
**Carlsbad, CA 92008 (US)**

(72) Inventors:
- **VONNEGUT, Chrisgen**
  **Carlsbad**
  **California 92011 (US)**
- **CLARKE, Scott**
  **Carlsbad**
  **California 92008 (US)**

(74) Representative: **Thermo Fisher Scientific-**
**Life Sciences Solutions Group**
**Life Technologies**
**Frankfurter Straße 129b**
**64293 Darmstadt (DE)**

(56) References cited:
**WO-A1-2014/089421** **WO-A2-2004/090780**
**CN-A- 104 630 348** **US-A1- 2005 208 534**

- **TZU-HSUEH YANG ET AL: "Determination of RNA degradation by capillary electrophoresis with cyan lightemitted diode-induced fluorescence", JOURNAL OF CHROMATOGRAPHY A, ELSEVIER, AMSTERDAM, NL, vol. 1239, 21 March 2012 (2012-03-21), pages 78-84, XP028420006, ISSN: 0021-9673, DOI: 10.1016/J.CHROMA.2012.03.070 [retrieved on 2012-03-30]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6809, C12Q 2563/107, C12Q 2565/102**

## Description

### CROSS-REFERENCE

This application claims the benefit under 35 U.S.C. § 119(e) of U.S. Provisional Application No. 62/568,896, filed October 6, 2017.

### FIELD OF THE INVENTION

The present invention relates to methods of determining the integrity and quality of RNA samples.

### BACKGROUND

RNA is extremely susceptible to degradation and RNase is ubiquitous in the environment. However, isolation of intact RNA is essential for many techniques used, for example, in gene expression analysis.

The most common method used to assess the integrity of total RNA is agarose or acrylamide electrophoresis. General information about RNA integrity can be obtained by observing the staining intensity of the major ribosomal RNA (rRNA) bands and any degradation products. For eukaryotic rRNA, a 28S: 18S ratio of 2:1 is generally representative of intact RNA, but it is not always accurate. However, while the cost of analyzing RNA by gel electrophoresis is relatively low, the analysis requires a significant amount of handling and hands on time. Furthermore, this method does not reliably give accurate results on the integrity of the RNA sample.

An alternative method uses UV absorbance to measure RNA concentration and purity. Absorbance at 260 nm is used to measure the amount of nucleic acid in a sample. However, this method cannot determine the integrity of RNA in a sample because all RNA, whether intact or degraded, contributes to the absorbance at 260 nm and therefore intact RNA cannot be distinguished from degraded RNA.

A third alternative, the Agilent 2100 Bioanalyzer (Agilent Technologies, Santa Clara, CA) uses microfluidics to analyze RNA using sample-specific chips (see, for example, U.S. Patent No. 8,346,486). The 2100 Bioanalyzer uses kits that are specific for the sample of interest; for example, kits are available for small RNA comprising 200 nucleotides or less and microRNA as well as total RNA and mRNA. The 2100 Bioanalyzer is able to measure RNA integrity which is displayed as an RNA Integrity Number

**[0007]** (RIN). To determine the RIN, the instrument uses a proprietary algorithm that takes into account the entire electrophoretic trace of the RNA, not just the 28S and 18S rRNAs. However, the 2100 Bioanalyzer has several disadvantages including: time-consuming sample preparation with considerable hands-on time, lack of sample flexibility due to the requirement of sample-specific chips, and the costs of the instrument, reagents and chips may be prohibitive to some labs.

Other methods similar to the 2100 Bioanalyzer RIN analysis have been developed for obtaining a metric for RNA integrity or quality. For example, a microfluidic assay has been developed using the Perkin Elmer LabChip® GX Touch microfluidics platform (PerkinElmer, Inc., Waltham, MA) to analyze RNA by electrophoretic separation on microfluidic sipper chips. The resulting electropherogram is used to calculate a RNA Quality Score (RQS) which correlates with Agilent's RIN and follows the same 0 to 10 scale rating.

Yet another method for determining RNA quality uses a direct functional RT-PCR based test to assess human or mouse RNA for its ability to produce full-length cDNA. This method uses two sets of primers to amplify the 5' and 3' end fragments of a long mRNA for a reference gene. The resulting amplification products are separated by electrophoresis and the ratio of the 3' and 5' amplification products is obtained. A 3':5' ratio of 1 indicates the RNA is intact, while a 3':5' ratio of >3 indicates degraded RNA. The disadvantages of this method include a time-consuming work flow and the sample of interest must contain the gene to which the primers bind.

In addition, the standard methods described above for assessing RNA quality and integrity are insufficient for RNA isolated from formalin-fixed, paraffin-embedded (FFPE) tissue samples due to the extensive degradation and modification of the RNA from long-term storage and fixation methods.

TZU-HSUEH YANG ET AL: "Determination of RNA degradation by capillary electrophoresis with cyan lightemitted diode-induced fluorescence" JOURNAL OF CHROMATOGRAPHY A, vol. 1239, 21 March 2012, pages 78-84, describes a method for determining the RNA quality in a sample by capillary electrophoreses.

Thus, there is a need for a faster, easier method for measuring RNA sample quality, including RNA isolated from FFPE samples. The RNA quality method described herein is a reliable, easy to use method for determining whether the RNA in a sample has degraded.

## BRIEF SUMMARY OF THE INVENTION

The present invention provides a method of determining the extent of degradation of RNA in a sample, expressed as an RNA quality value, the method comprising:

a) combining a sample suspected of containing RNA with a first nucleic acid binding dye that selectively binds small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
b) incubating the sample for a period of time sufficient to allow the first nucleic acid binding dye and the second nucleic acid binding dye to combine with the RNA in the sample to form a first nucleic acid binding dye-RNA complex and a second nucleic acid binding dye-RNA complex;
c) illuminating the first nucleic acid binding dye-RNA complex and the second nucleic acid binding dye-RNA complex with an appropriate wavelength of light to form an illuminated sample mixture, wherein the first nucleic acid binding dye-RNA complex emits a first fluorescent signal and the second nucleic acid binding dye-RNA complex emits a second fluorescent signal;
d) detecting the first fluorescent signal and the second fluorescent signal; and
e) calculating the ratio of the first fluorescent signal to the second fluorescent signal, thereby obtaining the RNA quality value, wherein the RNA quality value represents the extent of degradation of RNA in the sample;

wherein the first nucleic acid binding dye is selected from a compound described in Table 1 and the second nucleic acid binding dye is selected from the compounds in Table 2.

The present invention provides a method of determining the extent of degradation of RNA in a sample, expressed as an RNA quality value, the method comprising:

a) combining a sample suspected of containing RNA with a first nucleic acid binding dye that selectively binds small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
b) incubating the sample for a period of time sufficient to allow the first nucleic acid binding dye and the second nucleic acid binding dye to combine with the RNA in the sample to form a first nucleic acid binding dye-RNA complex and a second nucleic acid binding dye-RNA complex;
c) illuminating the first nucleic acid binding dye-RNA complex and the second nucleic acid binding dye-RNA complex with an appropriate wavelength of light to form an illuminated sample mixture, wherein the first nucleic acid binding dye-RNA complex emits a first fluorescent signal and the second nucleic acid binding dye-RNA complex emits a second fluorescent signal;
d) detecting the first fluorescent signal and the second fluorescent signal; and
e) calculating the relationship of the intensity of the first fluorescent signal to intensity of the second fluorescent signal, thereby obtaining the RNA quality value, wherein the RNA quality value represents the extent of degradation of RNA in the sample;

wherein the first nucleic acid binding dye is selected from a compound described in Table 1 and the second nucleic acid binding dye is selected from the compounds in Table 2.

In certain embodiments of the methods provided herein, the intact RNA with secondary and/or tertiary structureis rRNA, tRNA or mRNA. The small RNA comprises 200 nucleotides (nt) or fewer. In certain embodiments, the small RNA is selected from miRNA, siRNA, fragments of mRNA, fragments of tRNA, or fragments of rRNA. In preferred embodiments, the intact RNA with secondary and/or tertiary structure is indicative of intact RNA and the small RNA is indicative of degraded RNA.

In certain embodiments, the first nucleic acid binding dye and the second nucleic acid binding dye are added to the sample at the same time. In certain embodiments, the first nucleic acid binding dye and the second nucleic acid binding dye are added to the sample separately or sequentially. In certain preferred embodiments, a dye solution comprising the first nucleic acid binding dye and the second nucleic acid binding dye is prepared and is used to administer the first nucleic acid binding dye and the second nucleic acid binding dye to the sample.

I In the claimed embodiments, the first nucleic acid binding dye is selected from a compound described in Table 1. In certain more preferred embodiments, the first nucleic acid binding dye is Compound **16,** Compound **17,** Compound **23,** Compound **24,** Compound **29,** Compound **30,** Compound **45,** Compound **46,** Compound **47,** Compound **52,** Compound **53,** Compound **59,** Compound **60** or Compound **61.**

The second nucleic acid binding dye is a cyanine selected from a compound described in Table 2. In certain

more preferred embodiments, the second nucleic acid binding dye is Compound **1,** Compound **2,** Compound **3,** Compound **4,** Compound **5,** Compound **6,** Compound **7,** Compound **8,** Compound **9,** Compound **10,** Compound **11,** Compound **12,** Compound **13,** Compound **14** or Compound **15.**

In certain embodiments of the methods provided herein, the first nucleic acid binding dye is Compound **16,** Compound **17,** Compound **23,** Compound **24,** Compound **29,** Compound **30,** Compound **45,** Compound **46,** Compound **47,** Compound **52,** Compound **53,** Compound **59,** Compound **60** or Compound **61,** and the second nucleic acid binding dye is Compound **1,** Compound **2,** Compound **3,** Compound **4,** Compound **7,** Compound **9,** Compound **10,** Compound **12,** Compound **13,** Compound **14** or Compound **15.**

In certain embodiments of the methods provided herein, the second nucleic acid binding dye has a RNA/DNA fluorescence enhancement ratio of ≥ 7, ≥ 10 or ≥ 20. In certain embodiments of the methods provided herein, the first nucleic acid binding dye has a single-stranded RNA fluorescence enhancement of ≥ 30%.

In certain embodiments of the methods provided herein, the detecting step is performed by fluorometry.

In certain embodiments, the methods provided herein further comprise:

preparing:

a first standard solution by combining the first nucleic acid binding dye and the second nucleic acid binding dye with a buffer blank;
a second standard solution by combining the first nucleic acid binding dye and the second nucleic acid binding dye with a small RNA standard; and
a third standard solution by combining the first nucleic acid binding dye and the second nucleic acid binding dye with an intact RNA with secondary and/or tertiary structurestandard;

incubating the first standard solution, the second standard solution and the third standard solution for a time sufficient for the first nucleic acid binding dye and the second nucleic acid binding dye to combine with the RNA in the standard, if present, to form a first standard-dye complex, a second standard-dye complex and a third standard-dye complex, respectively;
illuminating the first standard-dye complex, the second standard-dye complex and the third standard-dye complex with an appropriate wavelength of light to form a first illuminated standard mixture, a second illuminated standard mixture and a third illuminated standard mixture, respectively; and
detecting a fluorescent signal from each of the first illuminated standard mixture, the second illuminated standard mixture and the third illuminated standard mixture.

In certain embodiments, the methods provided herein further comprise determining the extent of degradation of the RNA present in the illuminated sample mixture based on comparison of the detectable fluorescent signal in the illuminated sample mixture with a ratio of the fluorescent signal of the second standard solution and the fluorescent signal of the first standard solution. In certain preferred embodiments, the step of determining the extent of degradation of the RNA present in the illuminated sample mixture is calculated by Formula (**I**). In certain more preferred embodiments, the step of determining the extent of degradation of the RNA present in the illuminated sample mixture is calculated by Formula (**II**).

In certain embodiments, the present disclosure provides a kit comprising:

a dye solution comprising a first nucleic acid binding dye that selectively binds small RNA comprising 200 nucleotides or less and a second nucleic acid binding dye that selectively binds intact RNA with secondary and/or tertiary structure as defined above ; and
instructions for use according to any of the methods described herein.

In certain embodiments, the present disclosure provides a kit comprising:

a first nucleic acid binding dye that selectively binds small RNA comprising 200 nucleotides or less;
a second nucleic acid binding dye that selectively binds intact RNA with secondary and/or tertiary structure; and
instructions for use according to any of the methods described herein,
wherein the first nucleic acid binding dye and the second nucleic acid binding dye are detectably distinct.

In certain embodiments of the kits provided herein, the kits further comprise:

a first standard solution comprising a buffer and no nucleic acid;
a second standard solution comprising a small RNA standard, wherein the small RNA standard consists of fewer

than 200 nucleotides; and
a third standard solution comprising a large and/or structured RNA standard.

In certain embodiments, the present disclosure provides an RNA quality staining solution comprising:

a first nucleic acid binding dye that preferentially binds small RNA;
a second nucleic acid binding dye that preferentially binds intact RNA with secondary and/or tertiary structure;
a buffer; and
an organic solvent,
wherein the first nucleic acid binding dye and the second nucleic acid binding dye are detectably distinct

and wherein the first nucleic acid binding dye is Compound **16,** Compound **17,** Compound **23,** Compound **24,** Compound **29,** Compound **30,** Compound **45,** Compound **46,** Compound **47,** Compound **52,** Compound **53,** Compound **59,** Compound **60** or Compound **61** and the second nucleic acid binding dye is Compound **1,** Compound **2,** Compound **3,** Compound **4,** Compound **7,** Compound **9,** Compound **10,** Compound **12,** Compound **13,** Compound **14** or Compound **15.**

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 is a schematic of a certain embodiment of the methods provided herein for determining RNA quality and integrity and the resulting RNA Quality Score ("IQ Score") described herein performed on a QUBIT™ Fluorometer (Thermo Fisher Scientific, Waltham, MA).

FIG. 2A and FIG. 2B are graphical representations of different selectivities of certain embodiments of the methods provided herein using pure ribosomal RNA (rRNA) or pure small interfering RNA (siRNA).

FIG. 3A and FIG. 3B depict certain embodiments of the methods described herein demonstrating simultaneous measurement of large and/or structured RNA and small RNA. FIG. 3A graphically depicts rRNA solutions with increasing small RNA measured at both 644/673 nm (red emission channel, top panel) and 500/525 nm (green emission channel, bottom panel). FIG. 3B graphically depicts the ratio of the green and red channels of a QUBIT™ Fluorometer (the "G/R Ratio") demonstrating that the G/R Ratio correlates only to the percentage of small RNA.

FIG. 4A and FIG. 4B demonstrate the correlation of emission channel ratio ("G/R Ratio") with the percentage of small RNA in a sample, according to certain embodiments of the methods described herein. FIG. 4A graphically depicts the same assay as described in FIG. 3A, but with both variable rRNA (red emission channel, bottom graph) and siRNA (green emission channel, top graph) concentrations, and demonstrates a linear relationship for the G/R Ratio, although the red channel experiences some influence from small RNA content. FIG. 4B graphically represents the ratio of the two channels (the G/R Ratio), which is linear with the exception of 100% siRNA where the ratio correlation deviates from the trend.

FIG. 5A and FIG. 5B graphically demonstrate the RNA quality metric according to certain embodiments of the present disclosure. The linear relationship of the methods according to the present disclosure can be mapped as a quality metric, described herein as an RNA Quality Score ("IQ Score" of Formula (I), see below). By dividing the green emission-to-red emission (G/R) ratio (e.g., the ratio of small RNA to large RNA) by a constant to scale it to 10, and subtracting it from 10 to reverse the slope, the ratio becomes a scale from 10 to 0, with 10 indicating 100% large and/or structured RNA, which is indicative of intact RNA, and 0 indicating 100% small RNA, which is indicative of degraded RNA.

FIG. 6 graphically demonstrates real-time RNA degradation. Various concentrations of RNase A were added in triplicate and the solution fluorescence was monitored over time using certain embodiments of the methods described herein. The graphs demonstrate that the assay indicates real-time rRNA degradation, similar to qPCR growth curves, but in reverse. The top graph depicts the green emission channel of the fluorometer which corresponds to small RNA comprising 200 nucleotides or less and the bottom graph depicts the red emission channel which corresponds to large and/or structured RNA.

FIG. 7 is a graphical demonstration of the Green emission/Red emission (G/R) ratio of the results from FIG. 6 as a predictor of the level of RNA degradation.

FIG. 8 is a graphical representation of monitoring real-time RNA degradation using the methods according to certain embodiments of the present disclosure. The methods described herein monitor or assess RNA degradation as well as the nature of the process as RNase actively degrades the RNA. Initially, the signal corresponding to large and/or structured RNA (red emission channel) rapidly decreases as the rRNA degrades while the signal corresponding to small RNA (green emission channel) rapidly increases as the rRNA is linearized and shortened. Over time, the signal corresponding to small RNA decreases again as the nucleic acid fragments degrade into smaller and smaller fragments that are no longer able to bind to the small RNA nucleic acid binding dye.

FIGs. 9A, 9B, 9C and 9D are graphical representations of a comparison of the methods according to certain embodiments of the present disclosure to the 2100 Bioanalyzer RIN (Agilent Technologies, Santa Clara, CA). A solution of rRNA (185 μl of a 100 ng/μl solution) was treated with RNase A (20.6 ng, final concentration = 750 fM) at timepoint 0. Aliquots were removed and treated with RNaseOUT™ Recombinant Ribonuclease Inhibitor (Thermo Fisher Scientific, Waltham, MA), a high affinity RNase inhibitor protein, at various timepoints. Samples were then run on both the 2100 Bioanalyzer and a method for determining RNA quality and integrity described herein ("RNA Quality Assay"). FIG. 9A depicts degraded rRNA solutions measured at both 500/525 nm (green emission channel, top panel) and 644/673 nm (red emission channel, bottom panel) as measured by the methods described herein. FIG. 9B depicts the green emission/red emission (G/R) ratio of the RNA quality and integrity methods provided herein (top panel) and the RIN values from the 2100 Bioanalyzer (bottom panel). FIG. 9C shows the correlation between the RIN and G/R ratio of the RNA quality and integrity methods provided herein. FIG. 9D shows the correlation between the RIN and RNA Quality Score or "IQ Score" provided herein.

FIGs. 10A and 10B are graphical representations of the capability of the methods of the present disclosure to measure as little as 1 μl of sample with ≤20% CV. FIG. 10A shows the normalized fluorescence (RFU) of 1 μl, 10 μl and 20 μl of sample. FIG. 10B shows the fluorescence (RFU) values of the red emission channel (644/673 nm) and the green emission channel (500/525 nm) of 1 μl, 10 μl and 20 μl sample volume.

FIG. 11A and FIG. 11B show that the nucleic acid binding dye that selectively binds small RNA can also bind double-stranded DNA (dsDNA). FIG. 11A shows binding of the dye to rRNA and FIG. 11B shows binding of the dye to dsDNA.

FIG. 12 is a graphical correlation of RNA IQ values and RNA sequencing (RNA-Seq) mappable reads from formalin-fixed, paraffin-embedded (FFPE) clinical research samples. The upper left quadrant shows false negatives (6.7%), the lower left quadrant shows true negatives (10%), the upper right quadrant shows true positives (76.7%) and the lower right quadrant shows false positives (6.7%).

FIG. 13 is a graphical representation of the percent large and/or structured RNA vs IQ value and shows that the curve is asymptotic at the upper end and that the upper 50% of the x-range is distributed over the upper quarter of the IQ range, which results in a decrease in sensitivity for determining RNA quality.

FIG. 14 is a graphical representation of the effect of 2 mM $MgCl_2$ on the linearity of the RNA IQ curve in the region of high percentage large and/or structured RNA.

FIGs. 15A through 15F are graphical representations of the response in raw fluorescent data with 2 mM $MgCl_2$ (FIGs. 15D, 15E and 15F) or without MgCh (FIGs. 15A, 15B and 15C) for each emission channel (FIGs. 15A & 15D = green emission, FIGs. 15B & 15E = far red emission, FIGs. 15C & 15F = red emission). The linear response is improved with $MgCl_2$, especially with the green emission. The absolute signal intensities are reduced in the presence of $MgCl_2$.

## DETAILED DESCRIPTION OF THE INVENTION

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents, which may be included within the invention as defined by the appended claims.

To more clearly and concisely describe and point out the subject matter of the present disclosure, the following

definitions are provided for specific terms, which are used in the following description and the appended claims. Throughout the specification, exemplification of specific terms should be considered as non-limiting examples.

**Definitions:**

As used in this specification, the words "a" or "an" mean at least one, unless specifically stated otherwise. In this specification, the use of the singular includes the plural unless specifically stated otherwise. For example, but not as a limitation, "a nucleic acid" means that more than one nucleic acid can be present; for example, one or more copies of a particular nucleic acid species, as well as two or more different species of nucleic acid. The term "and/or" means that the terms before and after the slash can be taken together or separately. For illustration purposes, but not as a limitation, "X and/or Y" can mean "X or Y" or "X and Y".

It will be appreciated that there is an implied "about" prior to the temperatures, concentrations, times, *etc.* discussed in the present disclosure, such that slight and insubstantial deviations are within the scope of the present teachings herein. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the specification, embodiments in the specification that recite "comprising" various components are also contemplated as "consisting of" or "consisting essentially of" the recited components; embodiments in the specification that recite "consisting of' various components are also contemplated as "comprising" or "consisting essentially of" the recited components; and embodiments in the specification that recite "consisting essentially of' various components are also contemplated as "consisting of" or "comprising" the recited components (this interchangeability does not apply to the use of these terms in the claims).

The term "or combinations thereof' as used herein refers to all permutations and combinations of the listed terms preceding the term. For example, "A, B, C, or combinations thereof' is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

The section headings used herein are for organizational purposes only and are not to be construed as limiting the desired subject matter in any way. In the event that any of the cited literature contradicts any term defined in this specification, this specification controls. While the present teachings are described in conjunction with various embodiments, it is not intended that the present teachings be limited to such embodiments. On the contrary, the present teachings encompass various alternatives, modifications, and equivalents, as will be appreciated by those of skill in the art.

As used herein, "about" refers to a value that is 10% more or less than a stated value, gives results functionally equivalent to the stated value, or rounds to the stated value.

As used herein, colors and color channels such as near infrared, red, orange, yellow, green, cyan, blue, and violet have their ordinary and customary meaning in the arts of fluorescence detection in molecular and cell biology, including fluorescence microscopy and flow cytometry. Exemplary ranges for the colors and color channels are as follows: about 380 nm to about 435 or 440 nm for violet, about 435 or 440 nm to about 485 nm or about 435 or 440 nm to about 500 nm for blue, about 485 to 500 nm or about 500 nm to about 520 nm for cyan, about 500 nm or 520 nm to about 560 nm or about 500 nm or 520 nm to about 565 nm for green, about 560 or 565 nm to about 590 nm or about 560 or 565 nm to about 600 nm for yellow, about 590 nm or 600 nm to about 625 nm or about 590 nm or 600 nm to about 650 nm for orange, about 625 nm or 650 nm to about 700 nm or about 625 nm or 650 nm to about 740 nm for red, and about 700 nm or 740 nm to about 1000 nm or about 700 nm or 740 nm to about 2000 nm for near infrared.

As used herein, the term "kit" refers to a packaged set of related components, such as one or more compounds or compositions and one or more related materials such as solvents, solutions, buffers, instructions, desiccants, or cells.

"Substituted" as used herein refers to a molecule wherein one or more hydrogen atoms are replaced with one or more non-hydrogen atoms, functional groups or moieties. By example, an unsubstituted nitrogen is $-NH_2$, while a substituted nitrogen is $-NHCH_3$. Exemplary substituents include but are not limited to halogen, e.g., fluorine and chlorine, ($C_1$-$C_8$) alkyl, sulfate, sulfonate, sulfone, amino, ammonium, amido, nitrile, nitro, lower alkoxy, phenoxy, aromatic, phenyl, polycyclic aromatic, heterocycle, water-solubilizing group, linkage, and linking moiety.

A dashed line projecting from a substituent, such as:

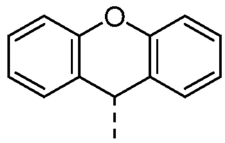

indicates the point of attachment to the base molecule. For a fused ring, dashed lines indicate portions of the base molecule where the fused ring is attached, such as:

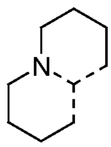

wherein the full molecule could have the structure:

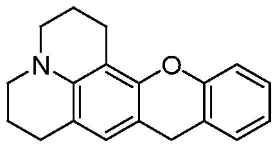
.

Unless indicated otherwise, the nomenclature of substituents that are not explicitly defined herein are arrived at by naming the terminal portion of the functionality followed by the adjacent functionality toward the point of attachment. For example, the substituent "arylalkyloxycarbonyl" refers to the group (aryl)-(alkyl)-O-C(O)-.

It is understood that in all substituted groups defined herein, polymers arrived at by defining substituents with further substituents to themselves (*e.g.*, substituted aryl having a substituted aryl group as a substituent which is itself substituted with a substituted aryl group, which is further substituted by a substituted aryl group *etc.*) are not intended for inclusion herein. In such cases, the maximum number of such substitutions is three. For example, serial substitutions of substituted aryl groups with two other substituted aryl groups are limited to -substituted aryl-(substituted aryl)-substituted aryl.

Similarly, it is understood that the definitions provided herein are not intended to include impermissible substitution patterns (*e.g.*, methyl substituted with 5 fluoro groups). Such impermissible substitution patterns are well known to the skilled artisan.

The compounds disclosed herein may exist in unsolvated forms as well as solvated forms, including hydrated forms. These compounds may exist in multiple crystalline or amorphous forms. In general, all physical forms are equivalent for the uses described herein and are intended to be within the scope of the present disclosure. The compounds disclosed herein may possess asymmetric carbon atoms (*i.e.,* chiral centers) or double bonds; the racemates, diastereomers, geometric isomers and individual isomers of the compounds described herein are within the scope of the present disclosure. The compounds described herein may be prepared as a single isomer or as a mixture of isomers.

Where substituent groups are specified by their conventional chemical formulae and are written from left to right, they equally encompass the chemically identical substituents, which would result from writing the structure from right to left, *e.g.,* $-CH_2O-$ is intended to also recite $-OCH_2-$.

It will be understood that the chemical structures that are used to define the compounds disclosed herein are each representations of one of the possible resonance structures by which each given structure can be represented. Further, it will be understood that by definition, resonance structures are merely a graphical representation used by those of skill in the art to represent electron delocalization, and that the present disclosure is not limited in any way by showing one particular resonance structure for any given structure.

Where a disclosed compound includes a conjugated ring system, resonance stabilization may permit a formal electronic charge to be distributed over the entire molecule. While a particular charge may be depicted as localized on a particular ring system, or a particular heteroatom, it is commonly understood that a comparable resonance structure can be drawn in which the charge may be formally localized on an alternative portion of the compound.

"Alkyl" refers to monovalent saturated aliphatic hydrocarbyl groups having from 1 to 10 carbon atoms and preferably 1 to 6 carbon atoms, *e.g.* 1, 2, 3, 4, 5 or 6 carbon atoms. This term includes, by way of example, linear and branched hydrocarbyl groups such as methyl ($CH_3$-), ethyl ($CH_3CH_2$-), *n*-propyl ($CH_3CH_2CH_2$-), isopropyl ($(CH_3)_2CH$-), *n*-butyl ($CH_3CH_2CH_2CH_2$-), isobutyl ($(CH_3)_2CHCH_2$-), sec-butyl ($(CH_3)(CH_3CH_2)CH$-), *t*-butyl ($(CH_3)3C$-), *n*-pentyl ($CH_3CH_2CH_2CH_2CH_2$-), and neopentyl ($(CH_3)_3CCH_2$-).

"Alkoxy" refers to the group -O-alkyl wherein alkyl is defined herein. Alkoxy includes, by way of example, methoxy, ethoxy, *n*-propoxy, isopropoxy, *n*-butoxy, *t*-butoxy, *sec*-butoxy, and *n*-pentoxy.

"Aryl" or "Ar" refers to a monovalent aromatic carboxylic group of from 6 to 14 carbon atoms having a single ring *(e.g.,* phenyl) or multiple condensed rings *(e.g.,* naphthyl or anthryl) which condensed rings may or may not be aromatic (*e.g.*, 2-benzoxazolinone, 2H-1,4-benzoxazin-3(4H)-one-7-yl, and the like) provided that the point of attachment is at an aromatic carbon atom. Preferred aryl groups include phenyl and naphthyl.

"Alkenyl" refers to alkenyl groups having from 2 to 6 carbon atoms and preferably 2 to 4 carbon atoms and having at least 1 and preferably from 1 to 2 sites of alkenyl unsaturation. Such groups are exemplified, for example, by vinyl, allyl, but-3-en-1-yl, and propenyl.

"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, cycloalkenyl-C(O)-, substituted cycloalkenyl-C(O)-, aryl-C(O)-, substituted aryl-C(O)-, heteroaryl-C(O)-, substituted heteroaryl-C(O)-, heterocyclic-C(O)-, and substituted heterocyclic-C(O)-, wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic and substituted heterocyclic are as defined herein. Acyl includes the "acetyl" group $CH_3C(O)$-.

"Amino" refers to the group $-NH_2$.

"Carbonyl" refers to the divalent group -C(O)- which is equivalent to -C(=O)-.

"Carboxyl" or "carboxy" refers to -COOH or salts thereof.

"Cycloalkyl" refers to cyclic alkyl groups of from 3 to 10 carbon atoms having single or multiple cyclic rings including fused, bridged, and spiro ring systems. Examples of suitable cycloalkyl groups include, for instance, adamantyl, cyclopropyl, cyclobutyl, cyclopentyl, and cyclooctyl.

"H" indicates hydrogen.

"Halo" or "halogen" refers to fluoro, chloro, bromo and iodo.

"Hydroxy" or "hydroxyl" refers to the group -OH.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring *(e.g.,* pyridinyl or furyl) or multiple condensed rings *(e.g.,* indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide ($N\rightarrow O$), sulfinyl, or sulfonyl moieties. Preferred heteroaryls include pyridinyl, pyrrolyl, indolyl, thiophenyl, and furanyl.

"Heterocycle" or "heterocyclic" or "heterocycloalkyl" or "heterocyclyl" refers to a saturated or unsaturated group having a single ring or multiple condensed rings, including fused bridged and spiro ring systems, from 1 to 10 carbon atoms and from 1 to 4 hetero atoms selected from the group consisting of nitrogen, sulfur or oxygen within the ring wherein, in fused ring systems, one or more the rings can be cycloalkyl, aryl or heteroaryl provided that the point of attachment is through the non-aromatic ring. In one embodiment, the nitrogen and/or sulfur atom(s) of the heterocyclic group are optionally oxidized to provide for the N-oxide, sulfinyl, or sulfonyl moieties.

Examples of heterocycle and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Sulfo" refers to the groups $-SO_3H$ or $-SO_3^-$.

"Sulfonyl" refers to the divalent group -S(O)z-.

"Thiol" refers to the group -SH.

"Alkylthio" refers to the group -S-alkyl wherein alkyl is as defined herein.

The term "assay" as used herein refers to an investigative analytic procedure for qualitatively assessing or quantitatively measuring the presence, amount or functional activity of a target entity. The methods of the present disclosure can be used in assays to determine the integrity and/or quality of RNA in a sample.

The term "detectable response" as used herein refers to an occurrence of or a change in, a signal that is directly or indirectly detectable either by observation or by instrumentation. Typically, the detectable response is an optical response resulting in a change in the wavelength distribution patterns or intensity of absorbance or fluorescence or a change in light scatter, fluorescence lifetime, fluorescence polarization, or a combination of the above parameters.

The term "dye" as used herein refers to a compound that emits light to produce an observable detectable signal.

As used herein, the term "fluorophore" or "fluorogenic" refers to a compound or a composition that demonstrates

a change in fluorescence upon binding to a biological compound or analyte of interest and/or upon cleavage by an enzyme. The fluorophores of the present disclosure may be substituted to alter the solubility, spectral properties or physical properties of the fluorophore.

As used herein, "large and/or structured RNA" includes large-sized RNA molecules and RNA molecules with secondary and/or tertiary structure. Exemplary large and/or structured RNA molecules include, but are not limited to, mRNA, rRNA and tRNA. The term "large and/or structured RNA" also includes intact RNA.

As used herein, "small RNA" includes small-sized RNA and RNA fragments. Exemplary small RNA molecules include, but are not limited to, miRNA, siRNA, mRNA fragments, rRNA fragments and tRNA fragments. The term "small RNA" also includes degraded RNA.

As used herein, "a pharmaceutically acceptable salt" or "a biologically compatible salt" is a counterion that is not toxic as used, and does not have a substantially deleterious effect on biomolecules. Examples of such salts include, among others, $K^+$, $Na^+$, $Cs^+$, $Li^+$, $Ca^{2+}$, $Mg^{2+}$, $Cl^-$, $AcO^-$, and alkylammonium or alkoxyammonium salts.

"Water-solubilizing group" means a substituent which increases the solubility of the compounds of the invention in aqueous solution. Exemplary water-solubilizing groups include but are not limited to quaternary amine, sulfate, sulfonate, carboxylate, phosphonate, phosphate, polyether, polyhydroxyl, and boronate.

**Methods of Determining RNA Integrity and Quality & Calculation of an RNA Integrity Score ("IQ Score"):**

The present disclosure provides methods, compositions and kits for determining the quality and/or integrity of RNA in a sample. The present disclosure provides reliable, easy to use methods for determining whether the RNA in a sample has degraded. These methods can be performed in 5 to 10 minutes using a fluorometer or microplate reader. Once such fluorometer that can be used in the methods of the present disclosure is a QUBIT™ Fluorometer (Thermo Fisher Scientific, Waltham, MA). The methods of the current disclosure provide the flexibility to run single samples or multiple samples. The end result, a measurement of RNA quality and/or integrity, herein termed an RNA "Quality Score" or "IQ Score", can be relied upon independent of analyst, instrument and time of analysis.

The present disclosure provides simple, easy-to-use fluorescence-based fluorometric methods and/or assays in which the ratio of the fluorescence of two nucleic acid binding dyes reliably indicates the extent of degradation of RNA in a sample. The methods require little hands-on time and are performed in a short amount of time, for example, less than 10 min. The methods can be used to analyze a single sample or multiple samples and are able to use starting materials containing a low concentration of RNA (see FIGs. 2A and 2B).

Size distributions of RNA polymers are a measure of the integrity and thus the quality of the RNA sample. The integrity of the RNA in the sample is significantly affected by contamination by RNA degrading enzymes (e.g., RNases) or mechanical shearing forces due to improper handling. A shift in length of the RNA polymer from long to short is observed when degradation has occurred.

The quality of an RNA molecule can be understood as a measure of its integrity. For example, an RNA sample will exhibit a high degree of integrity if its molecules have survived extraction from cells or tissues as a whole without suffering degradation or breakage due to shearing forces. A measure of the integrity can indicate, for example, to what extent the sample exhibits signs of degradation or shearing.

Certain embodiments of the present disclosure provide for ratiometric, fluorescence-based methods to determine the quality and/or integrity of RNA samples. The methods and resulting quality score (herein referred to as a "Quality Score" or "IQ Score") are independent of the source and type of the sample, e.g., independent of the biological species, the conditions of the cells, the types of tissues or organs and organisms involved, as well as the concentrations of the RNA in the sample and the methods by which they were prepared.

The methods for determining RNA integrity and/or quality described herein use a combination of two spectrally distinct fluorogenic nucleic acid binding dyes, a first nucleic acid binding dye that selectively binds to small RNA, such as miRNA or small RNA fragments, and a second nucleic acid binding dye that selectively binds to large and/or structured RNA, such as mRNA, tRNA and rRNA. In the methods of the present disclosure, the amount of small RNA present in the sample is indicative of degraded RNA and the amount of large and/or structured RNA is indicative of intact RNA. The methods of the present disclosure provide a ratiometric determination of the quality and/or integrity of RNA that utilizes the ratio of the fluorescence intensity of the emission of the first nucleic acid binding dye to the fluorescence intensity of the emission of the second nucleic acid binding dye as a measure of the size ratio of the RNA sample, which in turn is indicative of the extent of RNA degradation and is independent of RNA concentration.

In certain embodiments, the methods of the present disclosure provide a determination of the relationship of the intensity of the fluorescence emission of the first nucleic acid binding dye to the intensity of the fluorescence emission of the second nucleic acid binding dye as a measure of the size of the RNA present in the sample, which in turn is indicative of the extent of RNA degradation in the sample.

In certain embodiments, a method is provided for determining the extent of degradation of RNA in a sample, wherein the extent of RNA degradation is expressed as an RNA quality score, the method comprising:

a) combining a sample suspected of containing RNA with a first nucleic acid binding dye that selectively binds to small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
b) incubating the sample for a period of time sufficient to allow the first nucleic acid binding dye and the second nucleic acid binding dye to combine with the RNA in the sample to form a first nucleic acid binding dye-RNA complex and a second nucleic acid binding dye-RNA complex;
c) illuminating the first nucleic acid binding dye-RNA complex with a first appropriate wavelength of light and a second nucleic acid binding dye-RNA complex with a second appropriate wavelength of light to form an illuminated sample mixture, wherein the first nucleic acid binding dye-RNA complex emits a first fluorescent signal and the second nucleic acid binding dye-RNA complex emits a second fluorescent signal, wherein the first fluorescent signal is detectably distinct from the second fluorescent signal;
d) detecting the first fluorescent signal and the second fluorescent signal; and
e) calculating the ratio of the first fluorescent signal to the second fluorescent signal, thereby obtaining the RNA quality score, wherein the RNA quality score represents the extent of degradation of RNA in the sample.

The present disclosure provides a method of determining the extent of degradation of RNA in a sample, expressed as an RNA quality value, the method comprising:

a) combining a sample suspected of containing RNA with a first nucleic acid binding dye that selectively binds small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
b) incubating the sample for a period of time sufficient to allow the first nucleic acid binding dye and the second nucleic acid binding dye to combine with the RNA in the sample to form a first nucleic acid binding dye-RNA complex and a second nucleic acid binding dye-RNA complex;
c) illuminating the first nucleic acid binding dye-RNA complex and the second nucleic acid binding dye-RNA complex with an appropriate wavelength of light to form an illuminated sample mixture, wherein the first nucleic acid binding dye-RNA complex emits a first fluorescent signal and the second nucleic acid binding dye-RNA complex emits a second fluorescent signal;
d) detecting the first fluorescent signal and the second fluorescent signal; and
e) calculating the relationship of the intensity of the first fluorescent signal to intensity of the second fluorescent signal, thereby obtaining the RNA quality value, wherein the RNA quality value represents the extent of degradation of RNA in the sample.

This method utilizes two different nucleic acid binding dyes, each of which selectively binds different nucleic acids. In the methods provided herein, the two dyes can be combined in the same solution and added to the RNA sample simultaneously, or each dye can be in a separate solution and added to the RNA sample sequentially or simultaneously. In certain preferred embodiments provided herein, the first nucleic acid binding dye is selective for small RNAs and optionally, double-stranded DNA (dsDNA), but not larger RNA, and is excited at 500 nm and emits at 515 nm (e.g., the green emission channel of a fluorometer) and the second nucleic acid binding dye is selective for large and/or structured RNA, and is excited at 648 nm and emits at 666 nm (e.g., the red emission channel of a fluorometer). By combining these two dyes in the same method, a size profile of the RNA is obtained, with the green emission channel representative of the amount of smaller RNA present in the sample, and the red channel representative of the large, intact RNA present in the sample. With some manipulation of the data using the equation of Formula (**I**), the ratio of the emission of the two channels maps onto the fraction of small RNA in the sample (see FIGs 2A and 2B). Furthermore, by using both dyes in the same solution, a concentration independent size ratio of an RNA sample is obtained (see FIGs. 3A and 3B).

The methods for determining RNA quality and/or integrity provided herein create a new RNA quality metric. While microfluidics-based methods, such as the 2100 Bioanalyzer, provide a RNA Integrity Number (RIN) that is calculated using a proprietary algorithm to indicate the quality of an RNA sample, such RIN values are not entirely reliable as a quality or usability metric even though they are widely used. The linearity of the methods disclosed in the present teachings, can be mapped as a quality metric, herein termed the RNA Integrity Score or "IQ Score" of Formula (**I**) or Formula (**II**). By dividing the ratio of the fluorescence emission signal of the first nucleic acid binding dye (in this embodiment, the green emission channel) to the fluorescence emission signal of the second nucleic acid binding dye (in this embodiment, the red emission channel), herein termed the "Green/Red Ratio" or "G/R ratio" by a constant to scale it to 10, and subtracting it from 10 to reverse the slope, the ratio becomes a scale from 10 to 0, with 10 representing 100% large and/or structured RNA, which is indicative of intact RNA, and 0 representing 100% small RNA, which is indicative of degraded RNA (see FIG. 5 and Formula (I)).

$$IQ\ Score = 10 - \frac{GR}{0.3} \qquad \text{Formula (I)}$$

The results presented in FIGs. 6-8 demonstrate the ability of the methods provided herein to monitor or assay in real-time the integrity and/or quality of the RNA in the sample as RNase degrades the RNA. In these examples, the fluorescence of the second nucleic acid binding dye (in this embodiment, the signal of the red channel) decreases while the fluorescence of the first nucleic acid binding dye (in this embodiment, the signal of the green emission channel) initially increases and then decreases as the enzyme nicks the RNA into fragments that are too small to bind to the first nucleic acid binding dye. This demonstrates that the methods described herein measure the levels of decomposition as the RNA is degraded in a manner similar to a qPCR growth curve, but in reverse. The fluorescence of the second nucleic acid binding dye decreases as the large and/or structured RNA is degraded while the fluorescence of the first nucleic acid binding dye increases as smaller sized RNA is created. The fluorescence of the first nucleic acid binding dye eventually tails off as the RNA degrades to small sizes to which the dye cannot bind (see FIG. 8).

The methods of the present disclosure provide a linear relationship between the ratio of the emissions of the first nucleic acid binding dye and second nucleic acid binding dye (i.e., the "G/R Ratio") and the percent of small RNA (e.g., degraded RNA) in the sample. It was unexpectedly discovered that the RNA Quality Score or "IQ Score" correlates well with the RIN value determined from a 2100 Bioanalyzer (see FIGs. 9C-9D, wherein small RNA is detected in the green emission channel of the fluorometer and large and/or structured RNA is detected in the red emission channel of the fluorometer).

In certain embodiments of the method provided herein, three standard solutions are used to more precisely calculate the RNA Quality Score or "IQ Score": Standard 1, which is a buffer blank to determine background fluorescence; Standard 2, which is a small (<200 nt) RNA standard to represent fully degraded samples; and Standard 3, which is a large and/or structured RNA (e.g., mRNA, rRNA, or tRNA) to represent fully intact RNA. To calculate the RNA Quality Score (IQ Score), the green/red (G/R) ratio is measured for both Standard 3 (high fluorescence emission of the second nucleic acid binding dye) and Standard 2 (high fluorescence emission of the first nucleic acid binding dye and low fluorescence emission of the second nucleic acid binding dye). This value displays the most linearity, but as a result displays the opposite trend, with small RNA having the highest number. For a quality score, the opposite trend is needed, with large RNA displaying a high quality score. Therefore, in the RNA Quality Score (IQ Score) provided herein, the ratio of the green emission signal to the red emission signal (G/R) is divided by a constant to scale it to 10 which is subtracted from 10 to reverse the slope. Thus, the ratio becomes a scale from 10 to 0, with 10 representing 100% large and/or structured RNA, which is indicative of intact RNA, and 0 representing 100% small RNA, which is indicative of degraded RNA. The calculation described above results in the equation of Formula (**II**), a solution-based assay derived quality score.

$$Quality\ Score\ (IQ) = \frac{10(-GR_x+GR_2)}{(-GR_3+GR_2)} \qquad \text{Formula (II)}$$

wherein GRz represents the green/red ratio of Standard 2; $GR_3$ represents the green/red ratio of Standard 3; and $GR_x$ represents the green/red ratio of the sample. When $GR_x$ is equal to $GR_2$, the quality score is 0 (i.e., completely degraded) and when $GR_x$ is equal to $GR_3$, the quality score is 10 (i.e., fully intact).

The computed quality score can be a decimal number. For example, a quality score of 7.5 indicates that 75% of the sample contains large and/or structured RNA (e.g., intact RNA) and 25% of the sample contains small RNA (e.g., degraded RNA).

In certain embodiments, the FRET (Forster Resonance Energy Transfer) response, the nonradiative transfer of excitation energy from one of the dye-RNA complexes to another of the dye-RNA complexes, is also included in the calculations of RNA quality. This FRET complex is excited at about 500 nm and emits at about 666 nm, as distinct from the individual emission of either of the dye-RNA complexes alone. This emission channel, separate from either of the dye-RNA complex emissions alone, is mathematically combined with the other two emission channels for a refinement of the RNA quality metric. Inclusion of this third signal enables samples to be assessed in a three dimensional space, improving the specificity of the quality assessment. In some instances, dependent on fluorometer configuration, the signal from the FRET response is physically combined with one of the other emitted signals from the individual nucleic acid dyes, such as that which is excited at 500 nm and emits at 515 nm, or that which is excited at 648 nm and emits at 666 nm. In these cases the combined signal is then itself mathematically united with the remaining emission channel in such a fashion as to allow an additional refinement of the RNA quality assessment compared to that which is reached through the mathematical combination of the signals individually.

FRET requires two fluorophores to be not only in close proximity (usually ~10 nm) but to have spectral overlap between the donor's emission and the acceptor's excitation spectra, and an alignment of dipoles of the two fluorophores.

It was unexpectedly found that this response could be applied to the IQ assay. This FRET response depends entirely on the relative positions of the two dyes on nearby structures within RNA molecules, such that the signal when excited at 480 nm-500 nm and emission is measured from 640 nm-675 nm has a very interesting dependence on the sample composition. Without wishing to be bound by theory, this response as a function of RNA quality is likely due to some portions of RNA molecules being unraveled upon degradation, with the prevalence of structured fragments being present near the linear fragments decreasing as RNA degrades. The depletion of secondary and tertiary structure and linearization of structured regions of RNA molecules is a hallmark of RNA degradation, whether by environmental factors such as UV-induced chain breaks and basic hydrolysis, or via enzymatic action through ribonucleases. The reduction of tertiary and secondary structure is thus a useful marker of RNA degradation, enabling this FRET response as an indicator of RNA quality.

In situations where high amounts of large and/or structured RNA is present, the RNA IQ Scores as determined by Formula (**I**) and/or Formula (**II**) are asymptotic at the high-end of the curve thereby reducing the sensitivity of the RNA IQ Score (FIG. 13). However, it is at the high-end of the curve where the determination of RNA quality matters the most. Therefore, in order to make the curve more linear at higher concentrations of large and/or structured RNA, it was unexpectedly discovered that the addition of compounds that can melt or denature RNA secondary or tertiary structure, such as but not limited to NaCl, $MgCl_2$, CaClz, $ZnCl_2$, ammonium acetate, sodium acetate, EDTA, ethanol, SDS, phenol, bovine serum albumin (BSA), guanidine HCl, formamide, guanidine isothiocyanate, betaine, ethylene glycol and 1,2-propylene glycol, resulted in a more linear response in the region of high percentage of large and/or structured RNA. Without wishing to be bound by theory, addition of such denaturing compounds may alter the interaction of one or more of the nucleic acid binding dyes with the RNA to attenuate the signal to create a ratio with improved linearity and increased sensitivity at the high-end. (FIG. 14).

In certain embodiments, the addition of a denaturing agent, such as but not limited to $MgCl_2$, to the assay results in a more linear curve at higher concentrations of large and/or structured RNA and modifies the calculation of the RNA IQ Score wherein the ratio of the small RNA (e.g., the green channel) to the large and/or structured RNA (e.g., the red channel) is calculated using a random forest model with regression. In this embodiment of the RNA IQ Score calculation, the output is the same as the embodiments of the RNA IQ Score calculations described herein above, for example, wherein a value of 3 corresponds to 30% large and/or structured RNA, a value of 7 corresponds to 70% large and/or structured RNA, etc.

The advantages of the methods of determining the integrity and/or quality of RNA of the present disclosure are as follows: 1) able to assess RNA degradation in real-time; 2) simultaneous measurement of large and small RNA, alternatively the measurements can be done sequentially; 3) less sample manipulation than methods currently used; 4) significantly lower cost than microfluidics-based methods, such as the 2100 Bioanalyzer; 5) ability to analyze single samples or multiple samples; 6) ability to measure as little as 1 $\mu$l of sample with $\leq$ 20% CV (see FIGs. 10A and 10B); 7) the RNA Quality Score (IQ Score) is independent of the RNA concentration of the sample; and 8) the method can be used to measure differences in highly degraded samples such as those derived from formalin-fixed, paraffin-embedded (FFPE) tissue samples.

It was unexpectedly discovered that the ratio of two dyes that demonstrate selective binding for a particular type of RNA (large vs small), but not specific for either type, resulted in a highly reliable and easily interpretable calculation of RNA integrity that is independent of sample type, concentration, or method of preparation. Currently known methods that are considered to have a higher level of reliability regarding RNA quality use microfluidics and sophisticated algorithms that analyze the content of the entire electrophoretic trace in order to obtain a determination regarding the RNA quality. Methods that do not use such detailed analysis, such as determination of the 28S: 18S ratio, are considered to yield less reliable information. Thus, the current state of the art actually teaches away from the development of a method that measures only two variables, such as the RNA quality and integrity methods of the present teachings. However, the present disclosure clearly demonstrates that the method and quality score of Formula (I) and Formula (II) of the current teachings provide reliable and accurate determinations of the integrity and/or quality of the RNA in the sample.

**Nucleic Acid Binding Dyes:**

The methods of the present disclosure use at least two detectably distinct nucleic acid binding dyes. In the presently claimed embodiments, the first nucleic acid binding dye and the second nucleic acid binding dye are each a cyanine that are detectably distinct from each other and selected from the compounds of Table 1 and Table 2, respectively.

In the methods described herein, the first nucleic acid binding dye preferentially binds small RNA, and can also bind double-stranded DNA (dsDNA), and the second nucleic acid binding dye preferentially binds intact RNA with secondary and/or tertiary structure.

**First Nucleic Acid Binding Dye:**

The first nucleic acid binding dye exhibits enhanced fluorescence when bound to small RNA such as siRNA or small fragments of RNA. The first nucleic acid binding dye is an unsymmetrical cyanine dye including, but not limited to, any compound described in U.S. Patent No. 4,957,870; 4,883,867; 5,436,134; 5,658,751; 5,534,416; 5,863,753; and International Publication No. WO 2014/089421. The first nucleic acid binding dye may exhibit a fluorescence enhancement signal that is about 30% greater than the fluorescence signal of Compound **16** at 500 ng/ml siRNA (see WO 2014/089421 for description of determination of fluorescence enhancement). In the claimed embodiments, the first nucleic acid binding dye is selected from any of the compounds listed in Table 1 below. In certain preferred embodiments, the first nucleic acid binding dye is Compound 16, Compound **20,** Compound **26,** Compound **27,** Compound **32,** Compound **33,** Compound **48,** Compound **49,** Compound **50,** Compound **55,** Compound **56,** Compound **62,** Compound **63** or Compound **64** (See Table 1).

The first nucleic acid binding dye is selected from any of the compounds listed in Table 1 below wherein $\Psi^-$ is a biologically compatible counter ion. $\Psi^-$ balances the positive charge present. As used herein, a substance that is biologically compatible is not toxic as used, and does not have a substantially deleterious effect on biomolecules. Examples of $\Psi^-$ include, among others, chloride, bromide, iodide, sulfate, alkanesulfonate, arylsulfonate, phosphate, perchlorate, tetrafluoroboronate, tetraarylboride, nitrate, and anions of aromatic or aliphatic carboxylic acids. Preferred $\psi$- counterions are bromide, chloride, iodide, perchlorate, and various sulfonates.

In the claimed embodiments, the first nucleic acid binding dye selected from the compounds in Table 1.

**Table 1: Preferred Embodiments of the First Nucleic Acid Binding Dye**

| Compound | Structure |
|---|---|
| 16 | 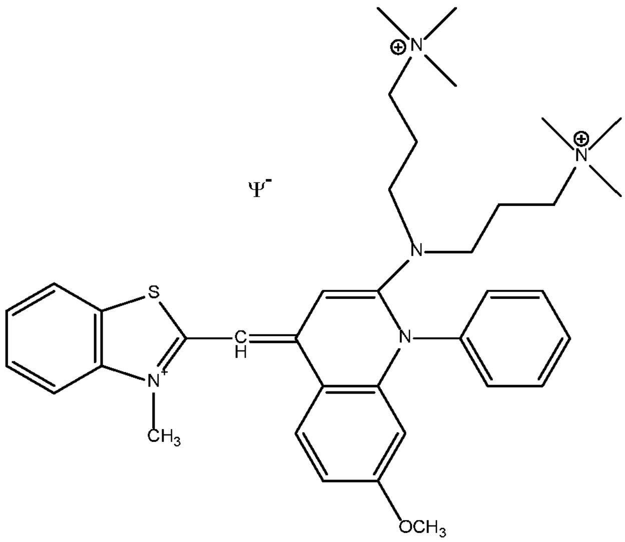 |
| 17 | 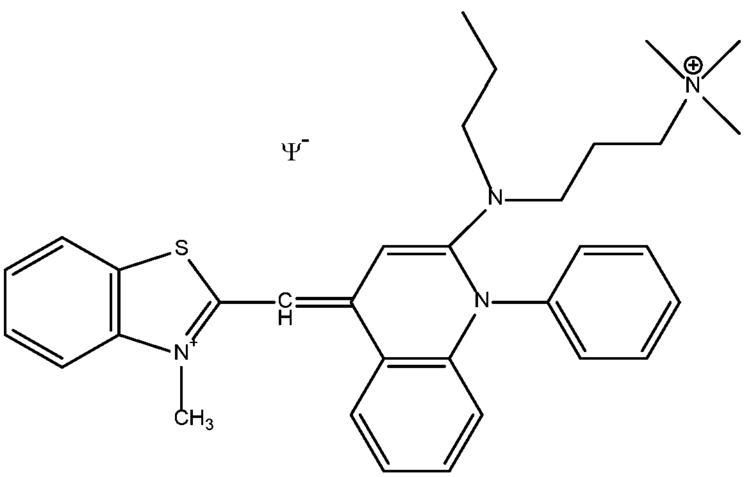 |

(continued)

| Compound | Structure |
|---|---|
| 18 | 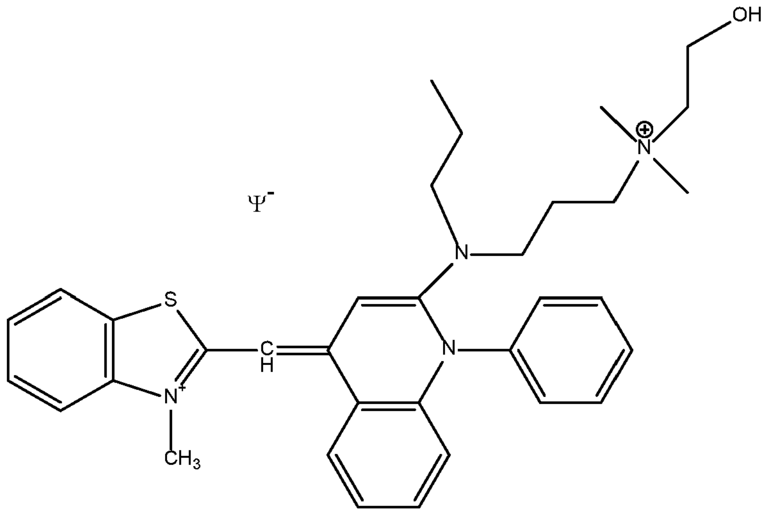 |
| 19 | 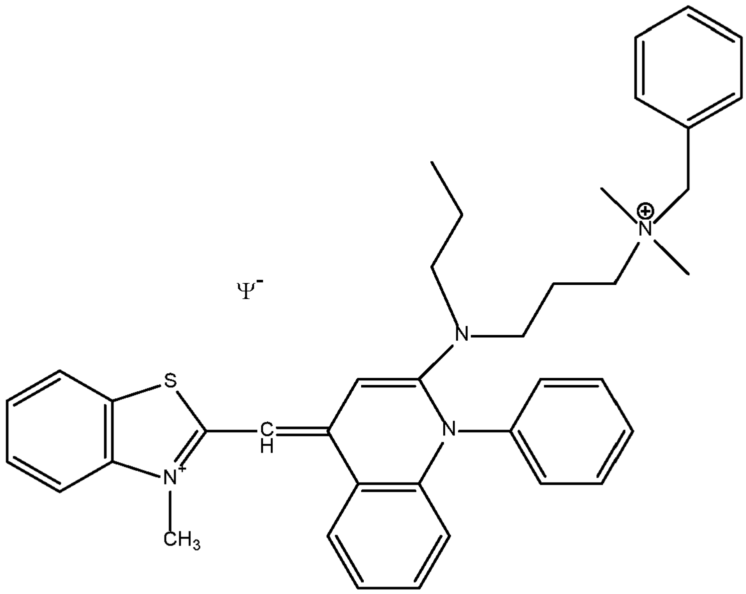 |

(continued)

| Compound | Structure |
|---|---|
| 20 | 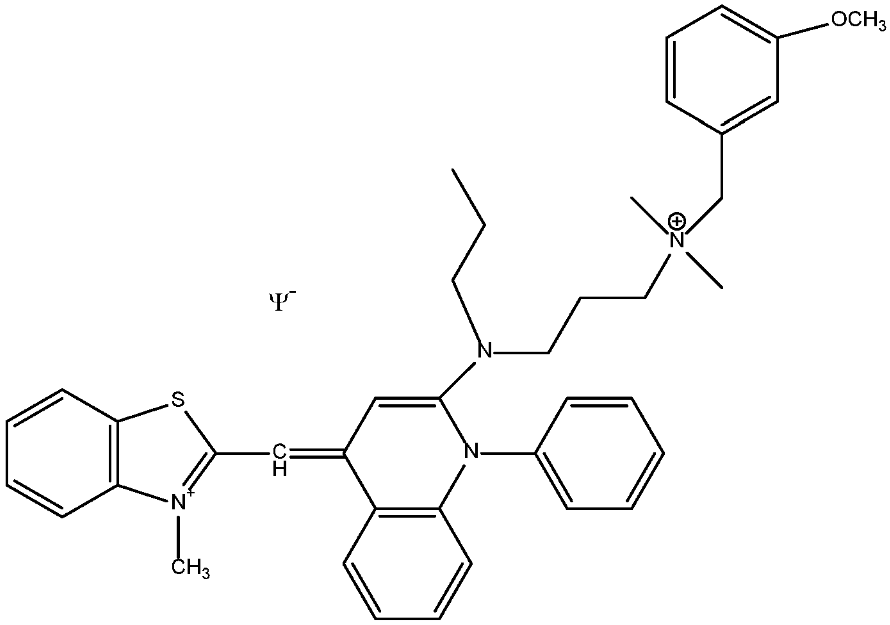 |
| 21 | 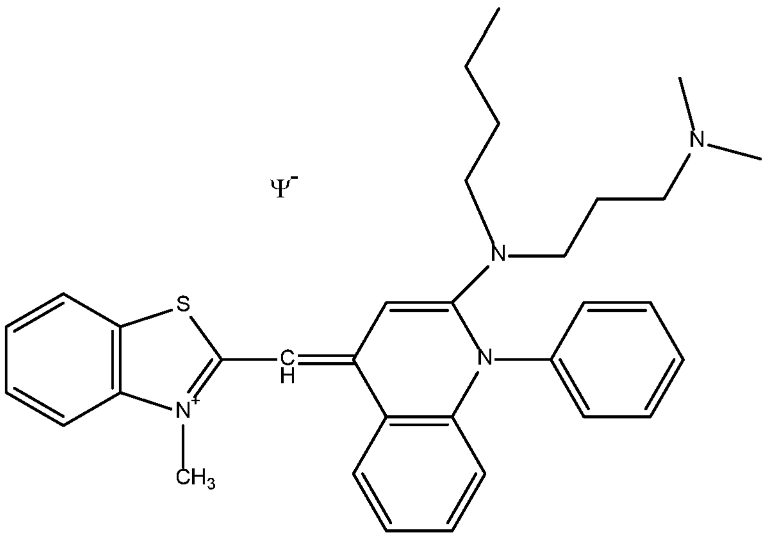 |

(continued)

| Compound | Structure |
|---|---|
| 22 | 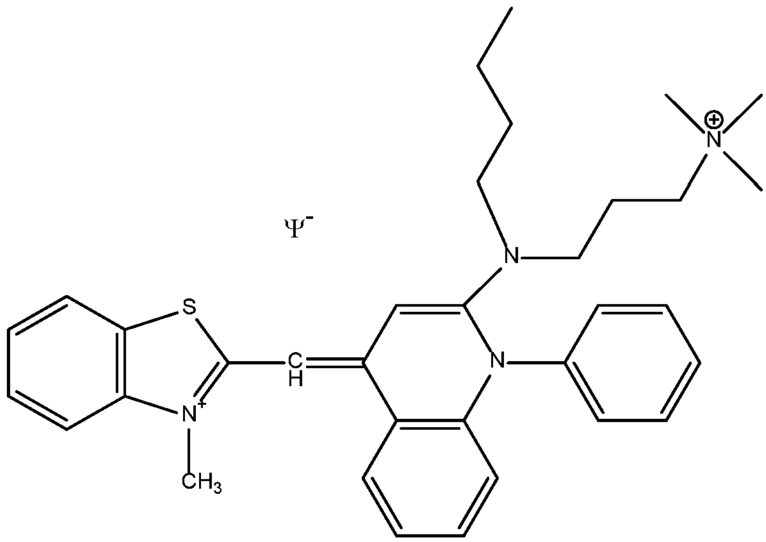 |
| 23 | 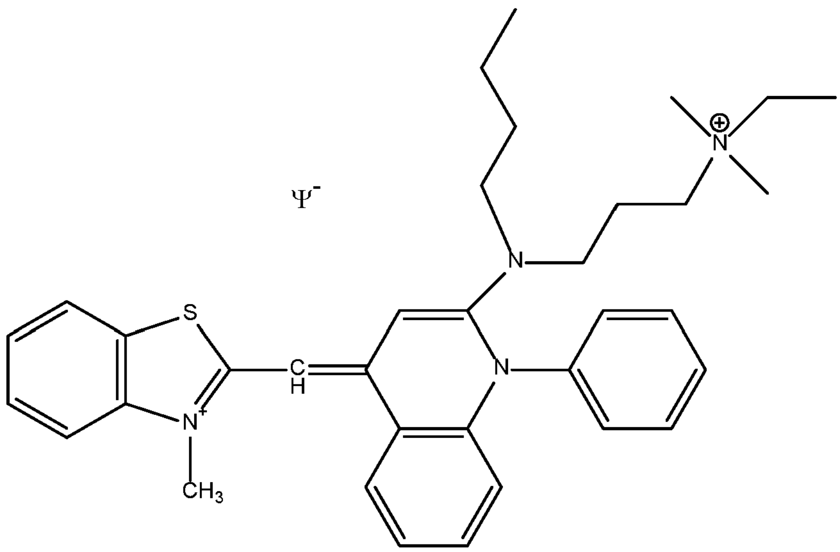 |

(continued)

| Compound | Structure |
|---|---|
| 24 | 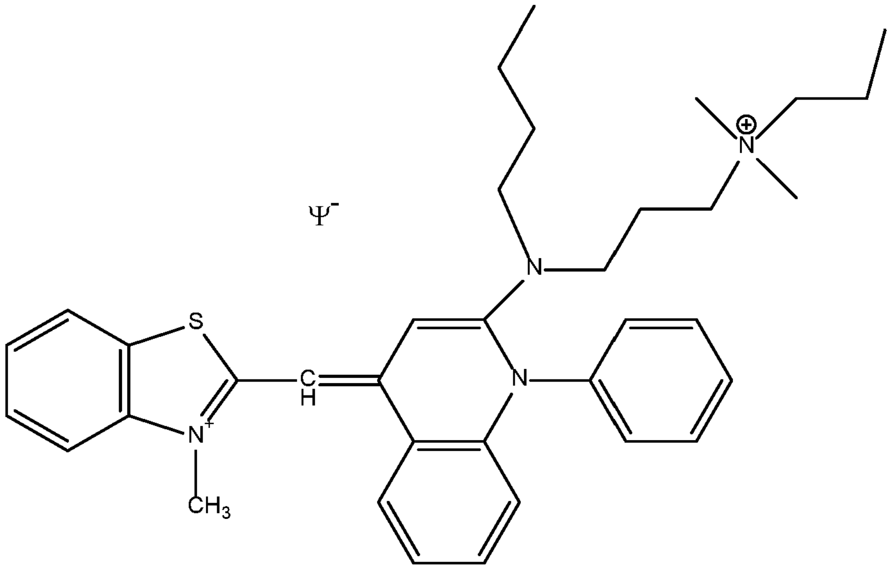 |
| 25 | 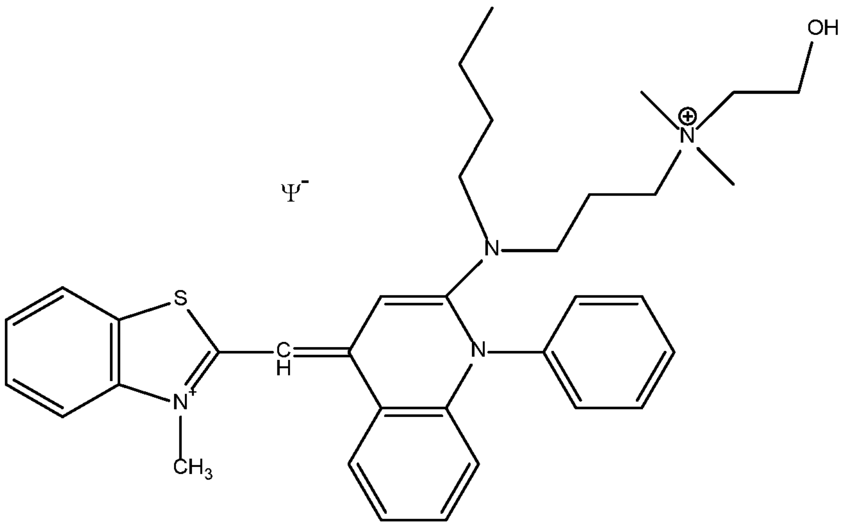 |
| 26 | 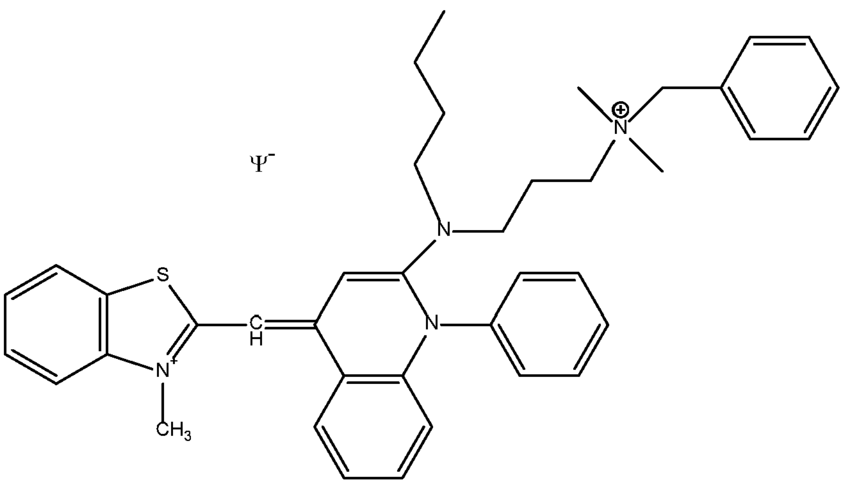 |

(continued)

| Compound | Structure |
|---|---|
| 27 | 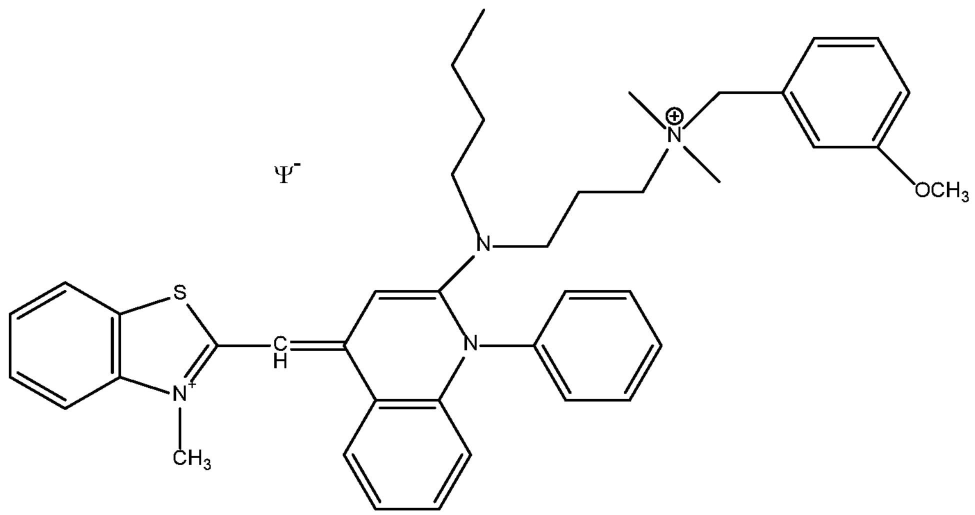 |
| 28 | 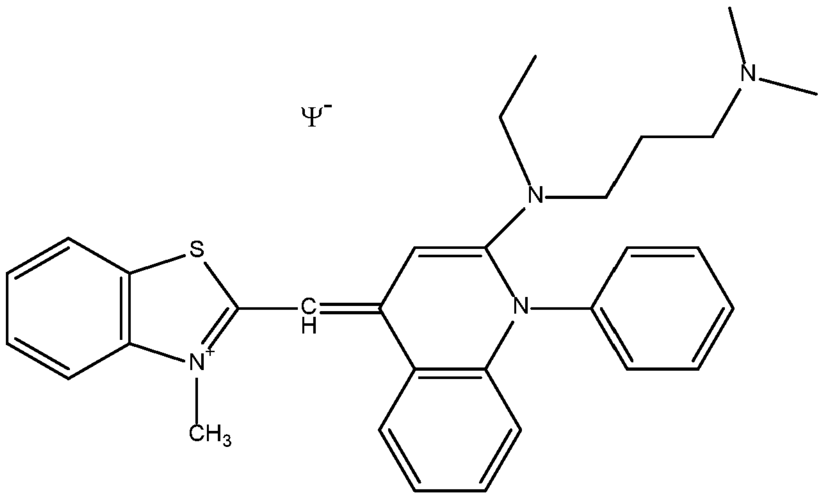 |
| 29 | 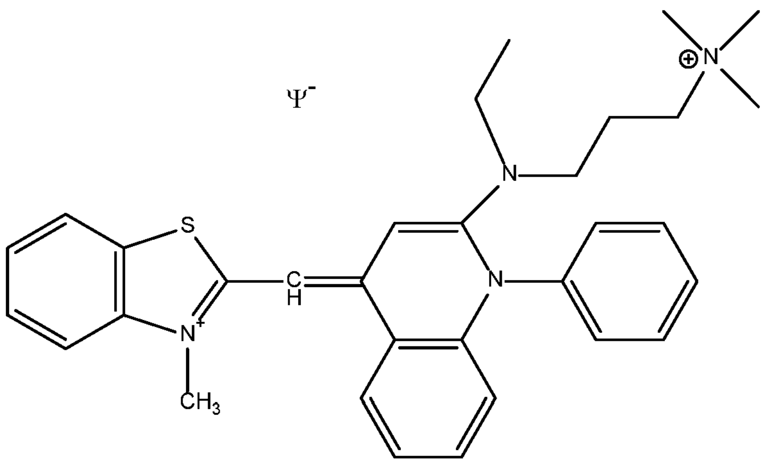 |

(continued)

| Compound | Structure |
|---|---|
| 30 | 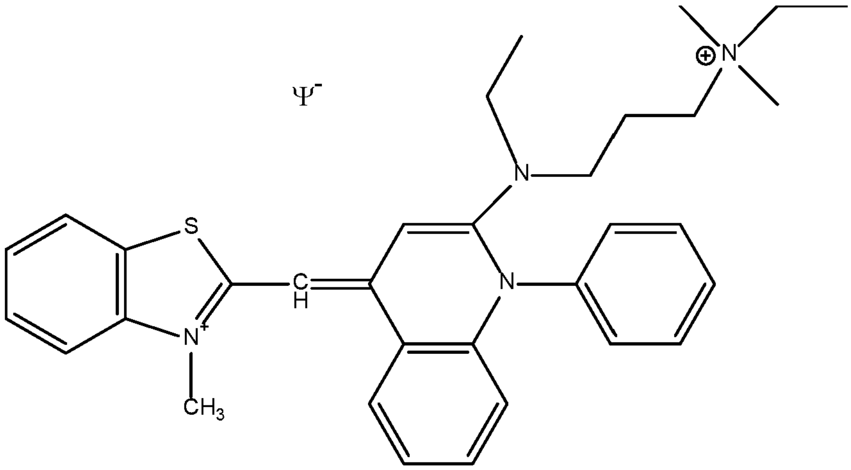 |
| 31 | 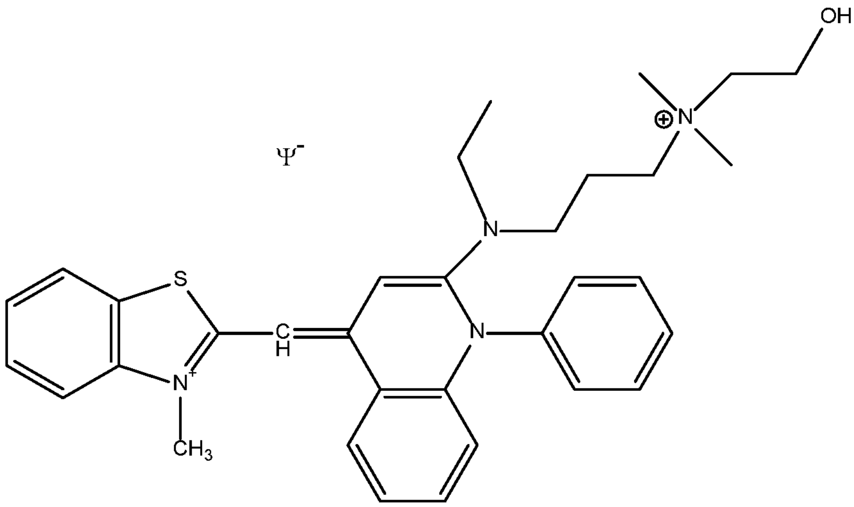 |
| 32 | 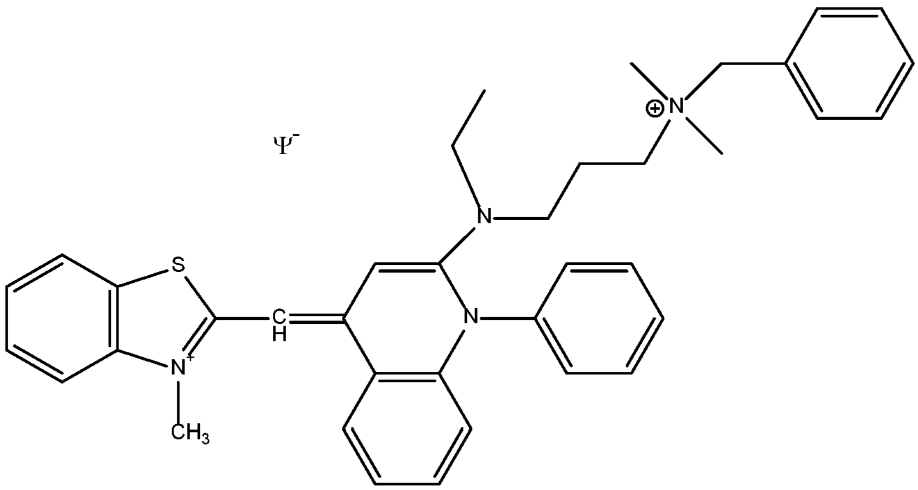 |

(continued)

| Compound | Structure |
|---|---|
| 33 | 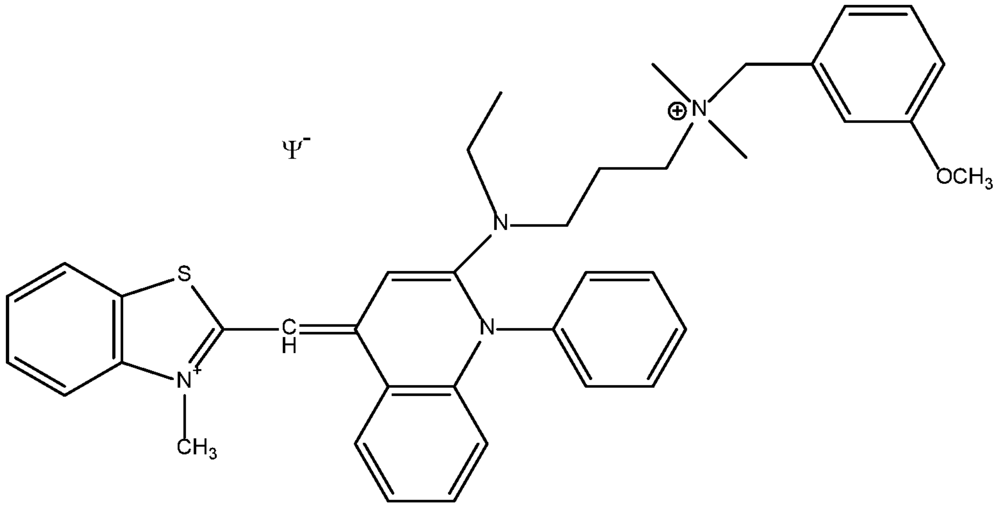 |
| 34 | 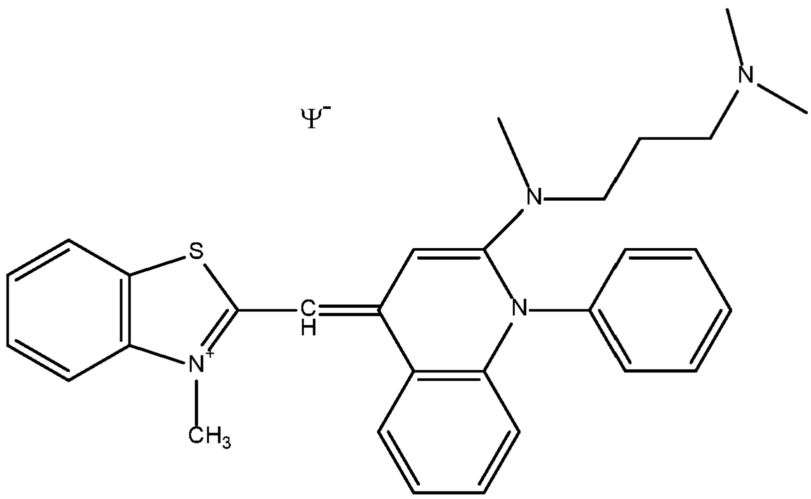 |
| 35 | 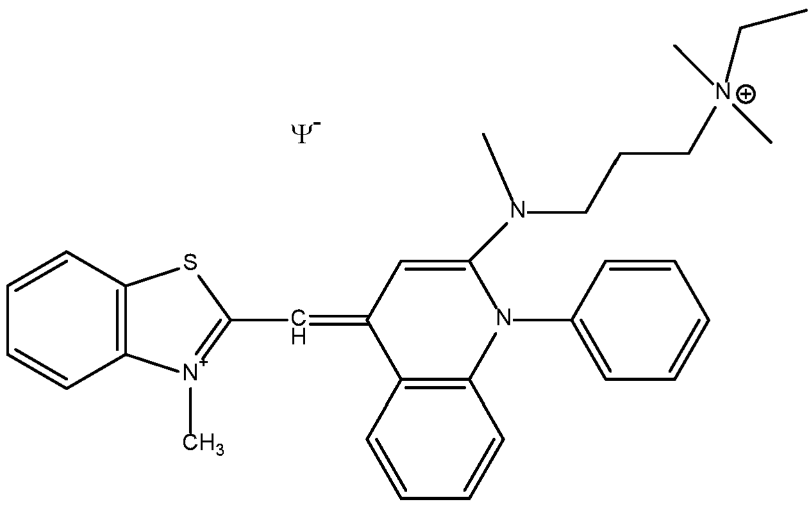 |

(continued)

| Compound | Structure |
|---|---|
| 36 | 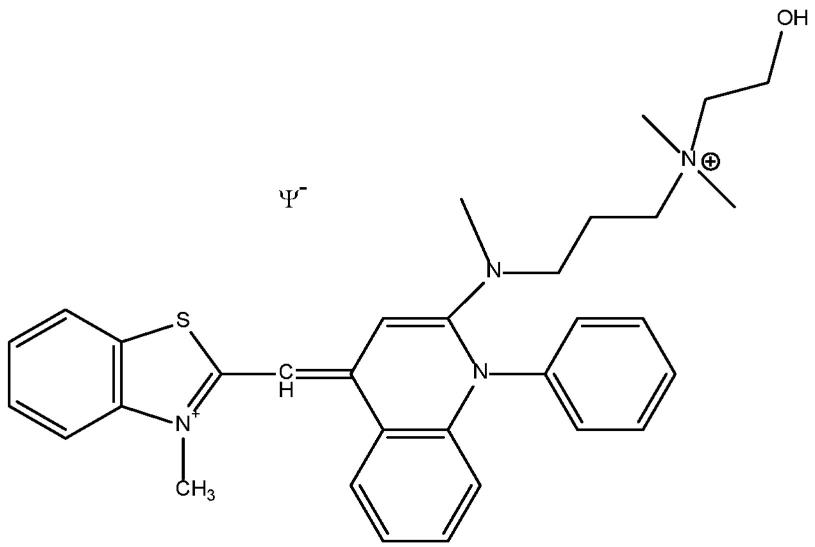 |
| 37 | 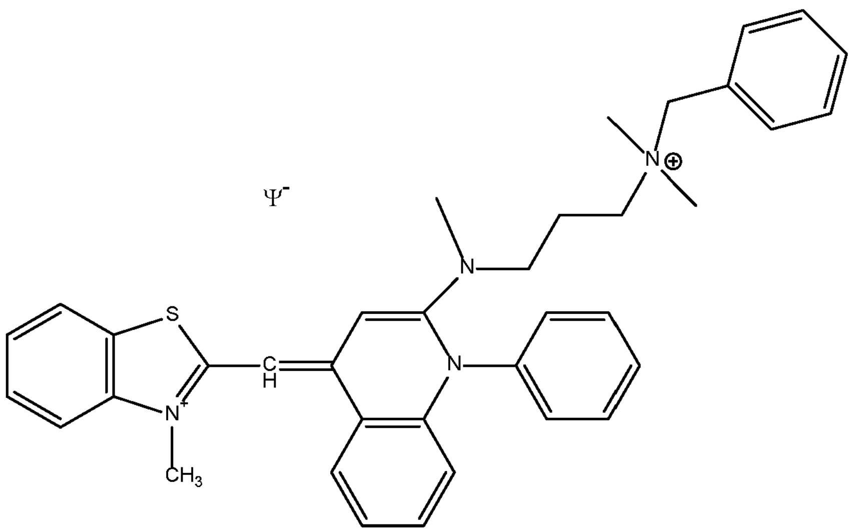 |
| 38 | 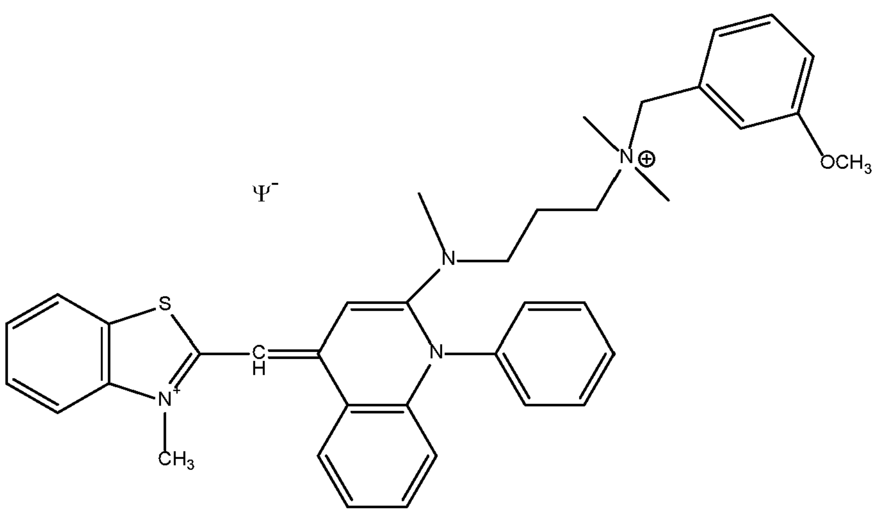 |

(continued)

| Compound | Structure |
|---|---|
| 39 | 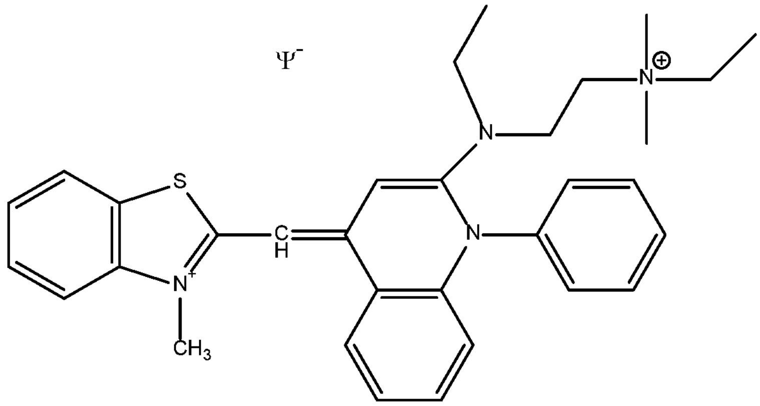 |
| 40 | 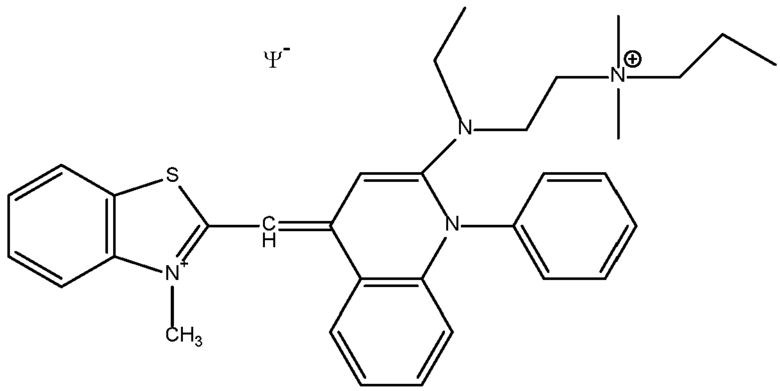 |
| 41 | 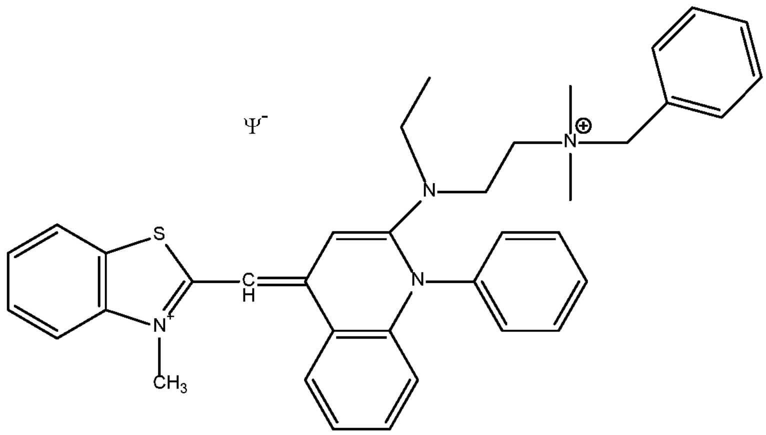 |

(continued)

| Compound | Structure |
|---|---|
| 42 | 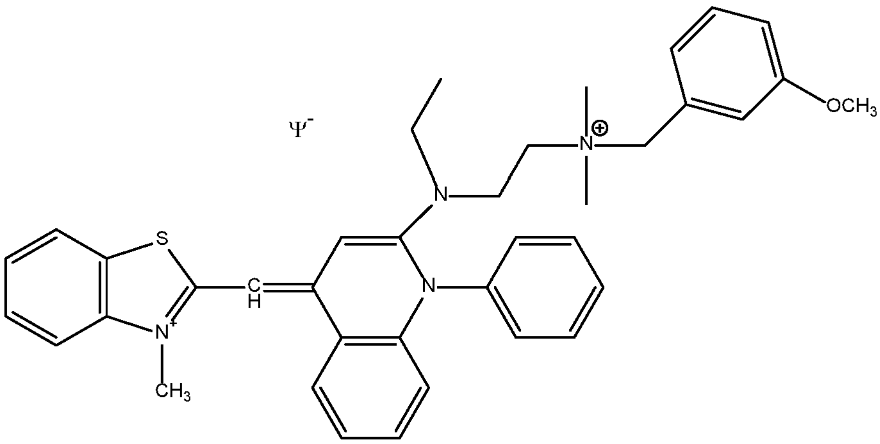 |
| 43 | 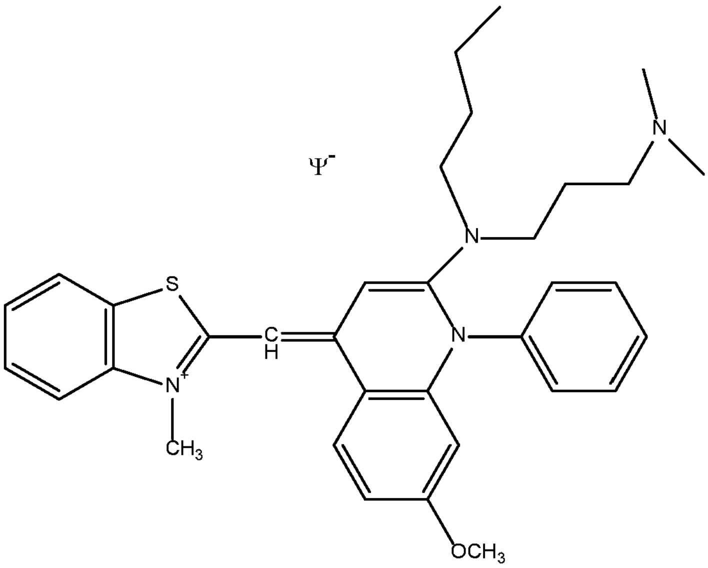 |
| 44 | 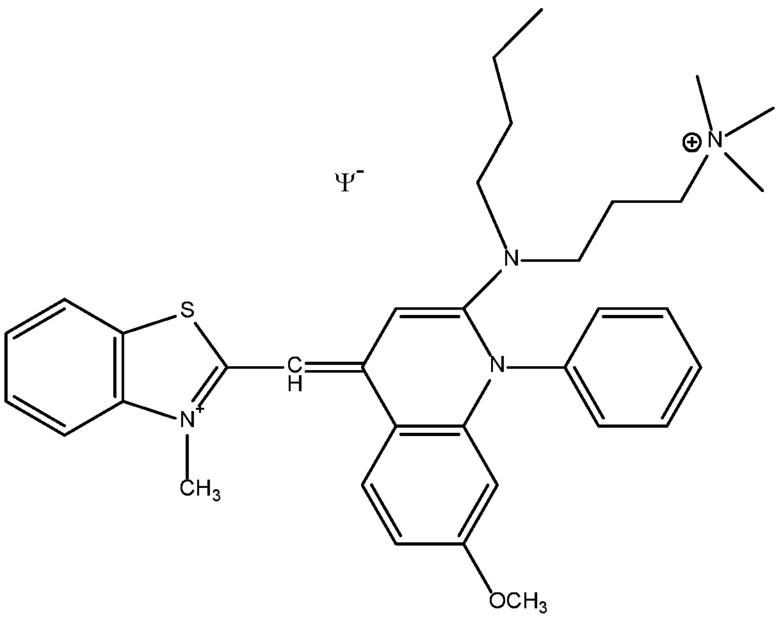 |

(continued)

| Compound | Structure |
|---|---|
| 45 | 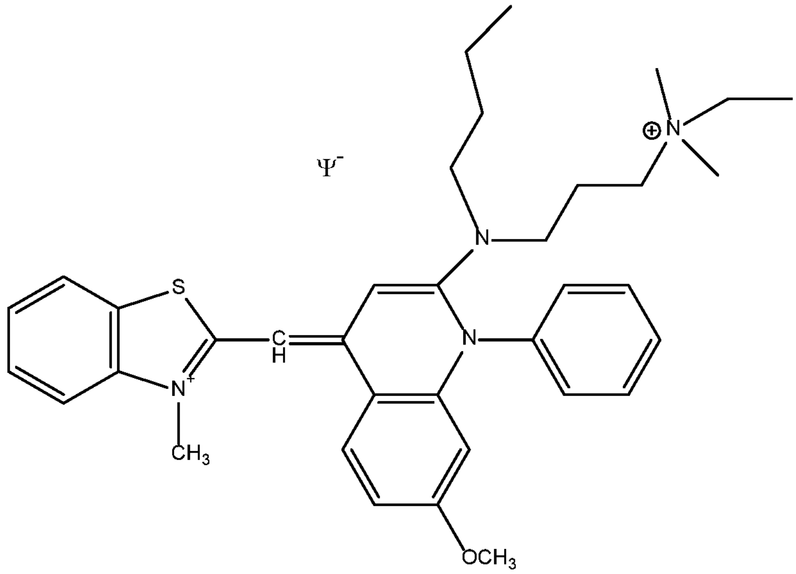 |
| 46 | 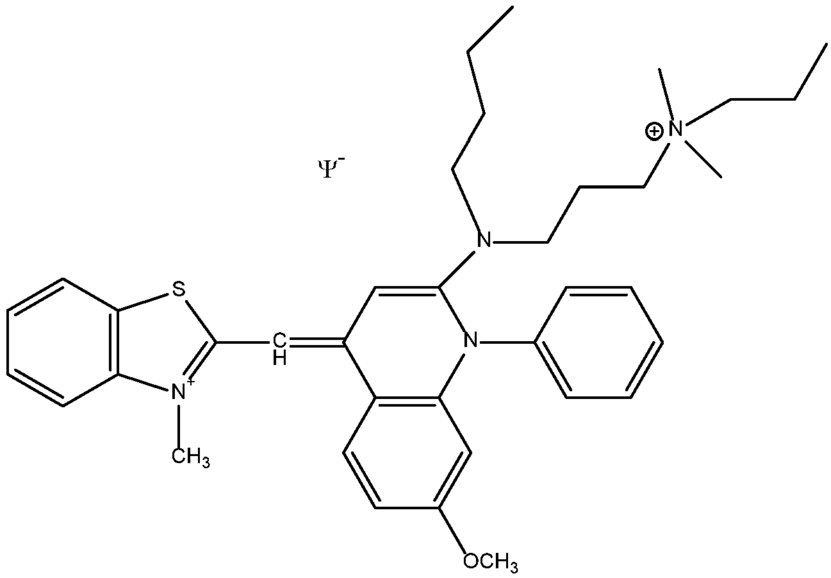 |

(continued)

| Compound | Structure |
|---|---|
| 47 | 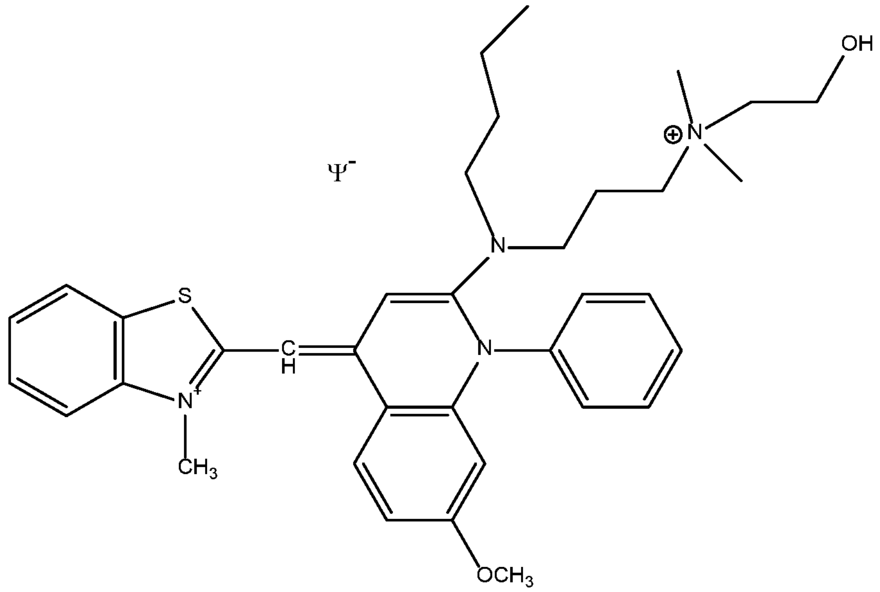 |
| 48 | 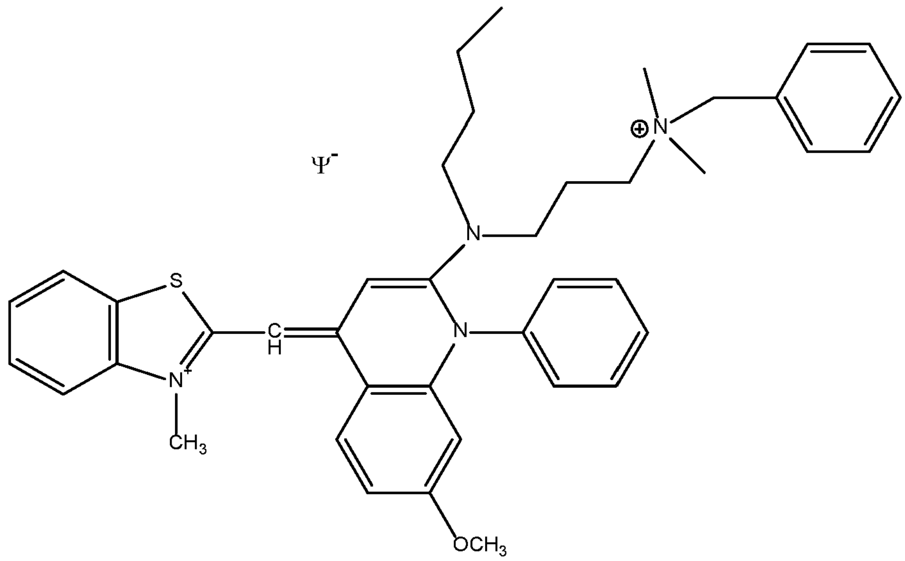 |

(continued)

| Compound | Structure |
|---|---|
| 49 | 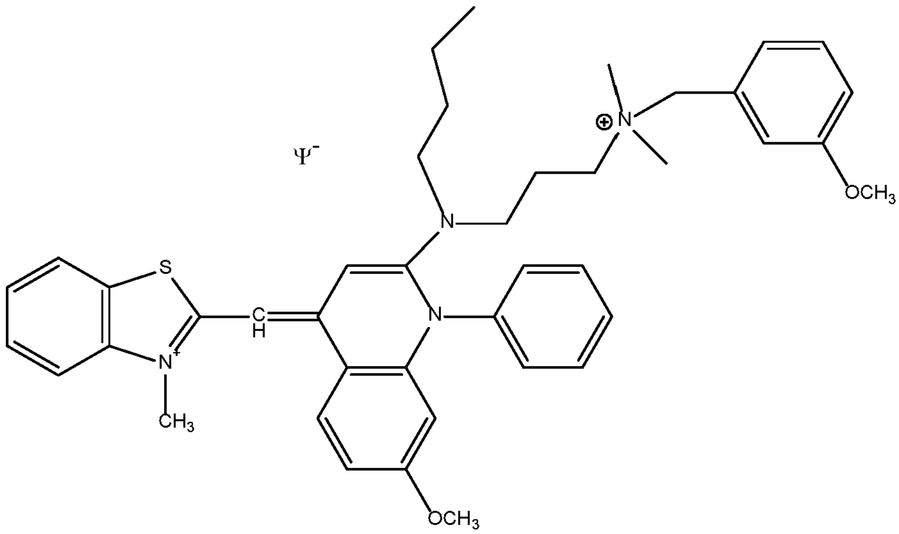 |
| 50 | 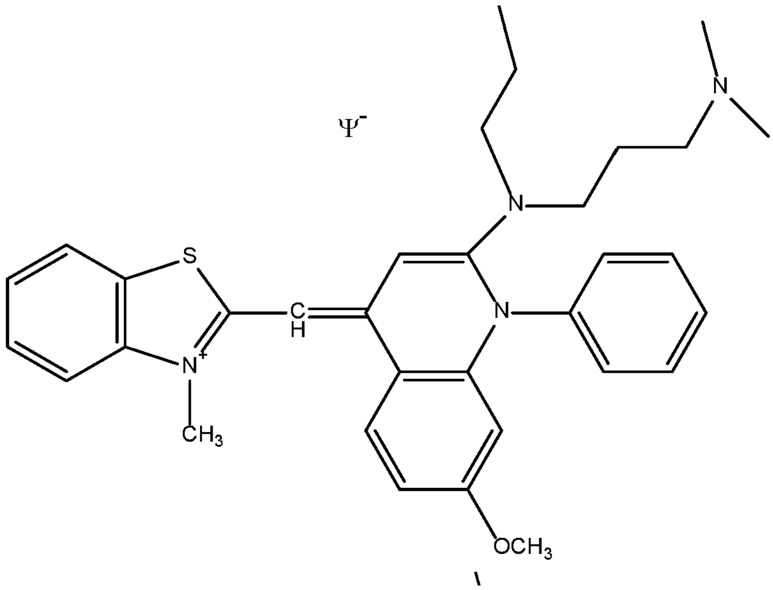 |

(continued)

| Compound | Structure |
|---|---|
| 51 | 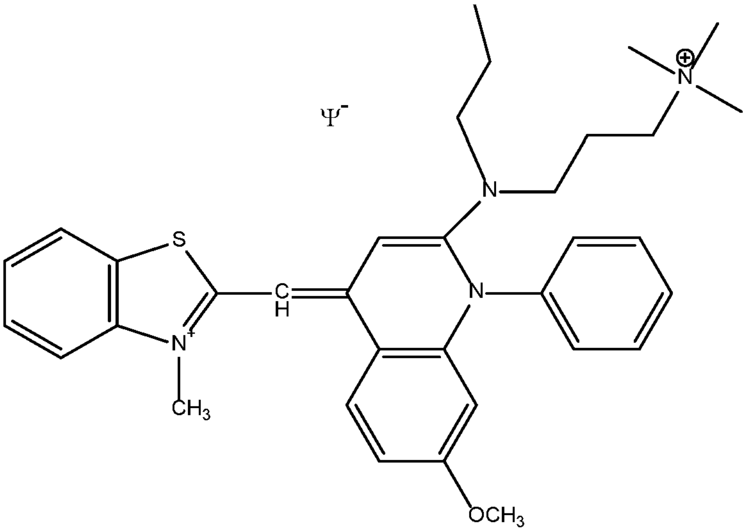 |
| 52 | 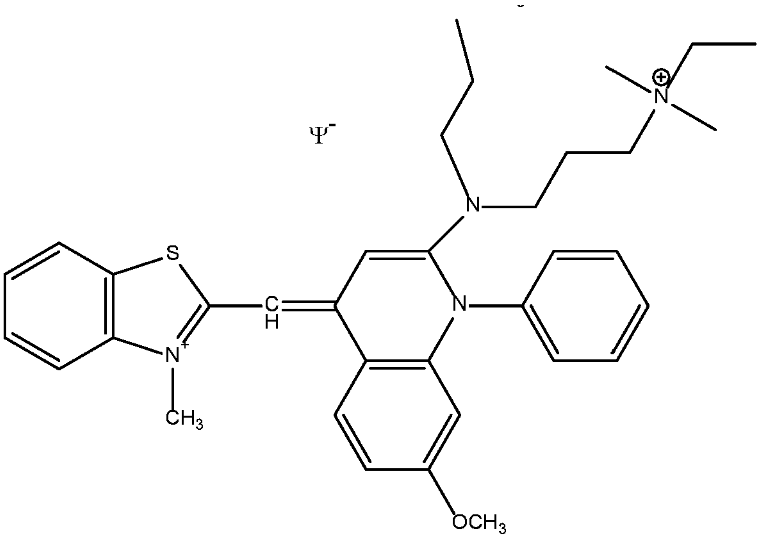 |
| 53 | 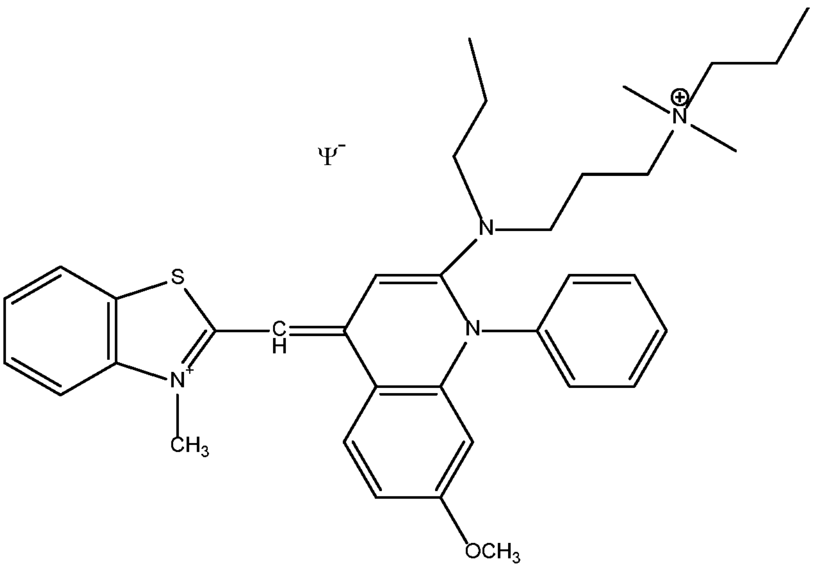 |

(continued)

| Compound | Structure |
|---|---|
| 54 | 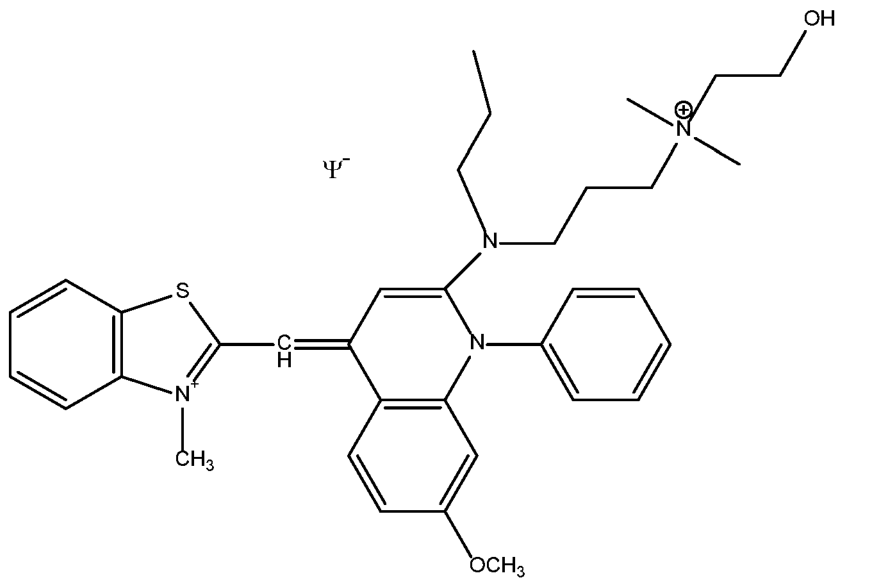 |
| 55 | 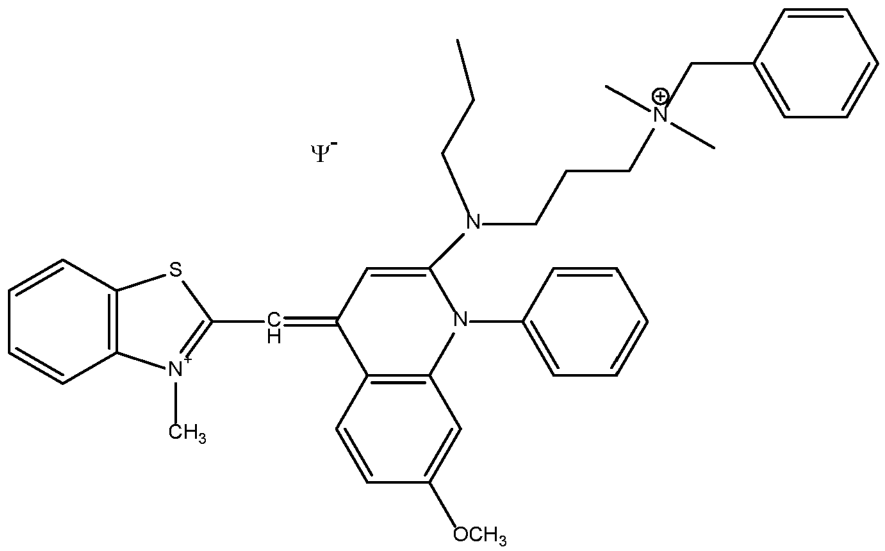 |
| 56 | 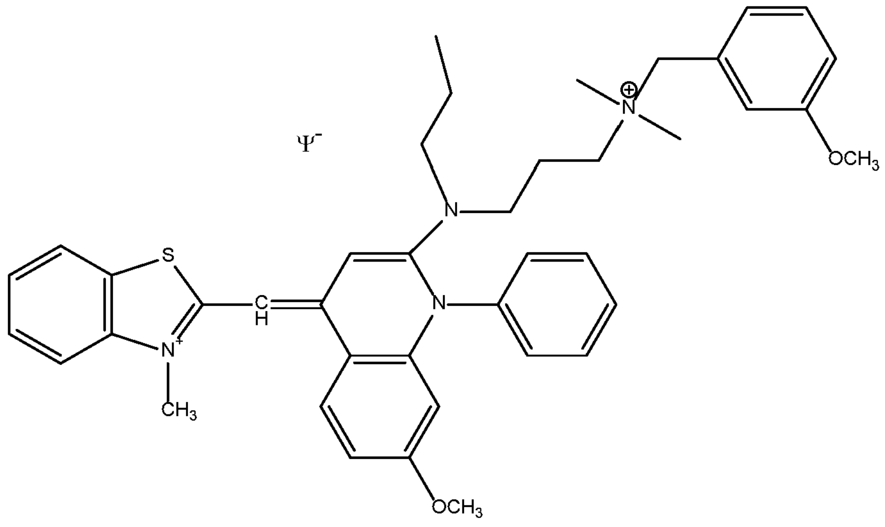 |

(continued)

| Compound | Structure |
|---|---|
| 57 | 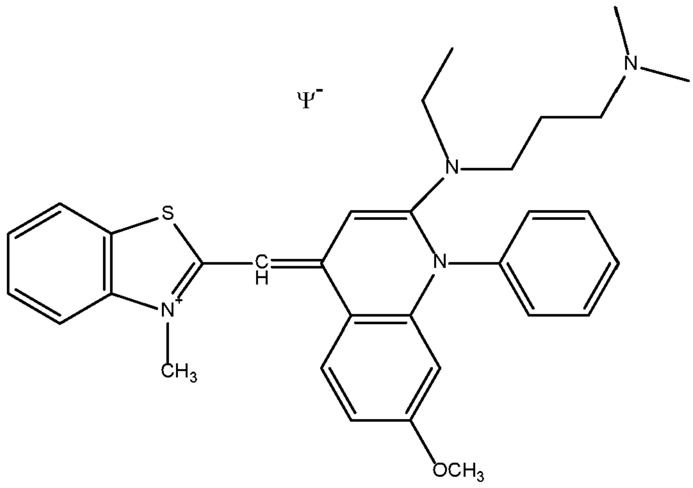 |
| 58 | 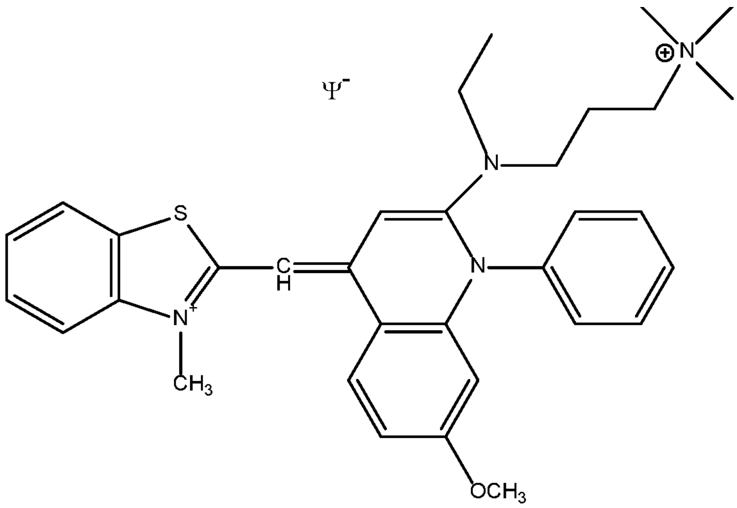 |
| 59 | 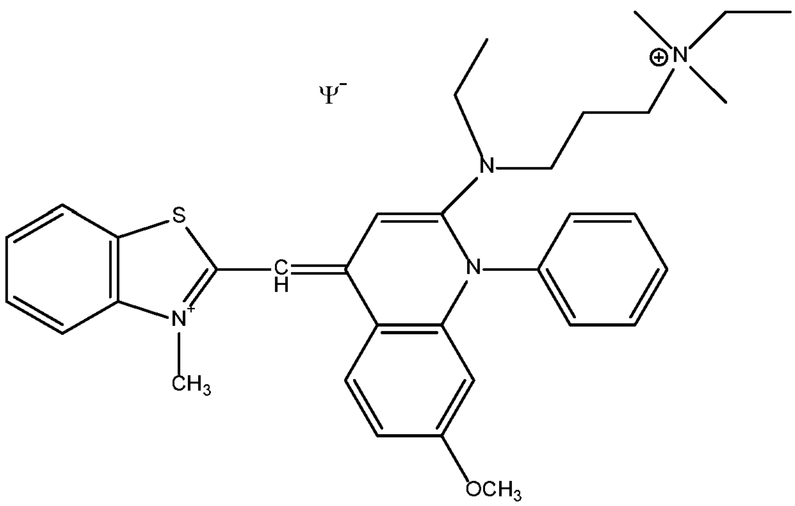 |

(continued)

| Compound | Structure |
| --- | --- |
| 60 | 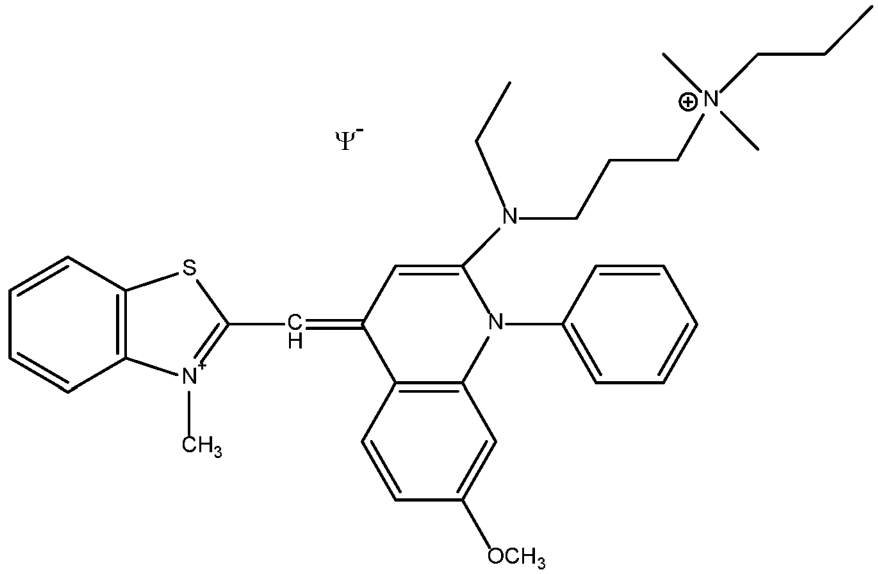 |
| 61 | 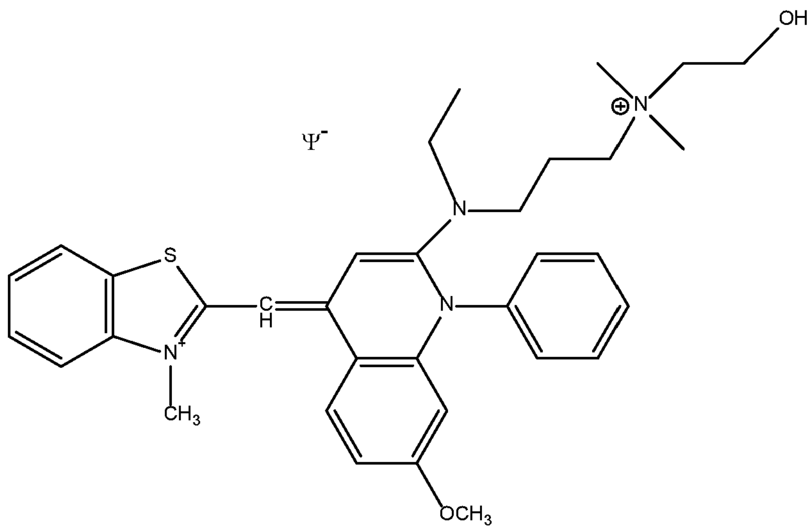 |
| 62 | 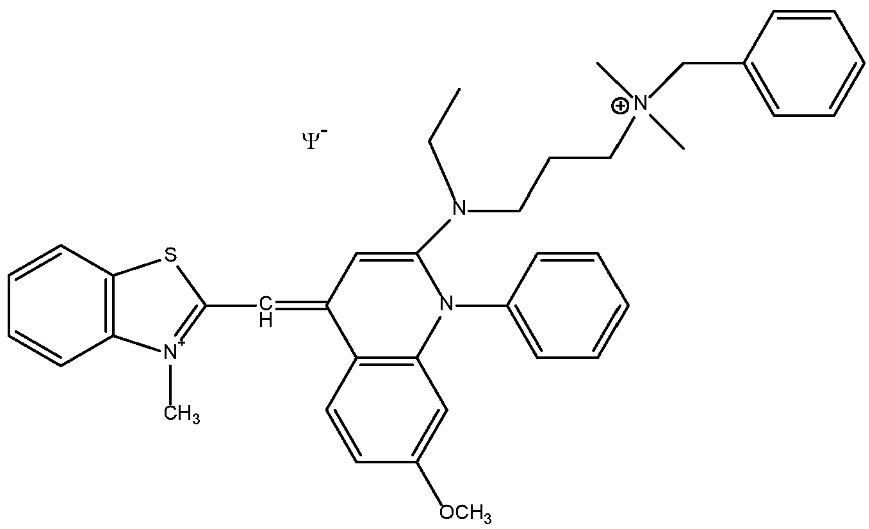 |

(continued)

| Compound | Structure |
|---|---|
| 63 | 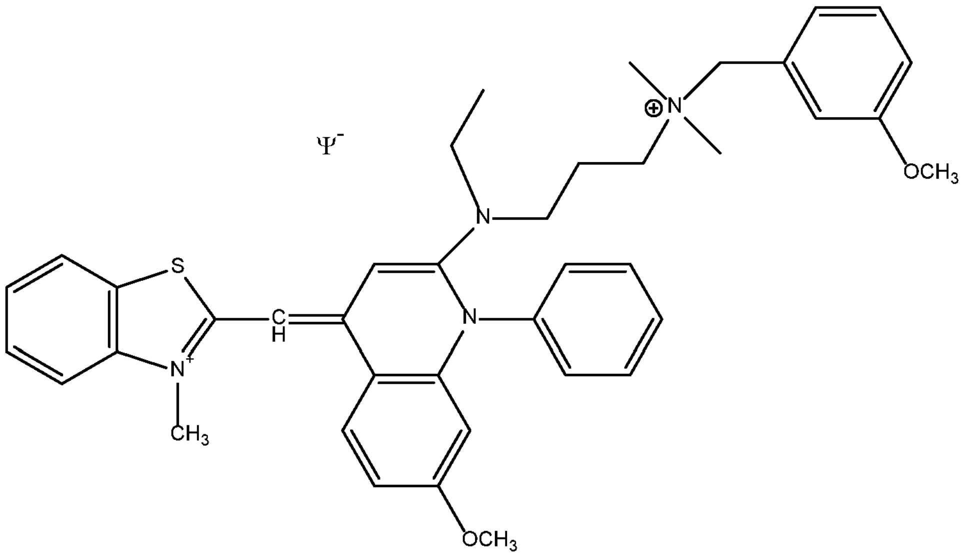 |
| 64 | 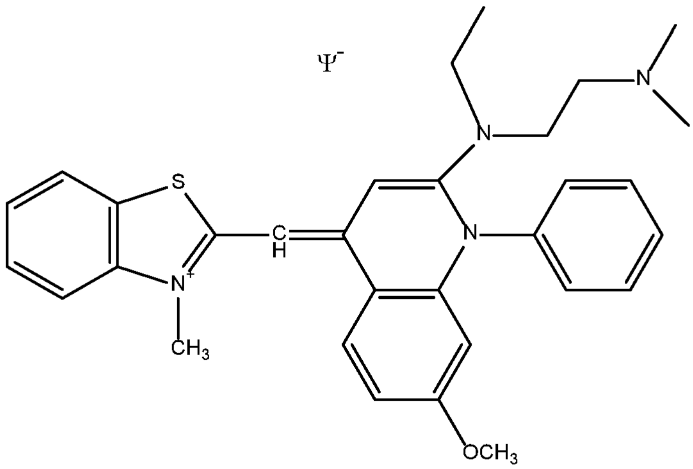 |
| 65 | 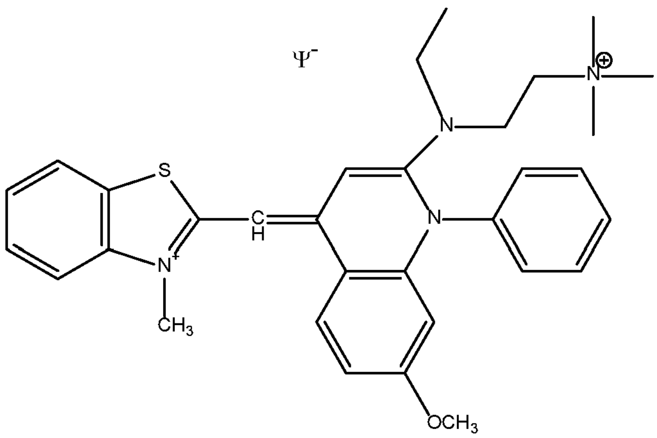 |

(continued)

| Compound | Structure |
|---|---|
| 66 | 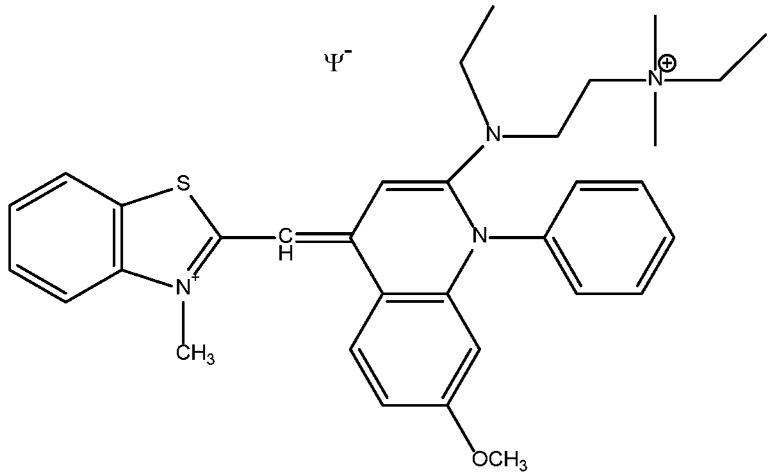 |
| 67 | 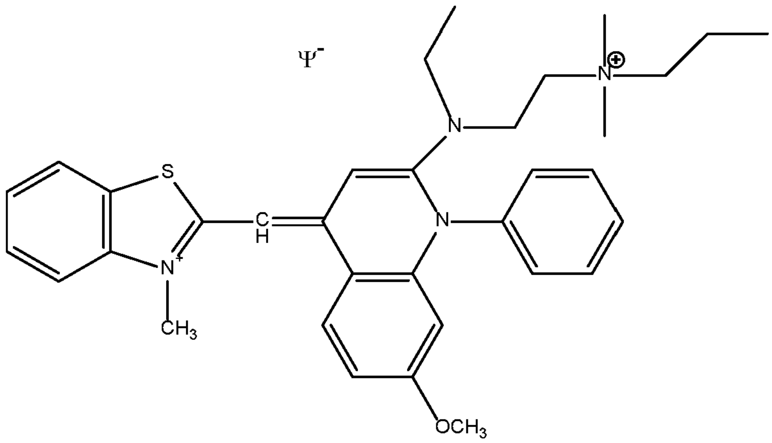 |
| 68 | 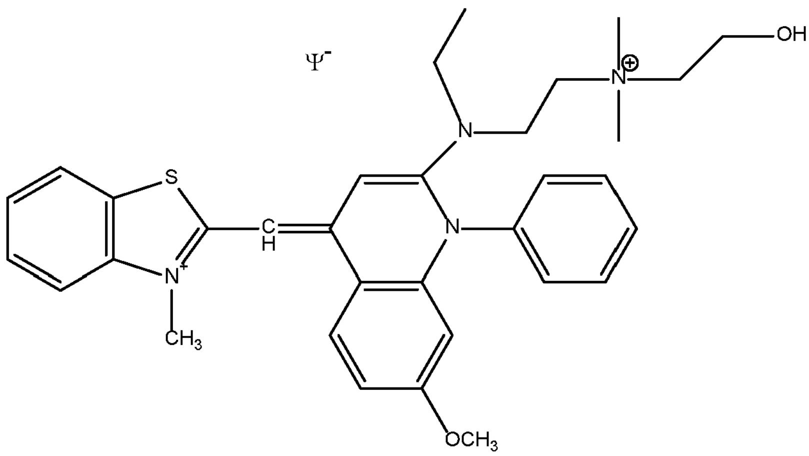 |

(continued)

| Compound | Structure |
|---|---|
| 69 | 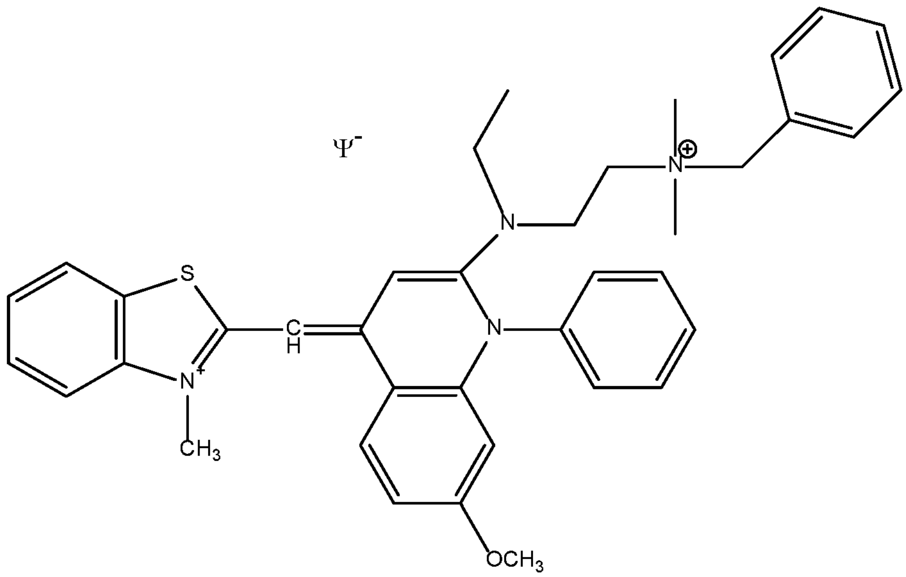 |

**Second Nucleic Acid Binding Dye:**

The second nucleic acid binding dye typically exhibits a fluorescence enhancement when noncovalently associated with a nucleic acid; specifically the fluorescence enhancement is greater when the nucleic acid is RNA than when the nucleic acid is DNA. The second nucleic acid binding dye comprises a substituted methine bridge. The second nucleic acid binding dye is an unsymmetrical cyanine dye including, but not limited to, any compound described in U.S. Patent No. 4,957,870; 4,883,867; 5,436,134; 5,658,751; 5,534,416; 5,863,753; 7,776,529; 8,470,529; 9,040,561 and 9,403,985. The second nucleic acid binding dye may exhibit a fluorescence enhancement ratio of RNA/DNA of ≥ 7 (See, U.S. Patent No. 8,470,529 for a description of the determination of the RNA/DNA fluorescence enhancement ratio). The second nucleic acid binding dye may exhibit a fluorescence enhancement ratio of RNA/DNA of ≥ 10. The second nucleic acid binding dye may exhibit a fluorescence enhancement ratio of RNA/DNA of ≥20. In the presently claimed embodiments, the first nucleic acid binding dye is Compound **1,** Compound **2,** Compound **3,** Compound **4,** Compound **7,** Compound **9,** Compound **10,** Compound **12,** Compound **13,** Compound **14** or Compound **15** (See Table 2).

.

In the presently claimed embodiments, the second nucleic acid binding dye is selected from the compounds in Table 2 below.

**Table 2: Preferred Embodiments of the Second Nucleic Acid Binding Dye**

| Compound | Ex/Em (nm)[1] | Fluorescence Enhancement Ratio (RNA/DNA)[2] |
|---|---|---|
| 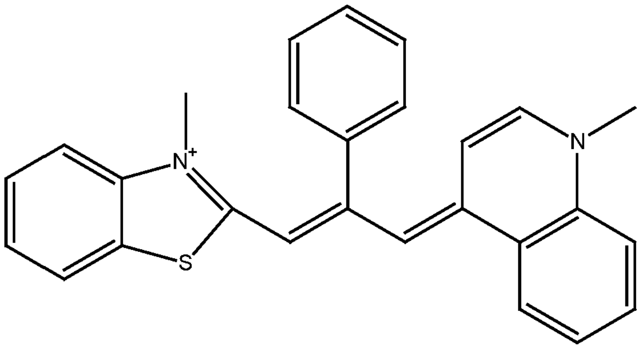 Compound **1** | 642/662 | 7 |
| 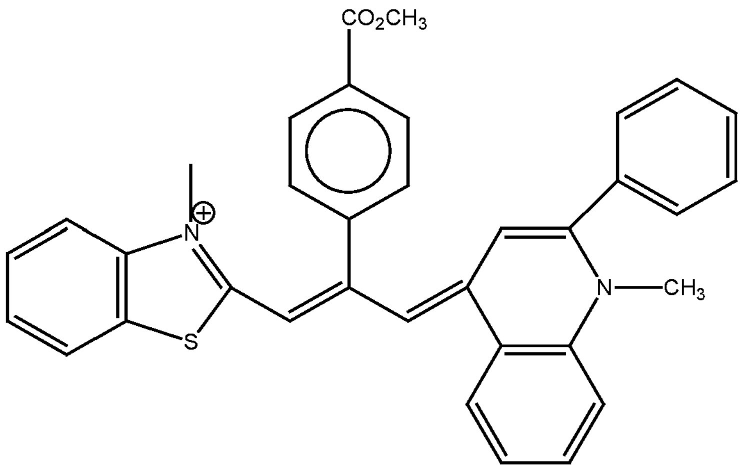 Compound **2** | 597/689 | 7.8 |
| 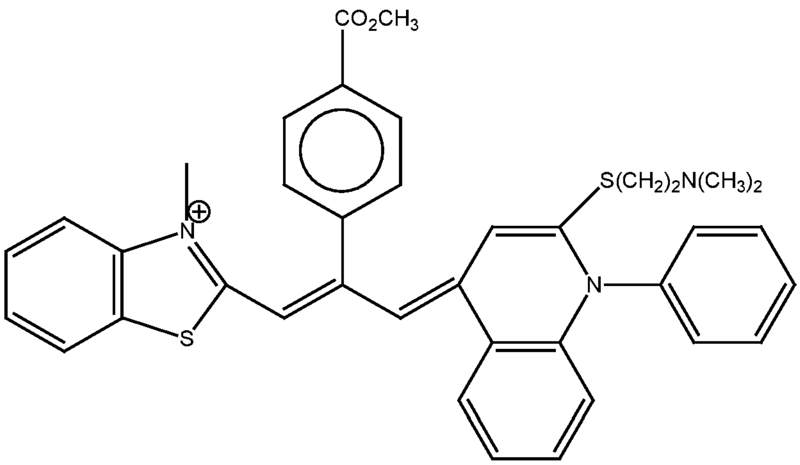 Compound **3** | 644/686 | 14.7 |

(continued)

| Compound | Ex/Em (nm)[1] | Fluorescence Enhancement Ratio (RNA/DNA)[2] |
|---|---|---|
| 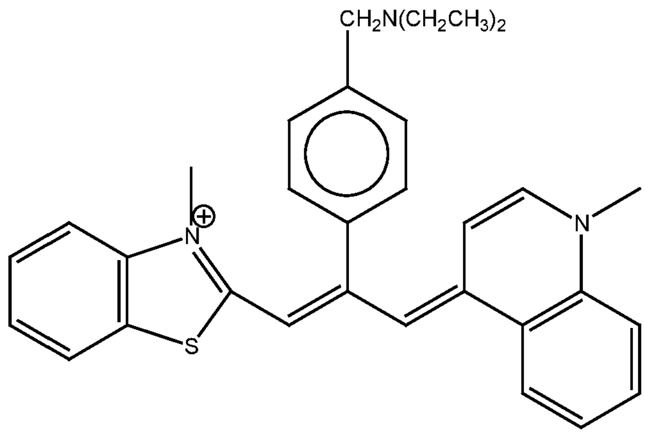<br>Compound **4** | 631/670 | 69 |
| 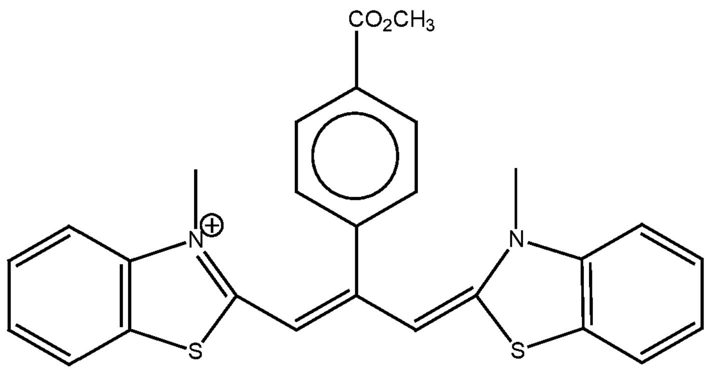<br>Compound **5** | 557/598 | 6.6 |
| 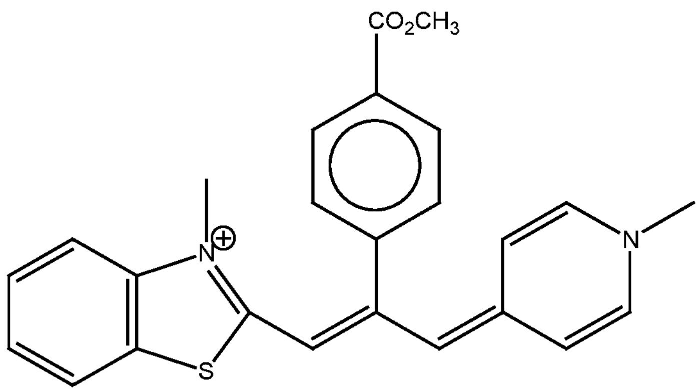<br>Compound **6** | 554/609 | 6.4 |

(continued)

| Compound | Ex/Em (nm)[1] | Fluorescence Enhancement Ratio (RNA/DNA)[2] |
|---|---|---|
| 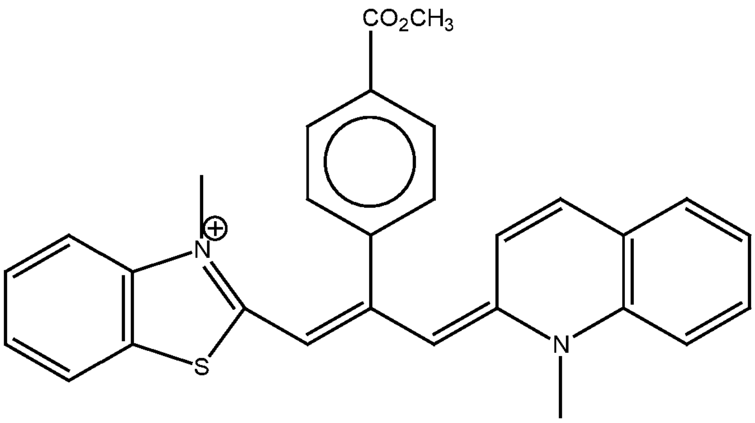 Compound **7** | 605/630 | 11.1 |
| 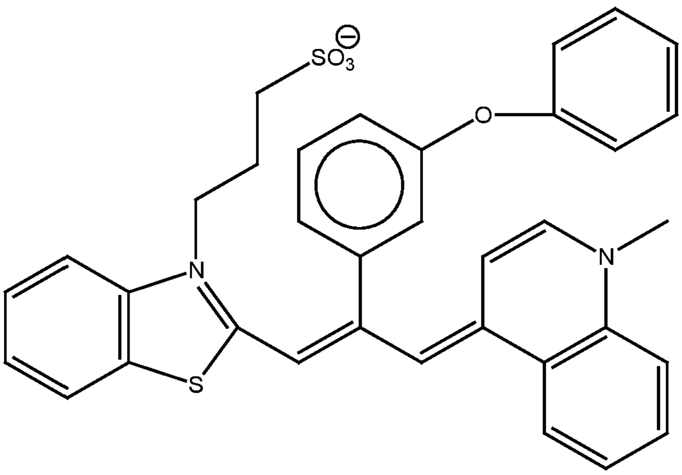 Compound **8** | 640/670 | 6 |
| 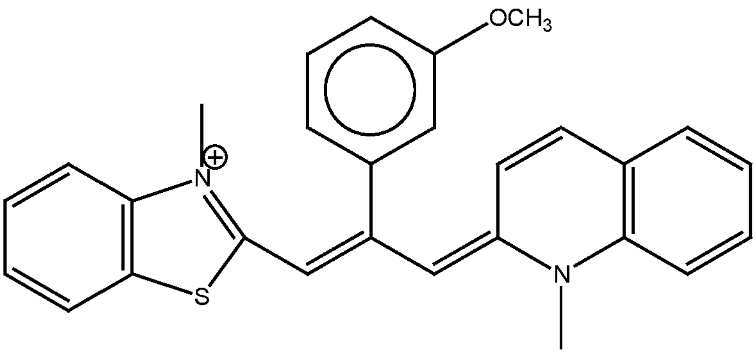 Compound **9** | 592/631 | 7.7 |

(continued)

| Compound | Ex/Em (nm)[1] | Fluorescence Enhancement Ratio (RNA/DNA)[2] |
|---|---|---|
| 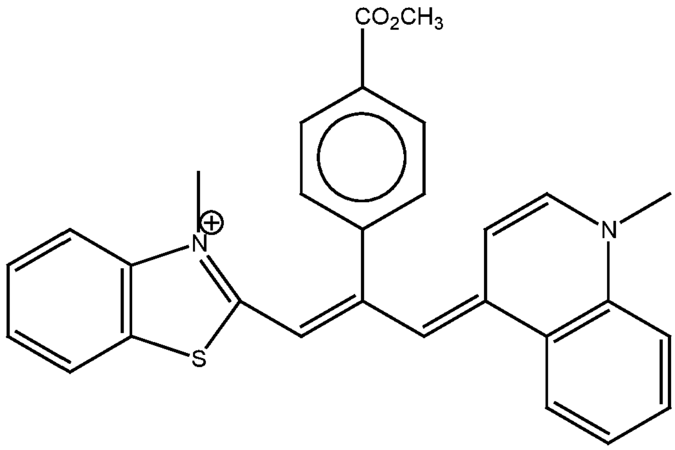 Compound **10** | 631/667 | 165 |
| 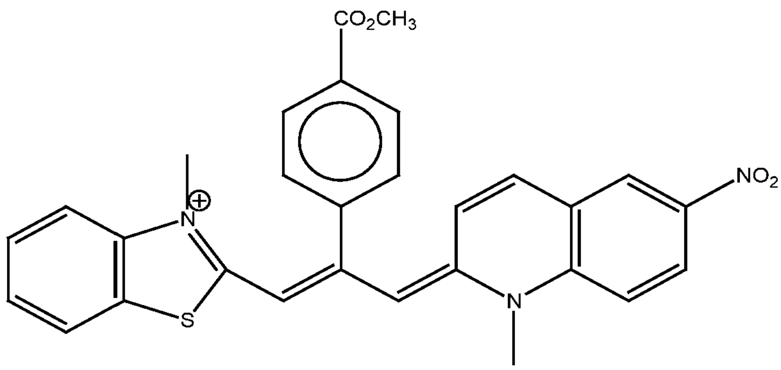 Compound **11** | 526/598 | 6 |
| 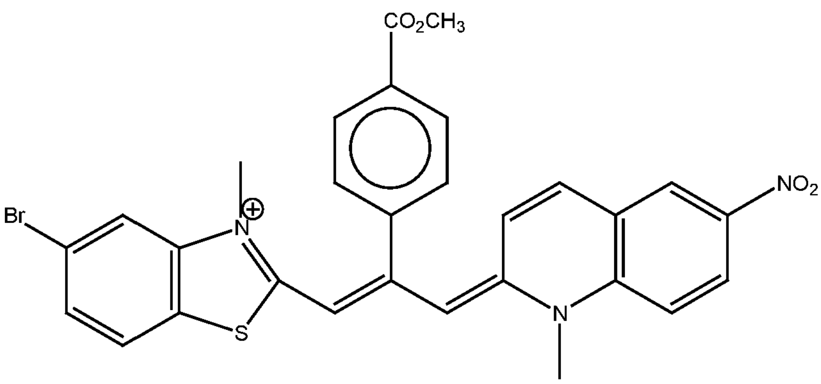 Compound **12** | 603/630 | 7 |

(continued)

| Compound | Ex/Em (nm)[1] | Fluorescence Enhancement Ratio (RNA/DNA)[2] |
|---|---|---|
| 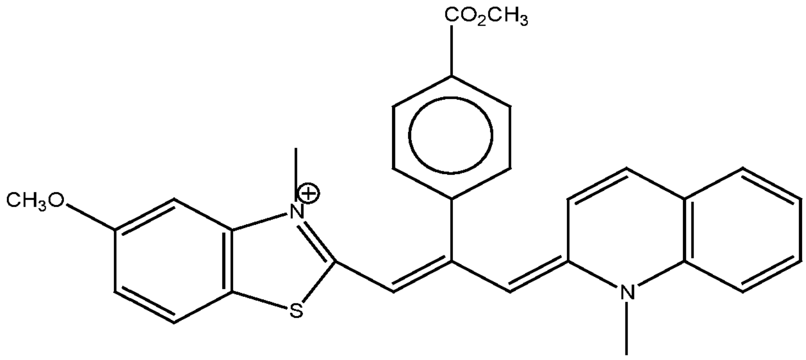 Compound **13** | 618/643 | 10 |
| 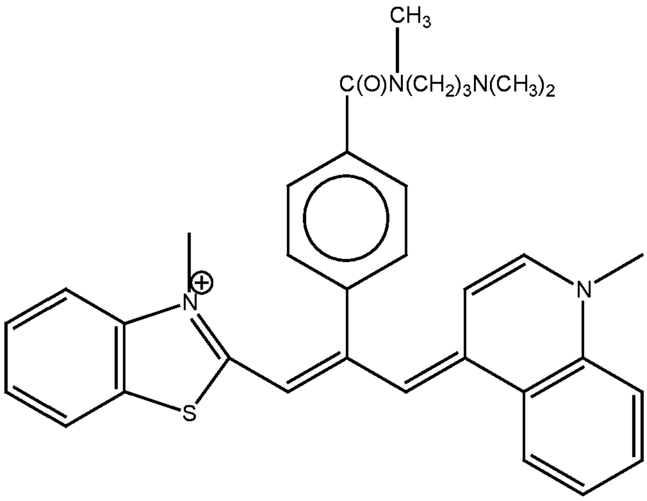 Compound **14** | 631/664 | 26.8 |
| 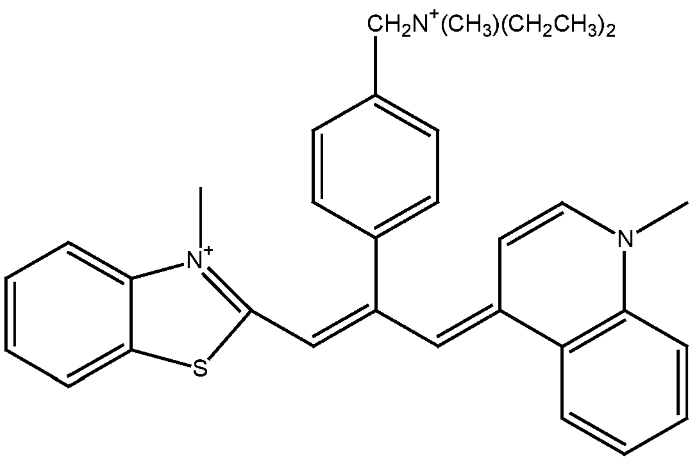 Compound **15** | 631/667 | 200 |

[1] Complex with nucleic acid.
[2] The ratio of the fluorescence enhancement of the compound when associated with RNA to the fluorescence enhancement of the compound when associated with DNA, as described in U.S. Patent No. 8,470,529.

**Instruments:**

Devices for measuring the quality of RNA in a sample are described herein. Devices that can be used in the methods of the present disclosure include fluorometers, including but not limited to those described in U.S. Patent No. 8,623,282, and microplate readers. Preferred instruments can run a single sample or multiple samples.

In a preferred embodiment, the methods of the present disclosure are performed on a QUBIT™ Fluorometer (Thermo Fisher Scientific, Waltham, MA). In certain preferred embodiments, the QUBIT™ fluorometer enables measurement of samples containing two dyes and enables measurements of samples for assays with high relative fluorescence units (RFUs). The methods described herein can be performed on the QUBIT™ fluorometer in less than 5 seconds per sample with high levels of accuracy using only 1 to 20 μl of sample, even when the sample is very dilute. The schematic shown in FIG. 1 describes the method of the present disclosure as performed on a QUBIT™ Fluorometer and the resulting read-out of the RNA IQ Score on a QUBIT™ Fluorometer. On the read-out, there are no units displayed; only the sample volume is required. If the results are within the instrument's assay range, the sample quality values are displayed. As can be seen in FIG. 1, the results presented on the display (shown in a circle in the center of the screen) are the total value for the RNA sample integrity and quality, e.g., RNA IQ Score (a value of 7.5 in FIG. 1), and also as the calculated percentage of large and/or structured RNA and small RNA in the sample. As shown in the schematic of FIG. 1, the display screen indicates below the circle that 75% of the sample contains large and/or structured (e.g., intact) RNA and 25% of the sample contains small (e.g., degraded) RNA.

**Kits:**

In another aspect, kits are provided comprising a dye solution comprising a first nucleic acid binding dye that selectively binds small RNA comprising 200 nucleotides or less and a second nucleic acid binding dye that selectively binds large RNA. In certain preferred embodiments, the first nucleic acid binding dye is selected from the compounds listed in Table 1 and the second nucleic acid binding dye is selected from the compounds listed in Table 2. In certain more preferred embodiments, the first nucleic acid binding dye is Compound **16,** Compound **17,** Compound **23,** Compound **24,** Compound **29,** Compound **30,** Compound **45,** Compound **46,** Compound **47,** Compound **52,** Compound **53,** Compound **59,** Compound **60** or Compound **61.** In certain more preferred embodiments, the second nucleic acid binding dye is Compound **1,** Compound **2,** Compound **3,** Compound **4,** Compound **5,** Compound **6,** Compound **7,** Compound **8,** Compound **9,** Compound **10,** Compound **11,** Compound **12,** Compound **13,**

Compound **14** or Compound **15.** In certain preferred embodiments, the first nucleic acid binding dye is Compound **16,** Compound **17,** Compound **23,** Compound **24,** Compound **29,** Compound **30,** Compound **45,** Compound **46,** Compound **47,** Compound **52,** Compound **53,** Compound **59,** Compound **60** or Compound **61,** and the second nucleic acid binding dye is Compound **1,** Compound **2,** Compound **3,** Compound **4,** Compound **7,** Compound **9,** Compound **10,** Compound **12,** Compound **13,** Compound **14** or Compound **15.** In certain embodiments, the kits further comprise instructions for use according to any of the methods provided herein. In certain embodiments, the kits further comprise one or more standards selected from a blank standard, a large and/or structured RNA standard, and a small RNA standard. Kits may include reagents useful to conduct the assay methods described herein. In some embodiments, kits comprise at least one of an organic solvent and a desiccant. In certain embodiments, the kits further comprise an assay buffer. In certain embodiments the kits further comprise one or more components that can melt or denature RNA secondary or tertiary structure and/or modify the binding of one or more of the nucleic acid binding dyes. These modifying components include, but are not limited to NaCl, $MgCl_2$, $CaCl_2$, $ZnCl_2$, ammonium acetate, sodium acetate, EDTA, ethanol, SDS, phenol, bovine serum albumin (BSA), guanidine HCl, formamide, urea, thiourea, guanidine isothiocyanate betaine, ethylene glycol and 1,2-propylene glycol.

In certain embodiments, kits are provided comprising:

a dye solution comprising a first nucleic acid binding dye that selectively binds small RNA comprising 200 nucleotides or less and a second nucleic acid binding dye that selectively binds intact RNA with secondary and/or tertiary structure; and
instructions for use according to any of the methods described herein.

In certain embodiments, kits are provided comprising:

a first nucleic acid binding dye that selectively binds small RNA comprising 200 nucleotides or less;
a second nucleic acid binding dye that selectively binds intact RNA with secondary and/or tertiary structure; and instructions for use according to any of the methods described herein,
wherein the first nucleic acid binding dye and the second nucleic acid binding dye are detectably distinct.

In certain embodiments, the kits further comprise:

a first standard solution comprising a buffer and no nucleic acid;
a second standard solution comprising a small RNA standard, wherein the small RNA standard has fewer than 200 nucleotides; and
a third standard solution comprising a large and/or structured RNA standard.

In another aspect, a staining solution is provided comprising a first nucleic acid binding dye, a buffer and optionally, an organic solvent. In some embodiments, the solvent is DMSO.

In certain embodiments of the present disclosure, an RNA quality staining solution is provided, comprising:

a first nucleic acid binding dye that preferentially binds small RNA comprising 200 nucleotides or less;
a second nucleic acid binding dye that preferentially binds intact RNA with secondary and/or tertiary structure;
a buffer; and
an organic solvent,
wherein the first nucleic acid binding dye and the second nucleic acid binding dye are detectably distinct.

**Examples:**

**Example 1: Exemplary Method for Determining RNA Quality and Calculation of an RNA Quality (IQ) Score**

The method described in this example provides a faster, easier measurement of the "integrity and quality" (IQ) of RNA samples and is described schematically in FIG. 1. Components used in this method were as follows:

1) a dye stock in DMSO containing:

a) a nucleic acid binding dye that selectively binds intact RNA with secondary and/or tertiary structure and emits in the red channel of the fluorometer; and
b) a nucleic acid binding dye that selectively binds small RNA comprising 200 nucleotides or less, but does not bind large and/or structured RNA;

2) Three Standards:

a) Standard 1 = buffer blank to determine background fluorescence;
b) Standard 2 = a small (< 200 nt) RNA standard to represent fully degraded RNA samples; and
c) Standard 3 = large and/or structured RNA to represent fully intact RNA; and

3) Buffer.

The user workflow for this method was as follows:

1) A working solution of the dye stock was prepared in buffer (herein termed "Dye Working Solution");
2) Three 0.5 ml microfuge tubes were labeled, one for each standard. For each tube, 10 $\mu$l of standard was added (Tube 1 contained Standard 1, Tube 2 contained Standard 2, Tube 3 contained Standard 3) and 190 $\mu$L of the Dye Working Solution was added to each tube (final volume = 200 $\mu$l).
3) To 0.5 ml microfuge tubes that were labeled for each test sample, 1-20 $\mu$l of test sample was added and a sufficient amount of the Dye Working Solution was added to achieve a final volume of 200 $\mu$l.
4) The tubes were mixed by vortexing for 2-3 seconds and incubated at room temperature for 2 minutes.
5) The tubes were inserted into a QUBIT™ Fluorometer and read by the fluorometer.
6) The fluorometer provided an IQ value of 7.5 which was calculated by the instrument using the equation of Formula (**II**). (See, FIG. 1)

The nucleic acid binding dye that is selective for large and/or structured RNA responds only to large, intact RNA whereas the nucleic acid binding dye that is selective for small RNA responds to single-stranded RNA, but minimally to large RNA. By measuring the ratio of green emission (small RNA) and red emission (large RNA), and the equation of Formula (**II**), a solution-based assay derived quality score was obtained:

$$Quality\ Score\ (IQ) = \frac{10(-GR_x+GR_2)}{(-GR_3+GR_2)} \quad \text{Formula (II)}$$

wherein $GR_2$ represents the green/red ratio of Standard 2; $GR_3$ represents the green/red ratio of Standard 3; and $GR_x$ represents the green/red ratio of the sample. When $GR_x$ is equal to $GR_2$, the quality score is 0 (i.e., completely degraded) and when $GR_x$ is equal to $GR_3$, the quality score is 10 (i.e., fully intact).

The readout shown in FIG. 1 was an IQ value of 7.5 which indicated that 75% of the sample contained large and/or structured RNA (e.g., intact RNA) and 25% of the sample contained small RNA (e.g., degraded RNA).

**Example 2: Determination of RNA quality using samples containing pure rRNA or pure siRNA**

A series of solutions were prepared of rRNA and siRNA such the final concentrations of nucleic acids were as follows in 1X TE buffer (either siRNA or rRNA): 0 ng/μL, 0.5 ng/μL, 1.0 ng/μL, 2.0 ng/μL, 4.0 ng/μL, 6.0 ng/μL, 8.0 ng/μL, and 10.0 ng/μL.

An assay solution was then made by 200X dilution of a DMSO solution comprised of the following in 1X TE buffer with 0.01% CHAPS: DMSO Stock: 0.2 mg/mL Compound **16**, 0.0047 mg/mL Compound **15**.

Each sample was diluted 20X with assay buffer by adding 10 μL sample to 190μL assay solution in triplicate in a 96-well plate. The sample and assay solution combination was then incubated for 2 minutes, then assayed on a Tecan Infinite® m1000 Pro plate reader (Tecan Trading AG, Switzerland), using the following settings:

| **Channel 1** | **Channel 2** |
|---|---|
| Excitation: 644 nm | Excitation 500 nm |
| Emission: 673 nm | Emission: 525 nm |
| Bandwidth: 5 nm | Bandwidth 5 nm |

As shown in FIGs. 2A and 2B, the RFUs collected at 644/673 were then plotted against the concentration of nucleic acid in the original sample, and can be seen to increase only as a function of rRNA concentration, while the RFUs collected at 500/525 can be seen to only increase as a function of siRNA concentration. FIG. 2A shows the RFUs collected in the red emission channel and FIG. 2B shows the RFUs collected in the green emission channel.

**Example 3: Determination of RNA quality - Simultaneous measurement of rRNA and siRNA**

A series of solutions were prepared containing both rRNA and siRNA such the final concentrations of nucleic acids were as follows in 1X TE buffer: 0 ng/μL, 0.5 ng/μL, 1.0 ng/μL, 2.0 ng/μL, 4.0 ng/μL, 6.0 ng/μL, 8.0 ng/μL, and 10.0 ng/μL (see Table 3 below).

Table 3

| **Solution ID** | **[rRNA]** | **[siRNA]** | **[Total RNA], ng/μL** | **%Small RNA (siRNA)** |
|---|---|---|---|---|
| 1 | 40 ng/μL | 0 ng/μL | 40 ng/μL | 0% |
| 2 | | 1 ng/μL | 41 ng/μL | 2% |

(continued)

| Solution ID | [rRNA] | [siRNA] | [Total RNA], ng/gL | %Small RNA (siRNA) |
|---|---|---|---|---|
| 3 | | 2 ng/μL | 42 ng/μL | 5% |
| 4 | | 4 ng/μL | 44 ng/μL | 9% |
| 5 | | 6 ng/μL | 46 ng/μL | 13% |
| 6 | | 8 ng/μL | 48 ng/μL | 17% |
| 7 | | 10 ng/μL | 50 ng/μL | 20% |
| 8 | | 20 ng/μL | 60 ng/μL | 33% |
| 9 | | 50 ng/μL | 90 ng/μL | 56% |
| 10 | | 75 ng/μL | 115 ng/μL | 64% |
| 11 | | 100 ng/μL | 140 ng/μL | 71% |

An assay solution was then made by 200X dilution of a DMSO solution comprised of the following in 1X TE buffer with 0.01% CHAPS: DMSO Stock: 0.2 mg/mL Compound **16**, 0.047 mg/mL Compound **15**.

Each sample was diluted 20X with assay buffer by adding 10 μL sample to 190 μL assay solution in triplicate in a 96-well plate. The sample and assay solution combination was then incubated for 2 minutes, then assayed on a Tecan Infinite® m1000 Pro plate reader, using the following settings:

| Channel 1 | Channel 2 |
|---|---|
| Excitation: 644 nm | Excitation 500 nm |
| Emission: 673 nm | Emission: 525 nm |
| Bandwidth: 5 nm | Bandwidth 5 nm |

The RFUs collected at 644/673 and 500/525 were then plotted against the concentration of total nucleic acid in the original sample (FIG. 3A). The 500/525 channel does increase significantly as a function of siRNA concentration, as expected. While the fluorescence emission in the 644/673 channel does increase as a function of total RNA concentration, this only occurs significantly for ratios of siRNA:rRNA > 1:2. Most interestingly, by taking the ratio of the emission from the 500/525 channel to the 644/673 channel the response can be seen to have a linear correlation with the % small RNA present in the sample (FIG. 3B).

**Example 4: Correlation of emission channel ratios with the percentage of small RNA present in a sample**

A series of samples were prepared, with varying concentrations of rRNA and siRNA in 1X TE buffer, with the following compositions (see Table 4 below):

Table 4

| Solution ID | [rRNA] | [siRNA] | [Total RNA], ng/μL | %Small RNA (siRNA) |
|---|---|---|---|---|
| 1 | 0 ng/μL | 0 ng/μL | 0 ng/μL | N/A |
| 2 | 0 ng/μL | 12.5 ng/μL | 12.5 ng/μL | 100% |
| 3 | 0 ng/μL | 25 ng/μL | 25 ng/μL | 100% |
| 4 | 0 ng/μL | 50 ng/μL | 50 ng/μL | 100% |
| 5 | 12.5 ng/μL | 0 ng/μL | 12.5 ng/μL | 0% |
| 6 | 12.5 ng/μL | 12.5 ng/μL | 25 ng/μL | 50% |
| 7 | 12.5 ng/μL | 25 ng/μL | 37.5 ng/μL | 67% |
| 8 | 12.5 ng/μL | 50 ng/μL | 62.5 ng/μL | 80% |
| 9 | 25 ng/μL | 0 ng/μL | 25 ng/μL | 0% |

(continued)

| Solution ID | [rRNA] | [siRNA] | [Total RNA], ng/μL | %Small RNA (siRNA) |
|---|---|---|---|---|
| 10 | 25 ng/μL | 12.5 ng/μL | 27.5 ng/μL | 33% |
| 11 | 25 ng/μL | 25 ng/μL | 50 ng/μL | 50% |
| 12 | 25 ng/μL | 50 ng/μL | 75 ng/μL | 67% |
| 13 | 50 ng/μL | 0 ng/μL | 50 ng/μL | 0% |
| 14 | 50 ng/μL | 12.5 ng/μL | 62.5 ng/μL | 20% |
| 15 | 50 ng/μL | 25 ng/μL | 75 ng/μL | 33% |
| 16 | 50 ng/μL | 50 ng/μL | 100 ng/μL | 50% |

An assay solution was then made by 200X dilution of a DMSO solution comprised of the following in 1X TE buffer with 0.01% CHAPS: DMSO Stock: 0.2 mg/mL Compound **16**, 0.047 mg/mL Compound **15**.

Each sample was diluted 20X with assay buffer by adding 10μL sample to 190μL assay solution in triplicate in a 96-well plate. The sample and assay solution combination was then incubated for 2 minutes and assayed on a Tecan Infinite® m1000 Pro plate reader, using the following settings:

| Channel 1 | Channel 2 |
|---|---|
| Excitation: 644 nm | Excitation 500 nm |
| Emission: 673 nm | Emission: 525 nm |
| Bandwidth: 5 nm | Bandwidth 5 nm |

The RFUs collected at 644/673 and 500/525 were then plotted against the concentration of each type of nucleic acid in the original sample. The 500/525 channel does increase significantly as a function of siRNA concentration, as expected (FIG. 4A, bottom panel). However, the emission from the 644/673 channel can be seen to have some effect from siRNA when [siRNA] > [rRNA] (FIG. 4A, top panel). And yet again, taking the ratio of the emission from the 500/525 channel to the 644/673 channel the response can be seen to have a linear correlation with the %small RNA present in the sample (FIG. 4B).

By a simple manipulation of the data from the aforementioned experiment using the equation of Formula (**I**), the ratio of emissions from the channels 500/525 and 644/673 can be mapped onto a quality score, such that higher numbers imply smaller amounts of small RNA in the sample, and thus less degradation (FIGs. 5A and 5B).

By dividing the ratio of the fluorescence emission signal of the first nucleic acid binding dye (in this embodiment, the green emission channel) to the fluorescence emission signal of the second nucleic acid binding dye (in this embodiment, the red channel), herein termed the Green/Red Ratio or "G/R ratio" by a constant to scale it to 10, and subtracting it from 10 to reverse the slope, the ratio becomes a scale from 10 to 0, with 10 representing 100% intact rRNA and 0 representing 100% degraded RNA (see FIG. 5B and Formula (**I**)).

$$IQ\ Score = 10 - \frac{GR}{0.3} \qquad \text{Formula (I)}$$

**Example 5: Demonstration of Real-time RNA Degradation**

An assay solution was made by 200X dilution of a DMSO solution comprised of the following in 1X TE buffer with 0.01% CHAPS: DMSO Stock: 0.2 mg/mL Compound **16**, 0.047 mg/mL Compound **15**.

250μL of a sample containing 100 ng/μL rRNA was diluted 20X with assay solution, and this solution was distributed to 21 wells in a 96 well plate such that each well contained 200μL of solution. To each of these solutions was then added volumes of a stock solution containing RNase A, such that the following final concentrations were reached, in triplicate: 0 fM RNase A, 50 fM RNase A, 100 fM RNase A, 250 fM RNase A, 500 fM RNase A, 750 fM RNase A, and 1000 fM RNase A.

These solutions were then measured on a Tecan Infinite m1000 Pro plate reader at the following timepoints: 0 minutes, 1 minute, 7 minutes, 13 minutes, 18 minutes, 26 minutes, 32 minutes, and 34 minutes.

| Channel 1 | Channel 2 |
|---|---|
| Excitation: 644 nm | Excitation 500 nm |
| Emission: 673 nm | Emission: 525 nm |
| Bandwidth: 5 nm | Bandwidth 5 nm |

The RFU at each channel for each timepoint for each solution was then normalized to T=0 minutes, and plotted as a function of time after addition of RNase A (FIG. 6).

Plotting the ratio of the 500/525 channel to the 644/673 channel of the above-mentioned experiment, the ratio of the two signals increases as a function of time and thus, of the RNA degradation (FIG. 7).

By normalizing the t=0 timepoint of the 644/673 channel to 1, and the t=0 timepoint of the 500/525 channel to 0 from the sample series of the above experiment treated with 50 fM RNase, a plot was obtained of the size profile of the rRNA while it was being digested by RNase A (FIG. 8). Initially the emission from the 500/525 channel increased sharply as the 644/673 channel decreased sharply, indicating linearization and degradation of the rRNA by RNase. Over time, the 500/525 channel signal depleted, indicating further degradation of the RNA to fragments with very low affinity for either dye molecule.

**Example 6: Stopping RNA Degradation at Distinct Timepoints**

To a sample comprised of 100 μL of 500 ng/μL of rRNA was added RNase A such that the final concentration of RNase A was 100 fM. From this solution, 5 μL aliquots were removed from the solution at these defined timepoints: T = 0 minutes, 4 minutes, 10 minutes, 13 minutes, 16 minutes, 19 minutes, 21 minutes, 24 minutes, 27 minutes, and 30 minutes.

Immediately upon removal from the reaction solution, each sample was treated with 5 units of RNaseOUT™ Recombinant Ribonuclease Inhibitor in 1 μL water, an inhibitor of RNase activity, and vortexed then spun down. Each sample was then diluted to a final volume of 50 μL with nuclease free water, such that the final concentration was 100 ng/μL RNA. This sample from each timepoint was then assayed via both Agilent 2100 Bioanalyzer using the RNA 6000 Nano kit and via fluorescence using the conditions described below.

An assay solution was then made by 200X dilution of a DMSO solution comprised of the following in 1X TE buffer with 0.01% CHAPS: DMSO Stock: 0.2 mg/mL Compound **16**, 0.047 mg/mL Compound **15**.

Each sample was diluted 20X with assay buffer by adding 10μL sample to 190μL assay solution in triplicate in a 96-well plate. The sample and assay solution combination was then incubated for 2 minutes and assayed on a Tecan Infinite® m1000 Pro plate reader, using the following settings:

| Channel 1 | Channel 2 |
|---|---|
| Excitation: 644 nm | Excitation 500 nm |
| Emission: 673 nm | Emission: 525 nm |
| Bandwidth: 5 nm | Bandwidth 5 nm |

The fluorescence from the 500/525 and 644/673 channels were plotted against time after the addition of RNase A, showing the RNA degradation with these two channels (FIG. 9A).

By plotting the ratio of emission from the 500/525 and 644/673 channels against the time after additions of RNase, the increase of this ratio as a function of RNA degradation can be seen (FIG. 9B, top). Similarly, the RIN value determined by the 2100 Bioanalyzer software can be seen to decrease as a function of time (FIG. 9B, bottom).

The linear relationship between the ratiometric emission measurement and the RIN determined via Bioanalyzer demonstrates a strong correlation (FIG. 9C).

Over many runs with concentrations of RNase ranging from 100 fM to 1 fM using similar reaction and assay conditions as described above, the RIN and RNA-IQ metrics can be seen to have a strong correlation up to RIN ≤ 8 (FIG. 9D).

**Example 7: Analysis of a Series of Sample Volumes**

Three solutions of rRNA were prepared at the following concentrations, 1000 ng/μL, 100 ng/μL and 50 ng/μL,

via dilution of a 1000 ng/μL stock in nuclease free water. Each solution was then diluted in triplicate with an appropriate dilution scheme such that the final concentration was 5 ng/μL in 200 μL assay solution (volume of sample used, 1 μL for 1000 ng/μL sample, 10 μL for 100 ng/μL sample, and 20 μL for 50 ng/μL sample). The diluent was comprised of 1X TE with 0.01% CHAPS, containing 0.5% of a DMSO solution containing 0.2 mg/mL Compound **16** and 0.047 mg/mL Compound **15**.

| RNA conc. (ng/μL) | 1000 | 100 | 50 |
|---|---|---|---|
| Sample Volume | 1 μL | 10 μL | 20 μL |
| Green RFU1 | 13803 | 14346 | 13741 |
| Green RFU2 | 14327 | 14300 | 13640 |
| Green RFU3 | 13905 | 14247 | 13397 |
| Mean | 14011.67 | 14297.67 | 13592.67 |
| SD | 277.8081 | 49.54123 | 176.8172 |
| %CV | 2.0% | 0.3% | 1.3% |
| G/R ratio 1 | 1.479316 | 1.598216 | 1.681901 |
| G/R ratio2 | 1.608501 | 1.647832 | 1.626686 |
| G/R ratio3 | 1.547141 | 1.659507 | 1.691274 |
| G/R ratio mean | 1.545783 | 1.635115 | 1.666511 |
| SD | 0.06462 | 0.032544 | 0.0349 |
| %CV | 4.2% | 2.0% | 2.1% |
| Red RFU1 | 20419 | 22928 | 23111 |
| Red RFU2 | 23045 | 23564 | 22188 |
| Red RFU3 | 21513 | 23643 | 22658 |
| Mean | 21659 | 23378.33 | 22652.33 |
| SD | 1319.074 | 391.9953 | 461.5261 |
| %CV | 6.1% | 1.7% | 2.0% |
| | 94.5% | 100.0% | 101.9% |
| Percent Deviation from Mean | 90.4% | 98.0% | 99.8% |
| | 98.7% | 102.0% | 104.0% |

Each solution was prepared in this way in triplicate, placed in QUBIT™ tubes, and measured on the QUBIT™ Fluorometer. Even for the sample with only 1 μL used, 4.2% CV was obtained for the green/red ratio (FIGs. 10A and 10B).

**Example 8: Analysis of the Nucleic Acid Binding Dye that preferentially binds small RNA**

The nucleic acid binding dye that binds to small RNA can also bind to DNA. This is beneficial because DNA is often a contaminant in RNA samples and can be problematic for almost all downstream applications of RNA analysis. Knowledge of the presence of DNA as a contaminant is indicative of potential issues using the RNA sample for further study.

In the experiment shown in FIG. 11A, an assay solution was made by 200X dilution of a DMSO solution comprised of the following in 1X TE buffer with 0.0025% CHAPS: DMSO Stock: 0.6 mg/mL Compound **16**, 0.047 mg/mL Compound **15**. This solution was used to dilute samples such that the final assay solution contained 2 ng/μL dsDNA, and the listed amounts of rRNA. The assay solution was then incubated for 2 minutes, then assayed on a Tecan Infinite® m 1000 Pro plate reader, using the following settings:

| **Channel 1** | **Channel 2** |
|---|---|
| Excitation: 644 nm | Excitation 500 nm |
| Emission: 673 nm | Emission: 525 nm |
| Bandwidth: 5 nm | Bandwidth 5 nm |

The RFUs collected at 644/673 and 500/525 were then plotted against the amount of nucleic acid in the sample (FIG. 11A).

In the experiment shown in FIG. 11B, an assay solution was made by 200X dilution of a DMSO solution comprised of the following in 1X TE buffer with 0.01% CHAPS: DMSO Stock: 0.2 mg/mL Compound **16**, 0.0047 mg/mL Compound **15**. This solution was used to dilute samples such that the final assay solution contained 0.25 ng/μL rRNA, and the listed amounts of dsDNA. The assay solution was then incubated for 2 minutes, then assayed on a Tecan Infinite® m 1000 Pro plate reader, using the following settings:

| Channel 1 | Channel 2 |
|---|---|
| Excitation: 644 nm | Excitation 500 nm |
| Emission: 673 nm | Emission: 525 nm |
| Bandwidth: 5 nm | Bandwidth 5 nm |

The RFUs collected at 644/673 and 500/525 were then plotted against the amount of nucleic acid in the sample (FIG. 11B).

**Example 9: Correlation of RNA IQ values and RNASEQ percent mappable reads of clinical FFPE samples**

Samples were prepared from FFPE preserved clinical tissue samples via the ONCOMINE™ kit protocol (Thermo Fisher Scientific, Waltham, MA). Tissue slides were deparaffinized using xylene and ethanol, then collected and digested with proteinase. The RNA and DNA were separated via filter cartridges, each collected individually. RNA was quantified via fluorometric measurement using the reagents supplied in the ONCOMINE™ kit via plate reader, then subjected to reverse transcription reaction to generate cDNA from the isolated RNA. The purified cDNA was then amplified, labeled, and combined in a library for sequencing. The library was loaded onto ION SPHERE™ particles (Thermo Fisher Scientific, Waltham, MA), then sequenced using the Ion PGM Dx System (Thermo Fisher Scientific, Waltham, MA). Sequencing results for the cDNA samples were then compared to reference sequences to determine the percentage of reads that could be matched to those in the reference sequences. This measure of reads that correspond to the reference sequences were presented as the percent of mappable reads.

RNA isolated from clinical FFPE tissue retains was subjected to RNA Seq on the Ion Torrent ONCOMINE™ platform (described herein above) and compared to RNA IQ results obtained using the method described in Example 1. It was found that sufficiently mapped reads (>50% mappable reads) correlated to RNA IQ > 4. With this guideline, only 4 out of 60 samples resulted in a false negative result, a 6.7% failure rate. The results are shown in FIG. 12.

**Example 10: Addition of $MgCl_2$ increases linearity of the curve for high concentrations of large and/or structured RNA**

To evaluate the effect of additives to the assay buffer on the linearity of the RNA IQ response, an eleven-point titration curve was made by diluting a range of standard 2 with a range of standard 3 such that the total amount of RNA was identical for each of the samples but the percentage of large and/or structured RNA ranged from 0-100%. To each of the samples a dye working solution was added for a total volume of 200 μL. Samples were well mixed by trituration and incubated at room temperature for a minimum of 2 minutes prior to reading on the QUBIT™ fluorometer instrument (Thermo Fisher Scientific, Waltham, MA). The RNA quality IQ Score was determined using the ratio of green to red response according to Formula (II). Results are shown in FIG. 14, where the percent of large and/or structured RNA is plotted against the calculated RNA IQ value. Individual fluorescence responses from each of the fluorescent emission channels were plotted and are shown in FIG. 15. The experiment was repeated over a range between 1/8 to 2 times of the total RNA contained in standard 2 and standard 3 to determine the change in RNA IQ over a range of RNA concentrations.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method of determining the extent of degradation of RNA in a sample, expressed as an RNA quality value, the method comprising:

   a) combining a sample suspected of containing RNA with a first nucleic acid binding dye that selectively binds small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
   b) incubating the sample for a time period sufficient to allow the first nucleic acid binding dye and the second nucleic acid binding dye to combine with the RNA in the sample to form a first nucleic acid binding dye-RNA complex and a second nucleic acid binding dye-RNA complex;
   c) illuminating the first nucleic acid binding dye-RNA complex and the second nucleic acid binding dye-RNA complex with an appropriate wavelength of light to form an illuminated sample mixture, wherein the first nucleic acid binding dye-RNA complex emits a first fluorescent signal and the second nucleic acid binding dye-RNA complex emits a second fluorescent signal;
   d) detecting the first fluorescent signal and the second fluorescent signal; and
   e) calculating a ratio of the first fluorescent signal to the second fluorescent signal, thereby obtaining the RNA quality value, wherein the RNA quality value represents the extent of degradation of RNA in the sample,
   wherein the first nucleic acid binding dye is selected from

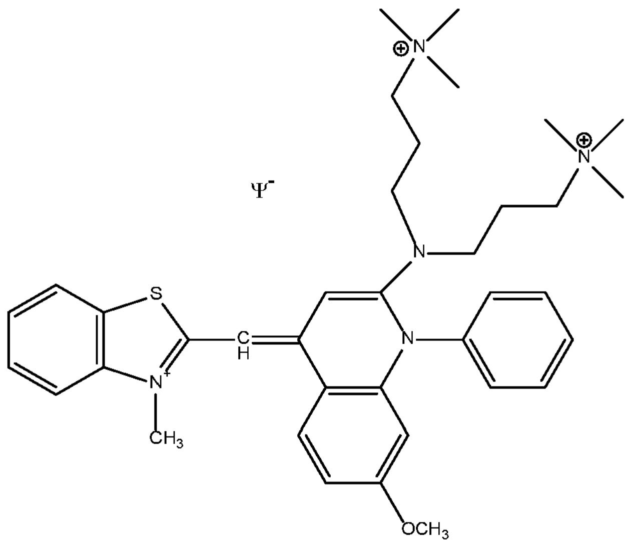

,

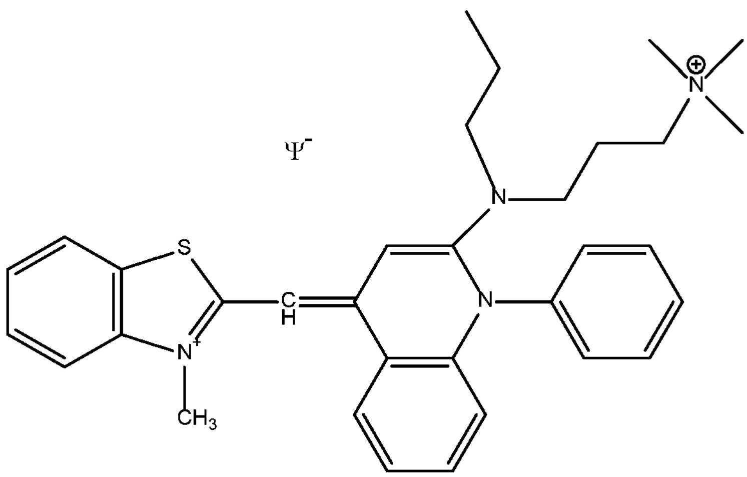

,

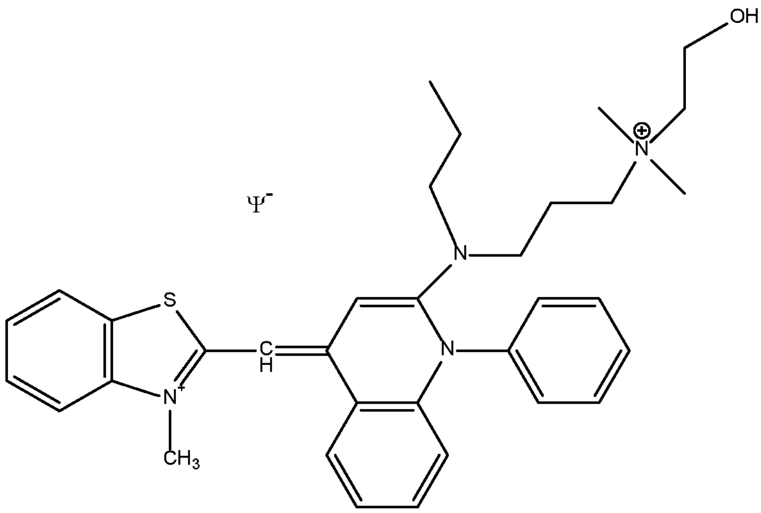

,

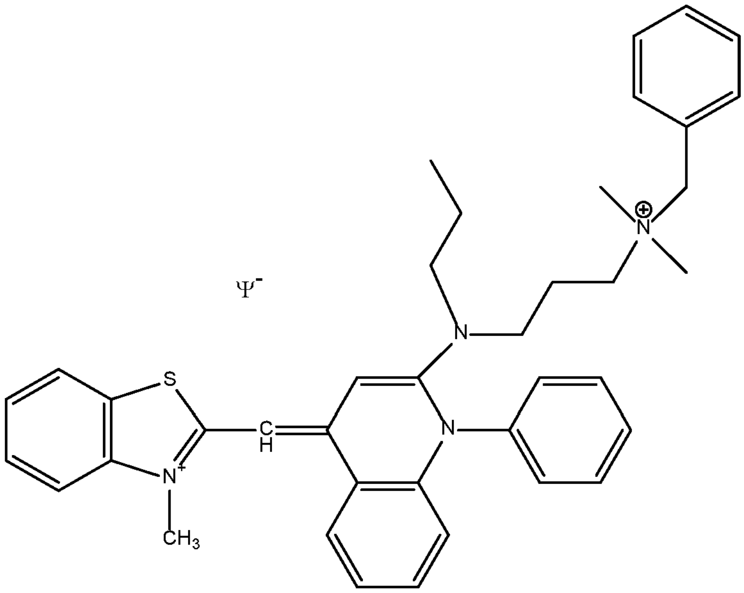

,

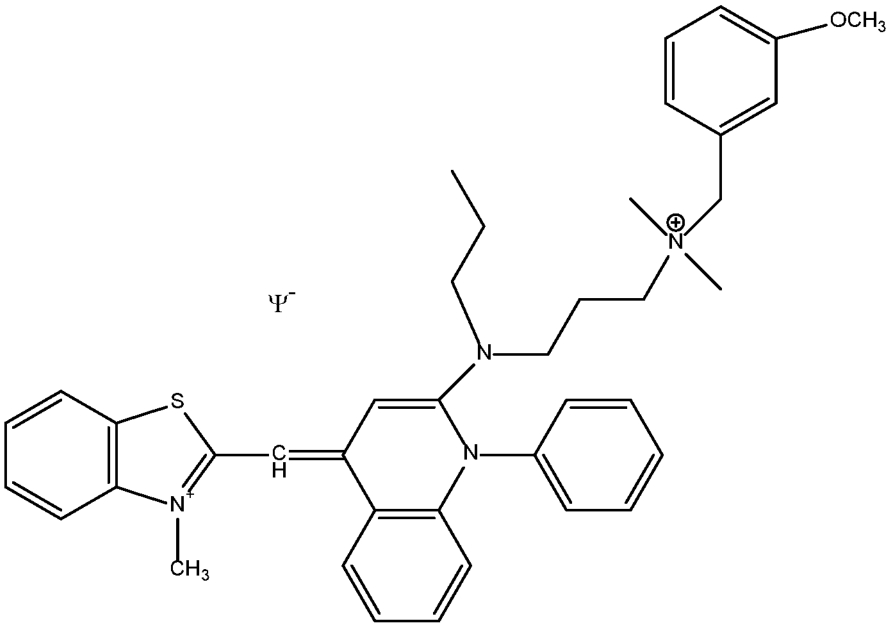

,

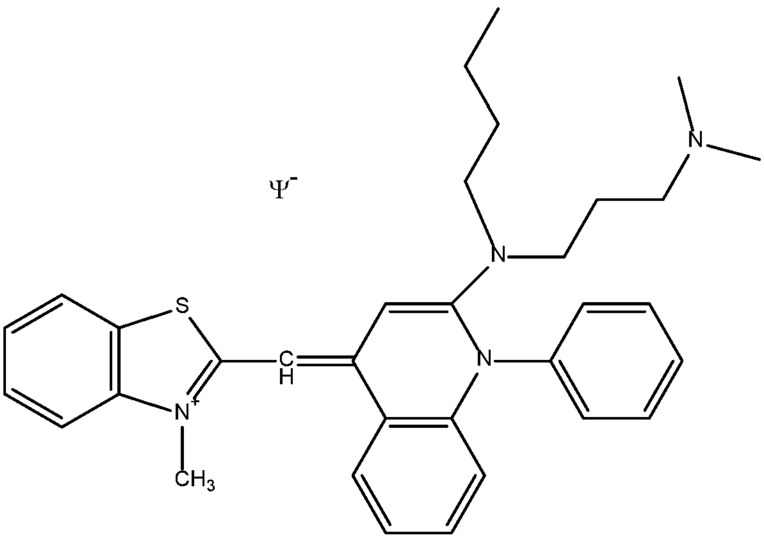

,

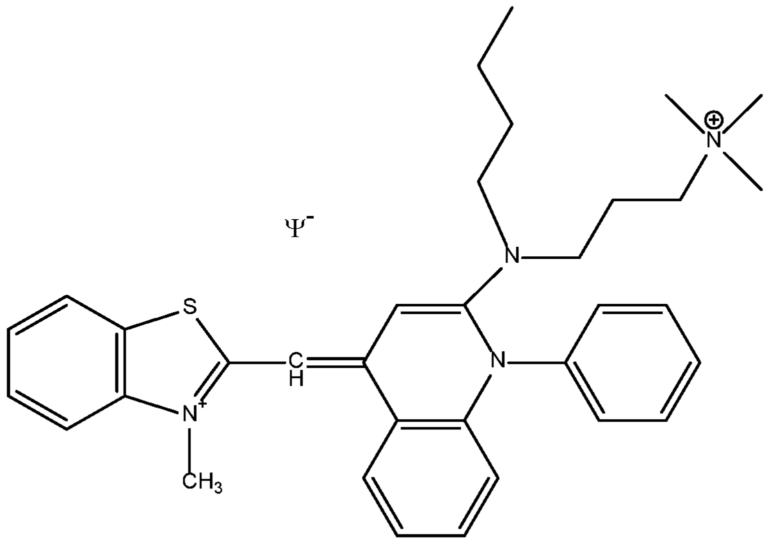

,

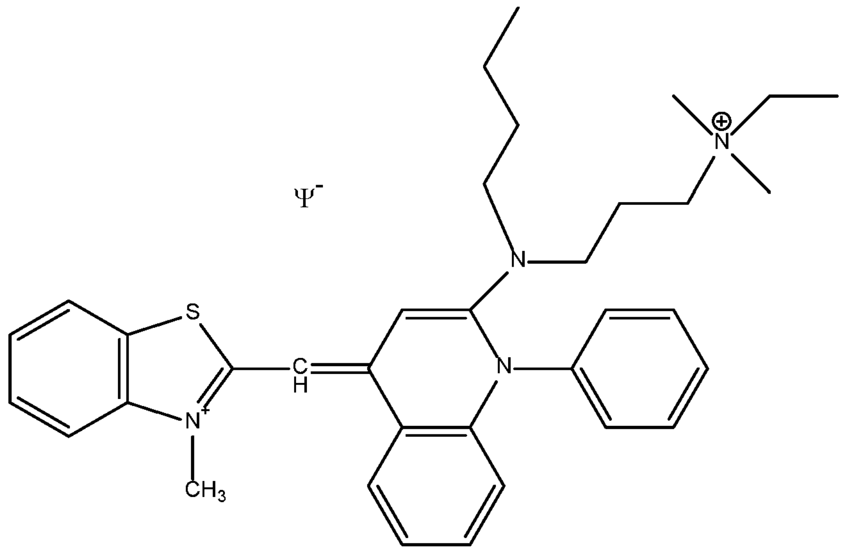

,

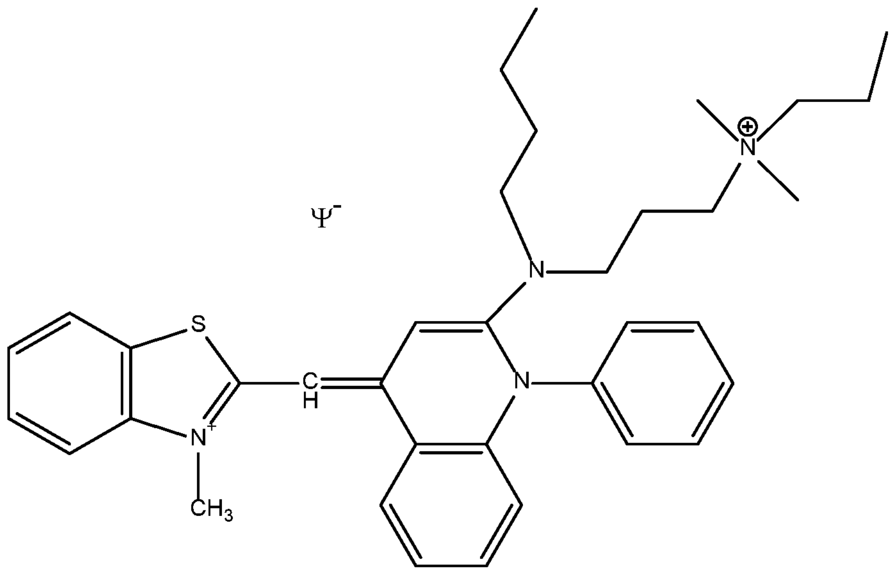

,

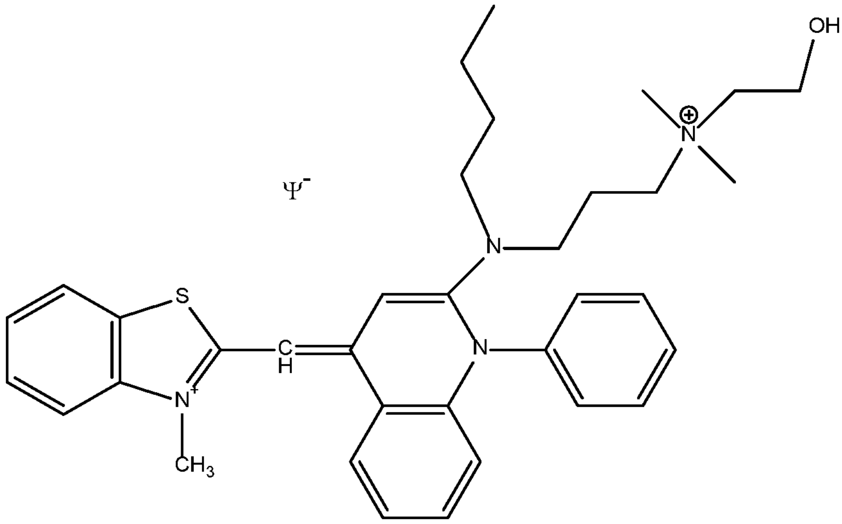

,

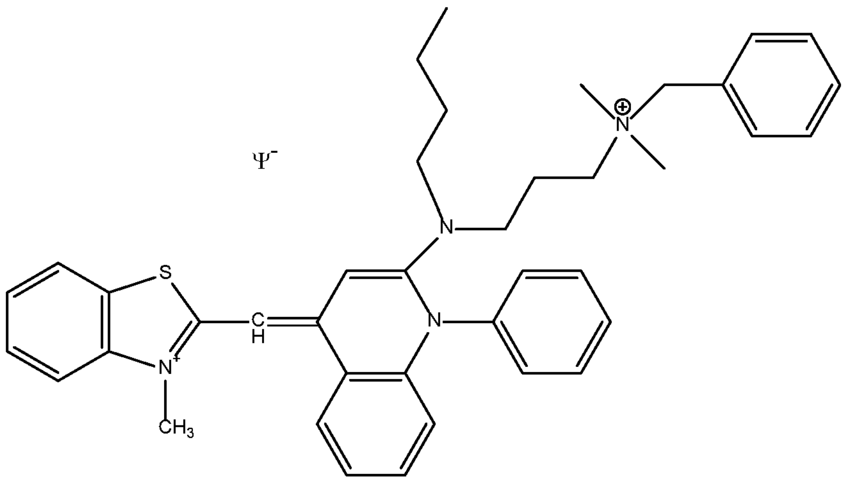

,

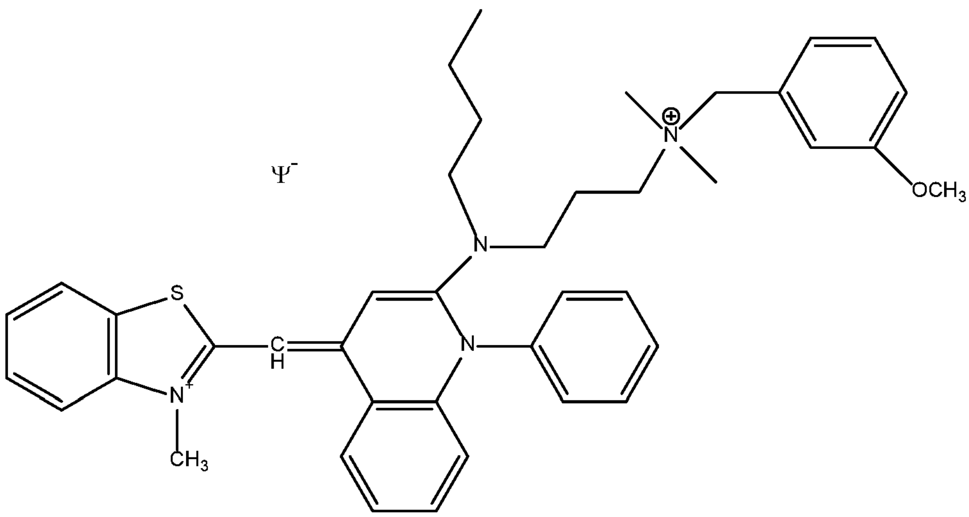

,

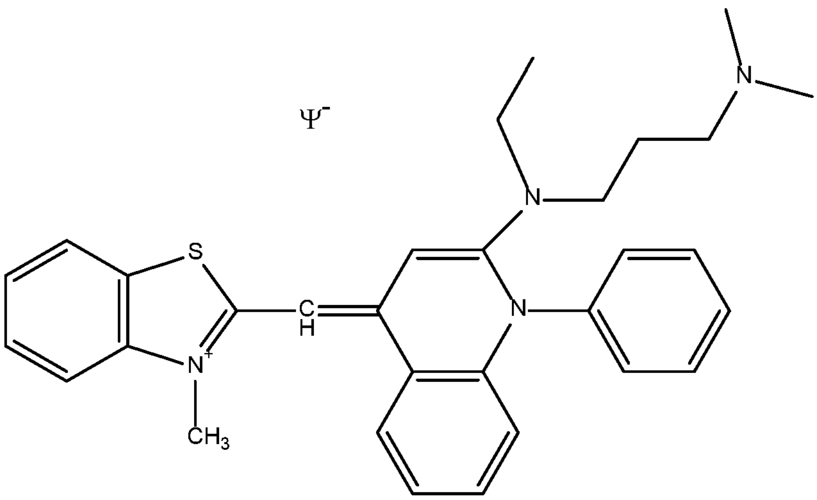

,

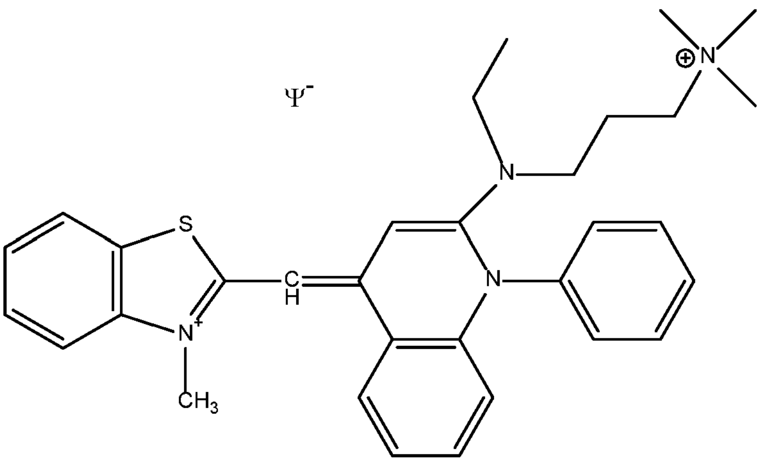

,

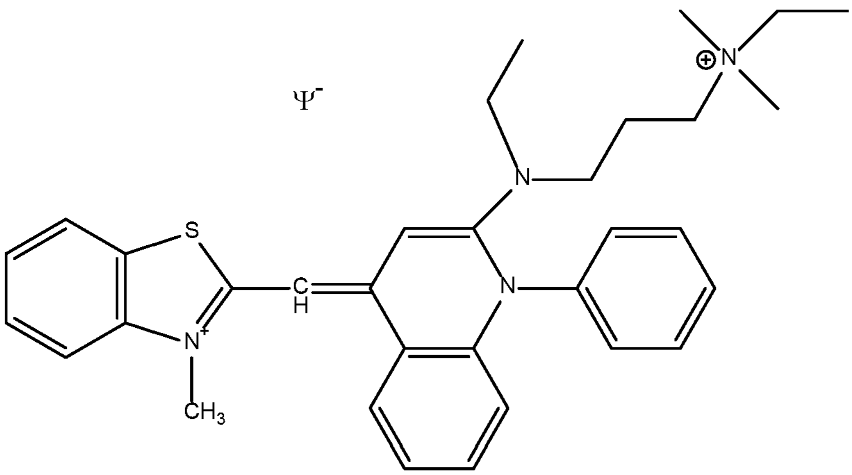

,

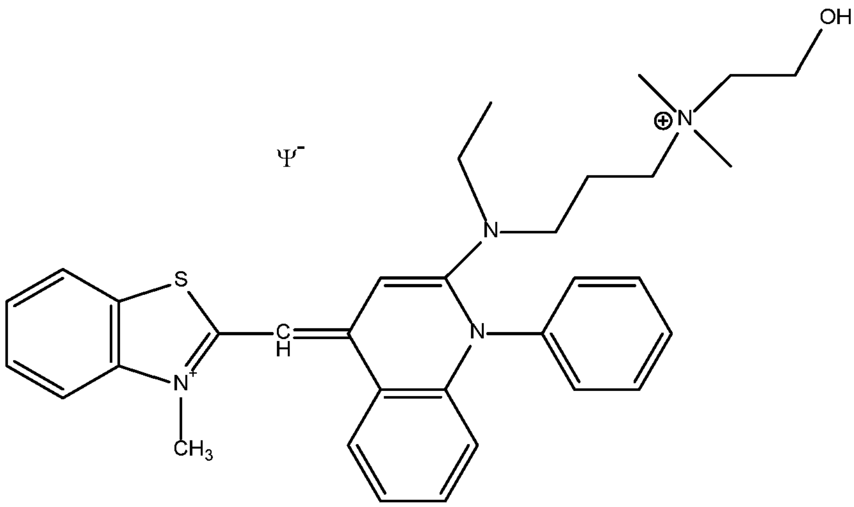

,

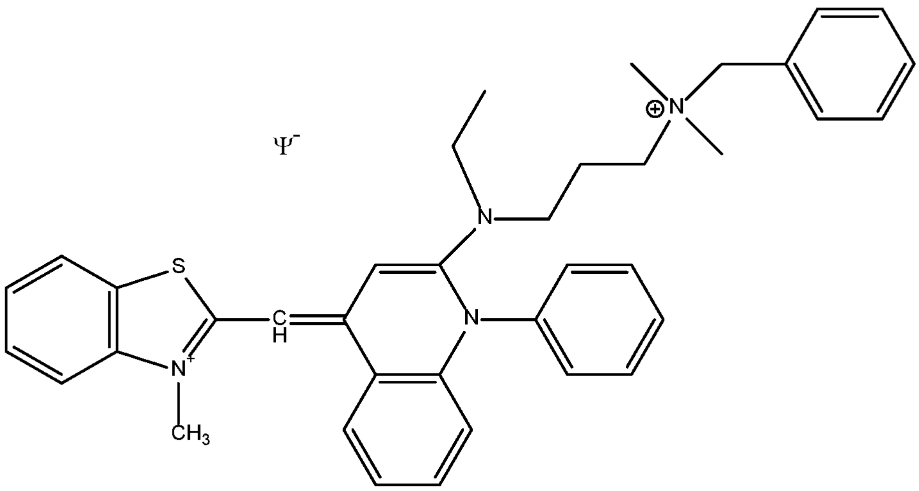

,

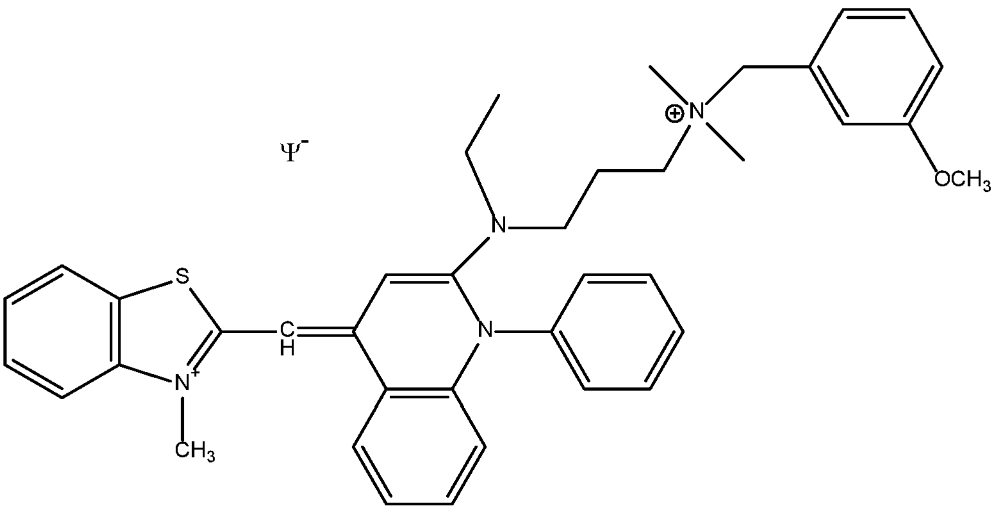

,

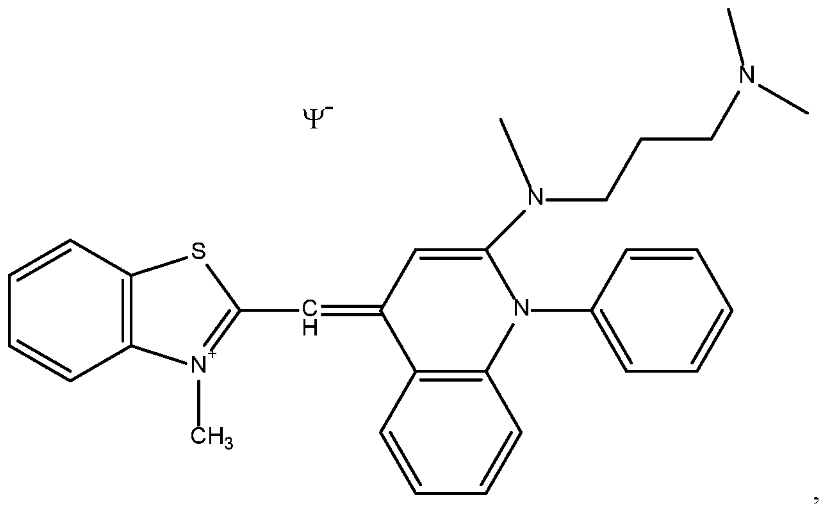

,

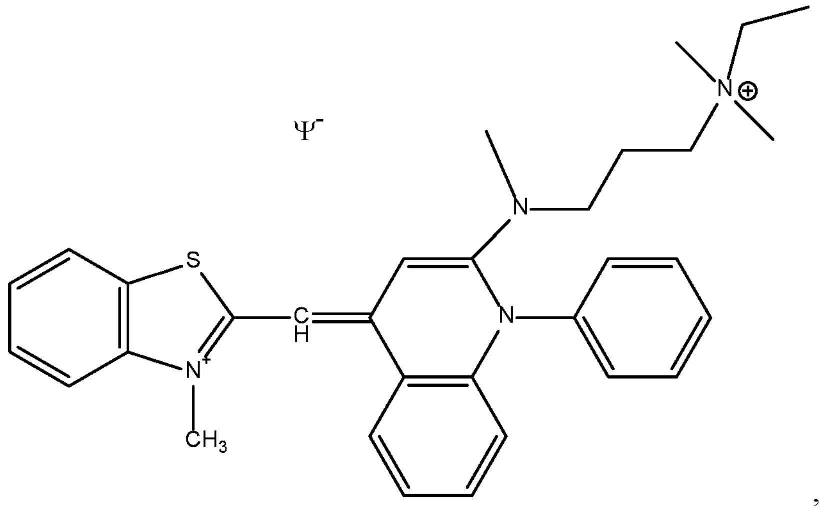

,

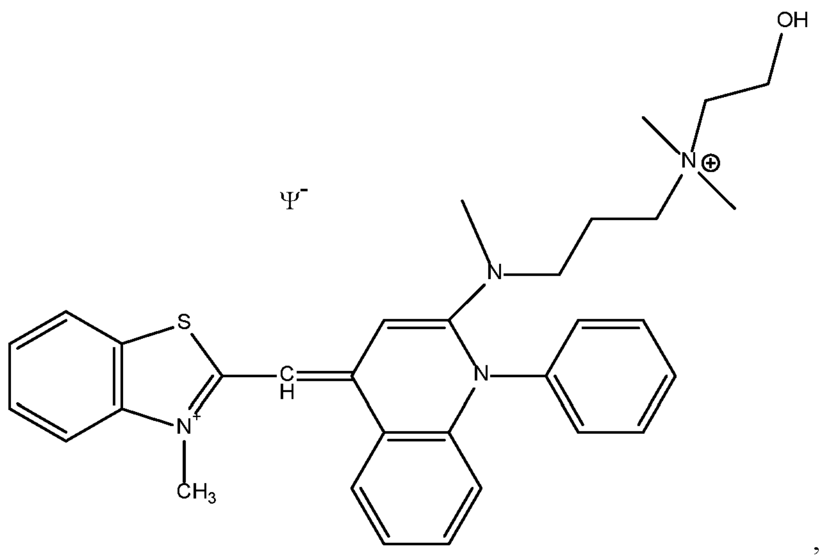

,

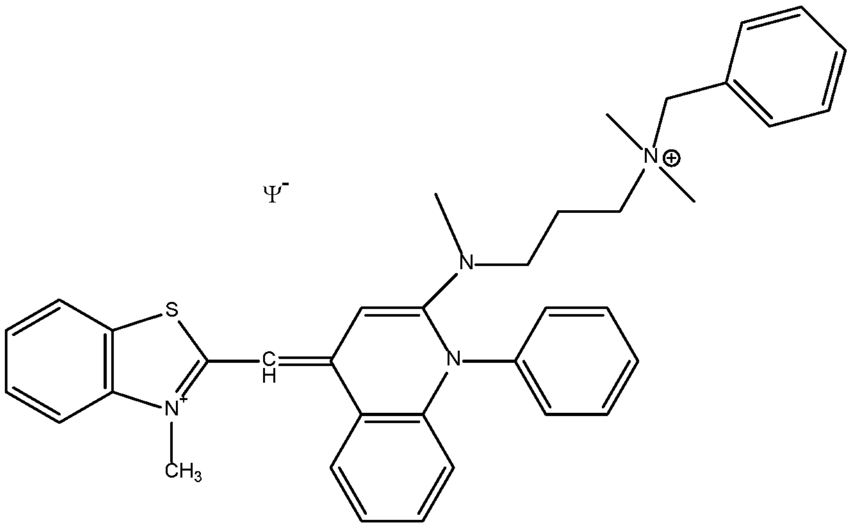

,

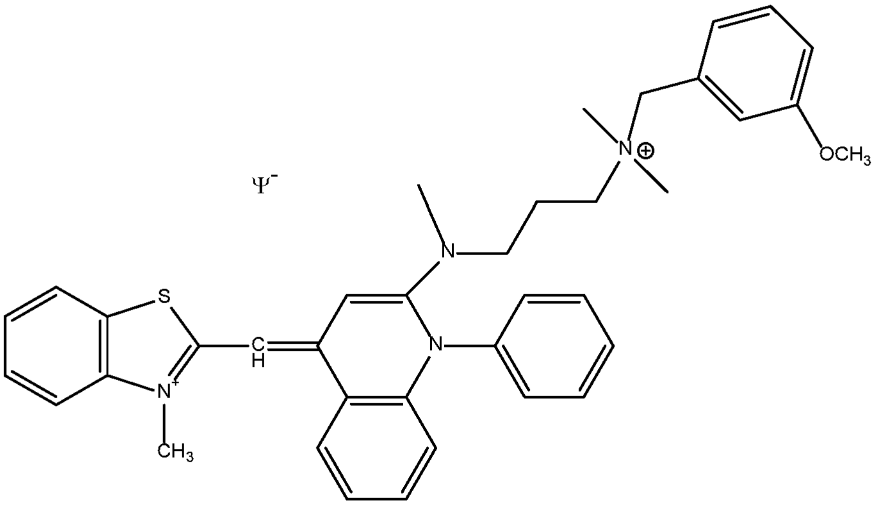

,

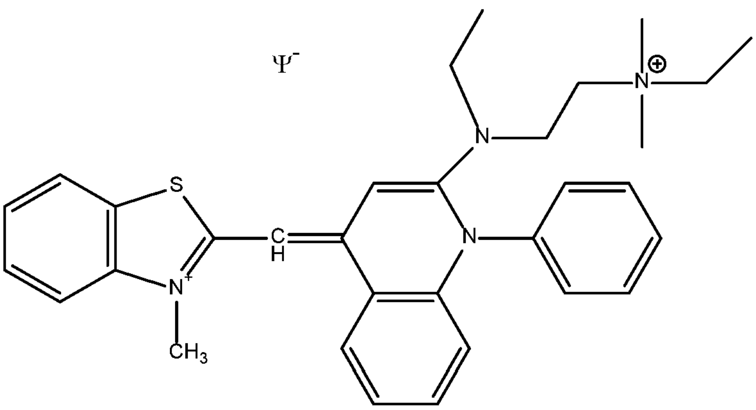

,

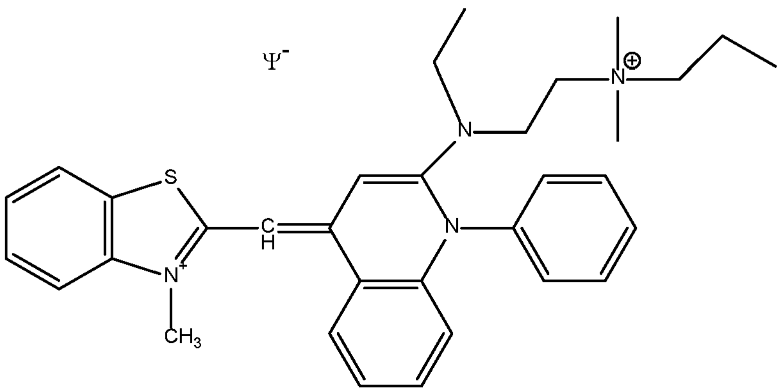

,

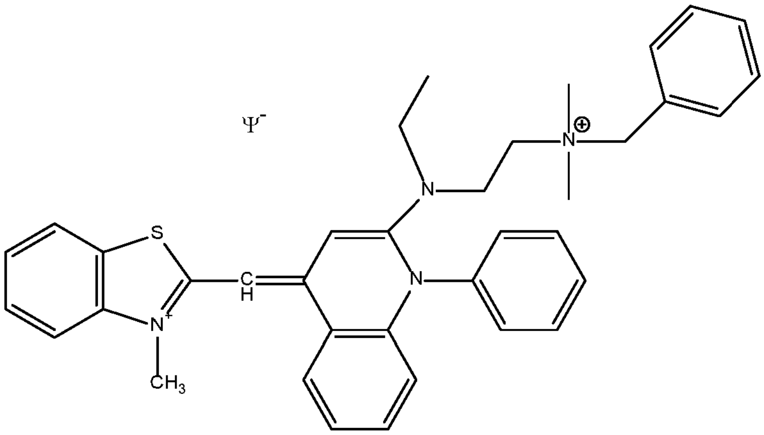

,

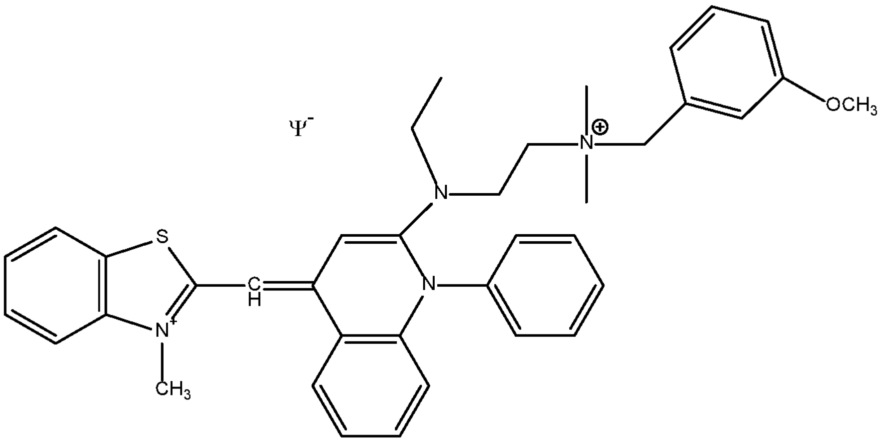

,

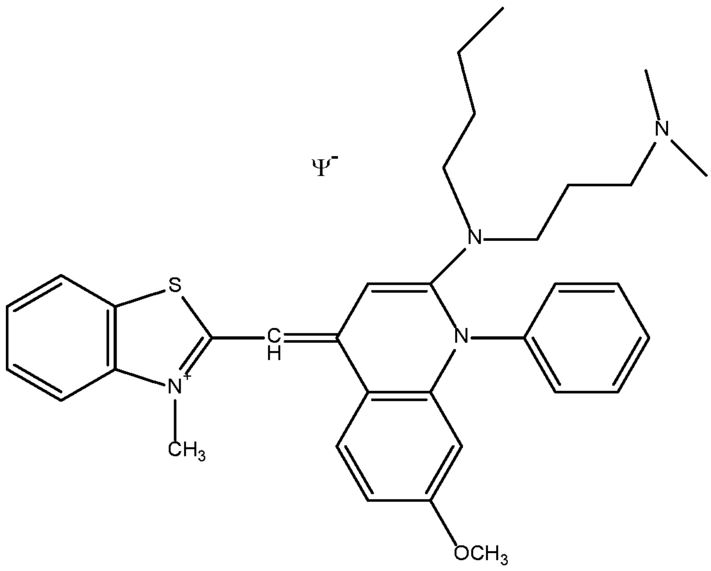

,

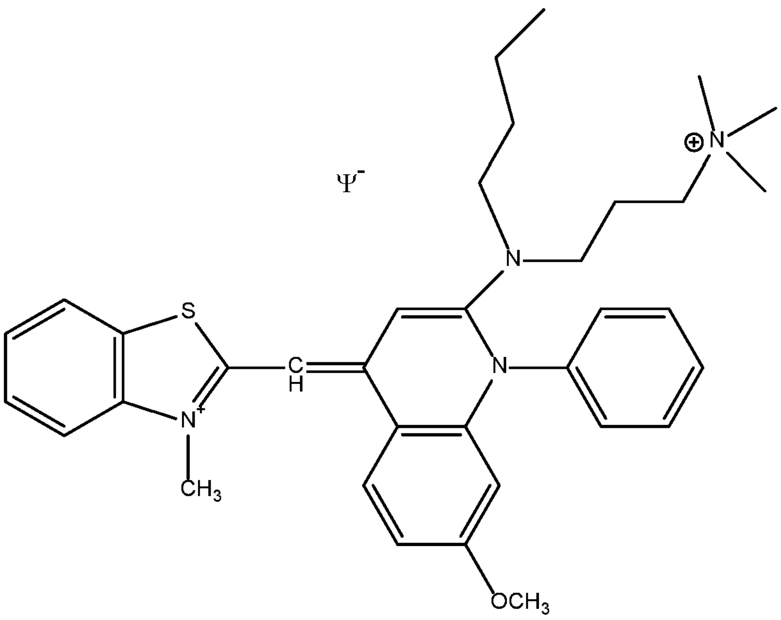

,

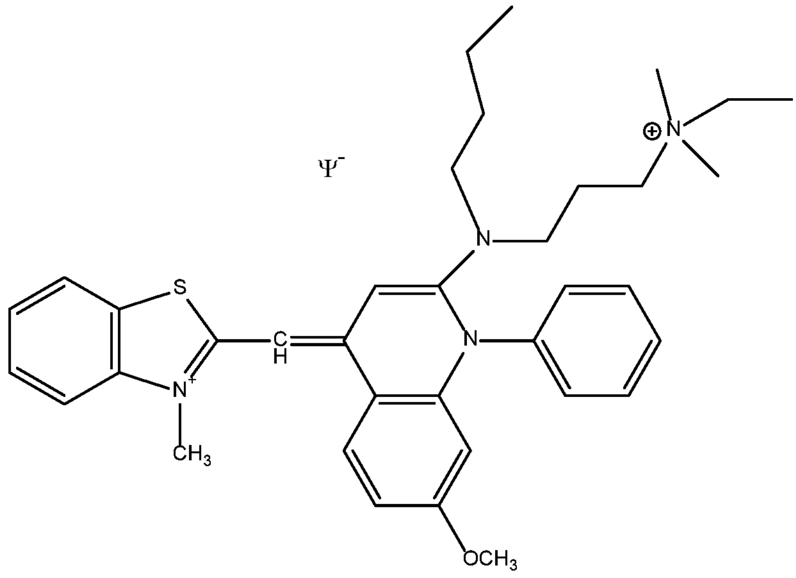

,

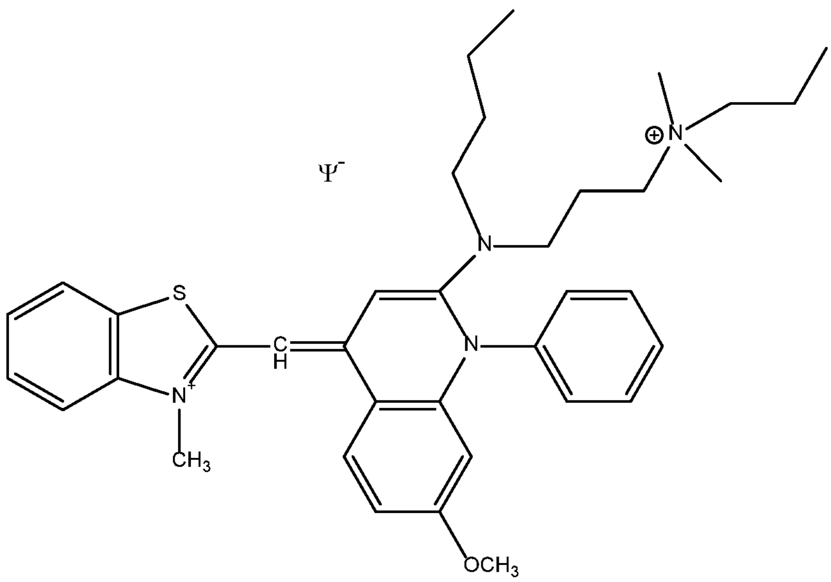

,

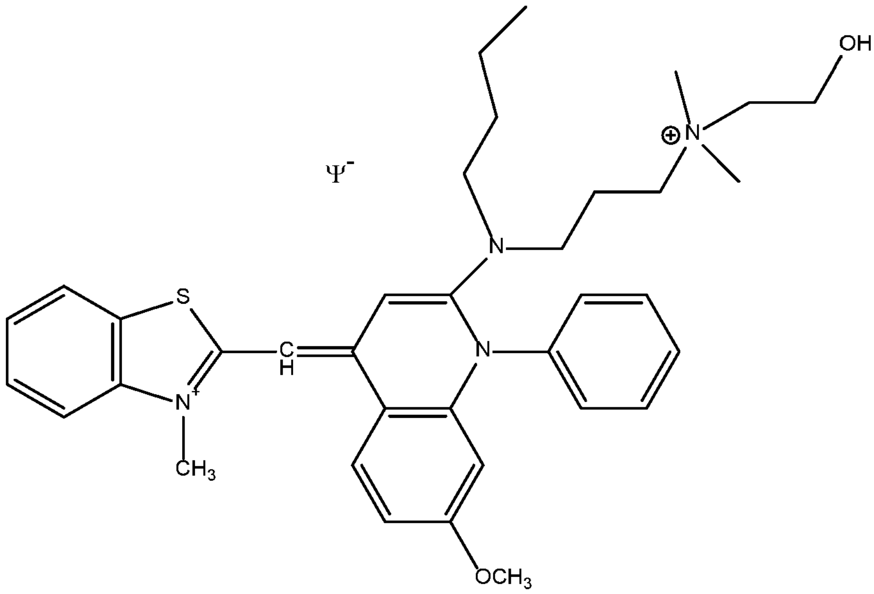

,

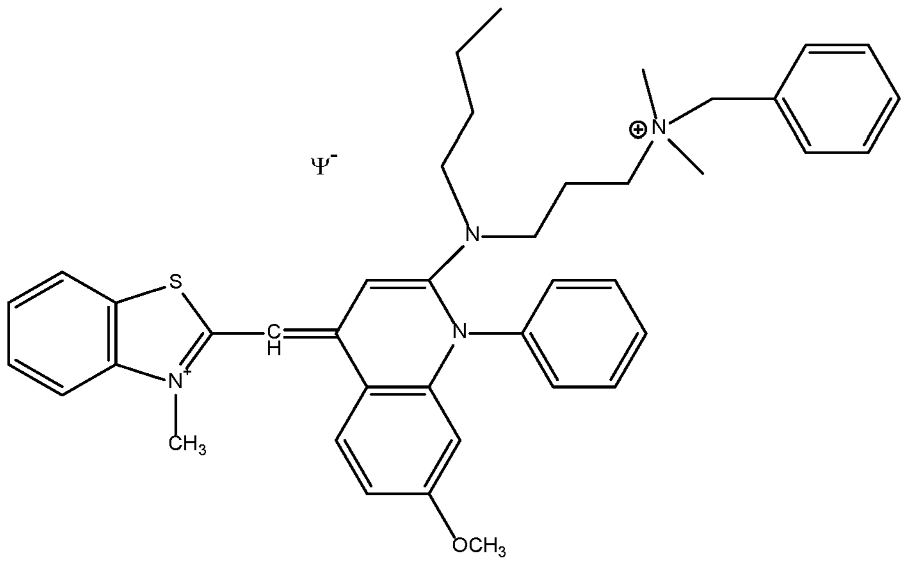

,

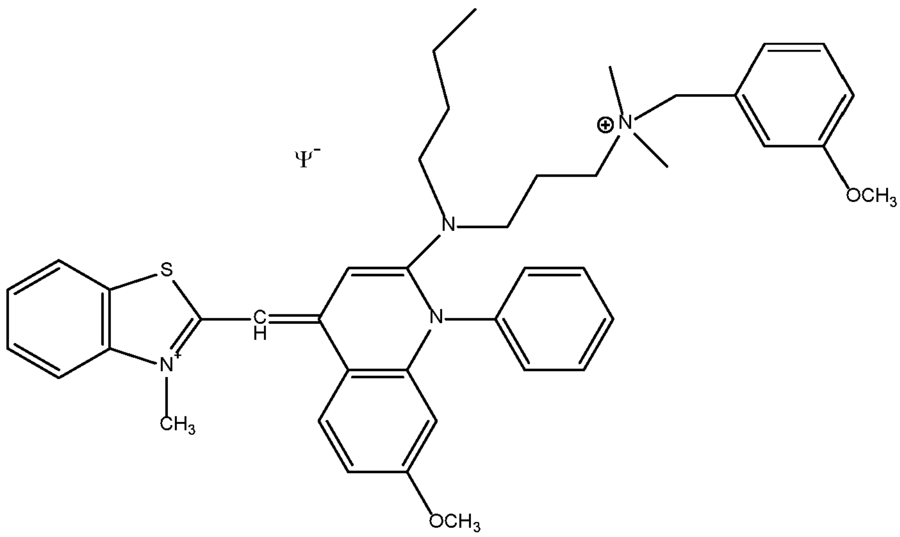

,

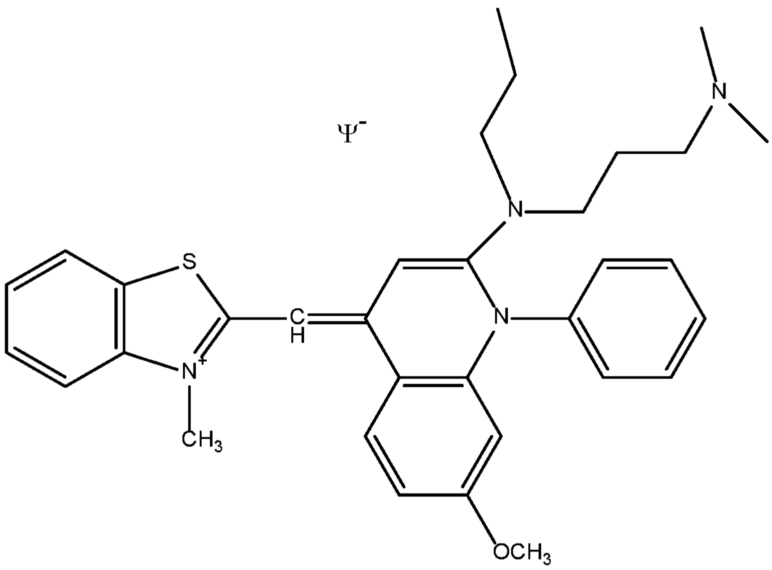

,

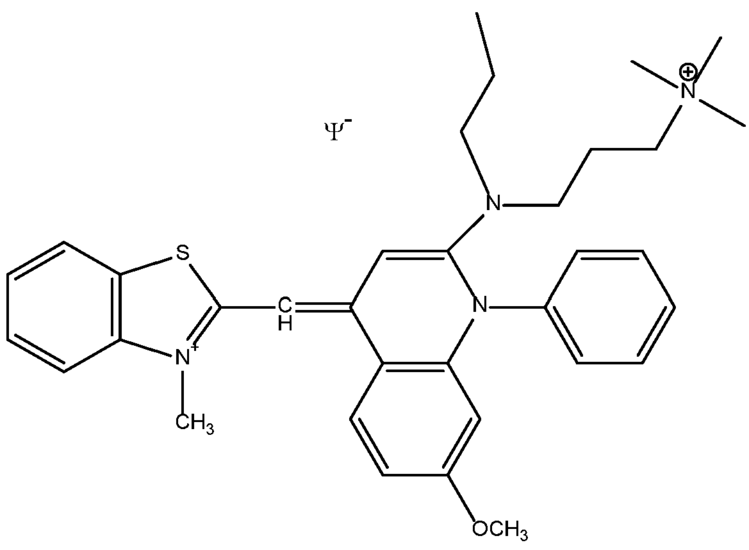

,

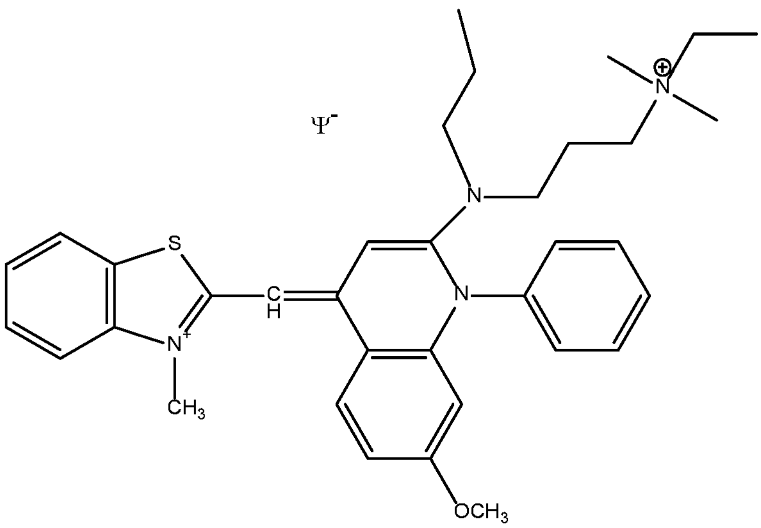

,

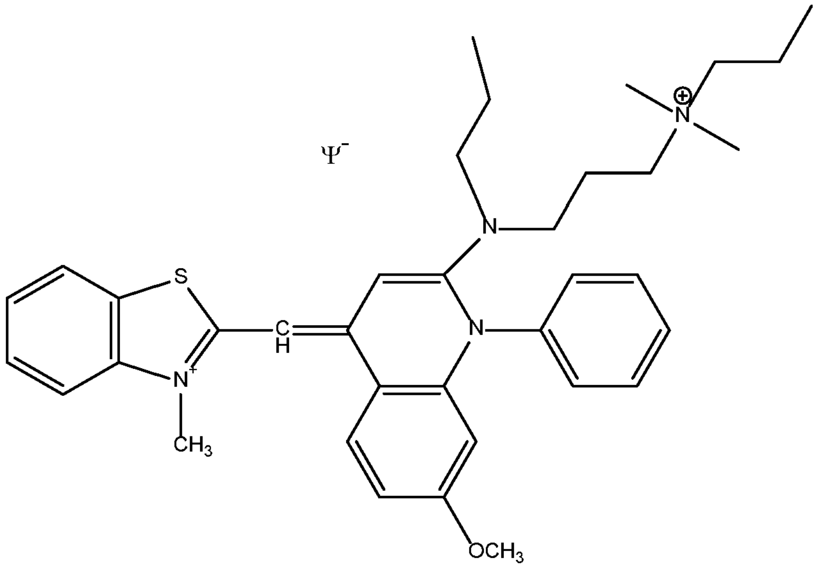

,

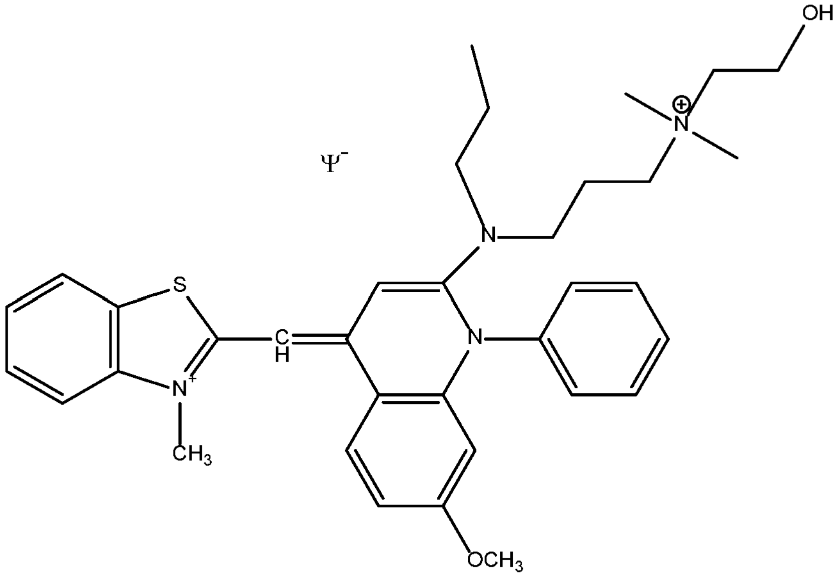

,

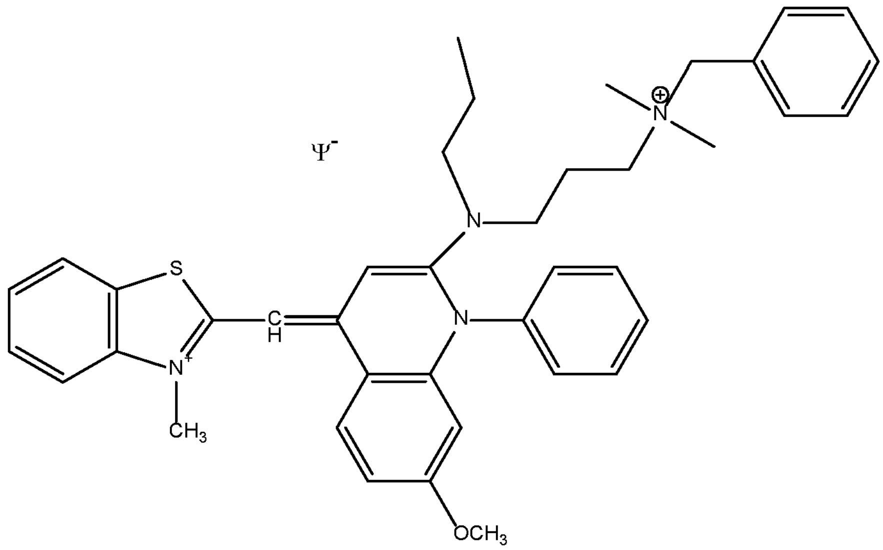

,

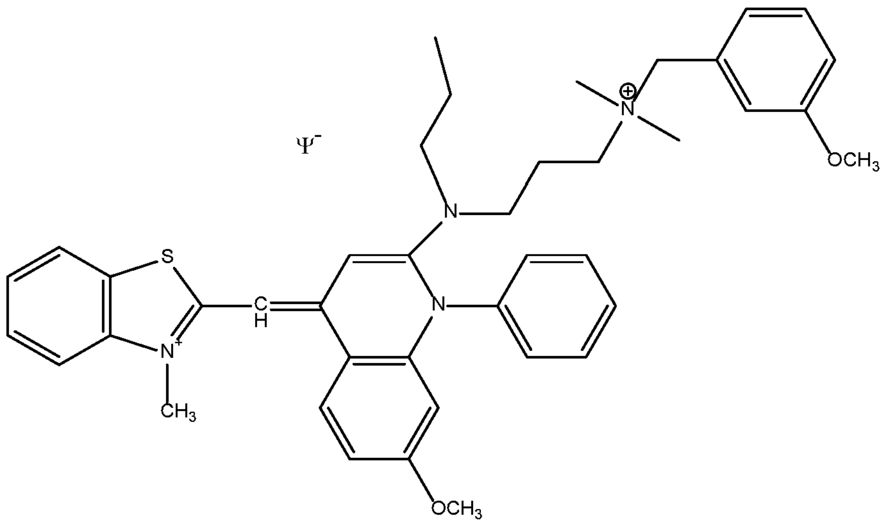

,

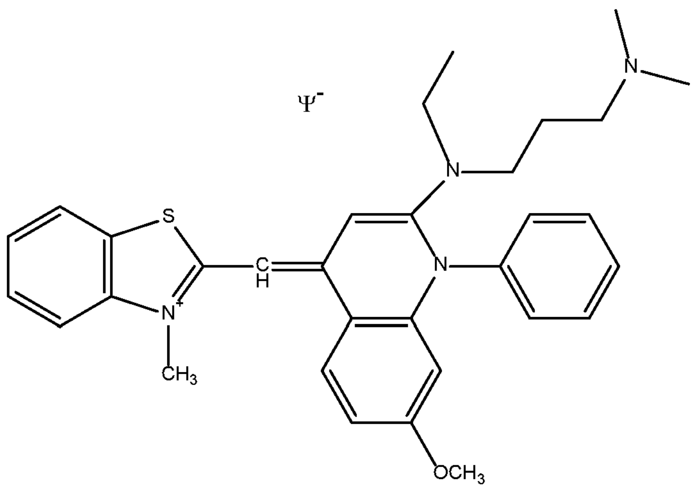

,

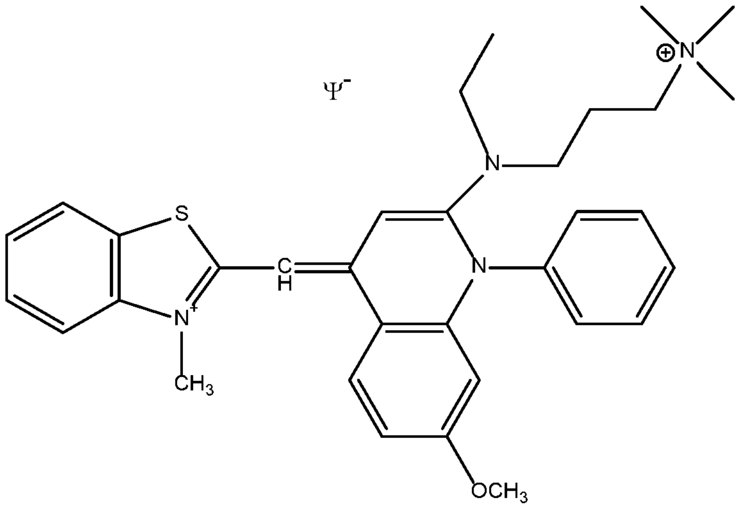

,

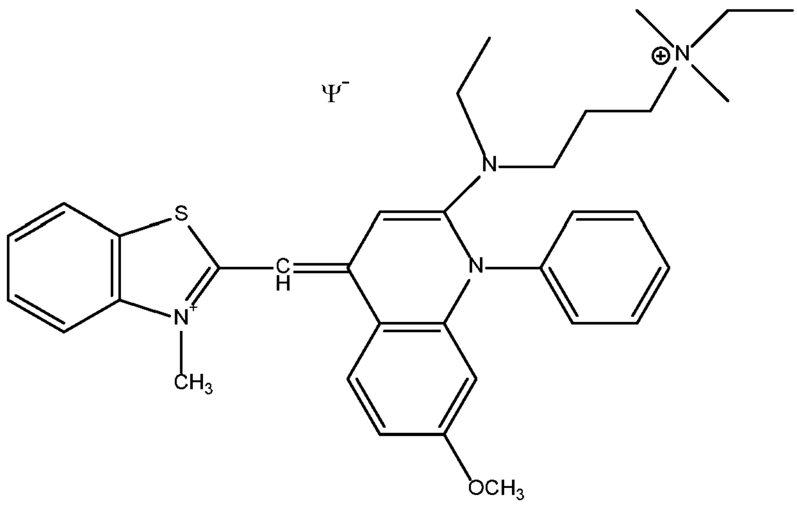

,

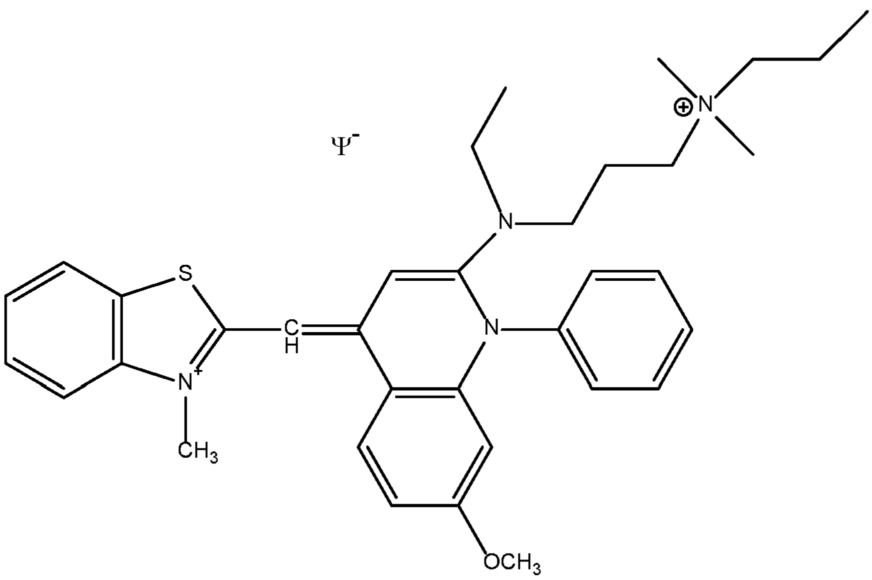

,

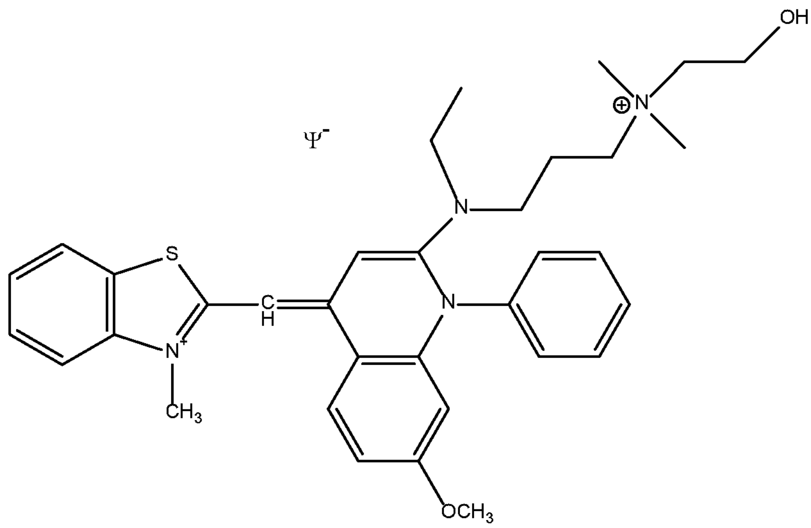

,

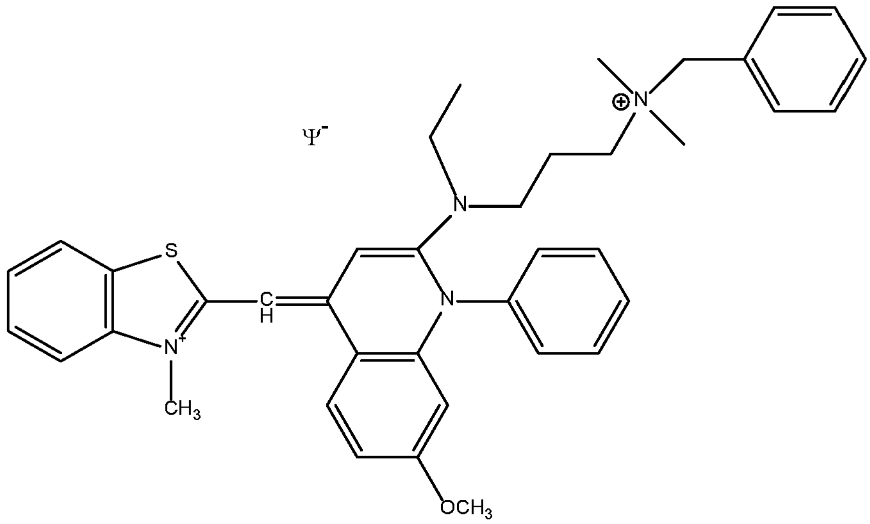

,

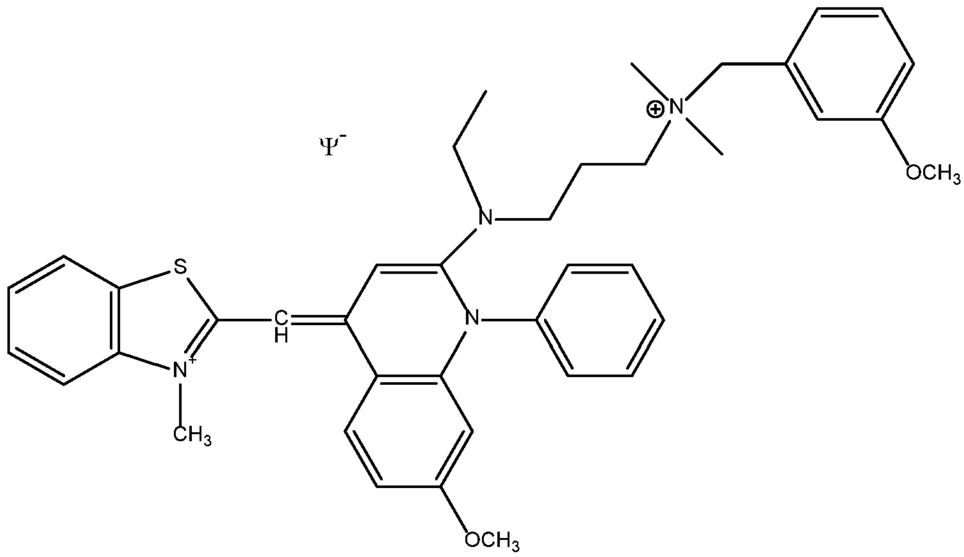

,

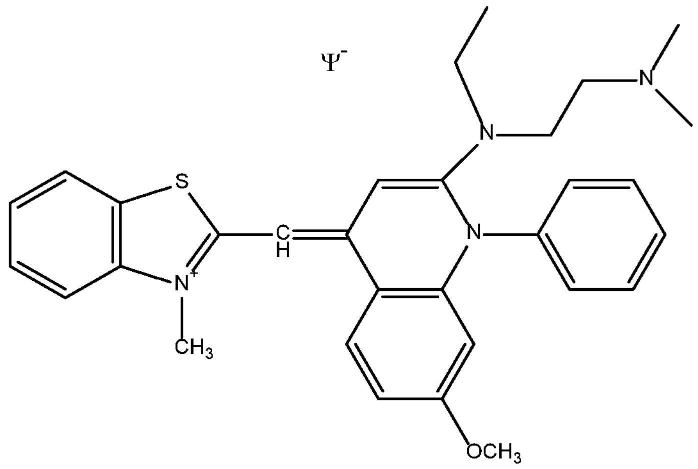

,

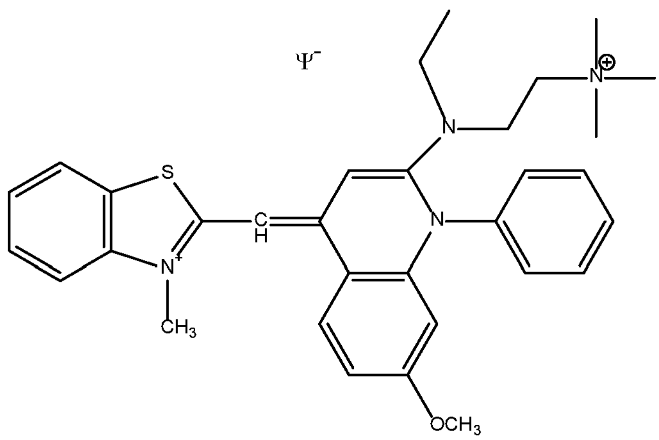

,

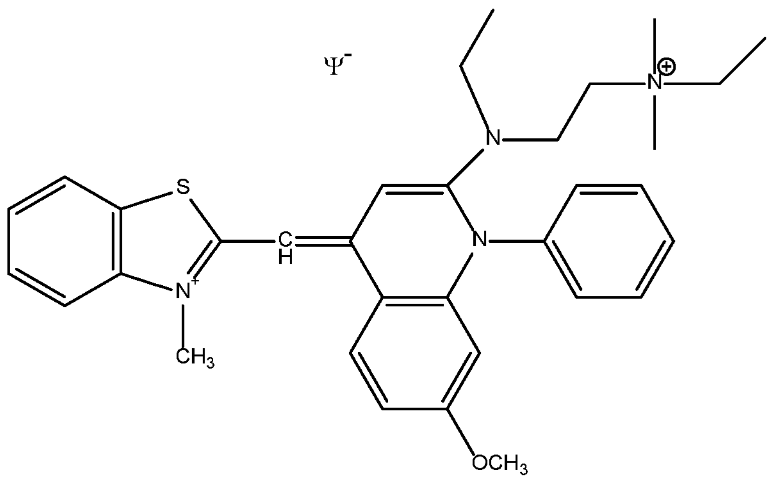

,

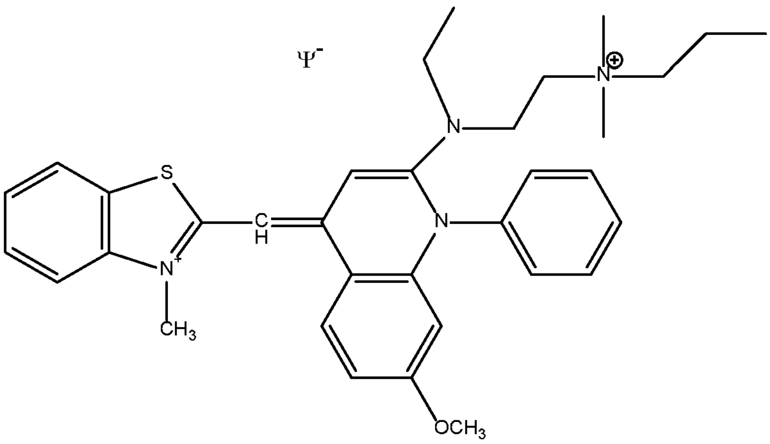

,

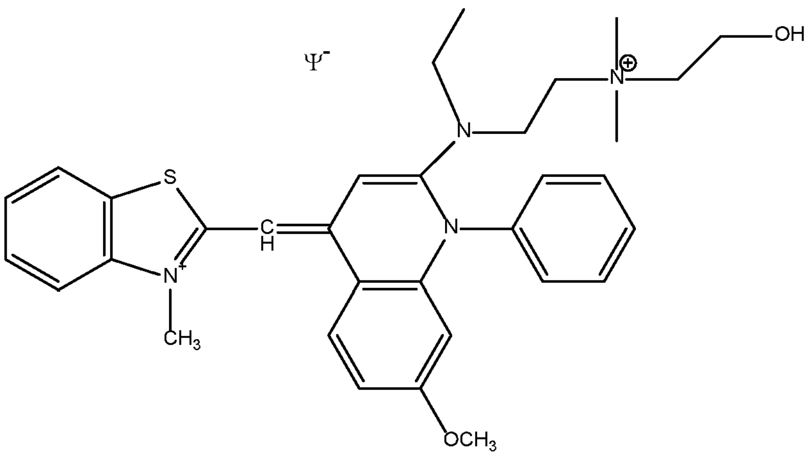

and

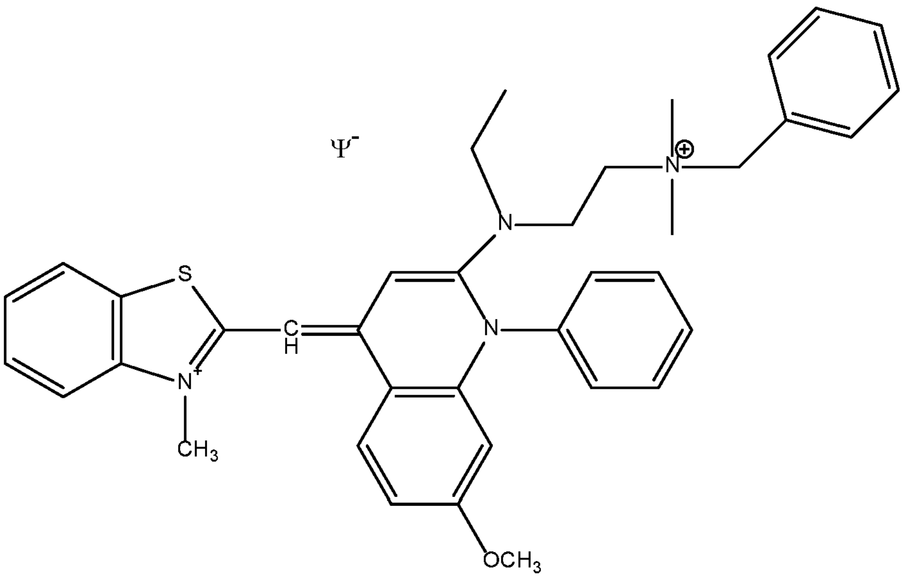

.

, wherein Ψ is a biologically compatible counterion and wherein the second nucleic acid binding dye is selected from:

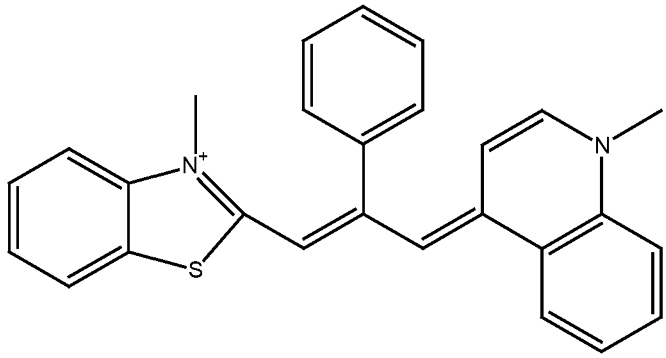

,

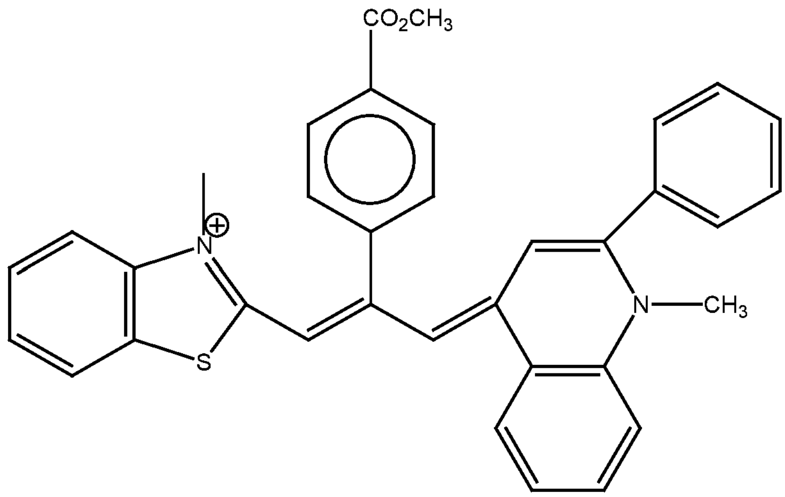

,

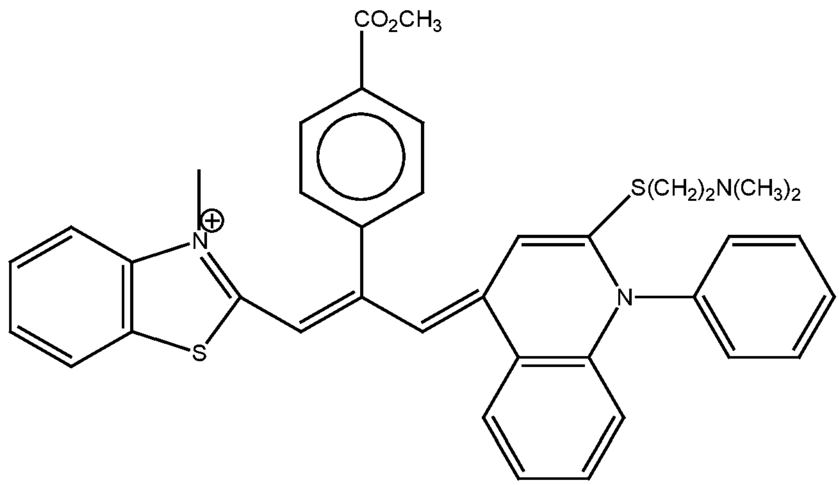

,

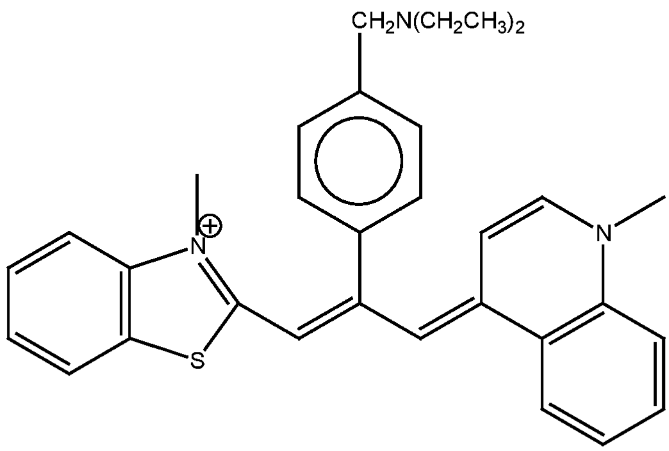

,

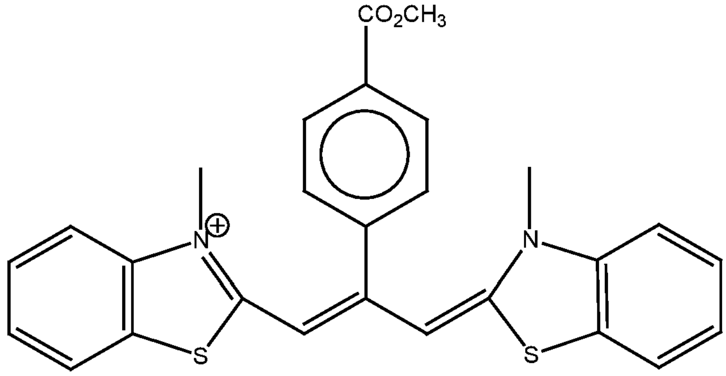

,

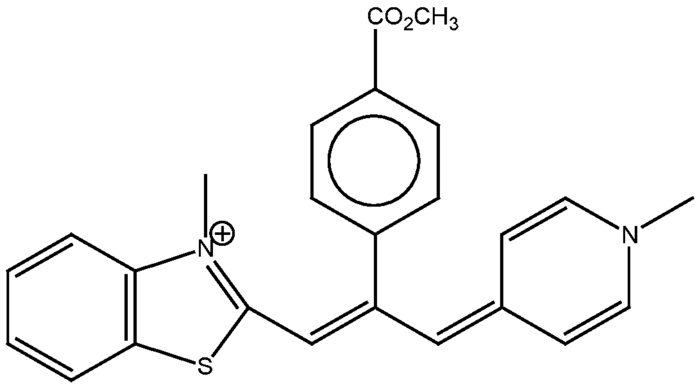

,

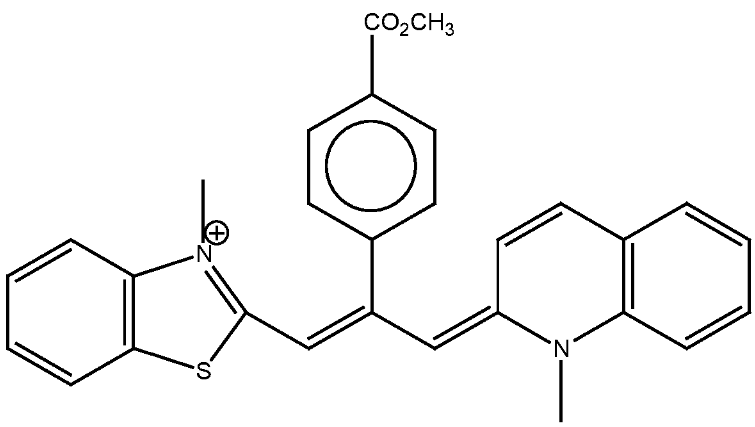

,

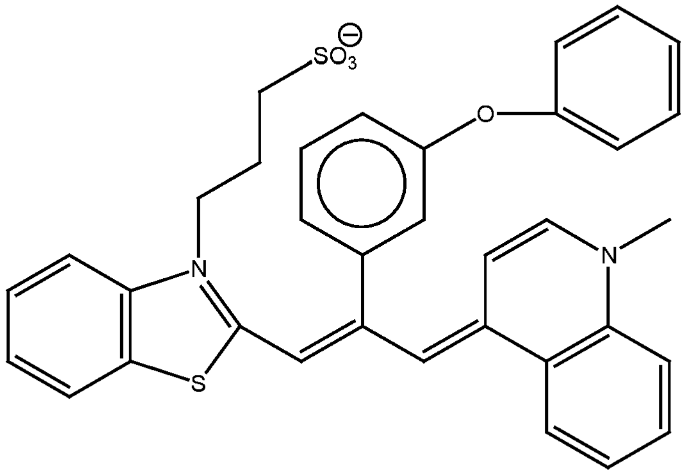

,

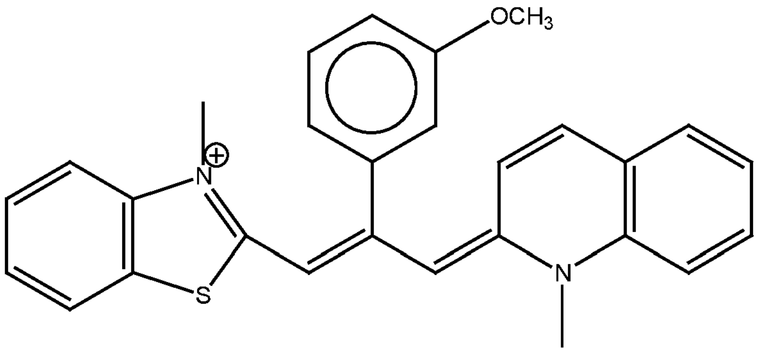

,

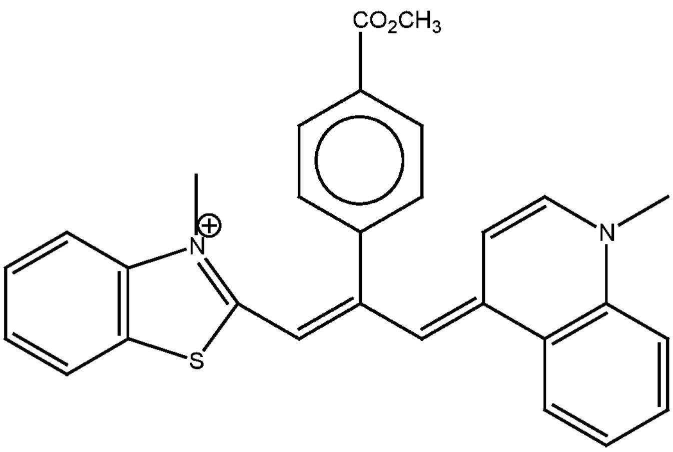

,

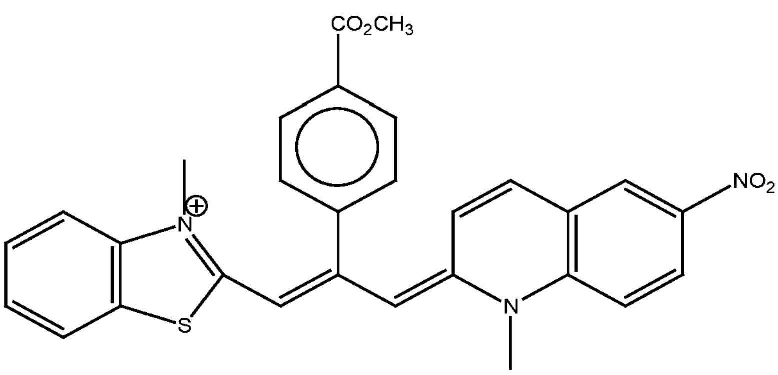

,

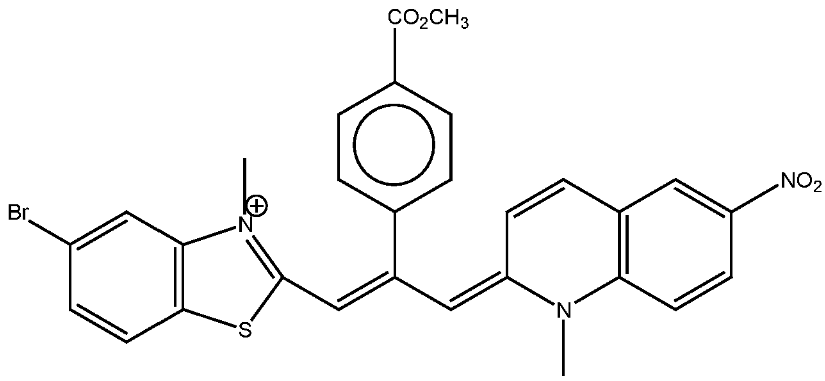

,

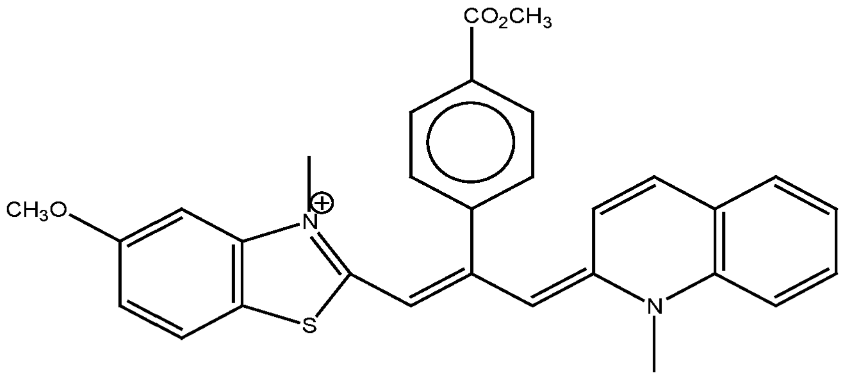

,

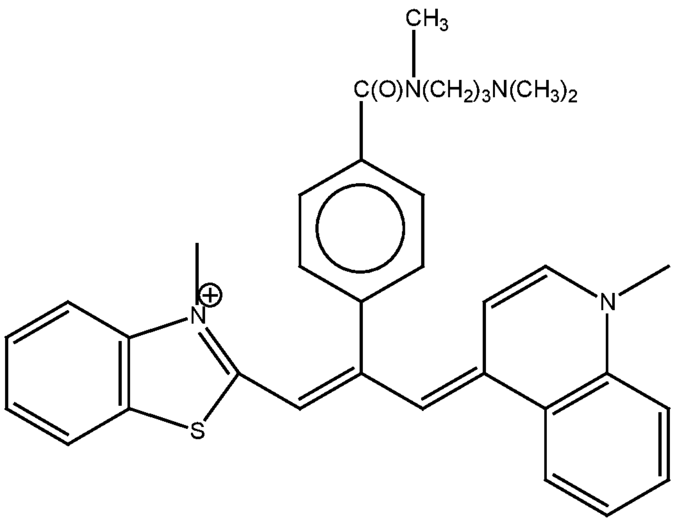

, ;

and

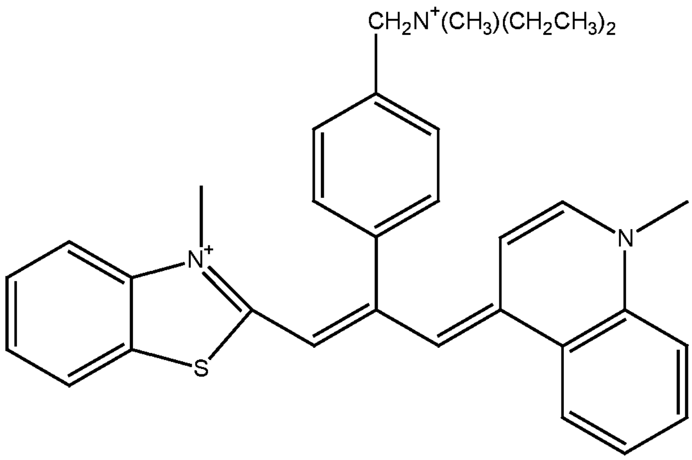

.

2. A method of determining the extent of degradation of RNA in a sample, expressed as an RNA quality value, the method comprising:

a) combining a sample suspected of containing RNA with a first nucleic acid binding dye that selectively binds small RNA comprising 200 nucleotides or less and a second nucleic acid binding dye that selectively binds intact RNA with secondary and/or tertiary structure, wherein the first nucleic acid binding dye and the second nucleic acid binding dye are detectably distinct;
b) incubating the sample for a time period sufficient to allow the first nucleic acid binding dye and the second nucleic acid binding dye to combine with the RNA in the sample to form a first nucleic acid binding dye-RNA complex and a second nucleic acid binding dye-RNA complex;
c) illuminating the first nucleic acid binding dye-RNA complex and the second nucleic acid binding dye-RNA complex with an appropriate wavelength of light to form an illuminated sample mixture, wherein the first nucleic acid binding dye-RNA complex emits a first fluorescent signal and the second nucleic acid binding dye-RNA complex emits a second fluorescent signal;
d) detecting the first fluorescent signal and the second fluorescent signal; and
e) calculating a relationship of the intensity of the first fluorescent signal to the intensity of the second fluorescent signal, thereby obtaining the RNA quality value, wherein the RNA quality value represents the extent of degradation of RNA in the sample,

wherein the first and second nucleic acid binding dyes are as defined in claim 1.

3. The method of **claim 1 or 2**, wherein the intact RNA with secondary and/or tertiary structure is rRNA.

4. The method of **claim 1 or 2**, wherein the first nucleic acid binding dye and the second nucleic acid binding dye are added to the sample simultaneously or wherein the first nucleic acid binding dye and the second nucleic acid binding dye are added to the sample sequentially.

5. The method of c**laim 1 or 2**, wherein the intact RNA with secondary and/or tertiary structure is tRNA

6. The method of c**laim 1 or 2**, wherein the intact RNA with secondary and/or tertiary structure is mRNA.

7. The method of **any of the preceding claims**, wherein the second nucleic acid binding dye has a RNA/DNA fluorescence enhancement ratio of ≥ 7, ≥ 10 or ≥ 20.

8. The method of **any of the preceding claims**, wherein the first nucleic acid binding dye has a single stranded RNA fluorescence enhancement of ≥ 30%.

9. The method of **any of the preceding claims**, wherein the detecting step is performed by fluorometry.

10. The method of **claim 1**, further comprising

preparing:

a first standard solution by combining the first nucleic acid binding dye and the second nucleic acid binding dye with a buffer blank;
a second standard solution by combining the first nucleic acid binding dye and the second nucleic acid binding dye with a small RNA standard; and
a third standard solution by combining the first nucleic acid binding dye and the second nucleic acid binding dye with an intact RNA with secondary and/or tertiary structure standard;

incubating the first standard solution, the second standard solution and the third standard solution for a time sufficient for the first nucleic acid binding dye and the second nucleic acid binding dye to combine with RNA in the standard, if present, to form a first standard-dye complex, a second standard-dye complex and a third standard-dye complex;
illuminating the first standard-dye complex, the second standard-dye complex and the third standard-dye complex with an appropriate wavelength of light to form a first illuminated standard mixture, a second illuminated standard mixture and a third illuminated standard mixture; and
detecting a fluorescent signal from each of the first illuminated standard mixture, the second illuminated standard mixture and the third illuminated standard mixture, and optionally further comprising determining the extent of degradation of the RNA present in the illuminated sample mixture based on comparison of the detectable fluorescent signal in the illuminated sample mixture with a ratio of the fluorescent signal of the second standard solution and the fluorescent signal of the third standard solution.

**11.** A kit comprising:

a dye solution comprising a first nucleic acid binding dye that selectively binds small RNA comprising 200 nucleotides or less and a second nucleic acid binding dye that selectively binds intact RNA with secondary and/or tertiary structure as defined in claim 1; and
instructions for use according to the methods of **any of the preceding claims**
wherein the first nucleic acid binding dye and the second nucleic acid binding dye are detectably distinct and optionally further comprising:

a first standard solution comprising a buffer and no nucleic acid;
a second standard solution comprising a small RNA standard, wherein the small RNA standard consists of fewer than 200 nt; and
a third standard solution comprising an intact RNA with secondary and/or tertiary structure standard.

**12.** A RNA quality staining solution comprising:

a first nucleic acid binding dye that preferentially binds small RNA comprising 200 nucleotides or less;
a second nucleic acid binding dye that preferentially binds intact RNA with secondary and/or tertiary structure;
a buffer; and
an organic solvent,
wherein the first nucleic acid binding dye and the second nucleic acid binding dye are detectably distinct, and wherein the first nucleic acid binding dye is

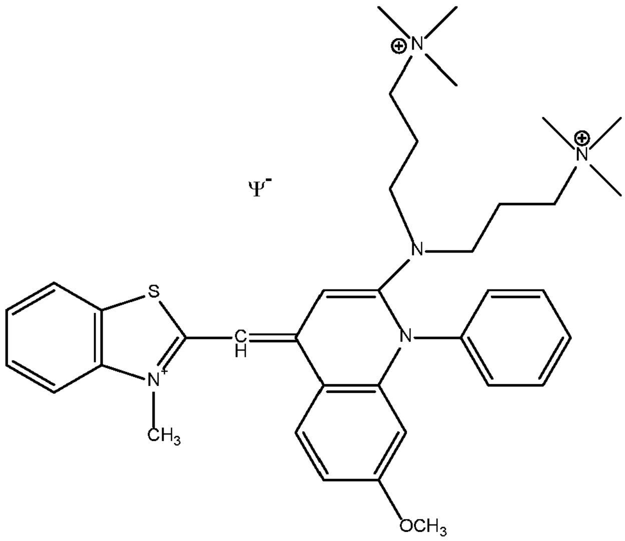

Compound **16**,

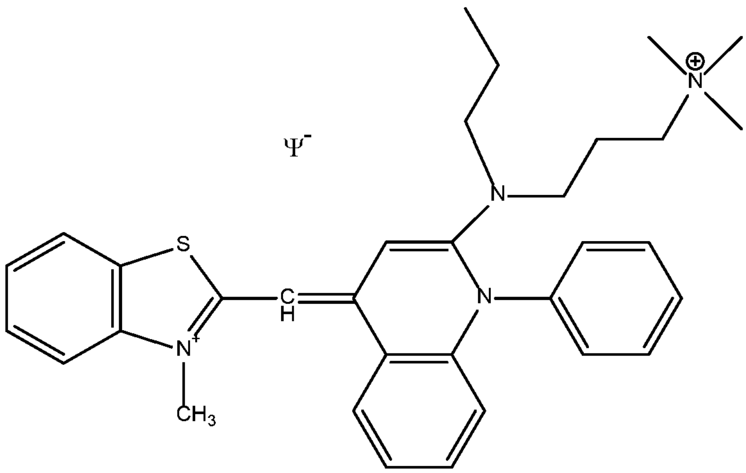

Compound **17**,

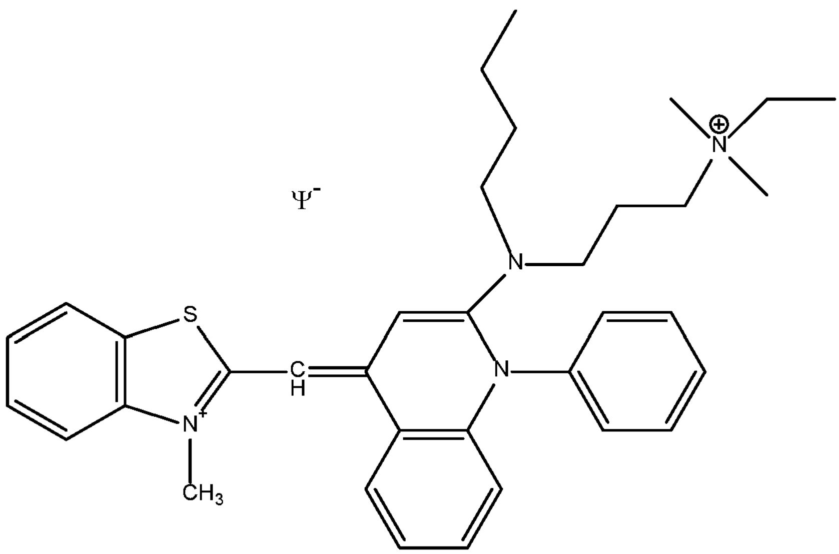

Compound **23**,

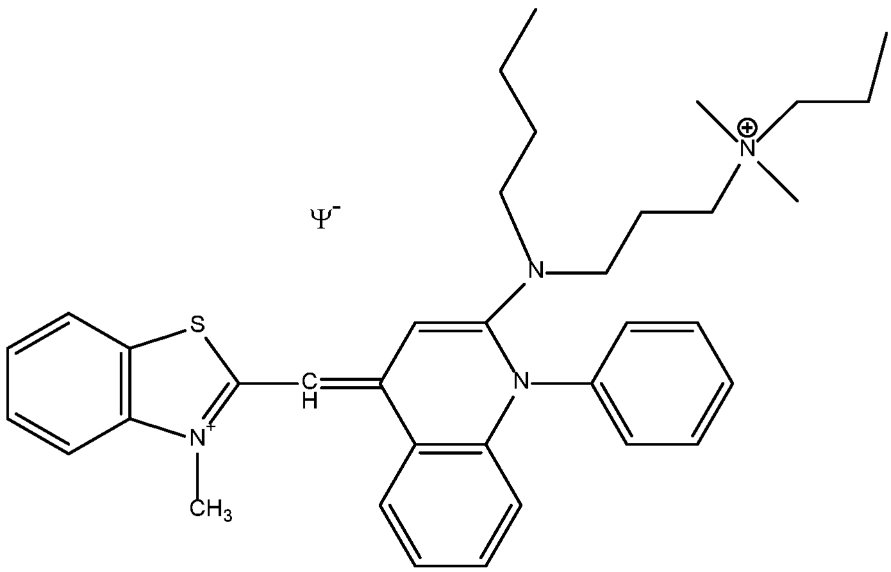

Compound **24**,

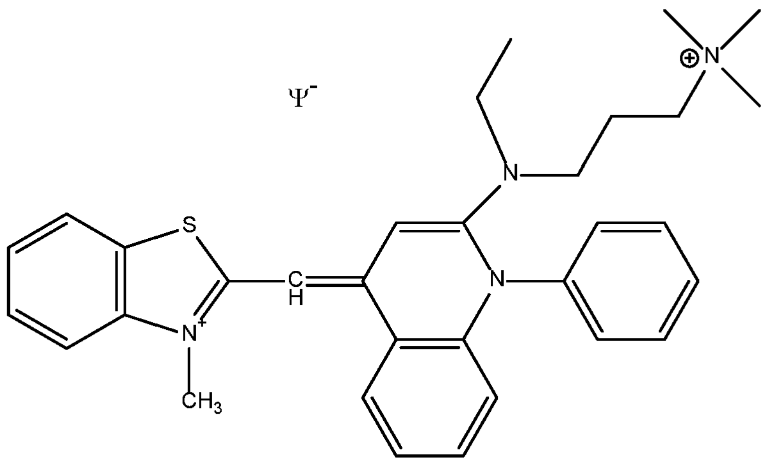

Compound **29**,

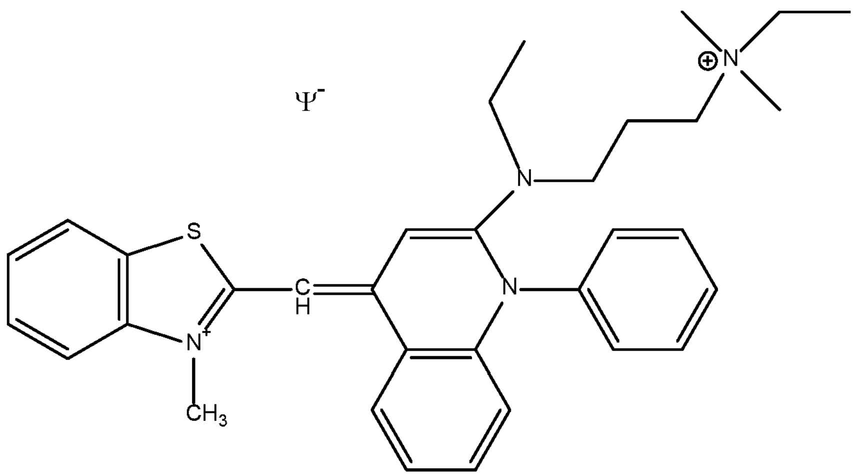

Compound **30**,

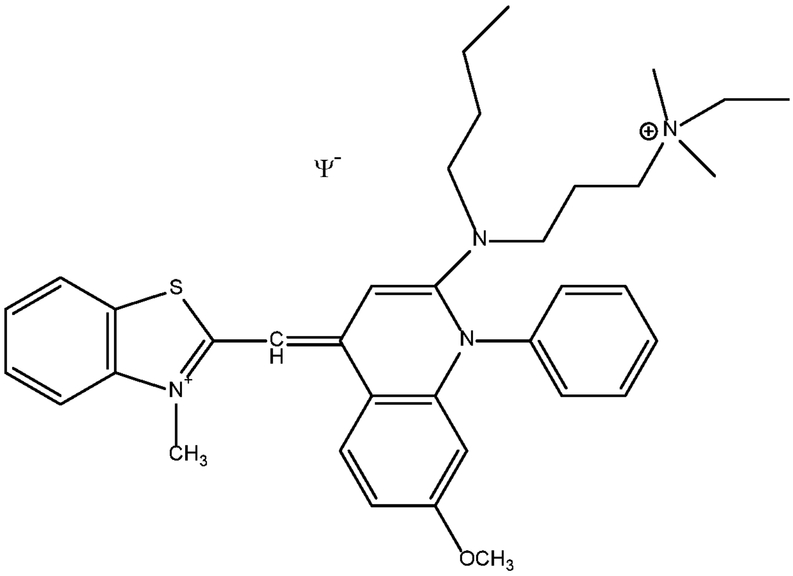

Compound **45**,

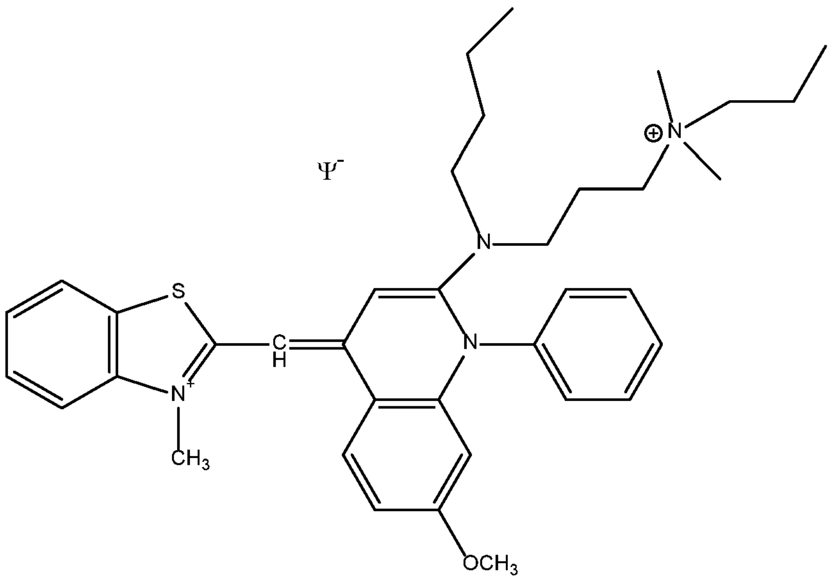

Compound **46**,

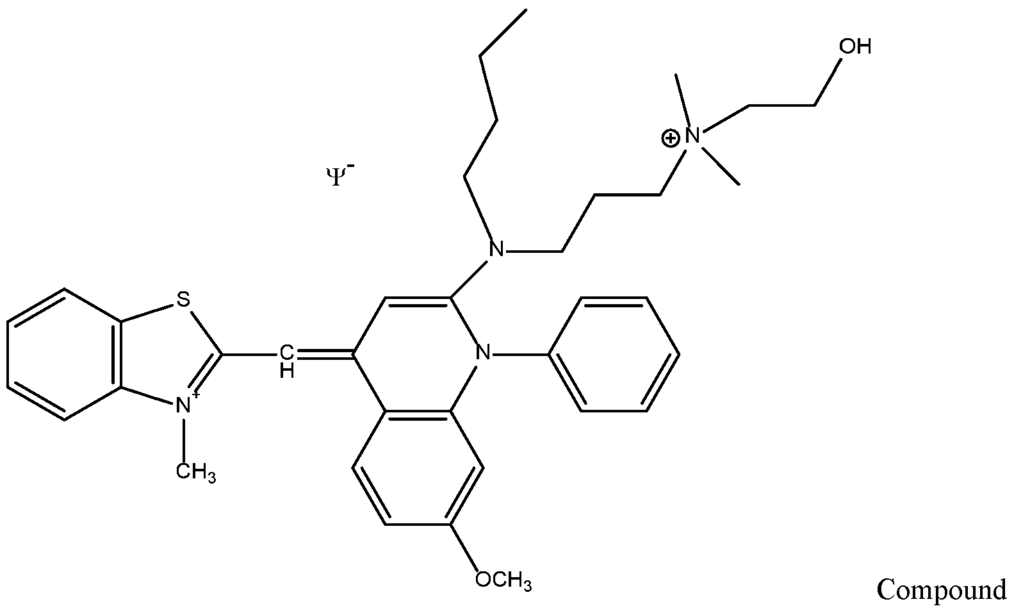

Compound **47**,

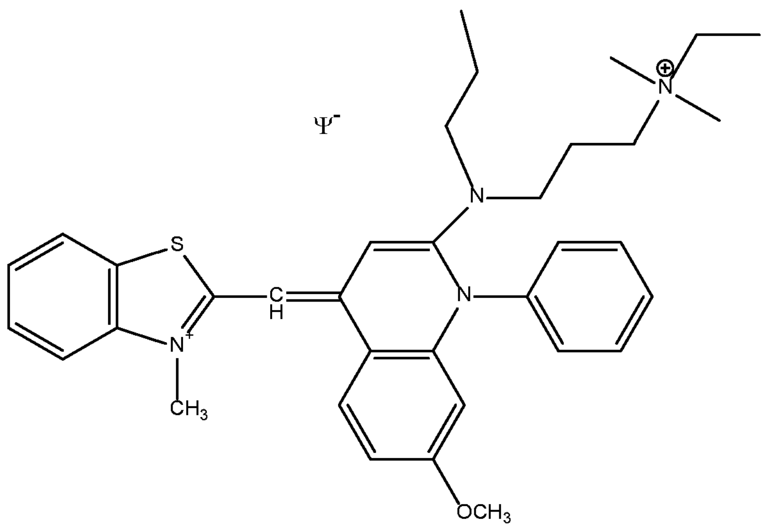

Compound **52**,

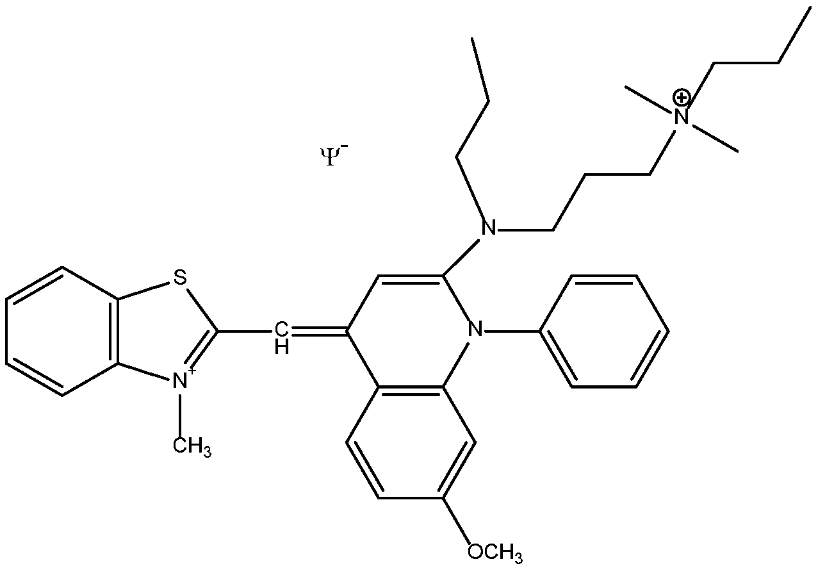

Compound **53**,

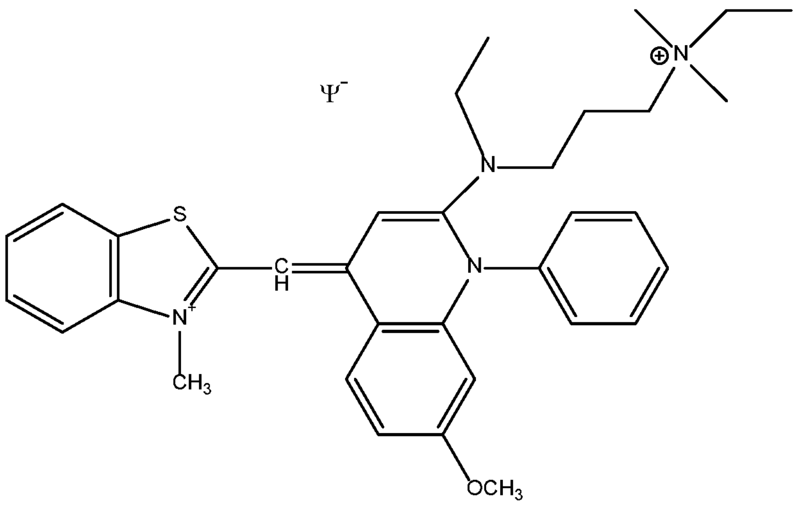

Compound **59**,

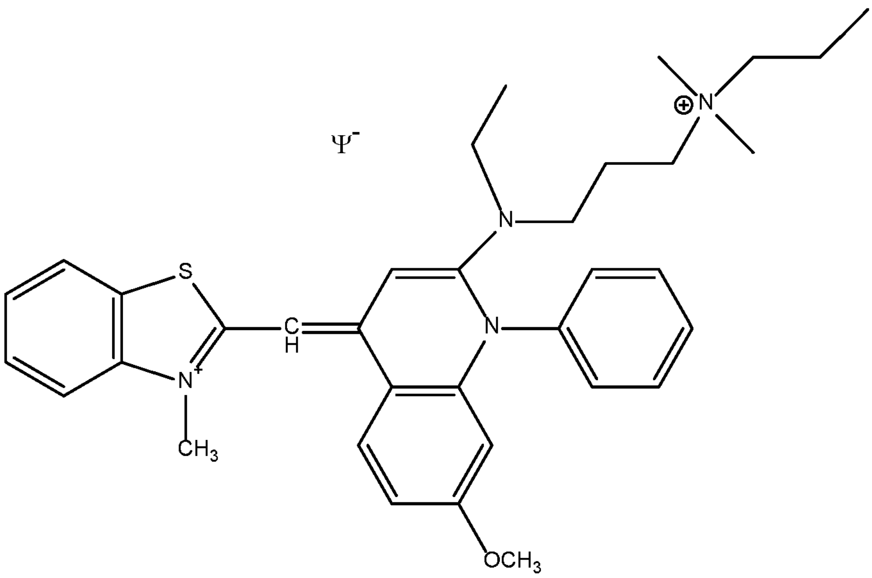

Compound **60** or

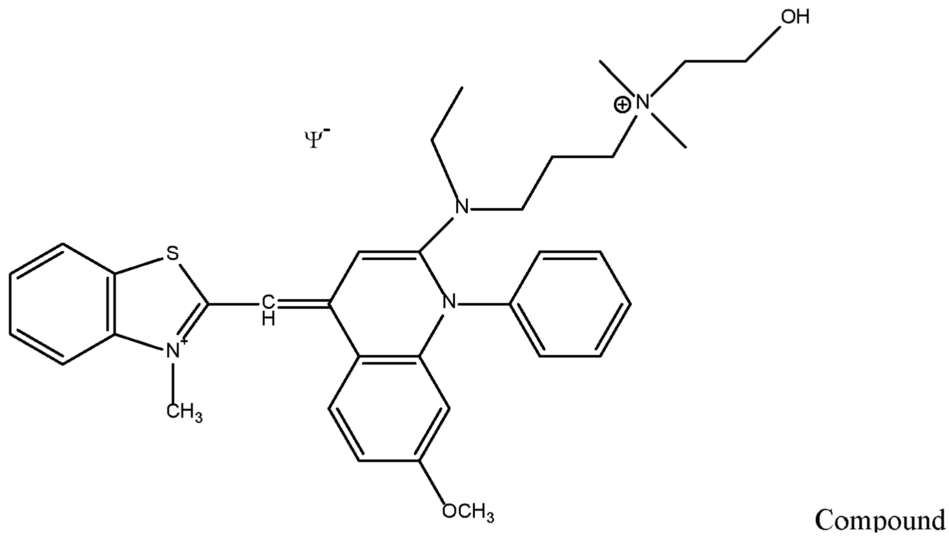

Compound **61**.

wherein Ψ is a biologically compatible counterion; and the second nucleic acid binding dye is

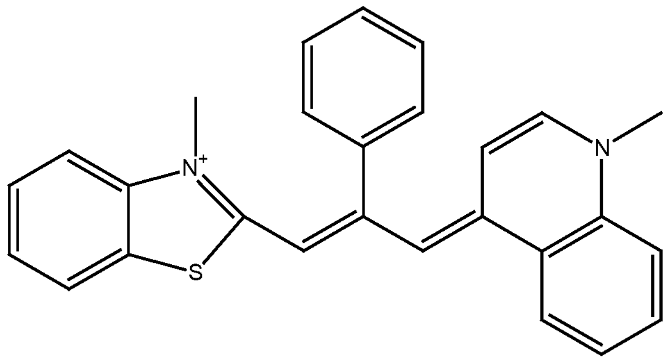

Compound **1**,

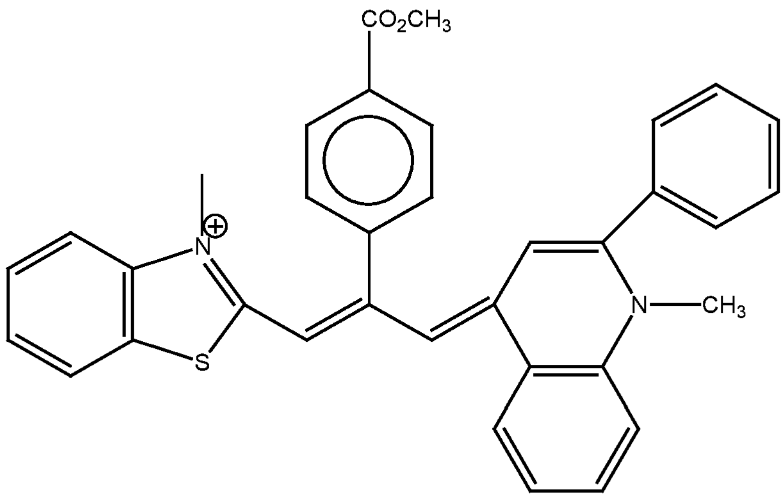

Compound **2**,

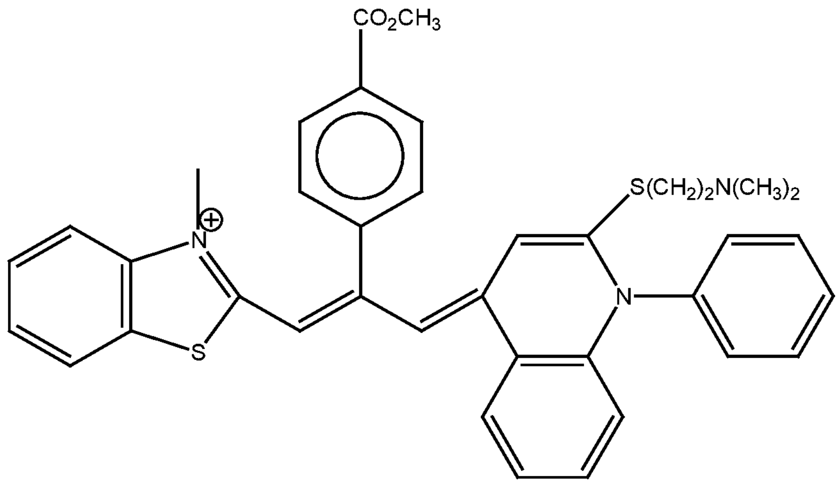

Compound **3**,

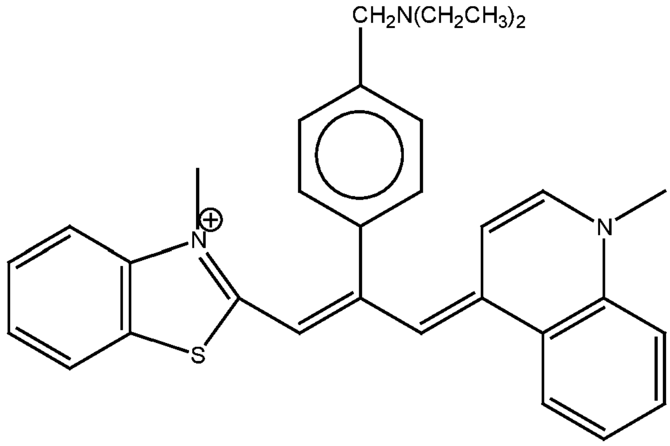

Compound **4**,

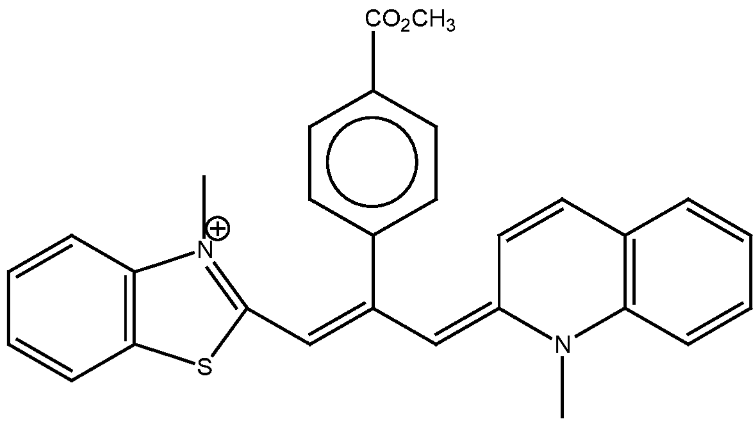

Compound **7**,

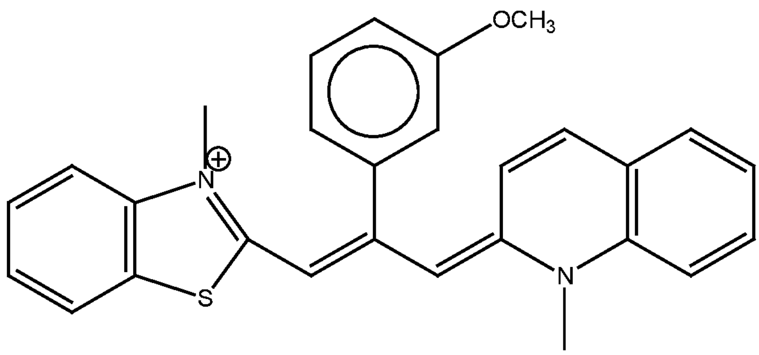

Compound **9**,

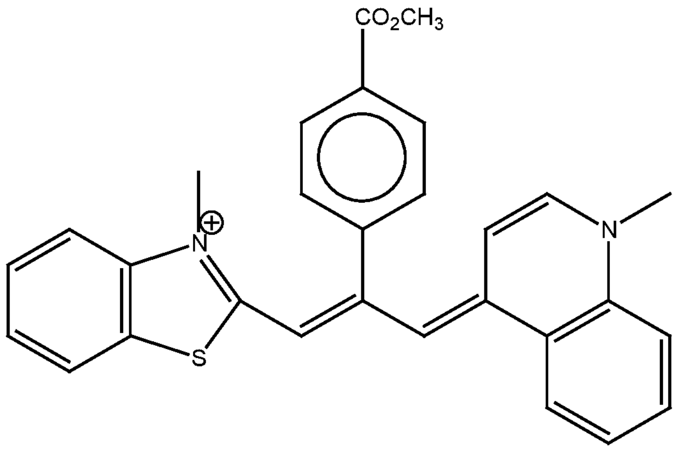

Compound **10**,

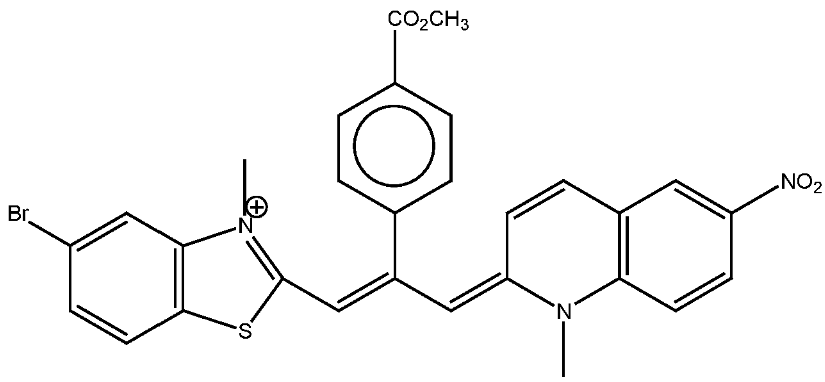

Compound **12**,

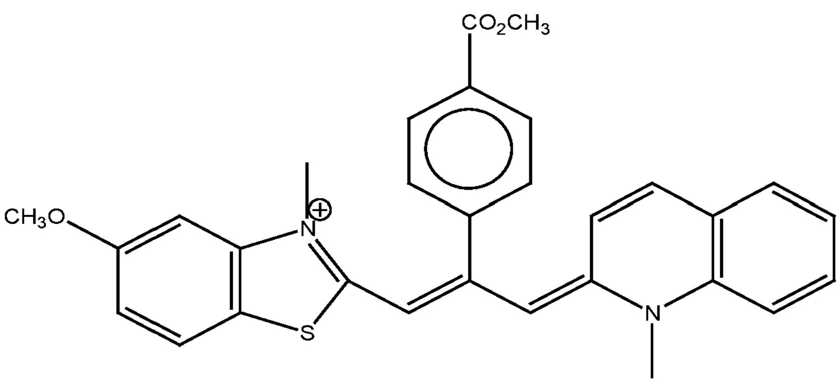

Compound **13**,

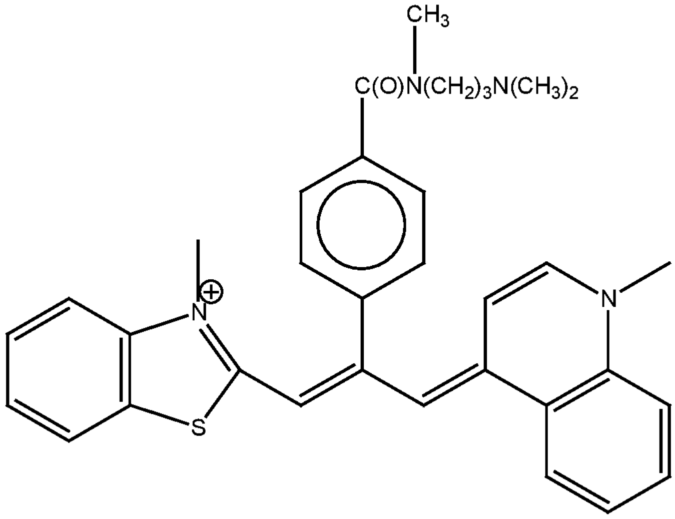

Compound **14** or

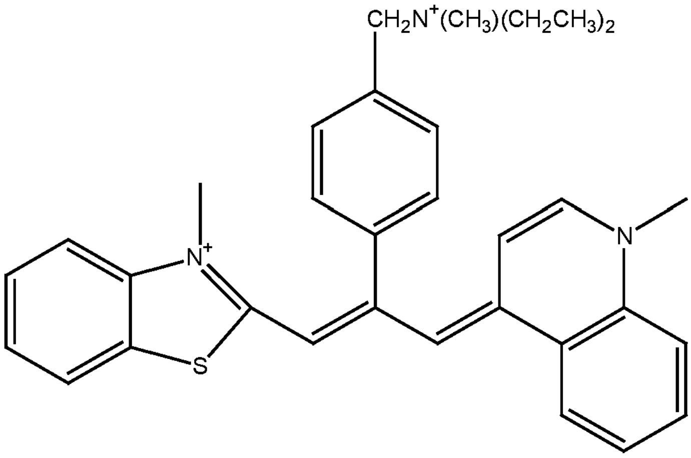

Compound **15**.

## Patentansprüche

1. Verfahren zum Bestimmen des Ausmaßes eines RNA-Abbaus in einer Probe, ausgedrückt als ein RNA-Qualitätswert, wobei das Verfahren Folgendes umfasst:

    a) Kombinieren einer Probe, die vermutlich RNA enthält, mit einem ersten nukleinsäurebindenden Farbstoff, der selektiv kleine RNA selektiv, umfassend 200 Nukleotide oder weniger, bindet und einem zweiten nukleinsäurebindenden Farbstoff, der selektiv intakte RNA mit sekundärer und/oder tertiärer Struktur selektiv bindet, wobei der erste nukleinsäurebindende Farbstoff und der zweite nukleinsäurebindende Farbstoff nachweisbar verschieden sind;
    b) Inkubieren der Probe für einen Zeitraum, der ausreicht, um dem ersten nukleinsäurebindenden Farbstoff und dem zweiten nukleinsäurebindenden Farbstoff zu ermöglichen, mit der RNA in der Probe zu kombinieren, um einen ersten nukleinsäurebindenden-Farbstoff-RNA-Komplex und einen zweiten nukleinsäurebindenden-Farbstoff-RNA-Komplex zu bilden;
    c) Beleuchten des ersten nukleinsäurebindenden-Farbstoff-RNA-Komplexes und des zweiten nukleinsäurebindenden-Farbstoff-RNA-Komplexes mit einer geeigneten Wellenlänge von Licht, um eine beleuchtete Probenmischung zu bilden, wobei der erste nukleinsäurebindende-Farbstoff-RNA-Komplex ein erstes Fluoreszenzsignal emittiert und der zweite nukleinsäurebindende-Farbstoff-RNA-Komplex ein zweites Fluoreszenzsignal emittiert;
    d) Nachweisen des ersten Fluoreszenzsignals und des zweiten Fluoreszenzsignals; und
    e) Berechnen eines Verhältnisses des ersten Fluoreszenzsignals zum zweiten Fluoreszenzsignal, wodurch der RNA-Qualitätswert erhalten wird, wobei der RNA-Qualitätswert das Ausmaß des RNA-Abbaus in der Probe darstellt,

wobei der erste nukleinsäurebindende Farbstoff ausgewählt ist aus

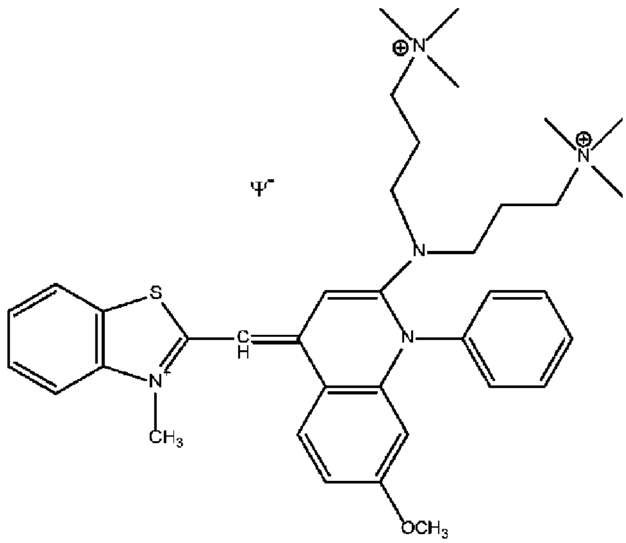

,

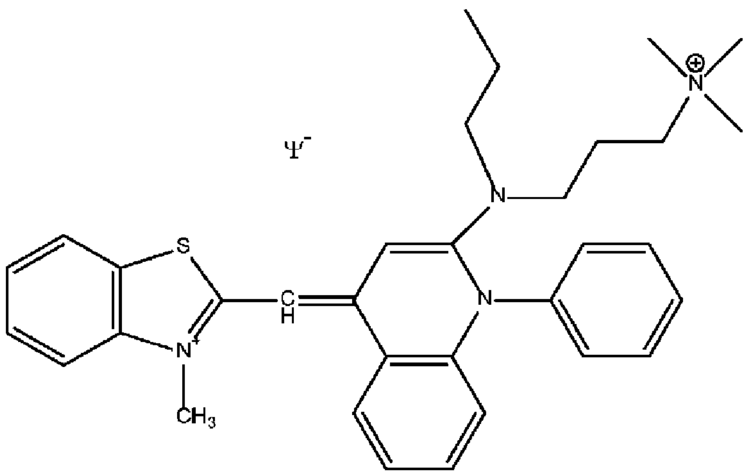

,

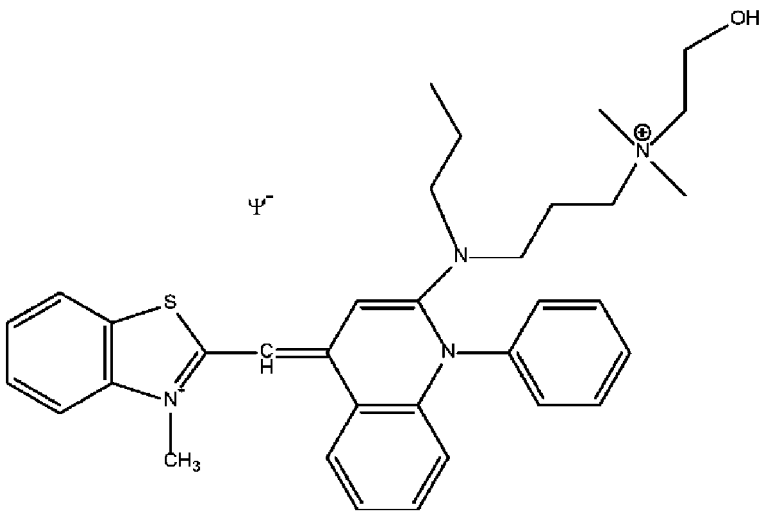

,

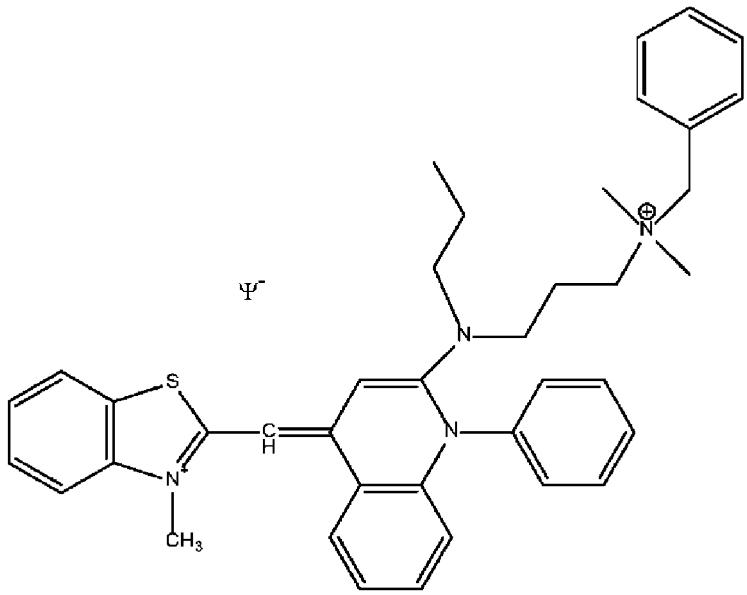

,

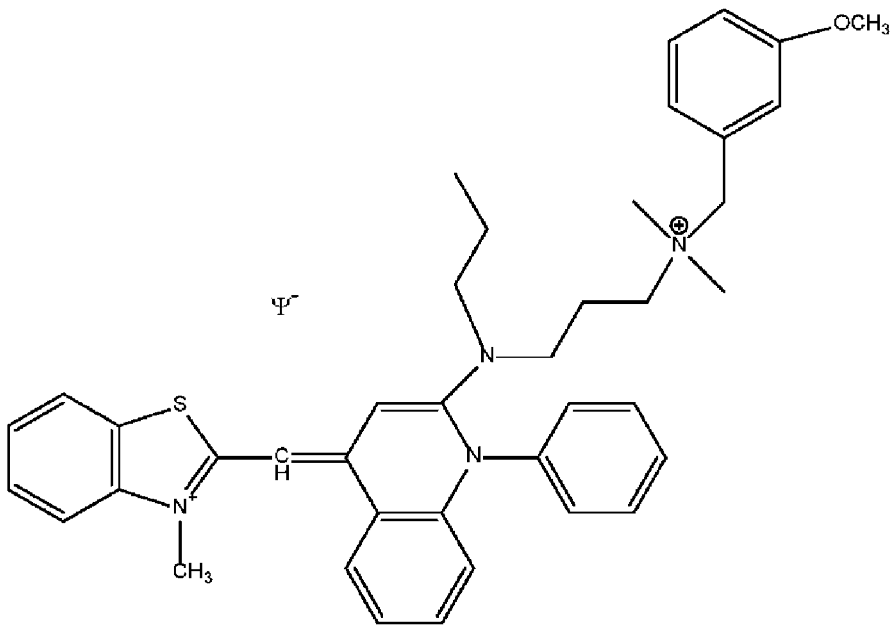

,

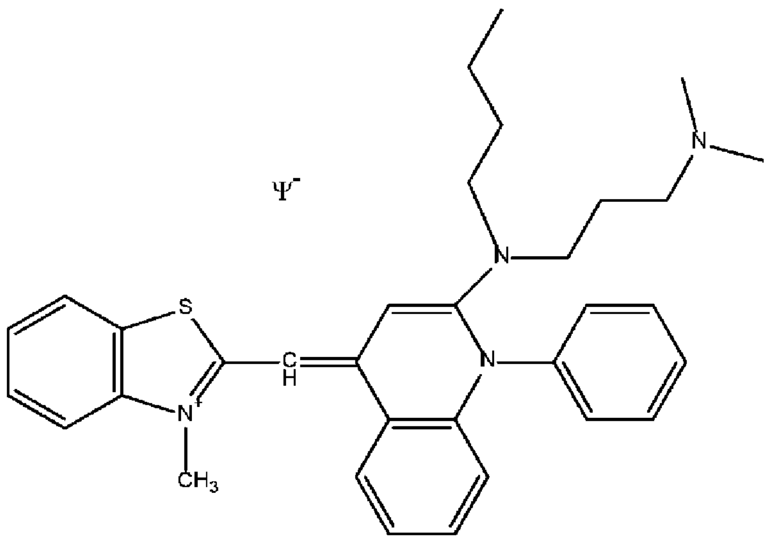

,

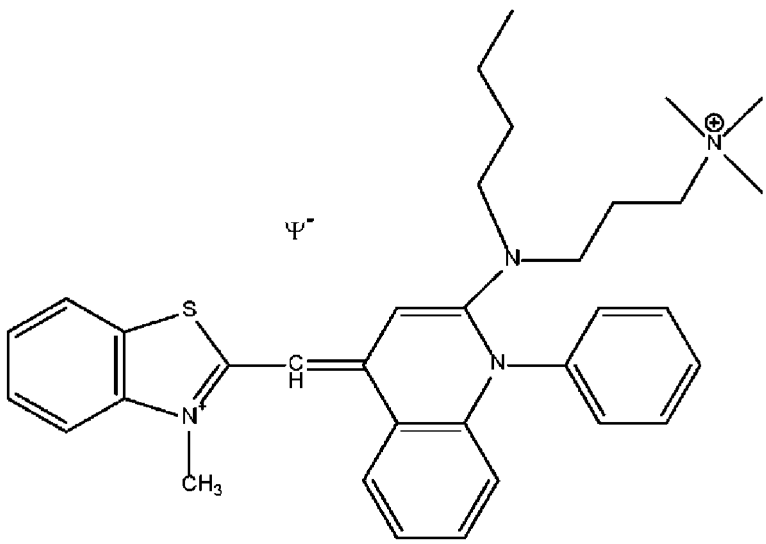

,

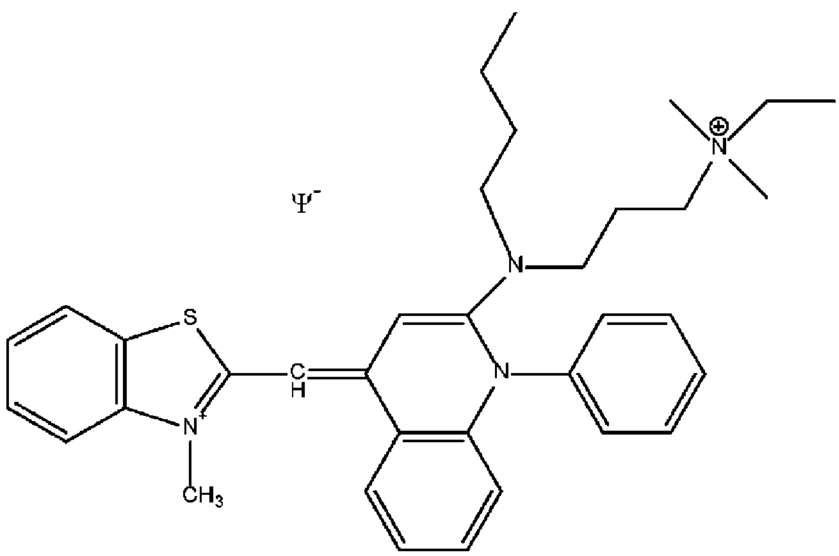

,

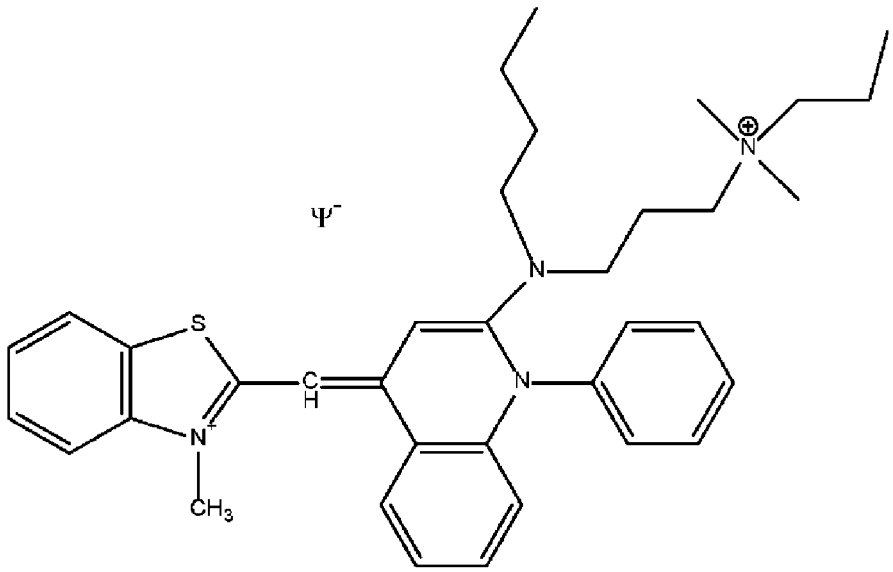

,

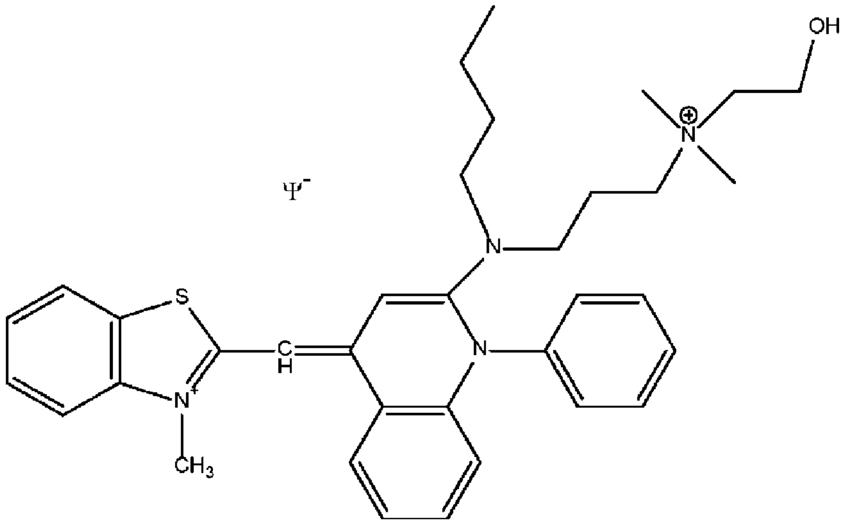

,

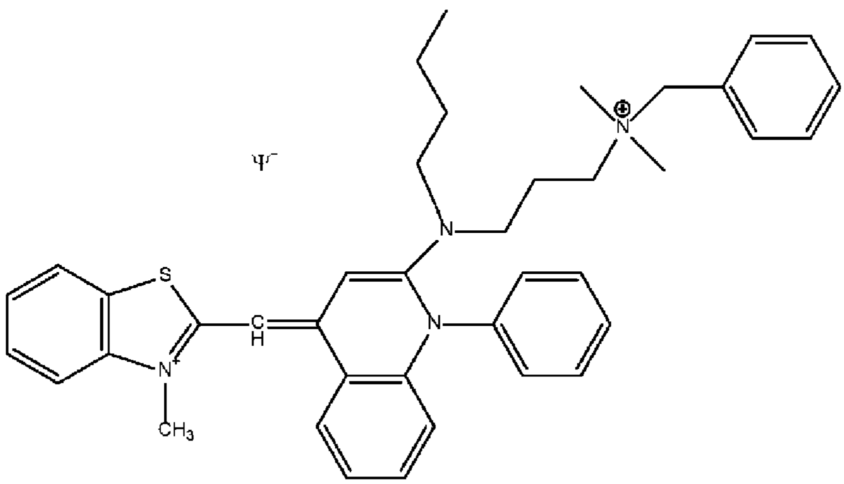

,

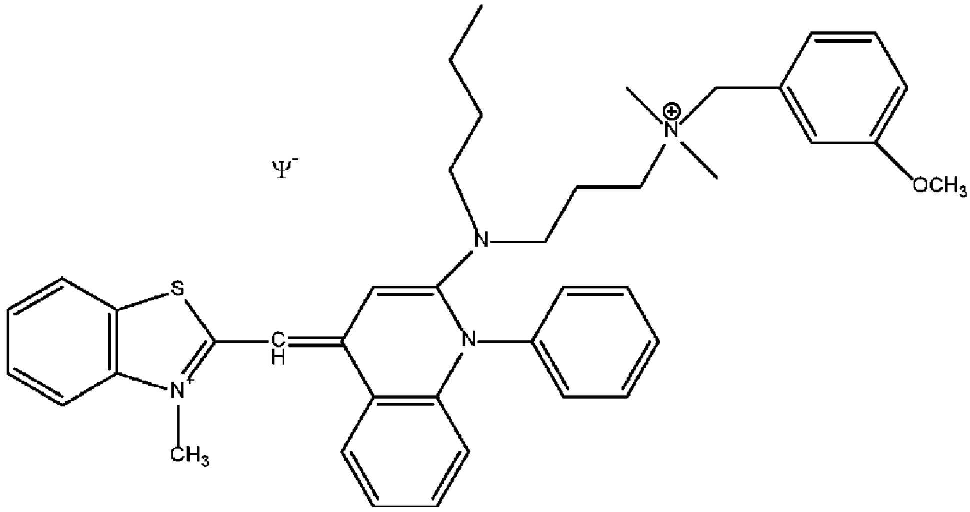

,

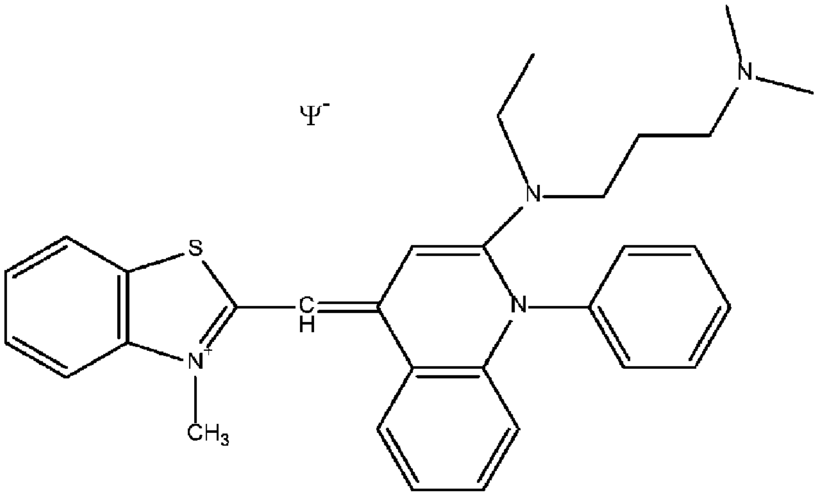

,

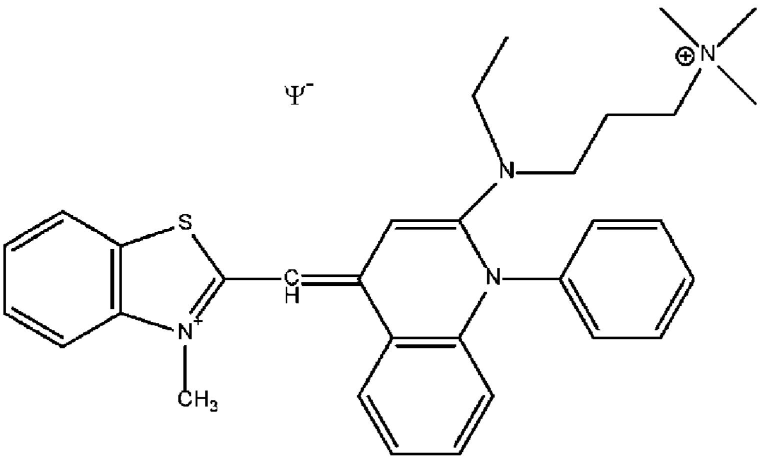

,

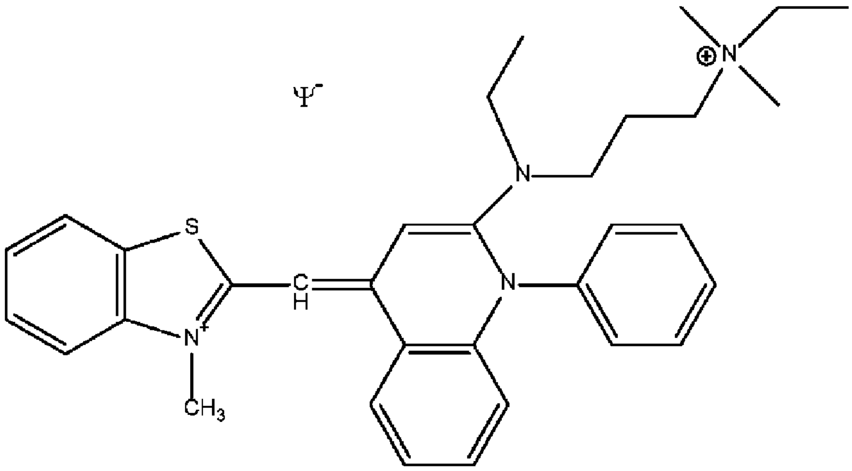

,

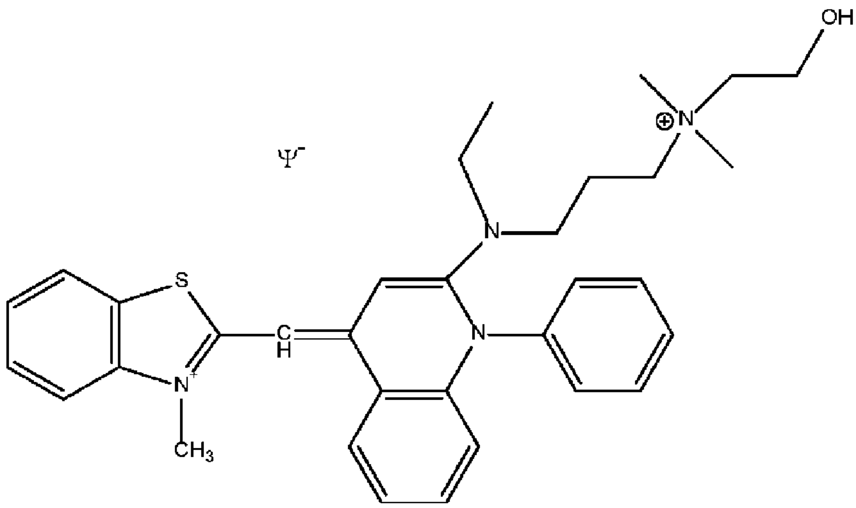

,

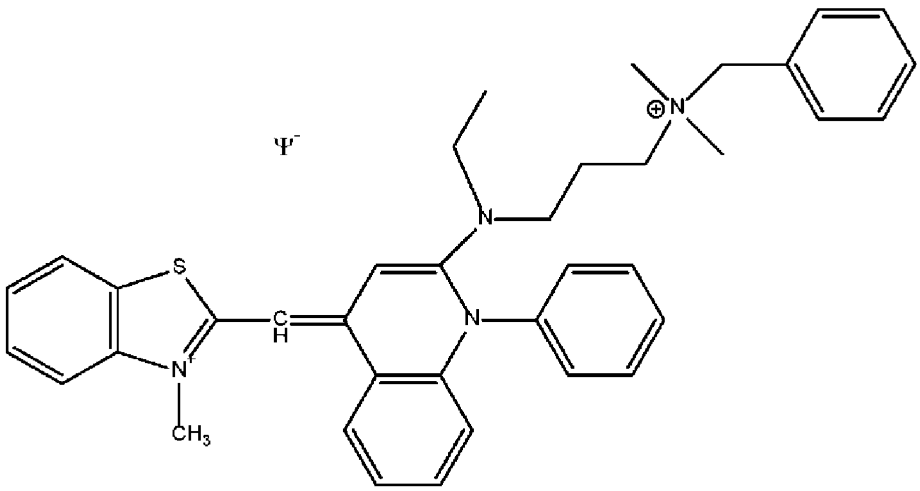

,

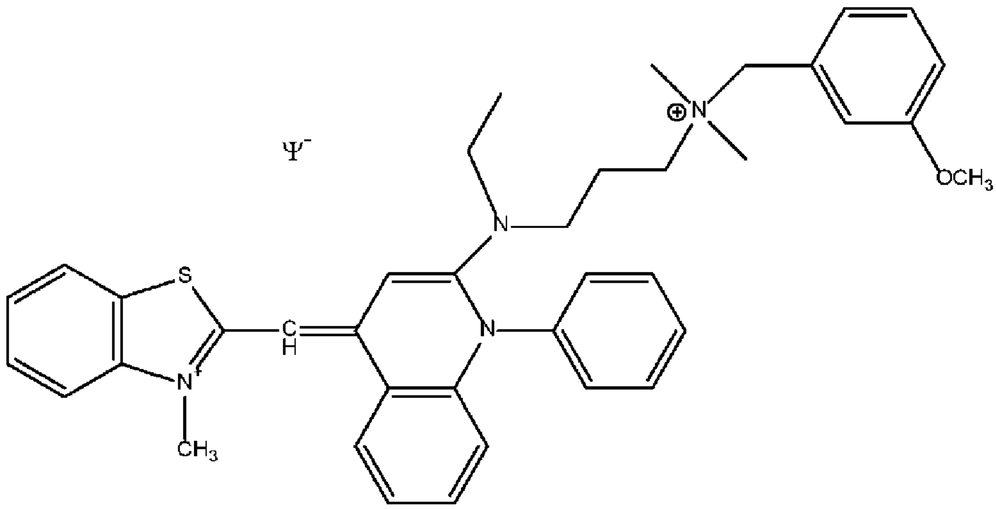

,

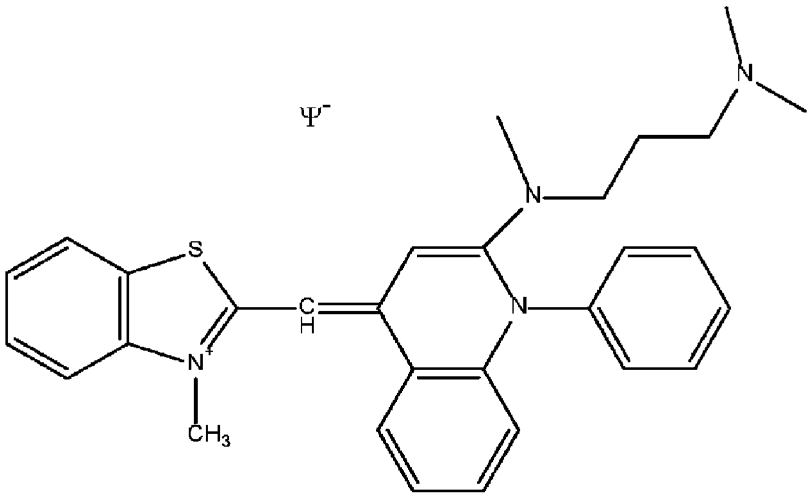

,

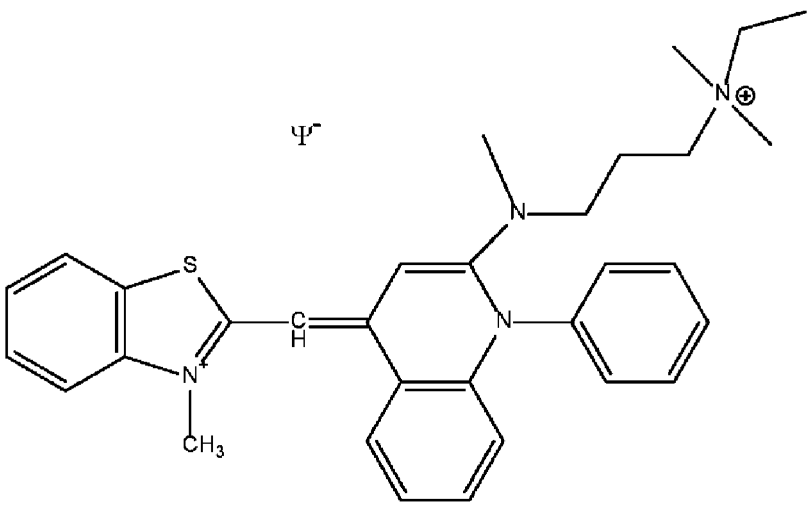

,

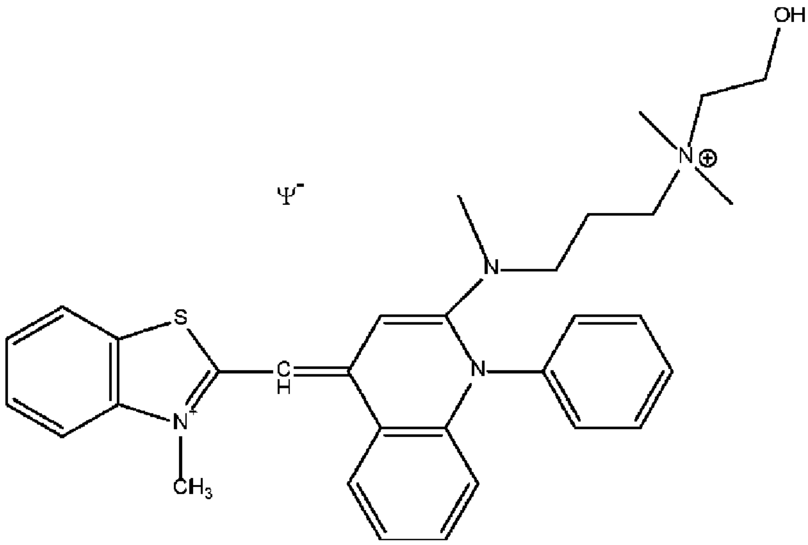

,

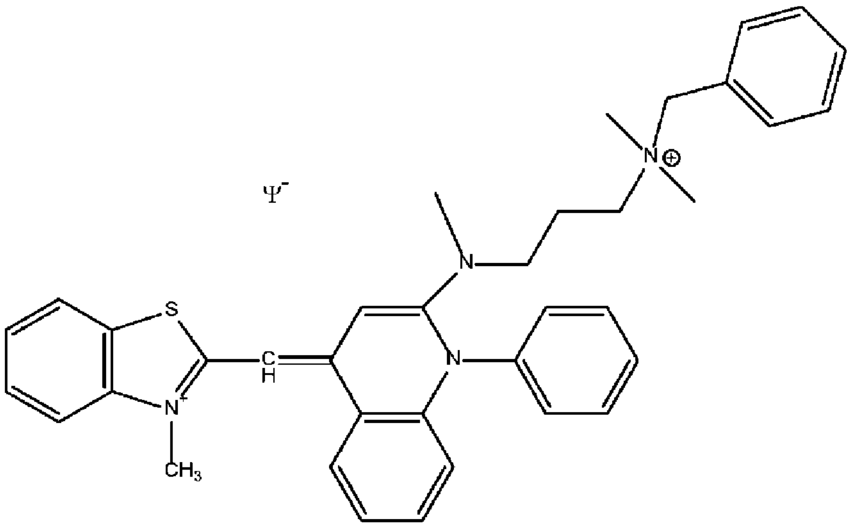

,

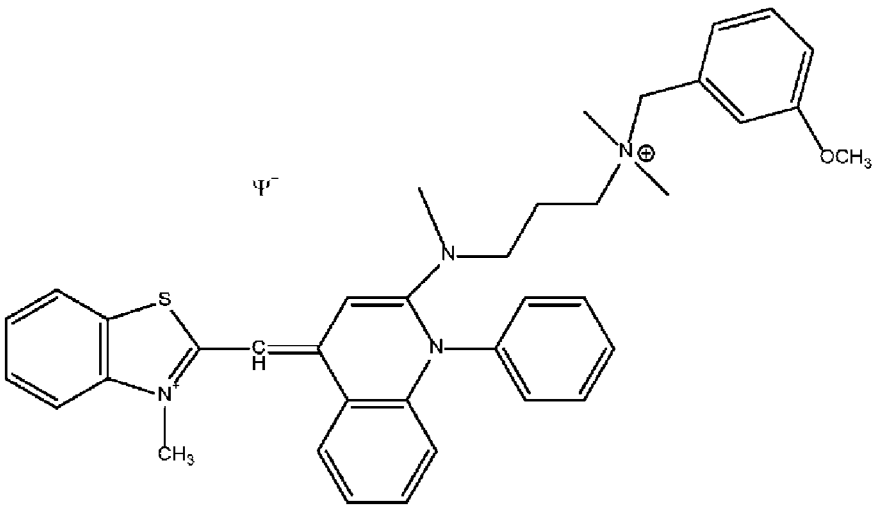

,

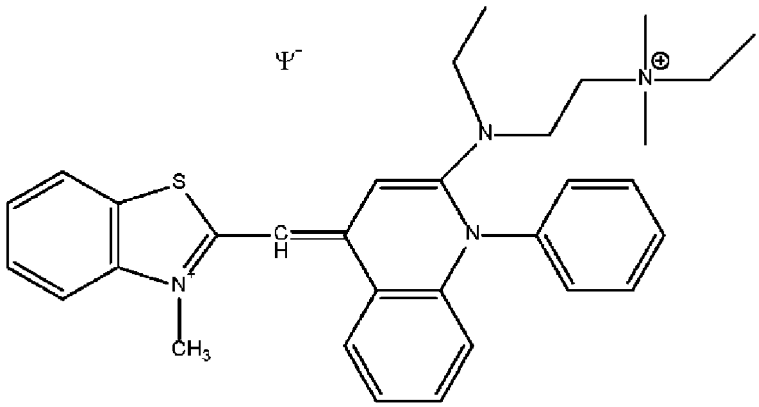

,

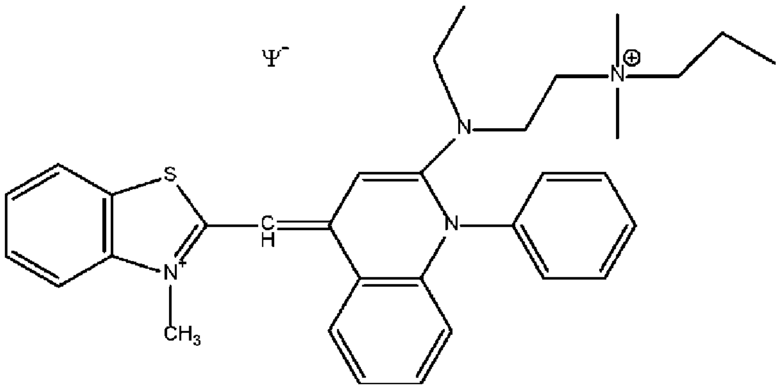

,

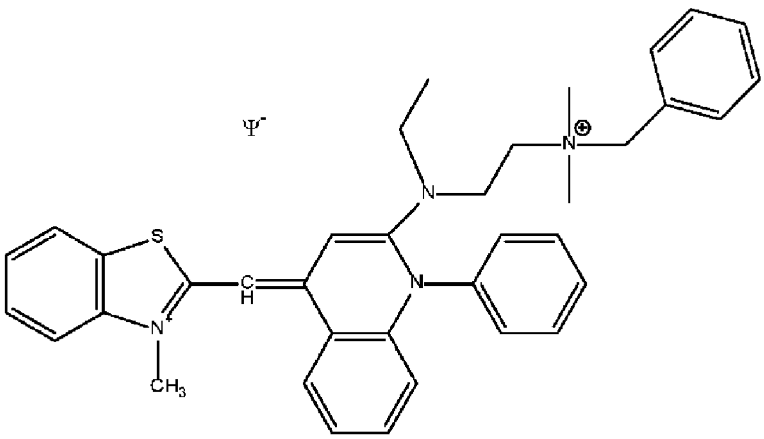

,

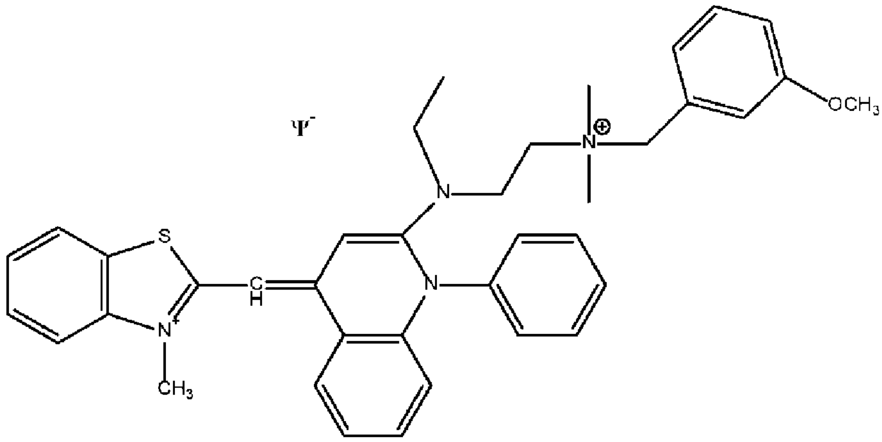

,

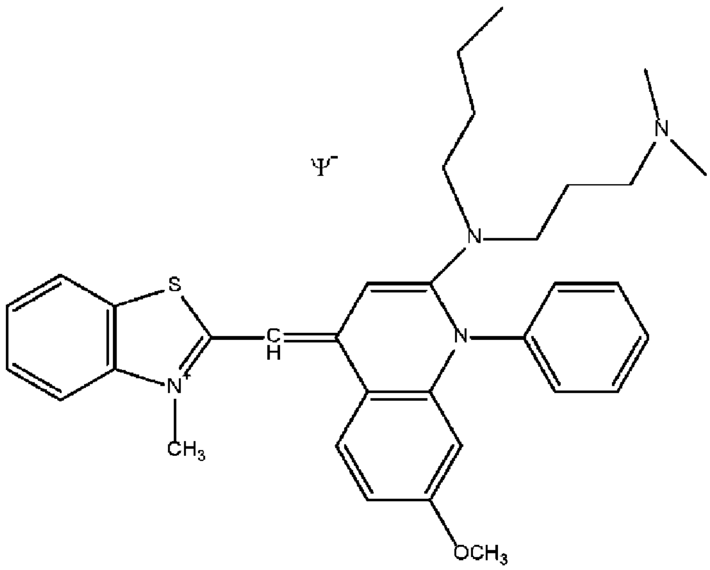

,

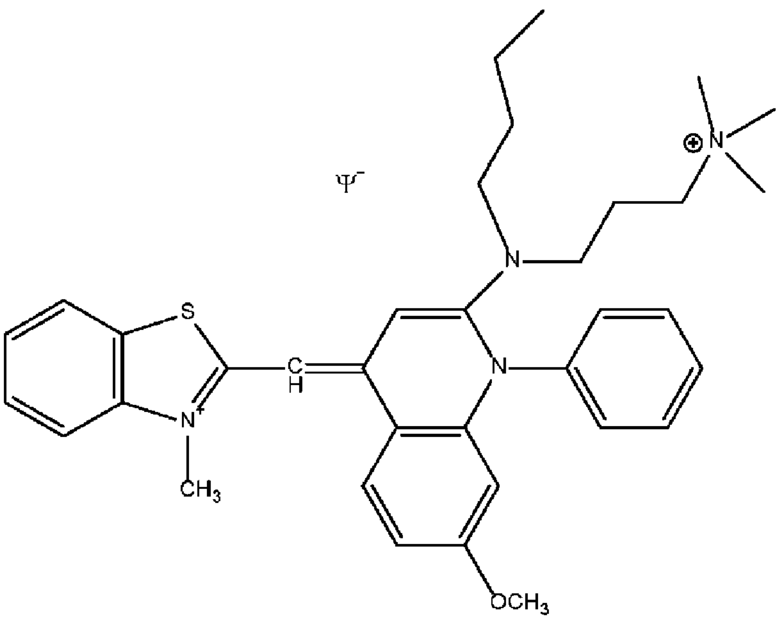

,

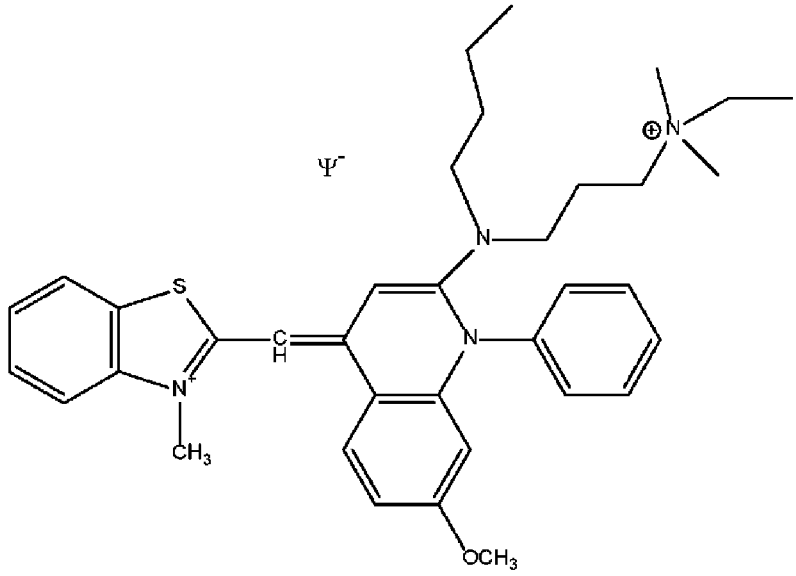

,

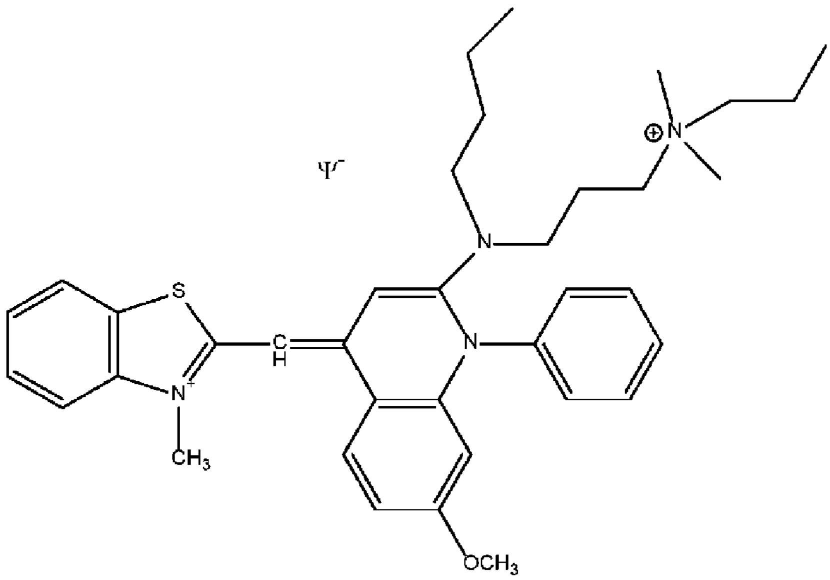

,

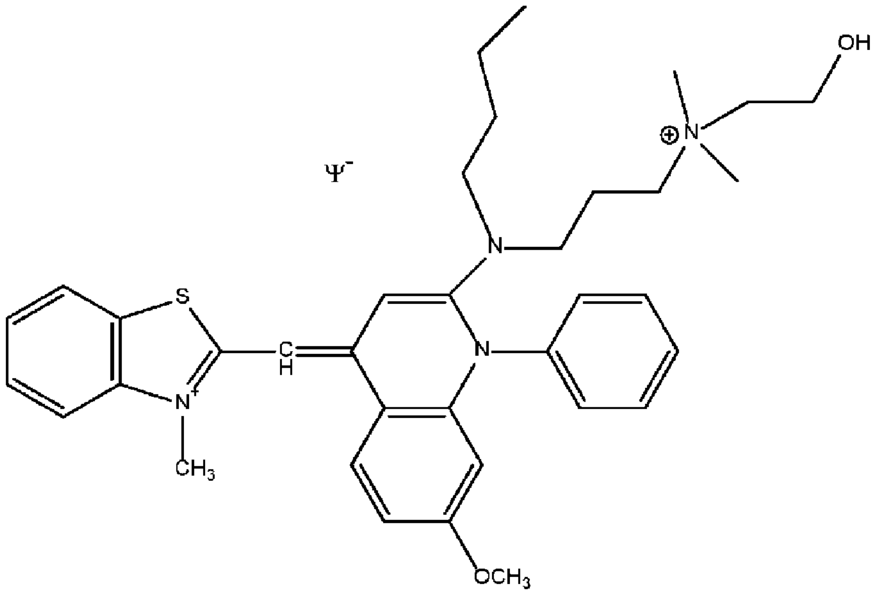

,

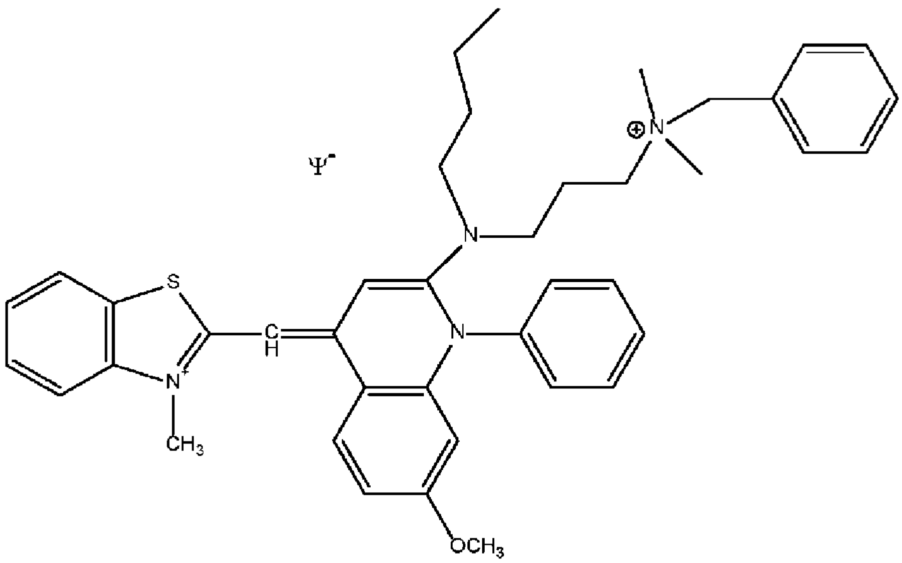

,

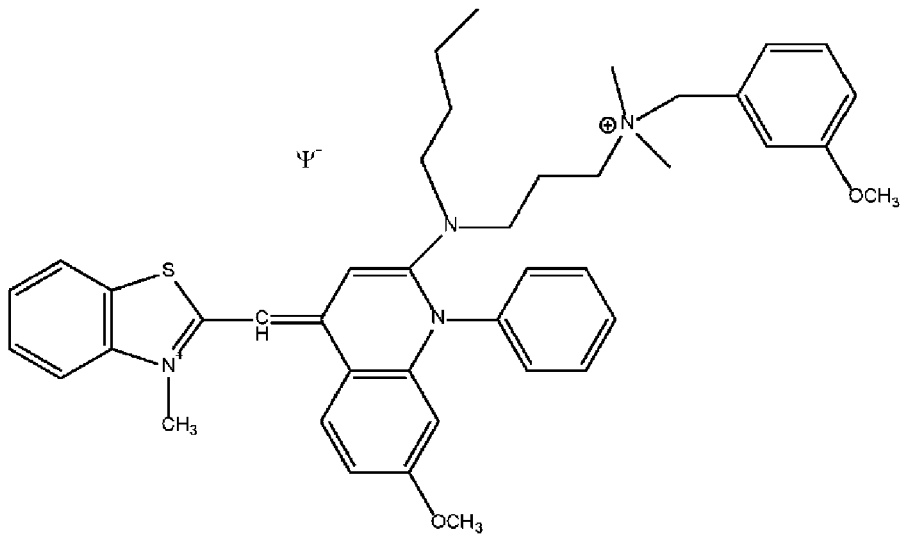

,

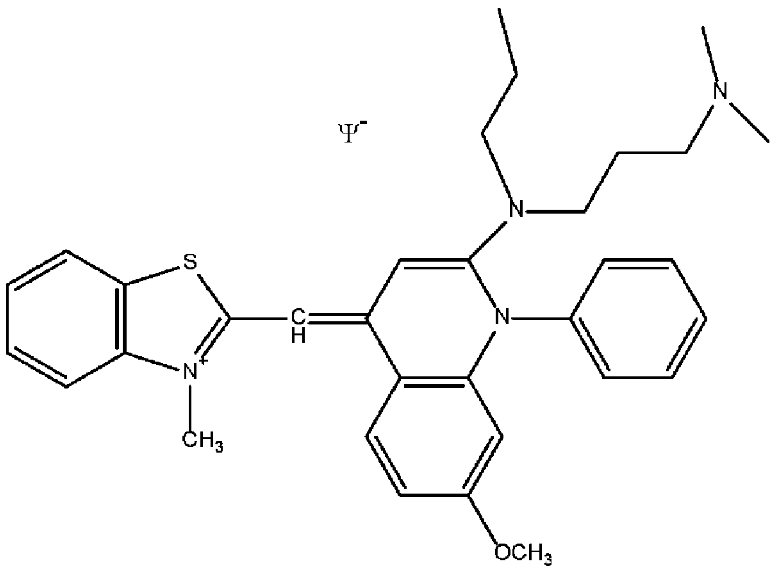

,

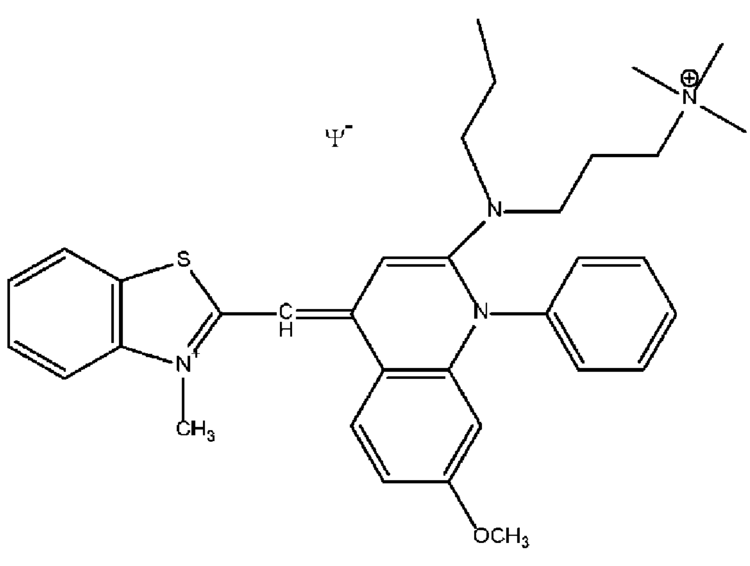

,

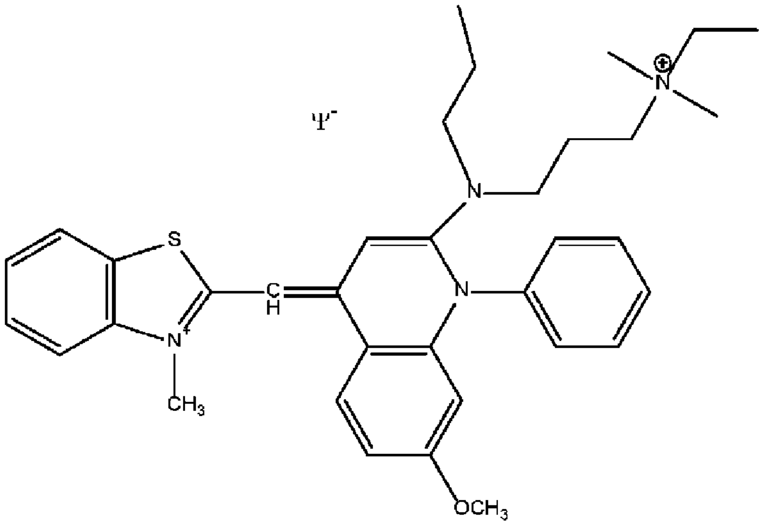

,

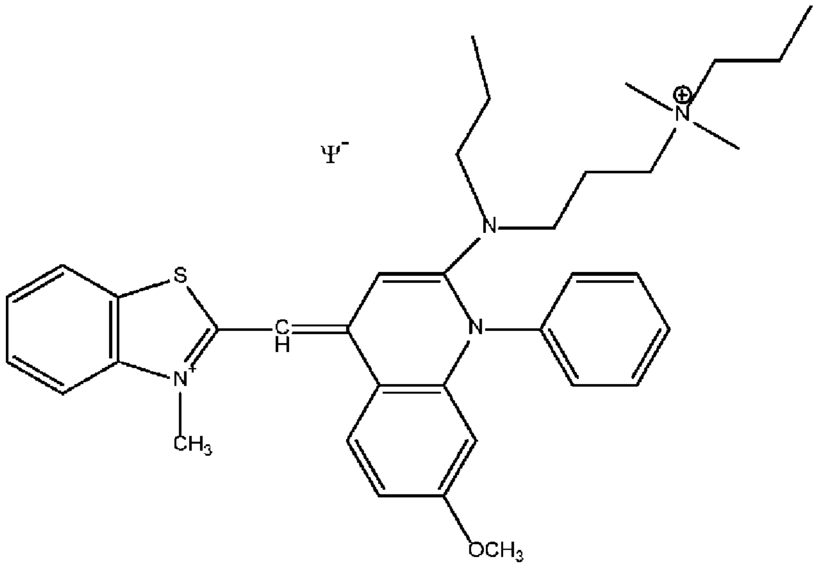

,

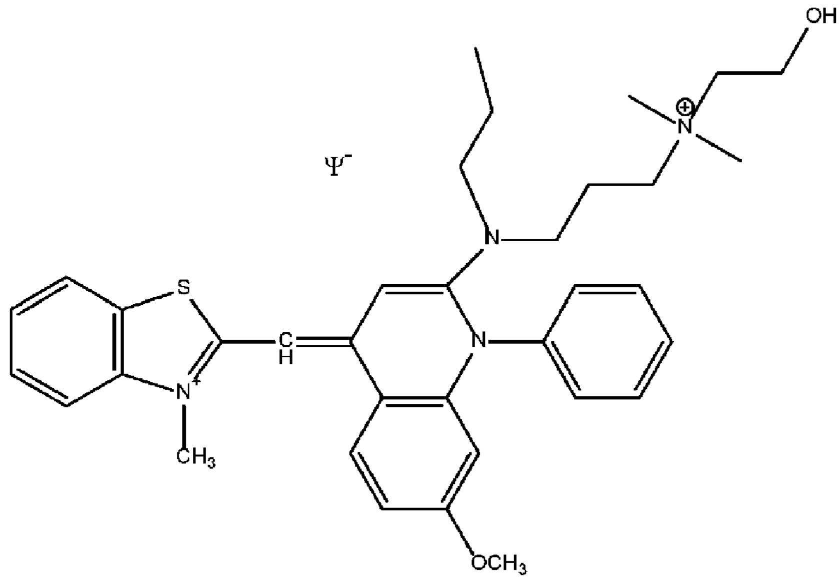

,

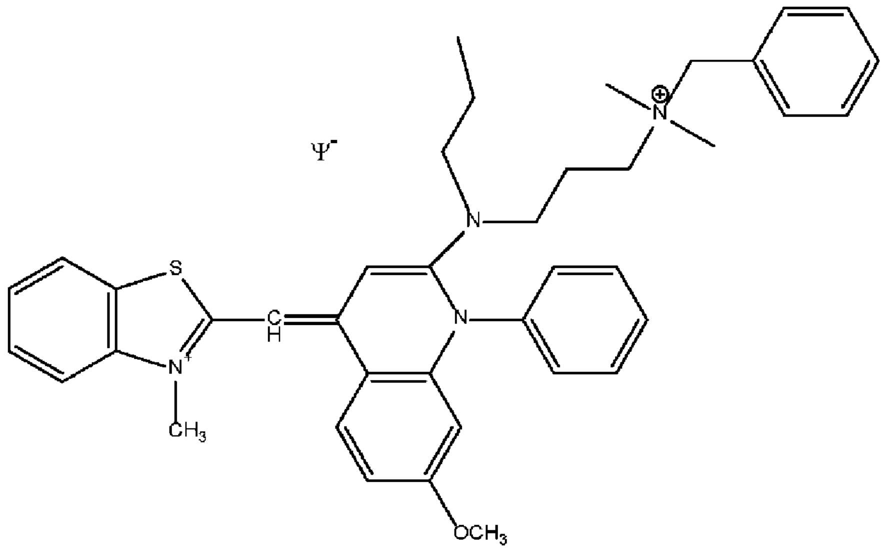

,

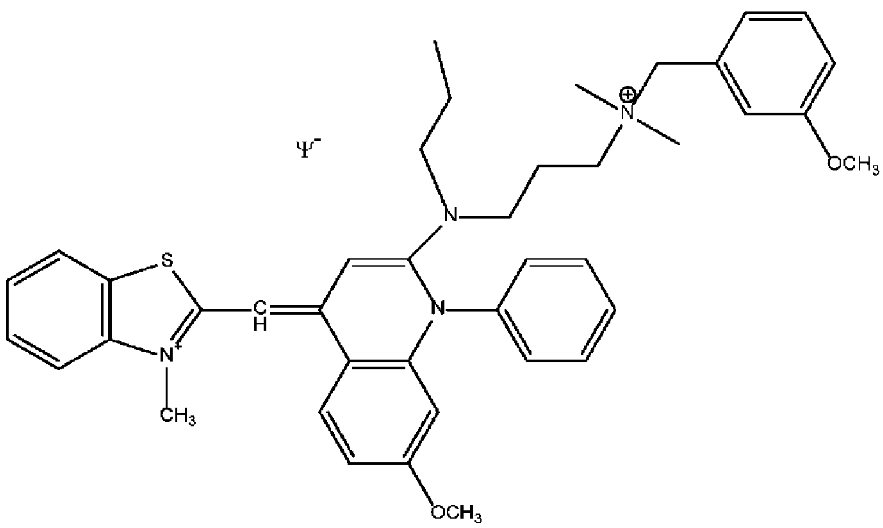

,

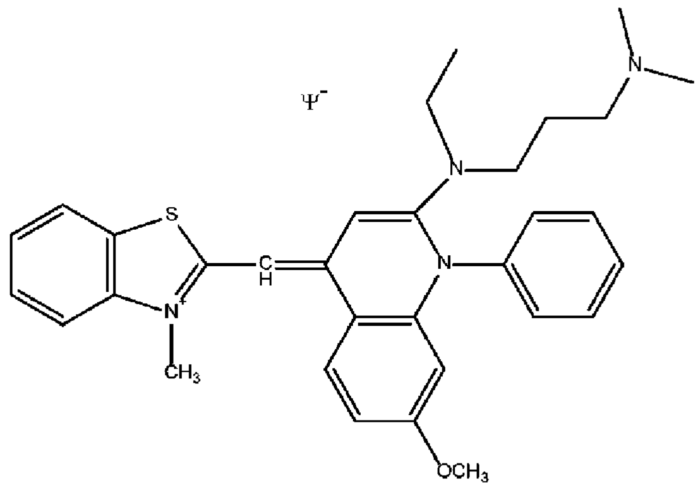

,

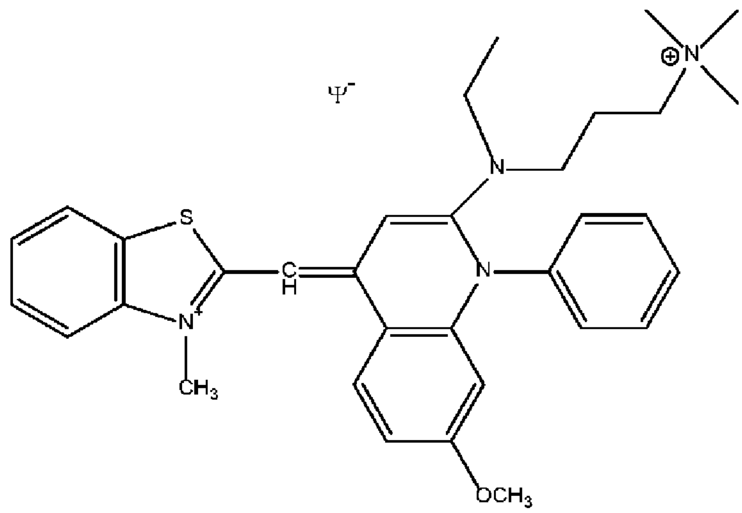

,

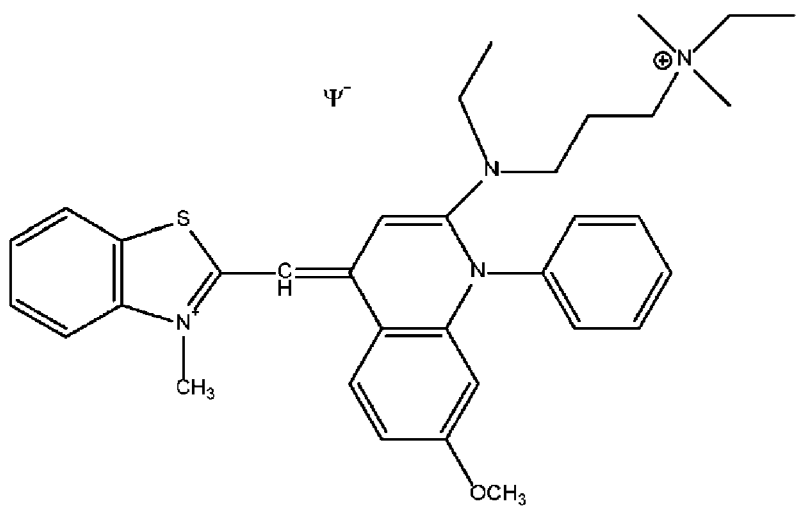

,

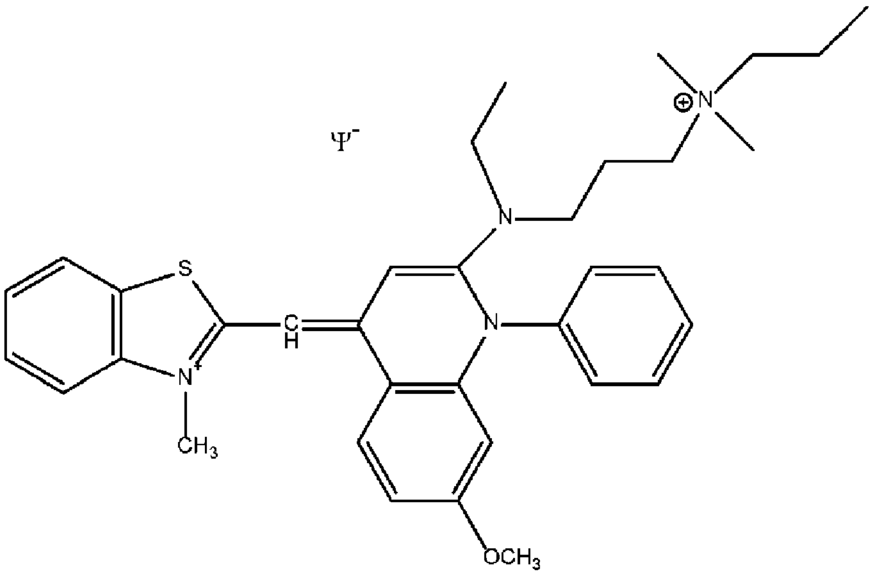

,

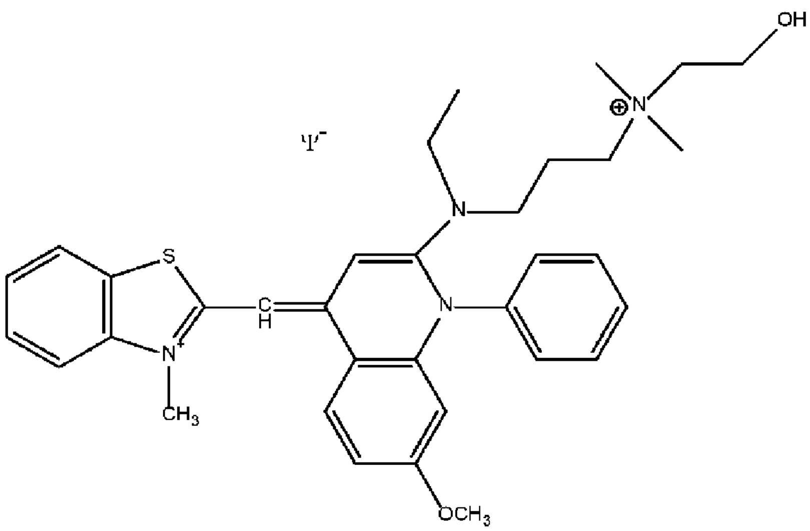

,

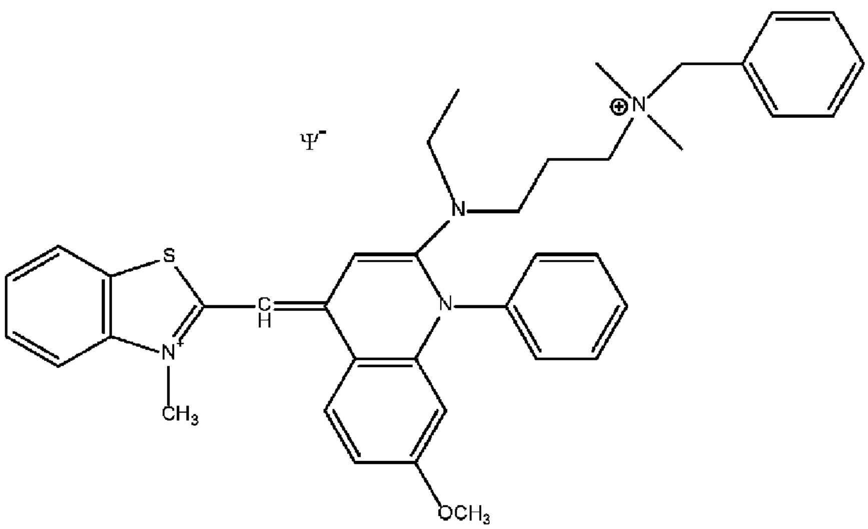

,

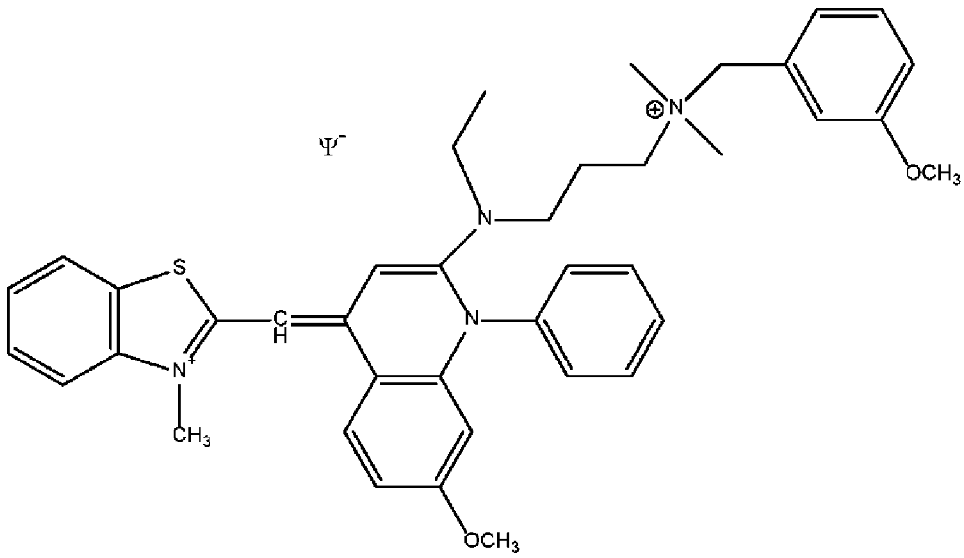

,

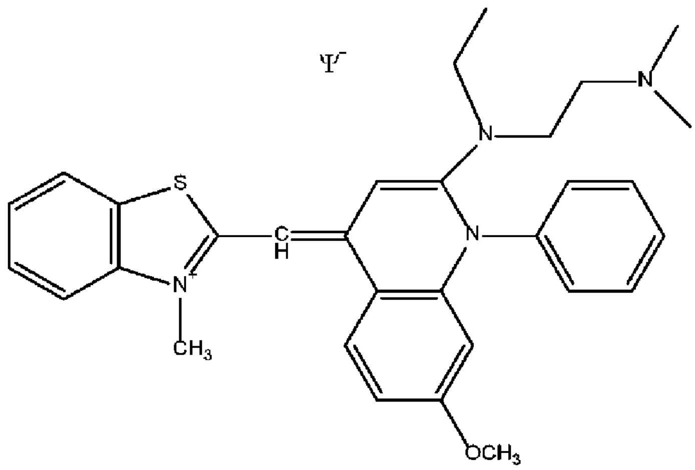

,

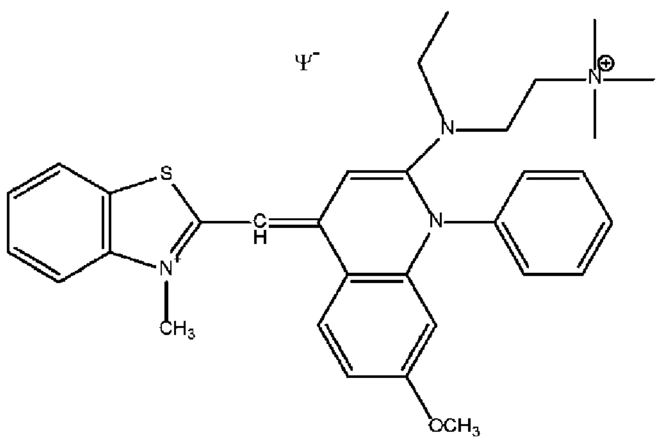

,

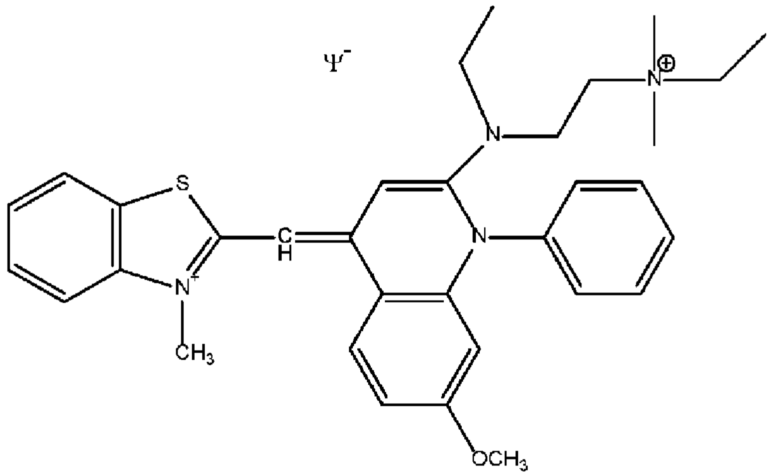

,

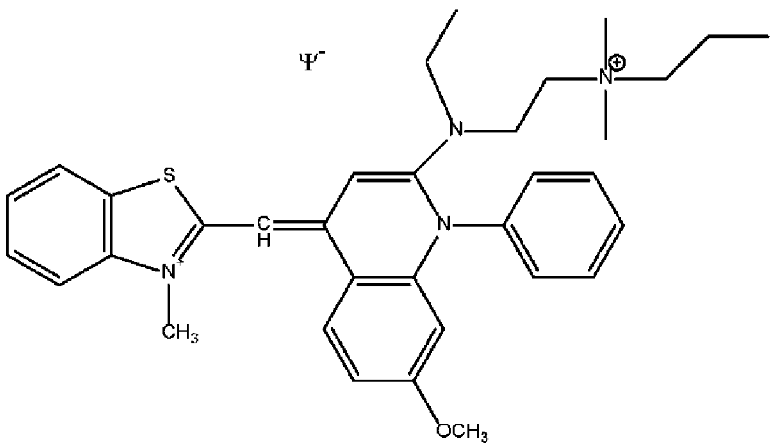

,

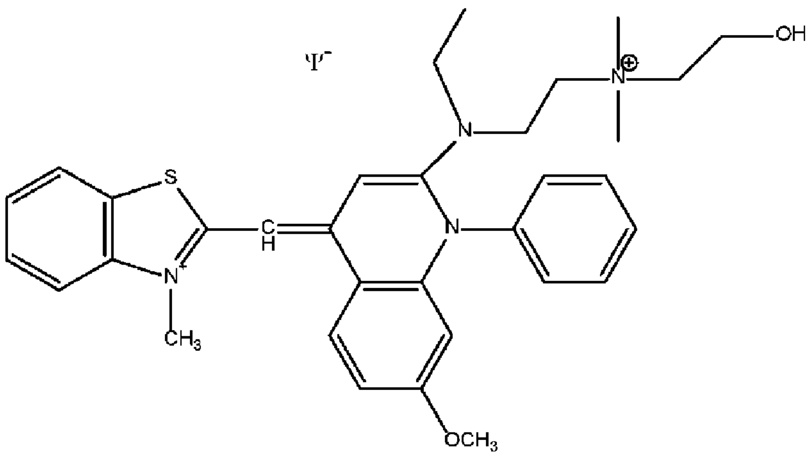

,

und

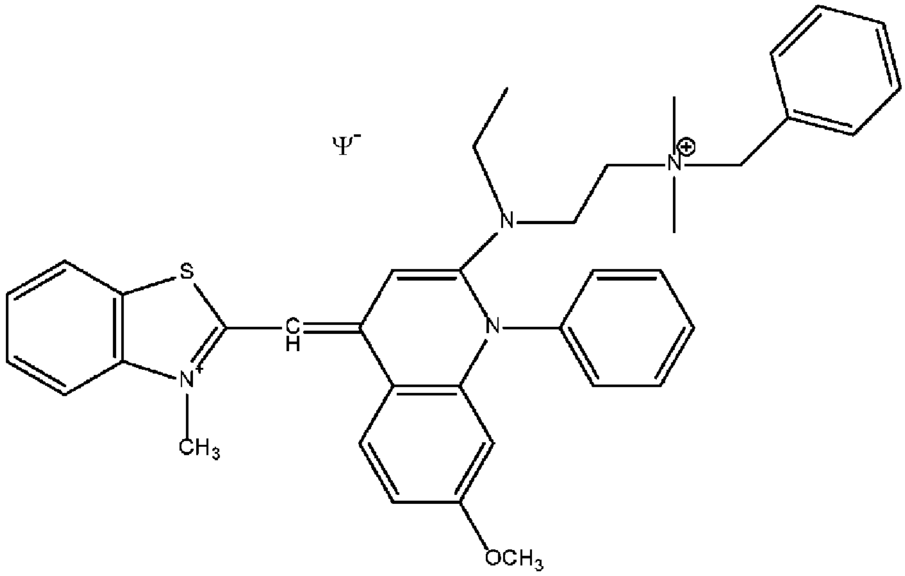

.

wobei Ψ ein biologisch kompatibles Gegenion ist
und wobei der zweite nukleinsäurebindende Farbstoff ausgewählt ist aus:

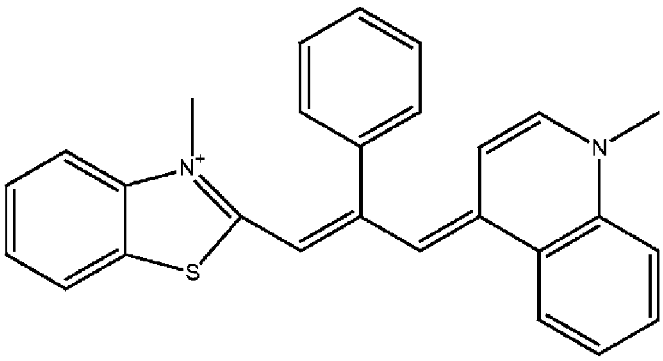

;

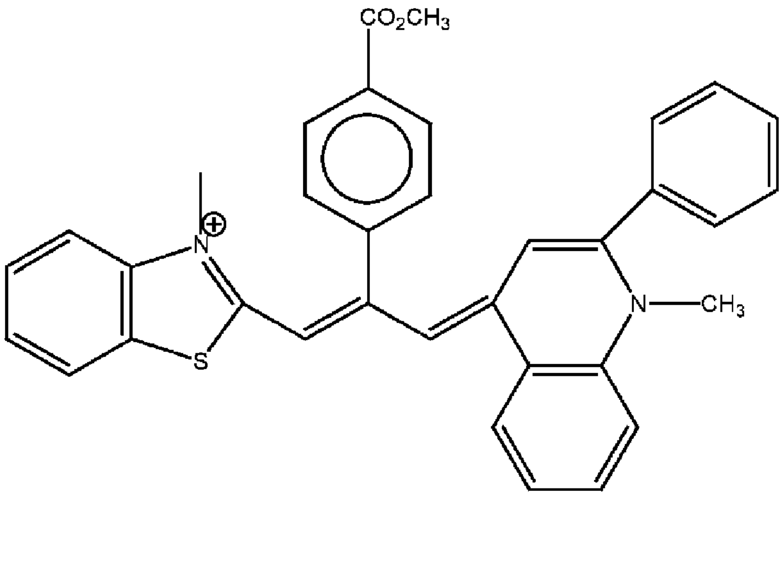

,

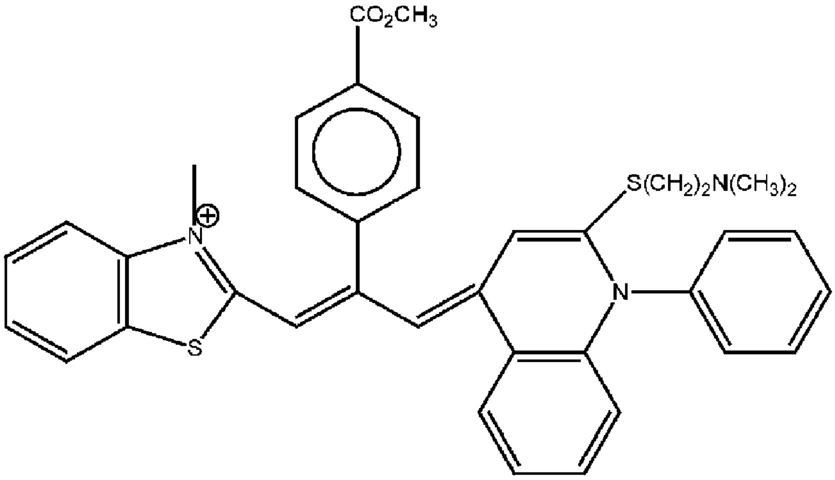

,

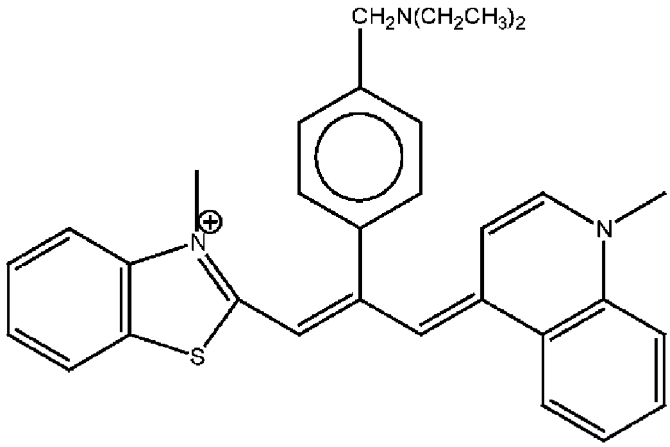

,

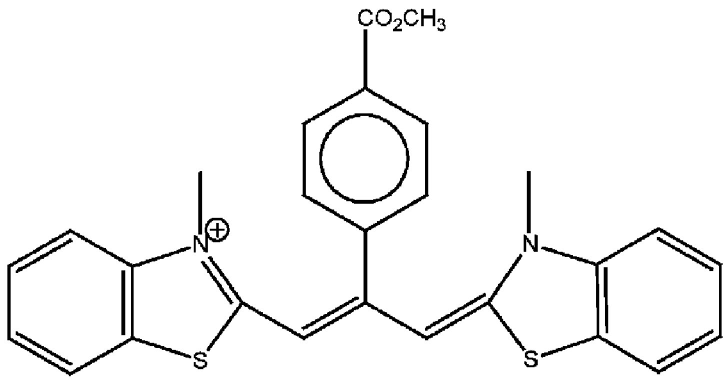

,

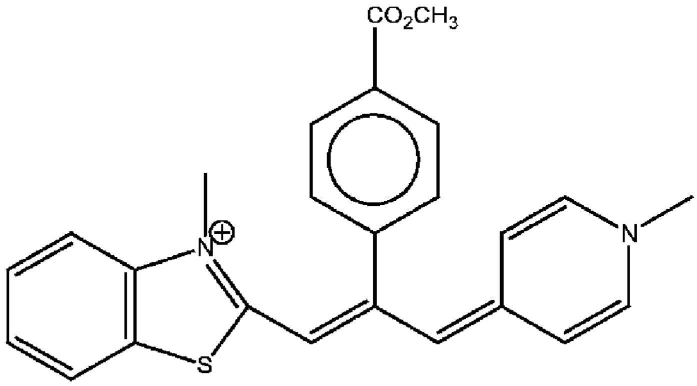

,

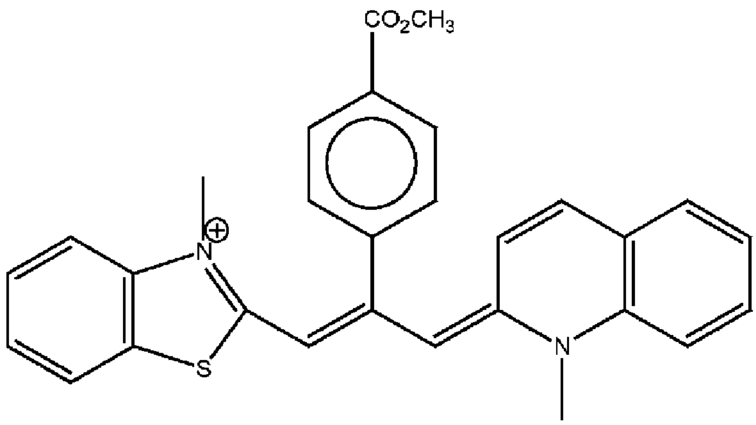

,

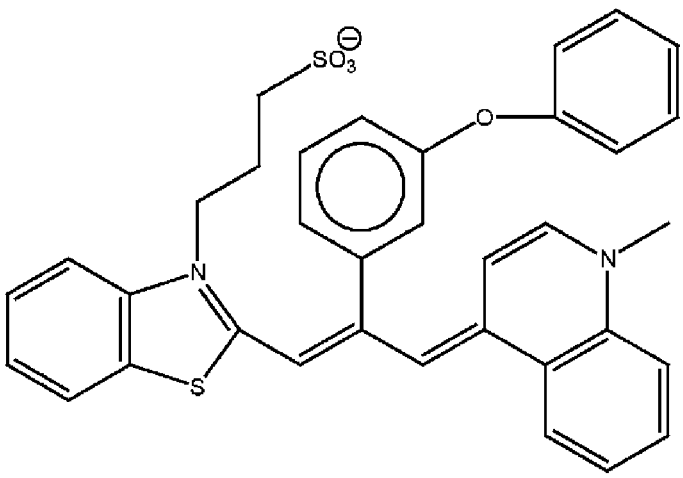

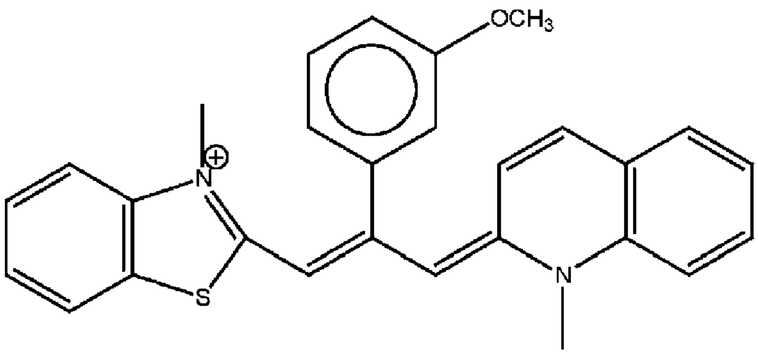

,

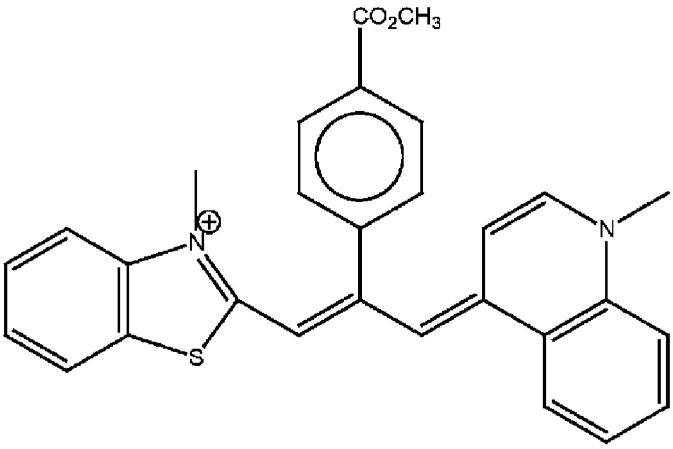

,

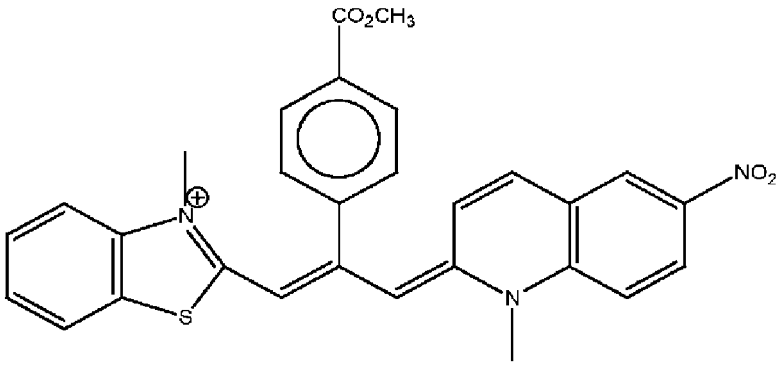

,

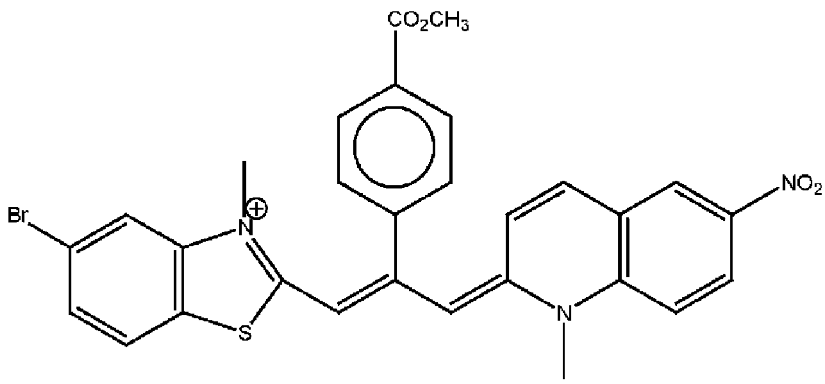

,

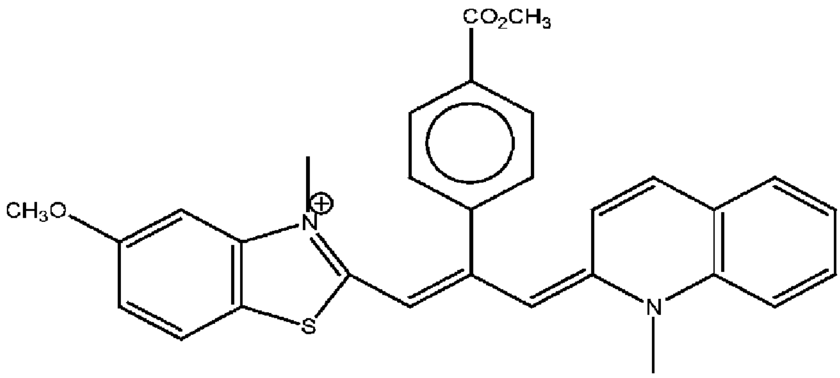

,

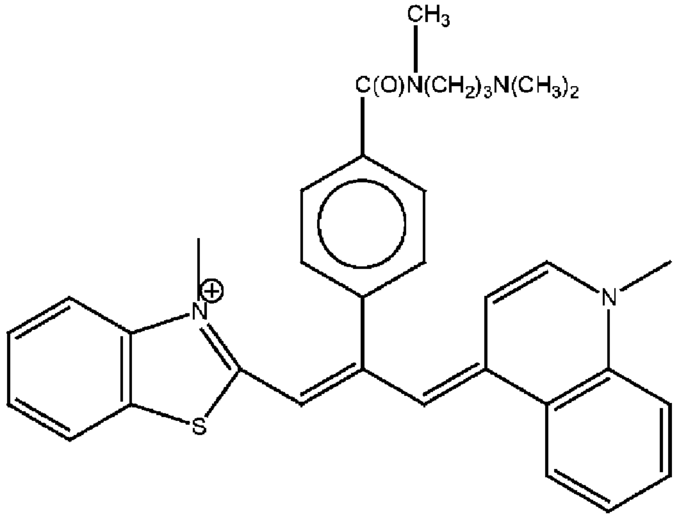

,

und

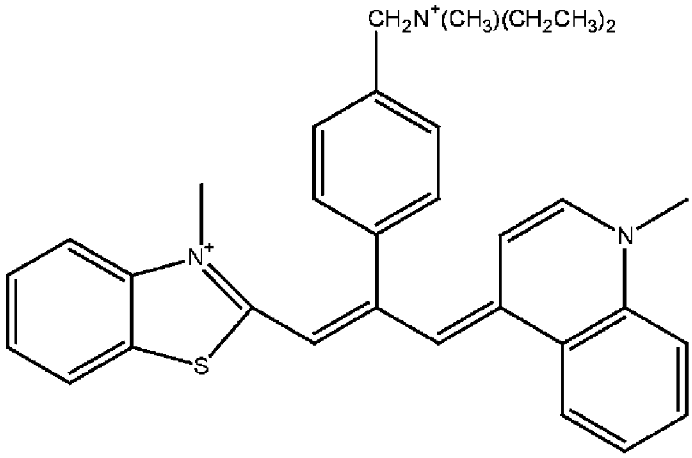

.

**2.** Verfahren zum Bestimmen des Ausmaßes des RNA-Abbaus in einer Probe, ausgedrückt als ein RNA-Qualitätswert, wobei das Verfahren Folgendes umfasst:

a) Kombinieren einer Probe, die vermutlich RNA enthält, mit einem ersten nukleinsäurebindenden Farbstoff, der selektiv kleine RNA, umfassend 200 Nukleotide oder weniger, bindet und einem zweiten nukleinsäurebindenden Farbstoff, der selektiv intakte RNA mit sekundärer und/oder tertiärer Struktur bindet, wobei der erste nukleinsäurebindende Farbstoff und der zweite nukleinsäurebindende Farbstoff nachweisbar verschieden sind;

b) Inkubieren der Probe für einen Zeitraum, der ausreicht, um dem ersten nukleinsäurebindenden Farbstoff und dem zweiten nukleinsäurebindenden Farbstoff zu ermöglichen, mit der RNA in der Probe zu kombinieren, um einen ersten nukleinsäurebindenden-Farbstoff-RNA-Komplex und einen zweiten nukleinsäurebindenden-Farbstoff-RNA-Komplex zu bilden;
c) Beleuchten des ersten nukleinsäurebindenden-Farbstoff-RNA-Komplexes und des zweiten nukleinsäurebindenden-Farbstoff-RNA-Komplexes mit einer geeigneten Wellenlänge von Licht, um eine beleuchtete Probenmischung zu bilden, wobei der erste nukleinsäurebindende-Farbstoff-RNA-Komplex ein erstes Fluoreszenzsignal emittiert und der zweite nukleinsäurebindende-Farbstoff-RNA-Komplex ein zweites Fluoreszenzsignal emittiert;
d) Nachweisen des ersten Fluoreszenzsignals und des zweiten Fluoreszenzsignals; und
e) Berechnen eines Verhältnisses der Intensität des ersten Fluoreszenzsignals zur Intensität des zweiten Fluoreszenzsignals, wodurch der RNA-Qualitätswert erhalten wird, wobei der RNA-Qualitätswert das Ausmaß des RNA-Abbaus in der Probe darstellt,

wobei der erste und der zweite nukleinsäurebindende Farbstoff wie in Anspruch 1 definiert sind.

**3.** Verfahren nach **Anspruch 1 oder 2,** wobei die intakte RNA mit sekundärer und/oder tertiärer Struktur rRNA ist.

**4.** Verfahren nach **Anspruch 1 oder 2,** wobei der erste nukleinsäurebindende Farbstoff und der zweite nukleinsäurebindende Farbstoff der Probe gleichzeitig hinzugefügt werden oder wobei der erste nukleinsäurebindende Farbstoff und der zweite nukleinsäurebindende Farbstoff der Probe sequentiell hinzugefügt werden.

**5.** Verfahren nach **Anspruch 1 oder 2,** wobei die intakte RNA mit sekundärer und/oder tertiärer Struktur tRNA ist.

**6.** Verfahren nach **Anspruch 1 oder 2,** wobei die intakte RNA mit sekundärer und/oder tertiärer Struktur mRNA ist.

**7.** Verfahren nach **einem der vorstehenden Ansprüche,** wobei der zweite nukleinsäurebindende Farbstoff ein RNA/DNA-Fluoreszenzverstärkungsverhältnis von $\geq 7$, $\geq 10$ oder $\geq 20$ aufweist.

**8.** Verfahren nach **einem der vorstehenden Ansprüche,** wobei der erste nukleinsäurebindende Farbstoff eine einzelsträngige RNA-Fluoreszenzverstärkung von $\geq 30$ % aufweist.

**9.** Verfahren nach **einem der vorstehenden Ansprüche,** wobei der Nachweisschritt durch Fluorometrie durchgeführt wird.

**10.** Verfahren nach **Anspruch 1**, ferner umfassend
Herstellen:

einer ersten Standardlösung durch Kombinieren des ersten nukleinsäurebindenden Farbstoffs und des zweiten nukleinsäurebindenden Farbstoffs mit einer Pufferblindprobe;
einer zweiten Standardlösung durch Kombinieren des ersten nukleinsäurebindenden Farbstoffs und des zweiten nukleinsäurebindenden Farbstoffs mit einem Standard für kleine RNA; und
einer dritten Standardlösung durch Kombinieren des ersten nukleinsäurebindenden Farbstoffs und des zweiten nukleinsäurebindenden Farbstoffs mit einem Standard für intakte RNA mit sekundärer und/oder tertiärer Struktur;
Inkubieren der ersten Standardlösung, der zweiten Standardlösung und der dritten Standardlösung für eine Zeit, die für den ersten nukleinsäurebindenden Farbstoff und den zweiten nukleinsäurebindenden Farbstoff ausreichend ist, um mit RNA in dem Standard, falls vorhanden, zu kombinieren, um einen ersten Standardfarbstoffkomplex, einen zweiten Standardfarbstoffkomplex und einen dritten Standardfarbstoffkomplex zu bilden;
Beleuchten des ersten Standardfarbstoffkomplexes, des zweiten Standardfarbstoffkomplexes und des dritten Standardfarbstoffkomplexes mit einer geeigneten Wellenlänge von Licht, um eine erste beleuchtete Standardmischung, eine zweite beleuchtete Standardmischung und eine dritte beleuchtete Standardmischung zu bilden; und
Nachweisen eines Fluoreszenzsignals von jeder der ersten beleuchteten Standardmischung, der zweiten beleuchteten Standardmischung und der dritten beleuchteten Standardmischung, und optional ferner umfassend das Bestimmen des Ausmaßes des RNA-Abbaus, der in der beleuchteten Probenmischung vorhanden ist, basierend auf einem Vergleich des nachweisbaren Fluoreszenzsignals in der beleuchteten Probenmischung mit einem Verhältnis des Fluoreszenzsignals der zweiten Standardlösung und des Fluoreszenzsignals der

dritten Standardlösung.

**11.** Kit, umfassend:

eine Farbstofflösung, umfassend einen ersten nukleinsäurebindenden Farbstoff, der selektiv kleine RNA bindet, die 200 Nukleotide oder weniger umfasst, und einen zweiten nukleinsäurebindenden Farbstof, der intakte RNA mit sekundärer und/oder tertiärer Struktur selektiv bindet; wie in Anspruch 1 definiert, und
Anweisungen zur Verwendung gemäß den Verfahren **nach einem der vorstehenden Ansprüche**
wobei der erste nukleinsäurebindende Farbstoff und der zweite nukleinsäurebindende Farbstoff nachweisbar verschieden sind

und optional ferner Folgendes umfasst:

eine erste Standardlösung, umfassend einen Puffer und keine Nukleinsäure;
eine zweite Standardlösung, umfassend einen Standard für kleine RNA, wobei der Standard für kleine RNA aus weniger als 200 nt besteht; und
eine dritte Standardlösung, umfassend einen Standard für intakte RNA mit sekundärer und/oder tertiärer Struktur.

**12.** RNA-Qualitätsanfärbelösung, die Folgendes umfasst:

einen ersten nukleinsäurebindenden Farbstoff, der vorzugsweise kleine RNA, die 200 Nukleotide oder weniger umfasst; bindet
einen zweiten nukleinsäurebindenden Farbstoff, der vorzugsweise intakte RNA mit sekundärer und/oder tertiärer Struktur bindet;
einen Puffer; und
ein organisches Lösungsmittel,
wobei der erste nukleinsäurebindende Farbstoff und der zweite nukleinsäurebindende Farbstoff nachweisbar verschieden sind, und
wobei der erste nukleinsäurebindende Farbstoff ist:

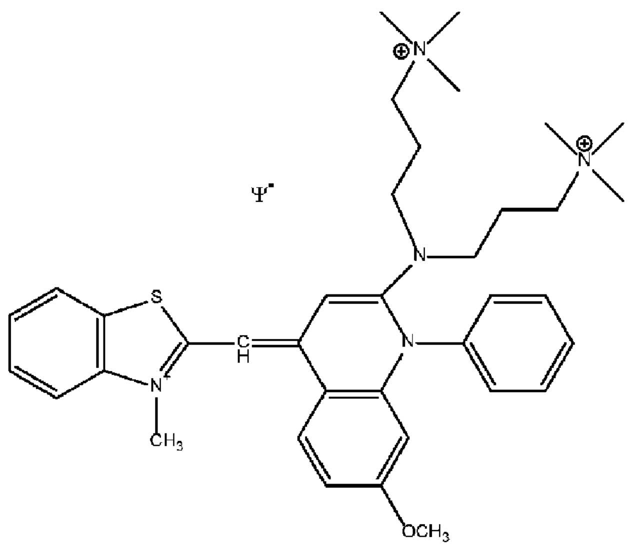

Verbindung **16**,

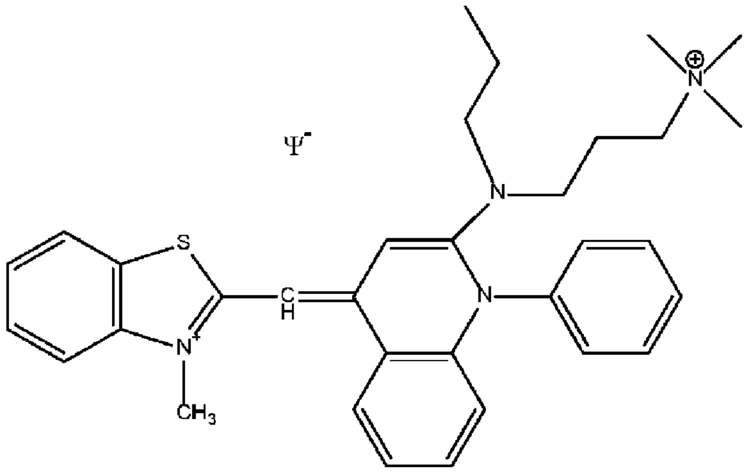

Verbindung **17**,

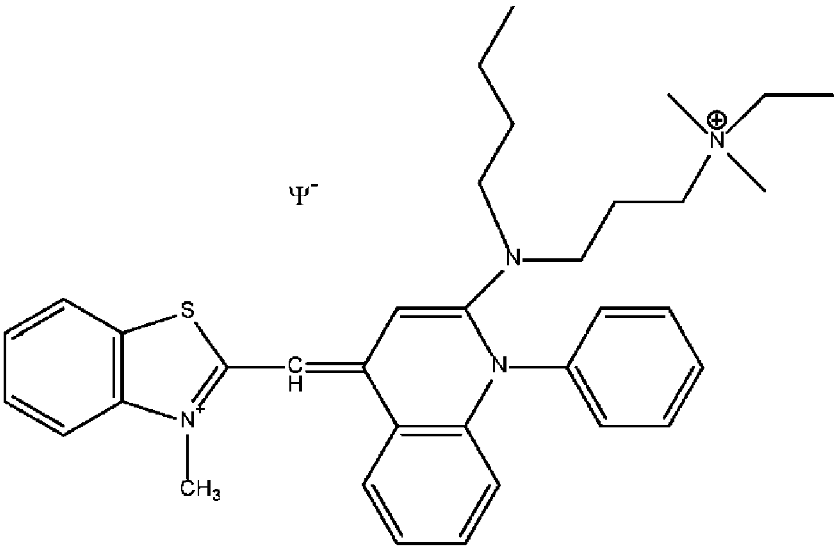

Verbindung **23**,

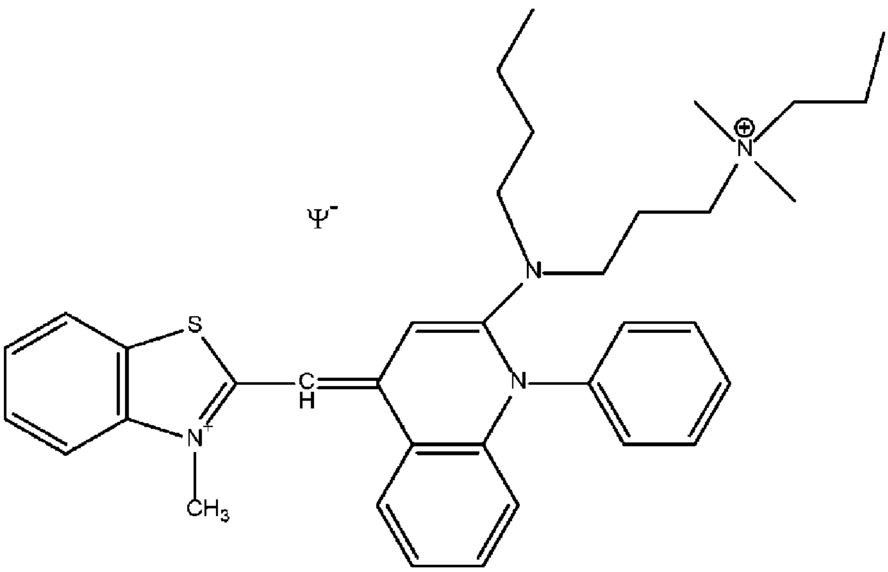

Verbindung **24**,

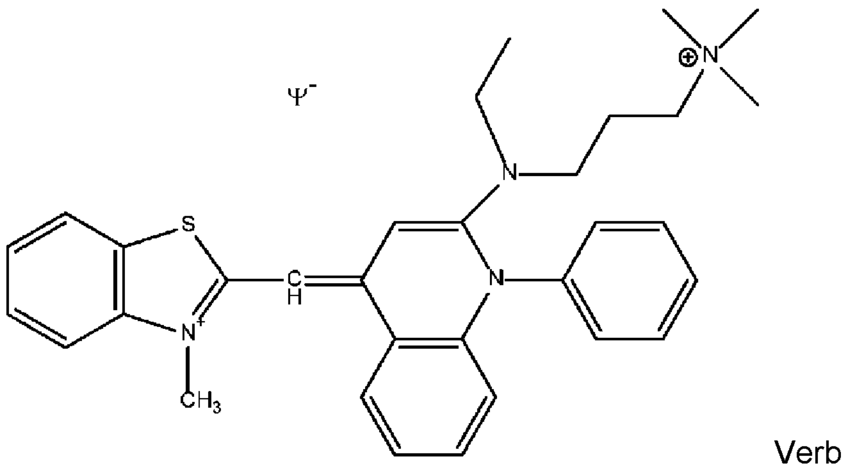

Verbindung **29**,

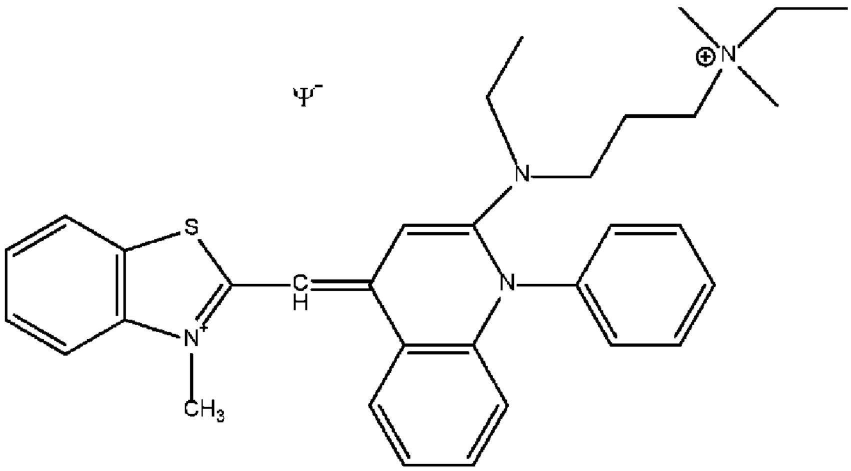

Verbindung **30**,

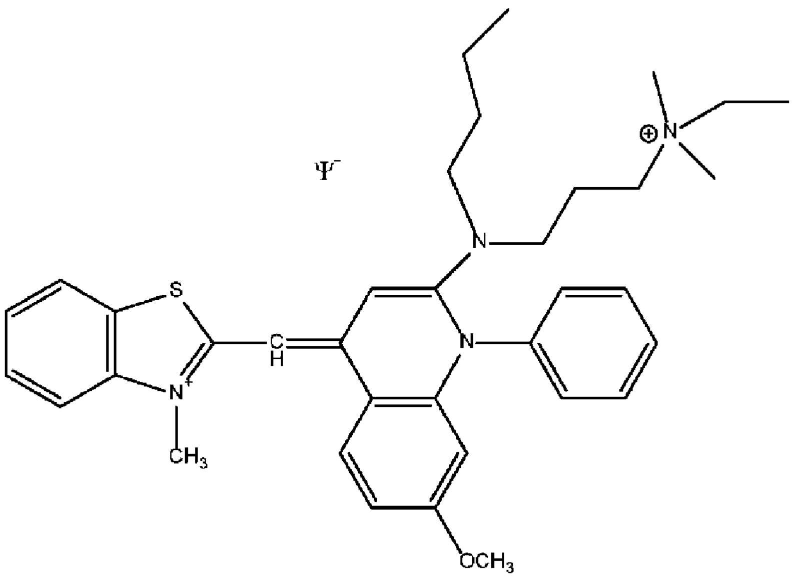

Verbindung **45**,

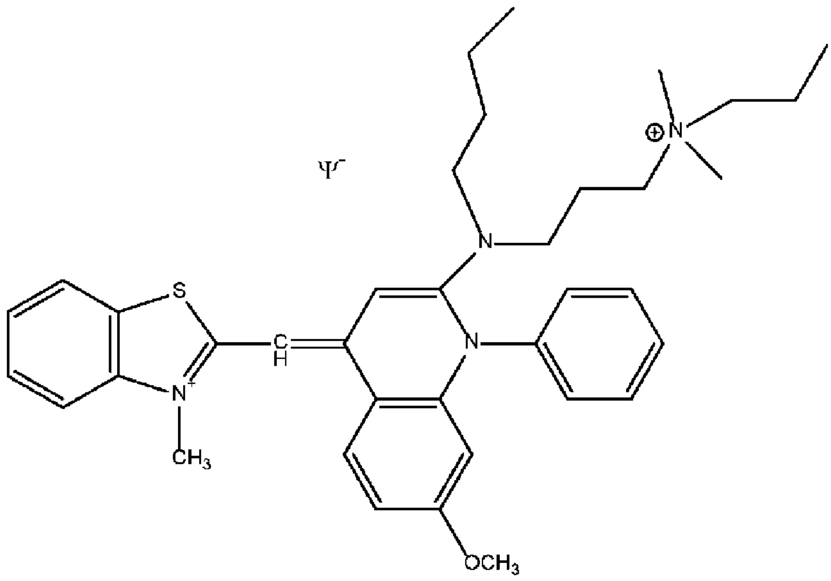

Verbindung **46**,

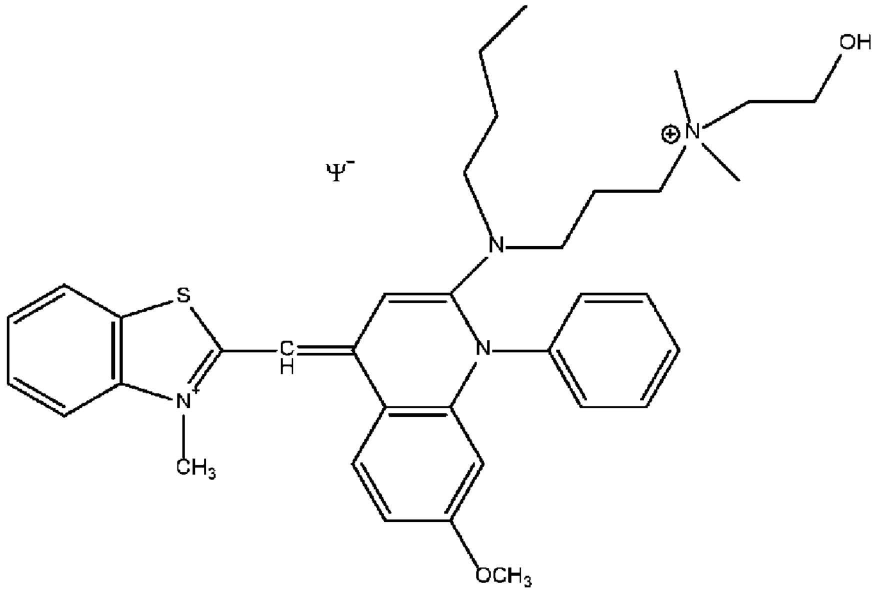

Verbindung **47**,

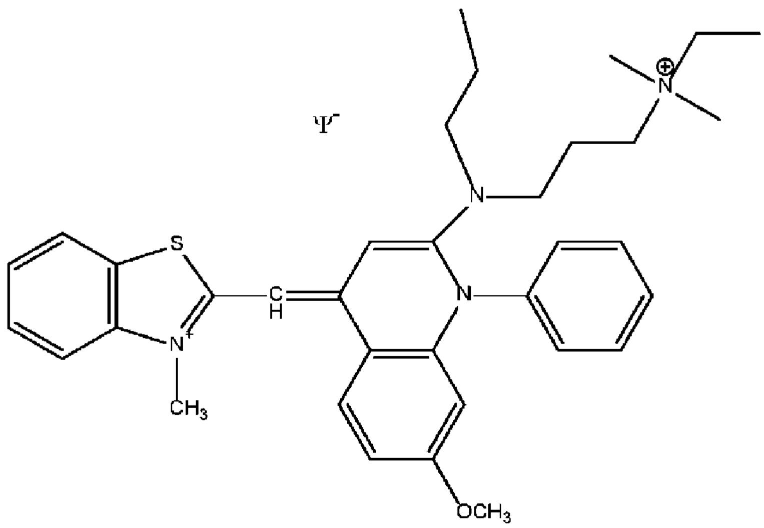

Verbindung **52**,

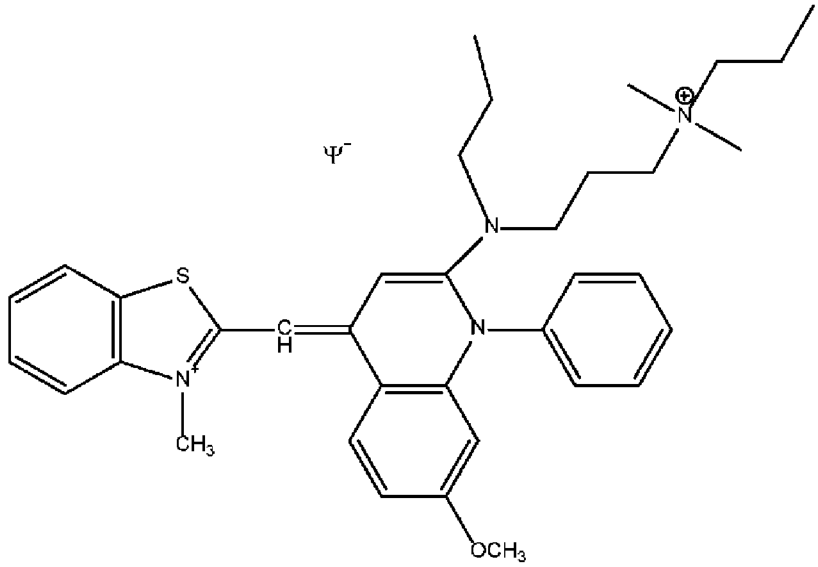

Verbindung **53**,

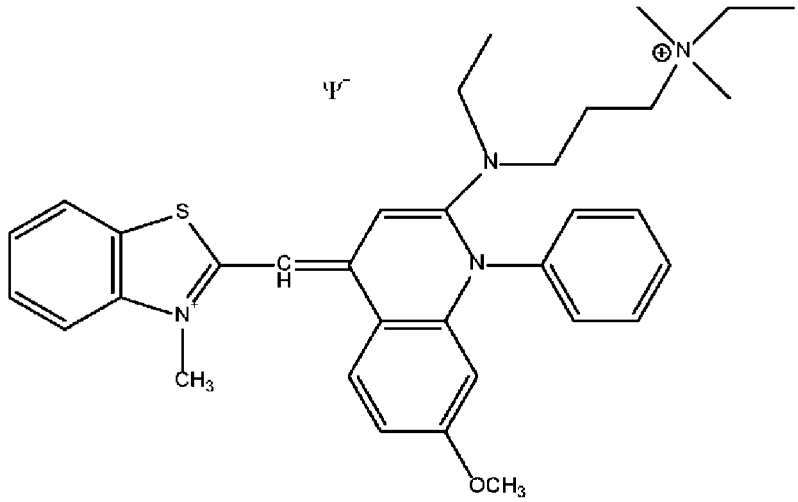

Verbindung **59**,

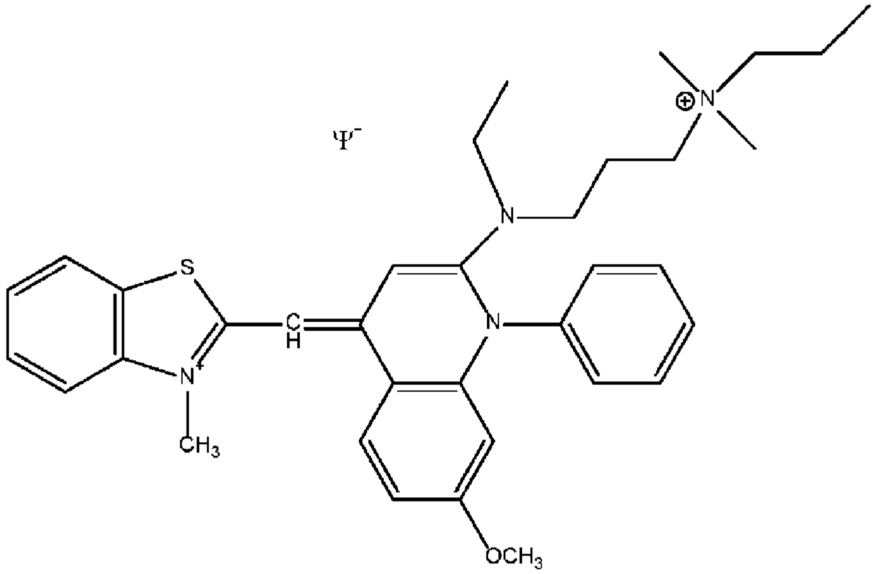

Verbindung **60** oder

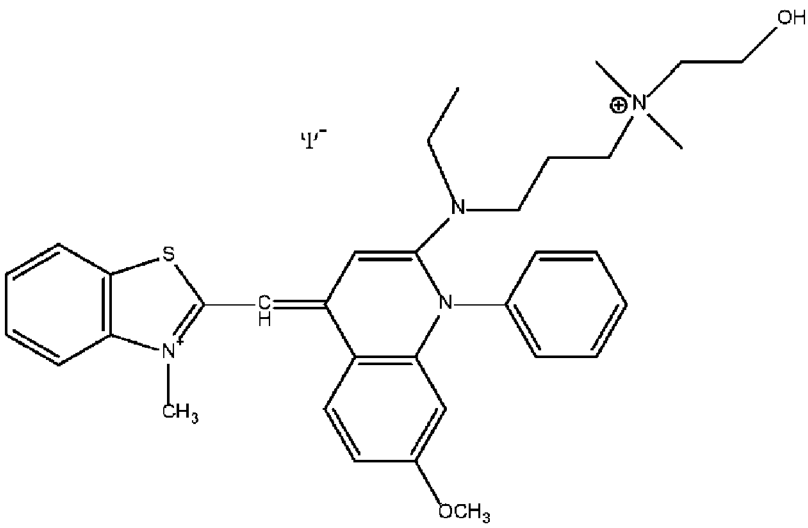

Verbindung **61**,

wobei Ψ ein biologisch kompatibles Gegenion ist; und der zweite nukleinsäurebindende Farbstoff ist:

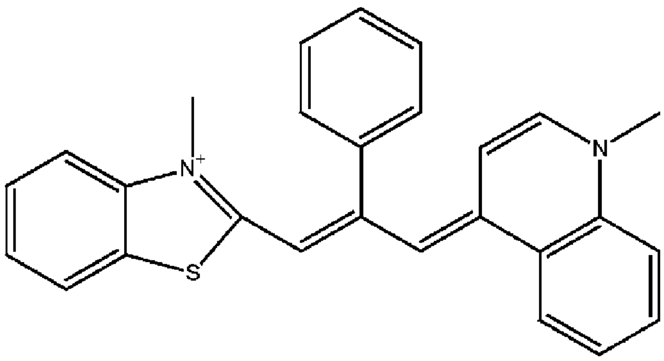

Verbindung **1**,

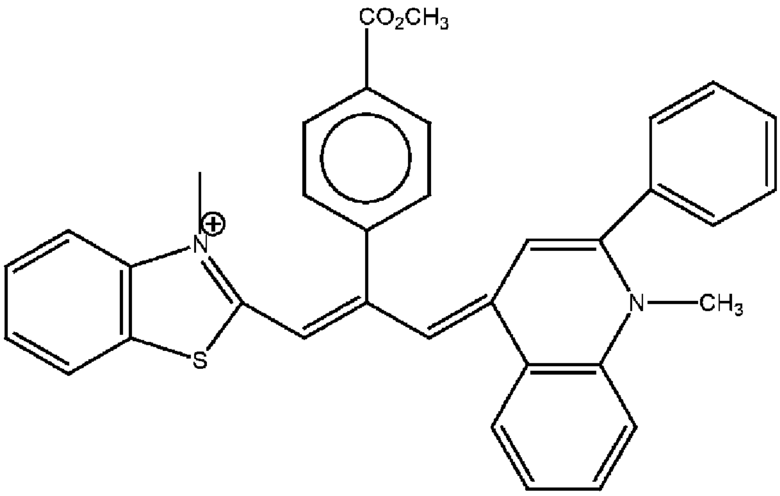

Verbindung **2**,

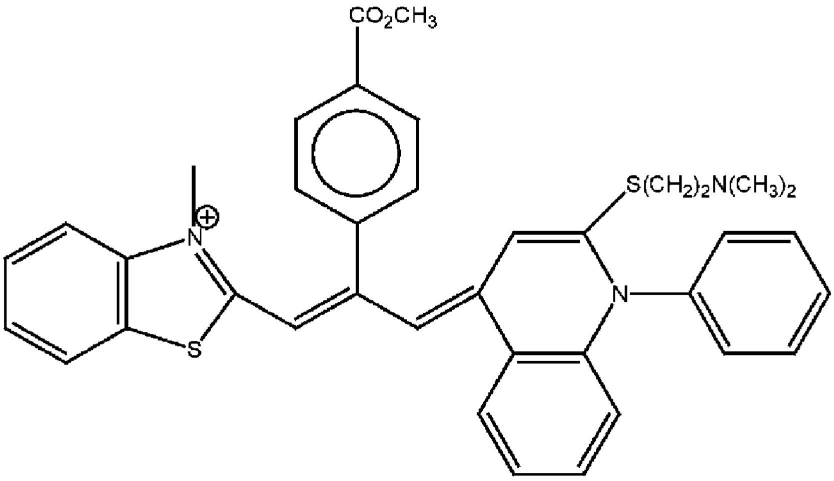

Verbindung **3**,

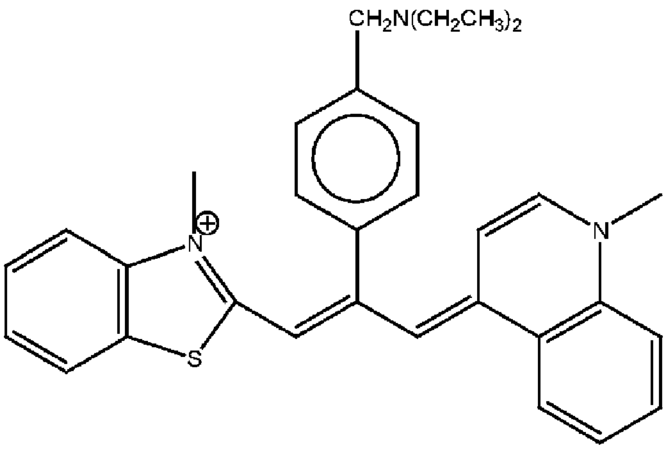

Verbindung **4**,

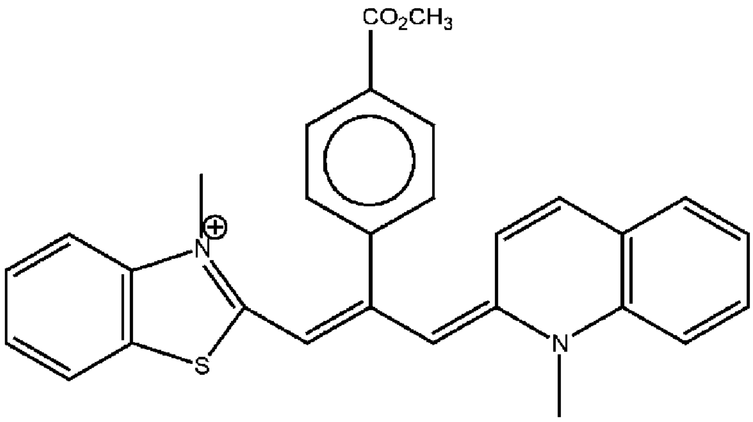

Verbindung **7**,

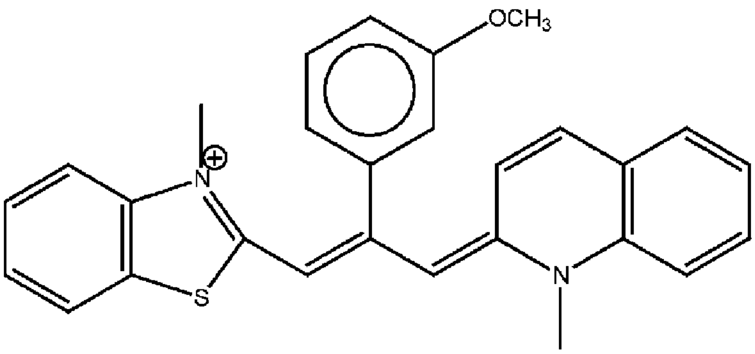

Verbindung **9**,

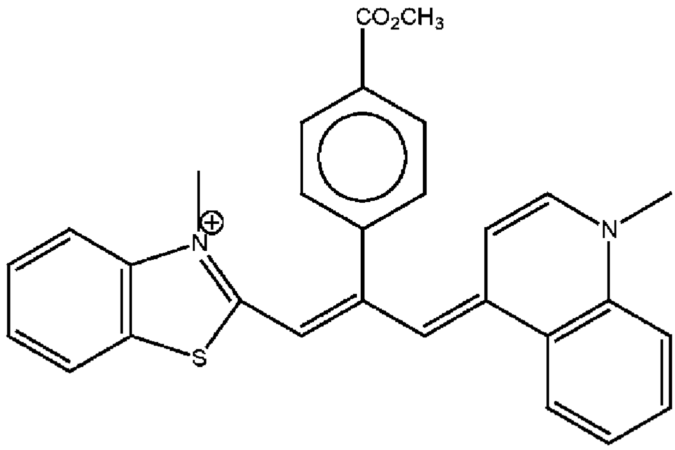

Verbindung **10**,

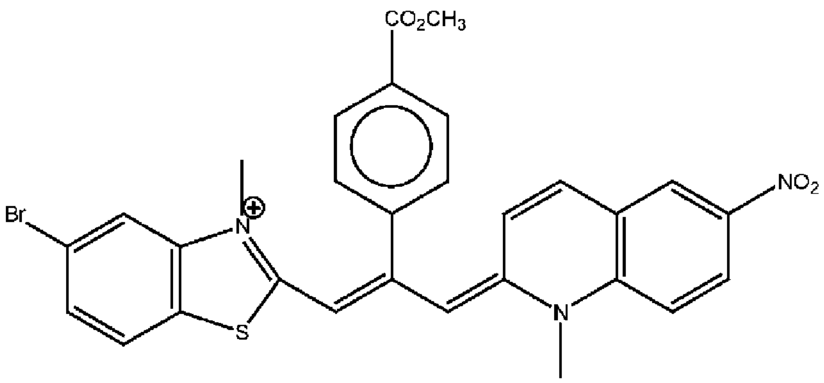

Verbindung **12**,

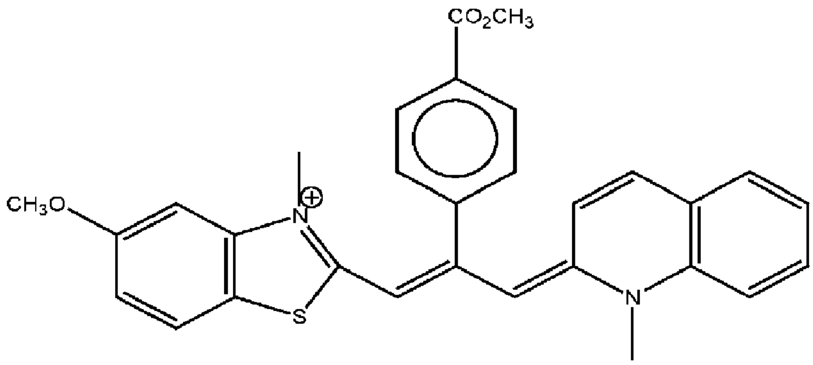

Verbindung **13**,

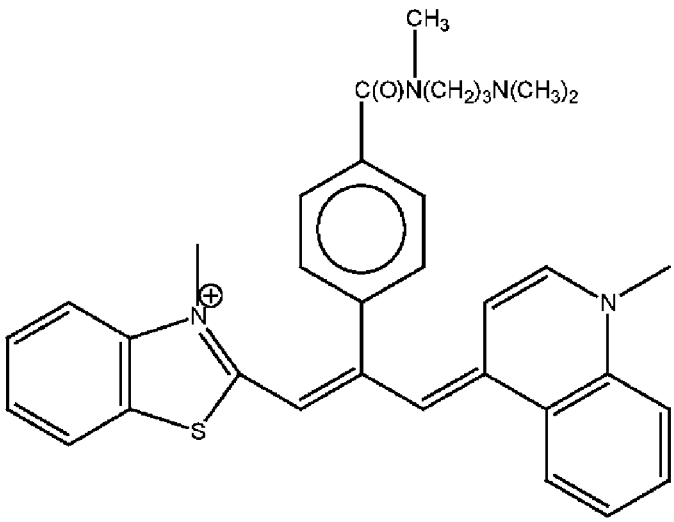

Verbindung **14**

oder

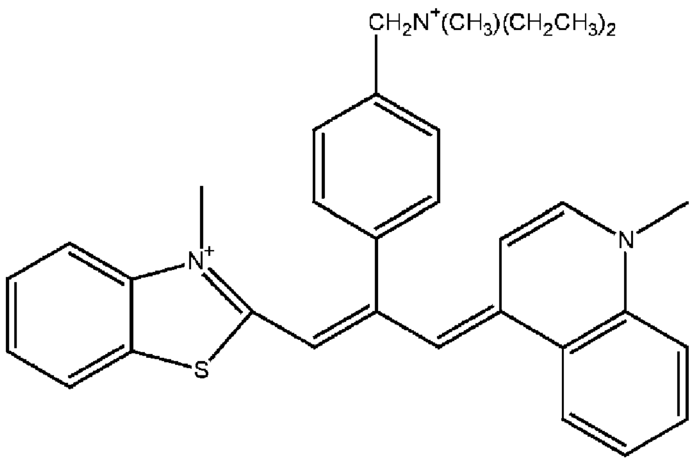

Verbindung **15**.

**Revendications**

1. Procédé de détermination de l'étendue de dégradation d'ARN dans un échantillon, exprimée en tant que valeur de qualité d'ARN, le procédé comprenant :

   a) la combinaison d'un échantillon soupçonné de contenir de l'ARN avec un premier colorant de liaison d'acide nucléique qui se lie sélectivement à un petit ARN comprenant 200 nucléotides ou moins et un second colorant de liaison d'acide nucléique qui se lie sélectivement à un ARN intact avec une structure secondaire et/ou tertiaire,

dans lequel le premier colorant de liaison d’acide nucléique et le second colorant de liaison d’acide nucléique sont distincts de manière détectable ;

b) l’incubation de l’échantillon pendant une période de temps suffisante pour permettre au premier colorant de liaison d’acide nucléique et au second colorant de liaison d’acide nucléique de se combiner avec l’ARN dans l’échantillon pour former un complexe premier colorant de liaison d’acide nucléique-ARN et un complexe second colorant de liaison d’acide nucléique-ARN ;

c) l’éclairage du complexe premier colorant de liaison d’acide nucléique-ARN et du complexe second colorant de liaison d’acide nucléique-ARN avec une longueur d’onde appropriée de lumière pour former un mélange d’échantillon éclairé, dans lequel le complexe premier colorant de liaison d’acide nucléique-ARN émet un premier signal fluorescent et le complexe second colorant de liaison d’acide nucléique-ARN émet un second signal fluorescent ;

d) la détection du premier signal fluorescent et du second signal fluorescent ; et

e) le calcul d’un rapport du premier signal fluorescent au second signal fluorescent, permettant ainsi d’obtenir la valeur de qualité d’ARN, dans lequel la valeur de qualité d’ARN représente l’étendue de dégradation d’ARN dans l’échantillon,

dans lequel le premier colorant de liaison d’acide nucléique est choisi parmi

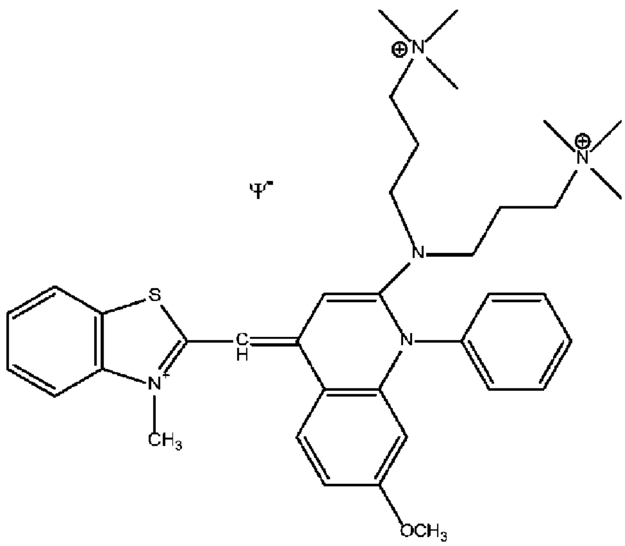

,

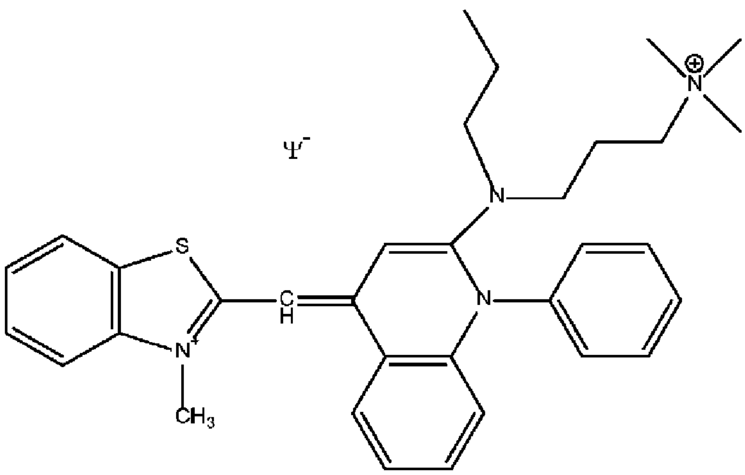

,

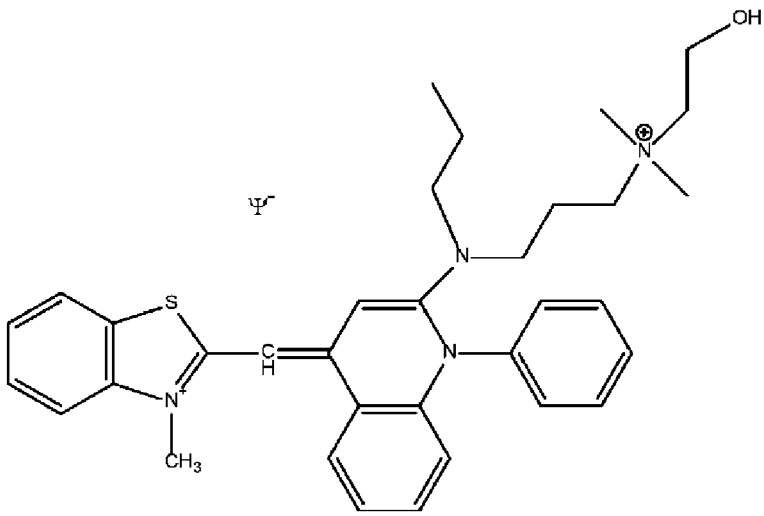

,

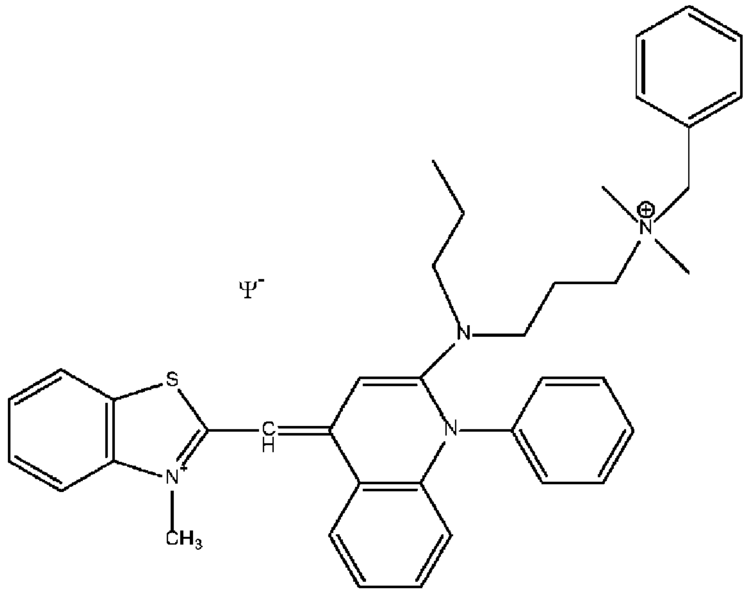

,

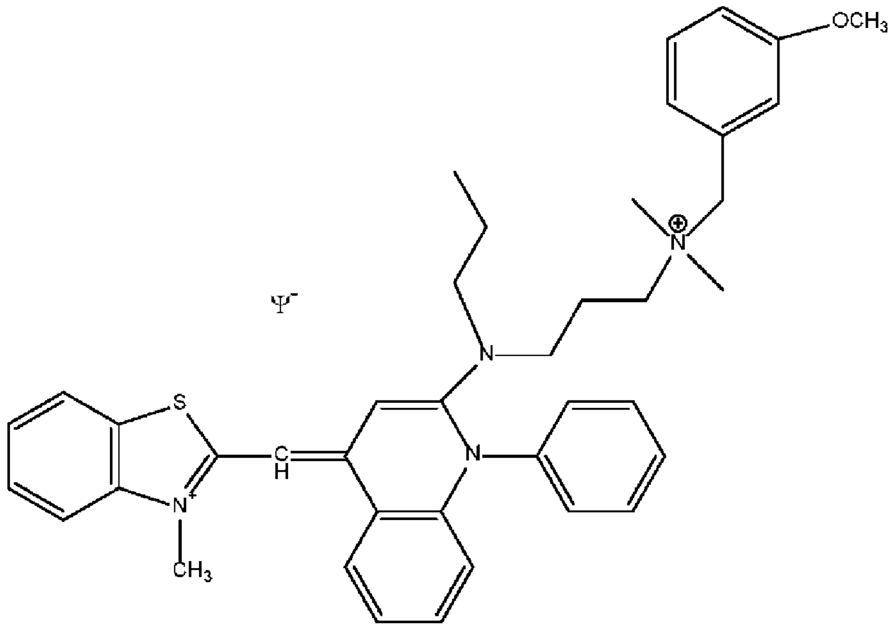

,

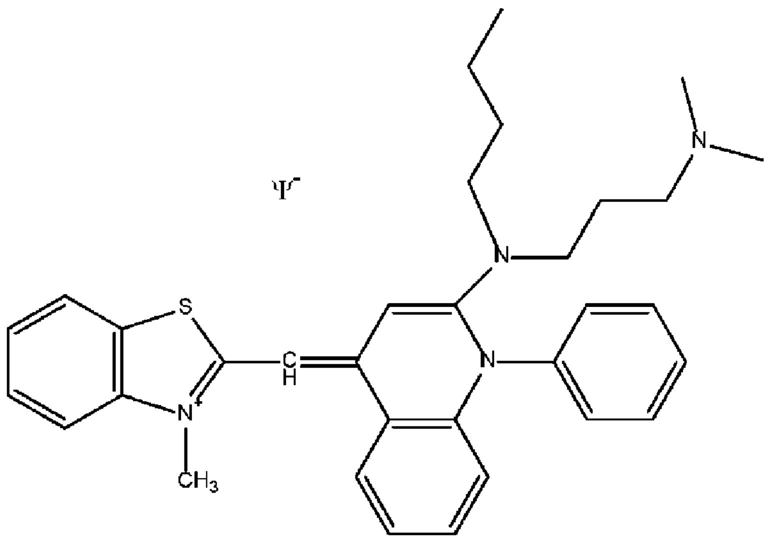

,

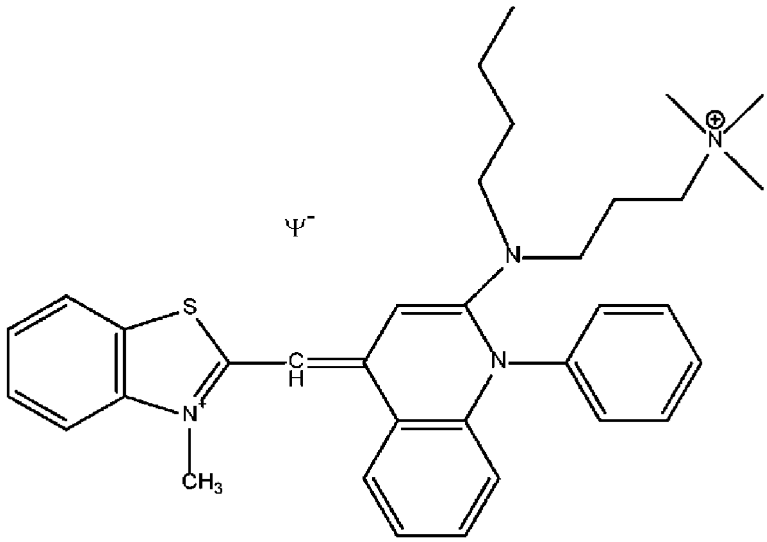

,

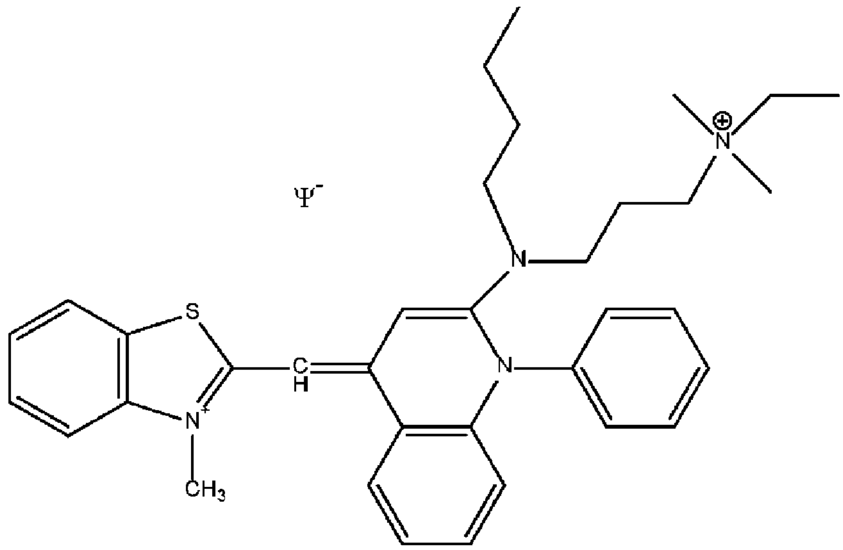

,

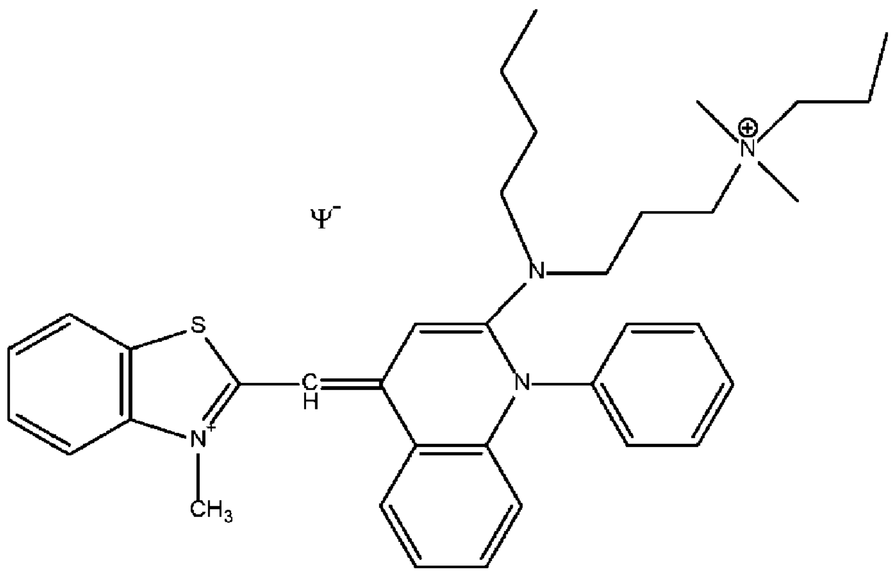

,

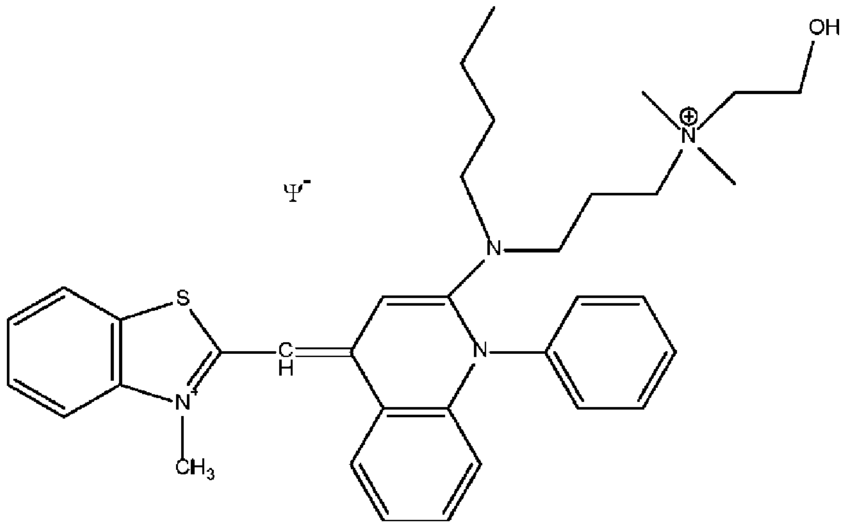

,

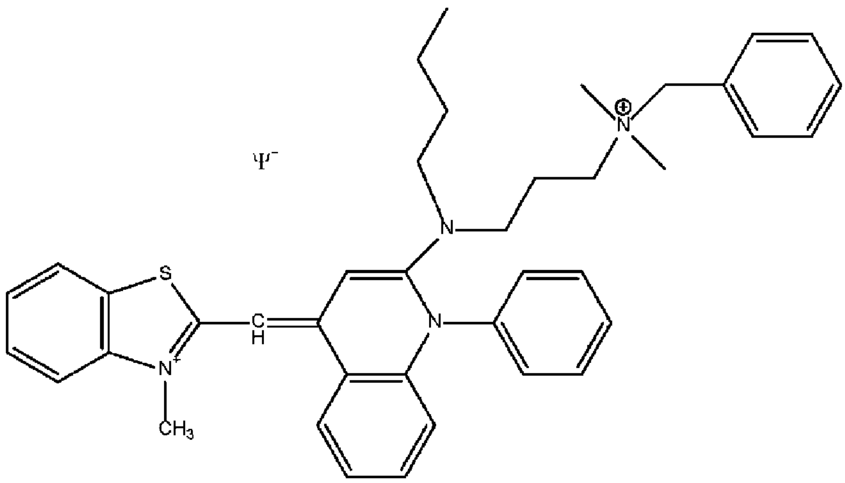

,

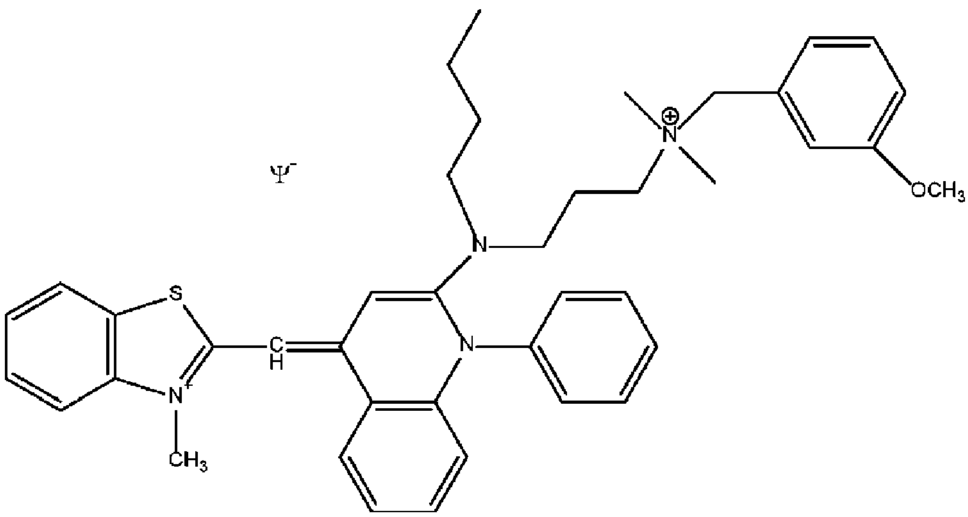

,

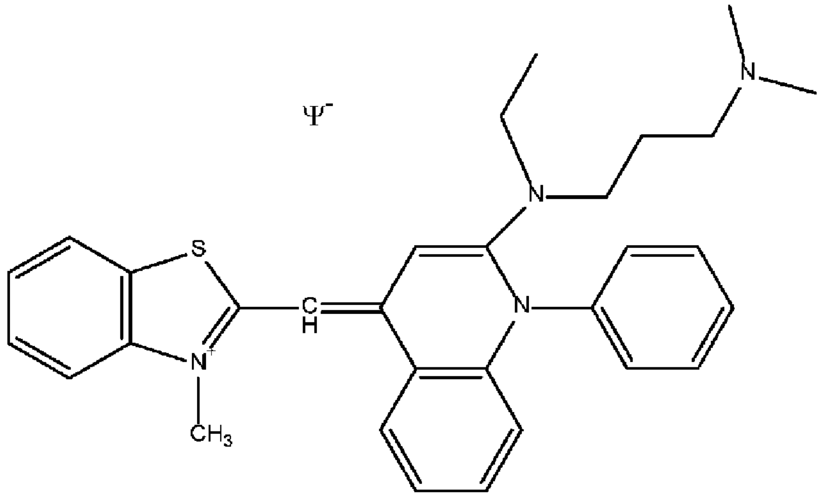

,

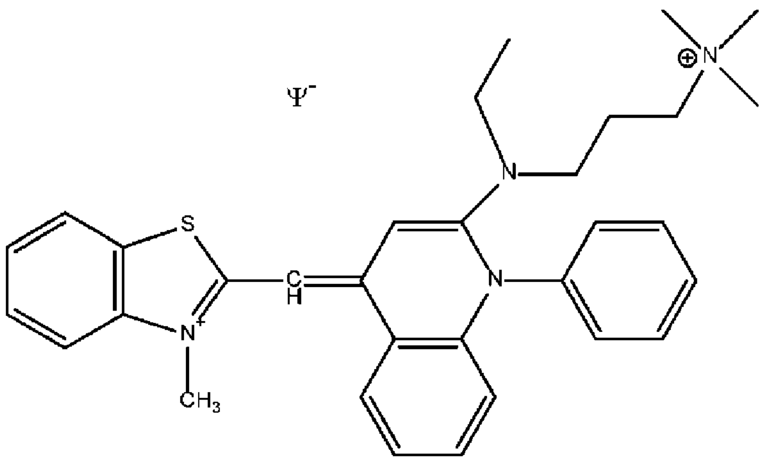

,

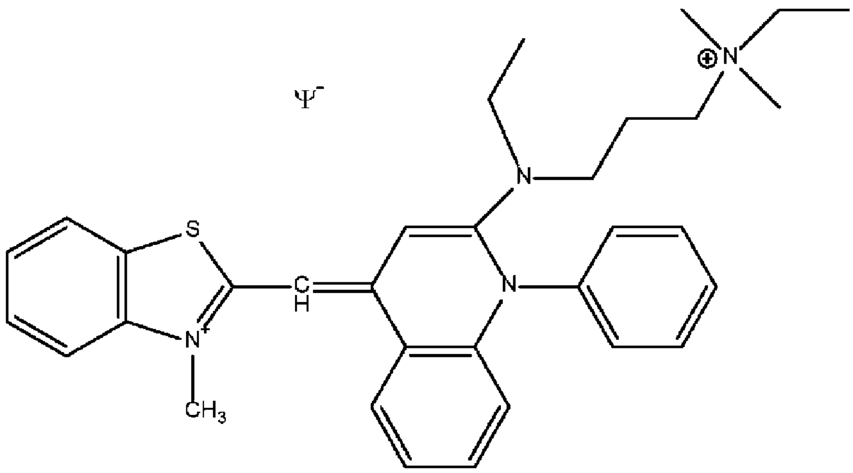

,

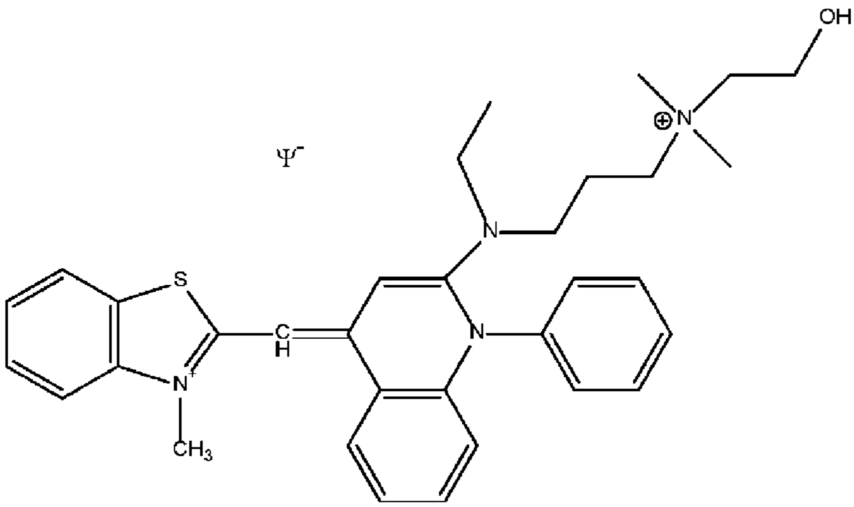

,

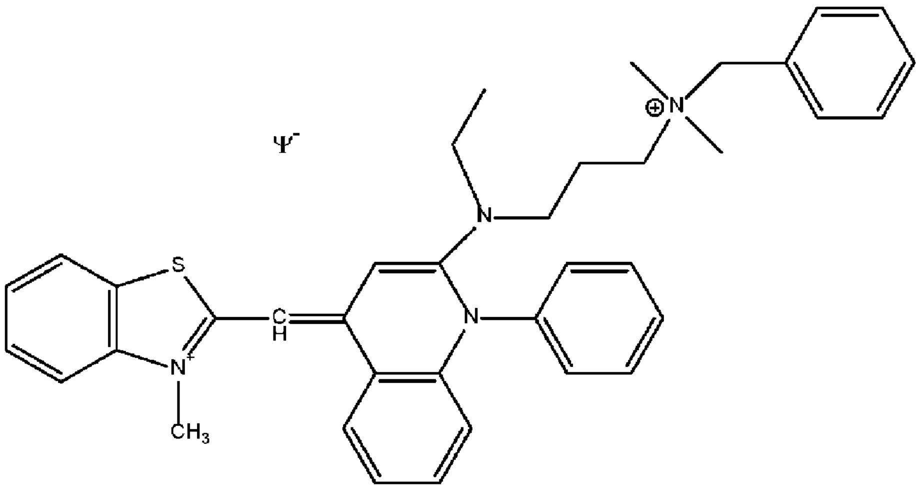

,

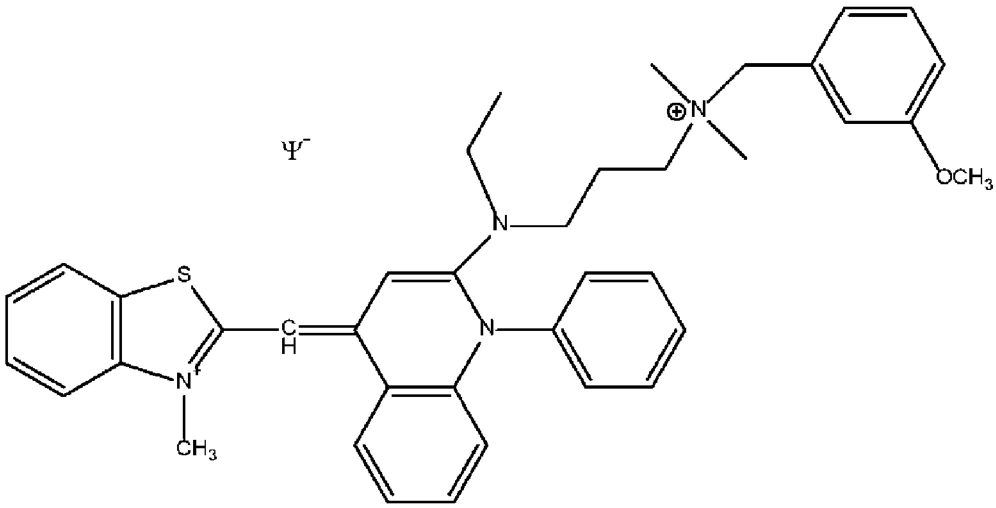

,

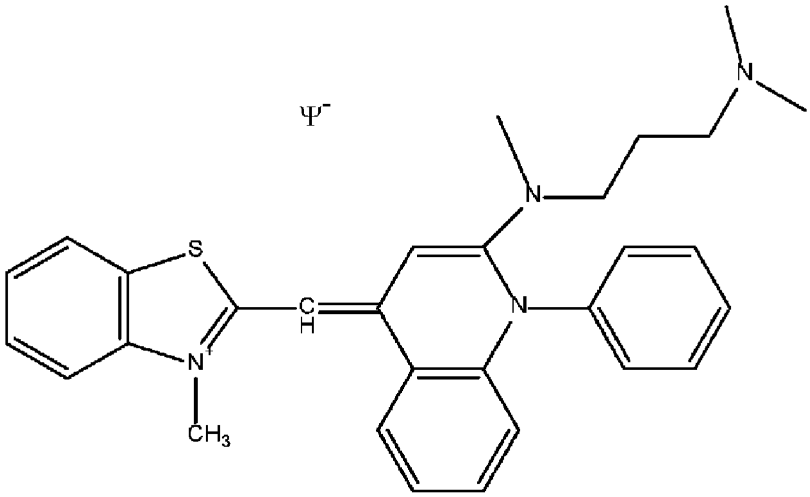

,

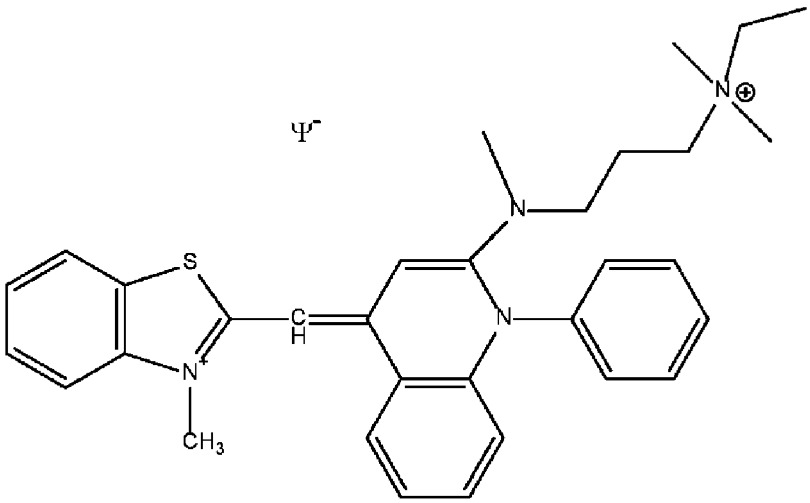

,

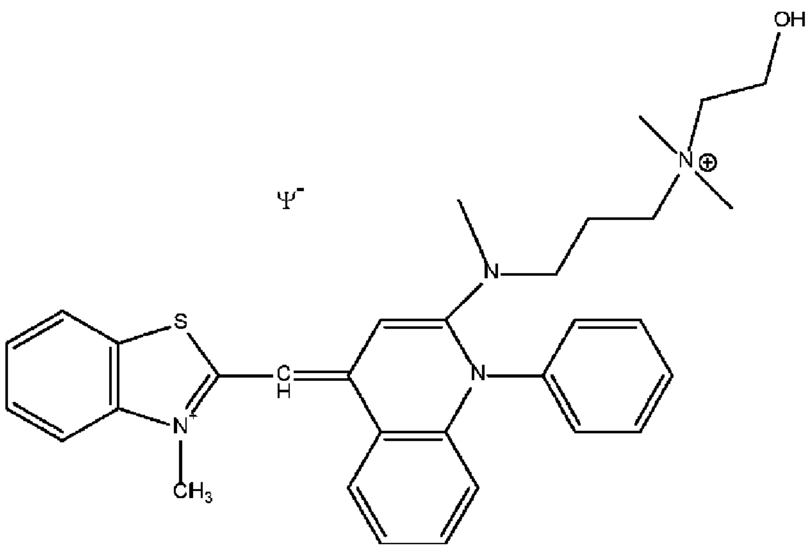

,

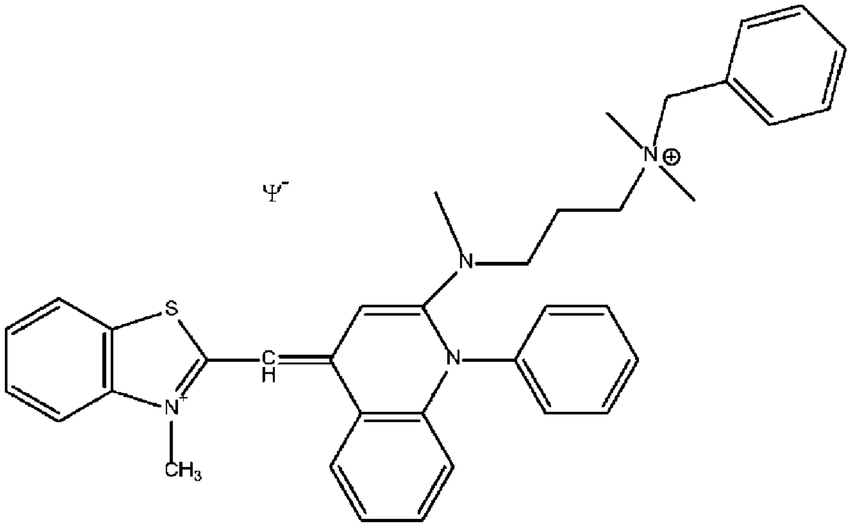

,

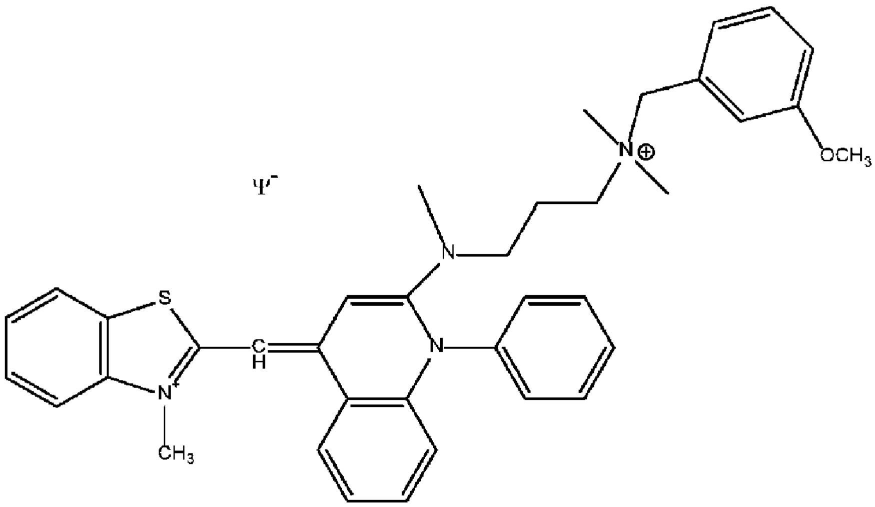

,

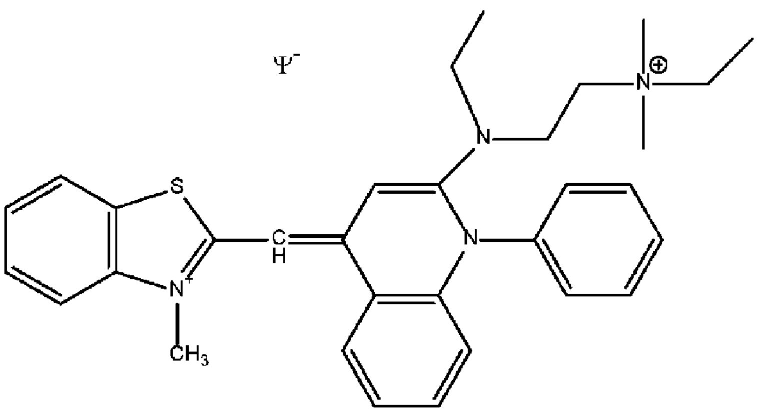

,

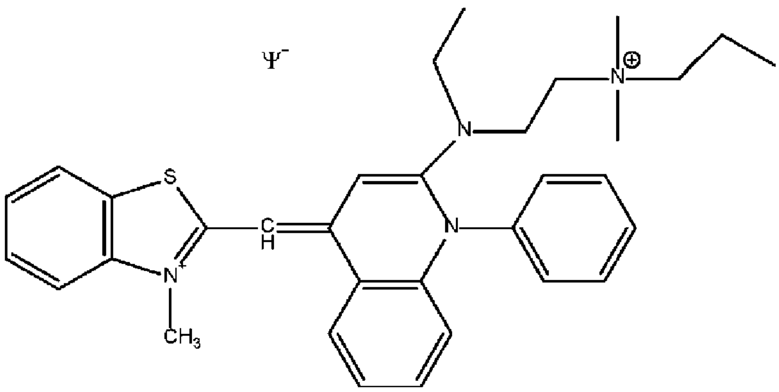

,

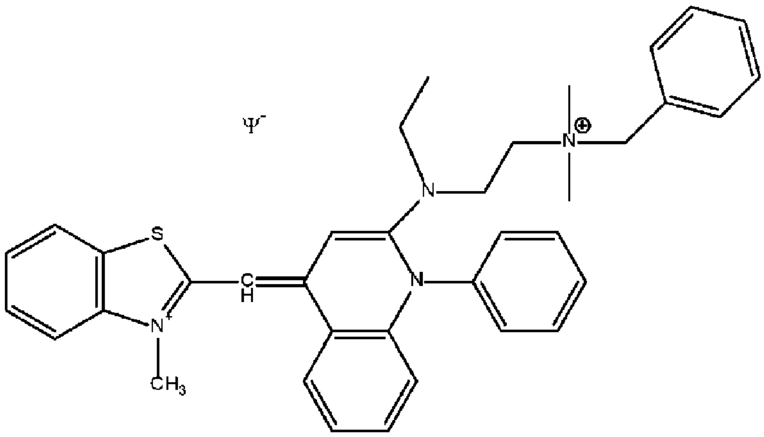

,

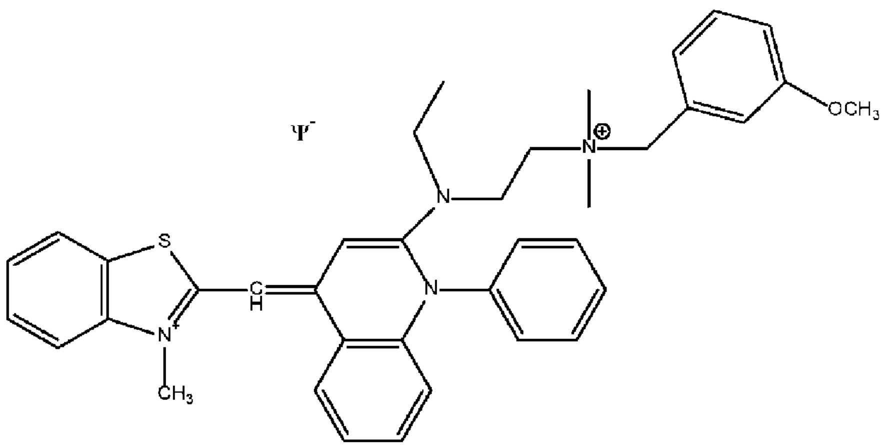

,

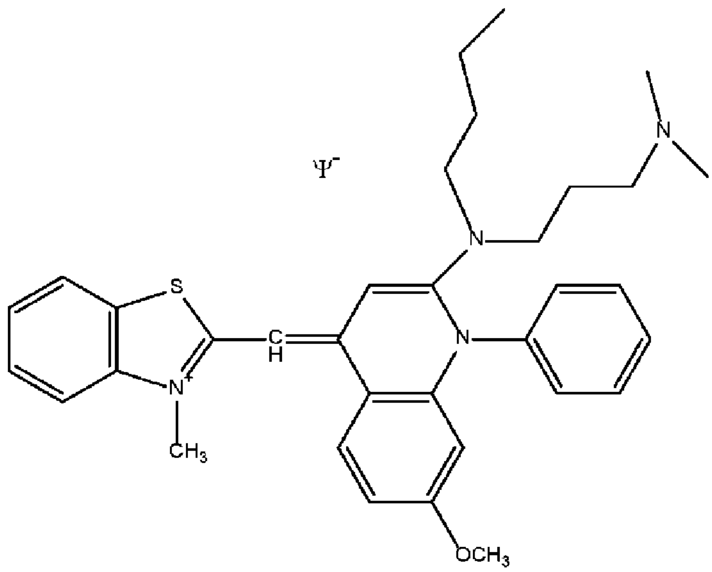

,

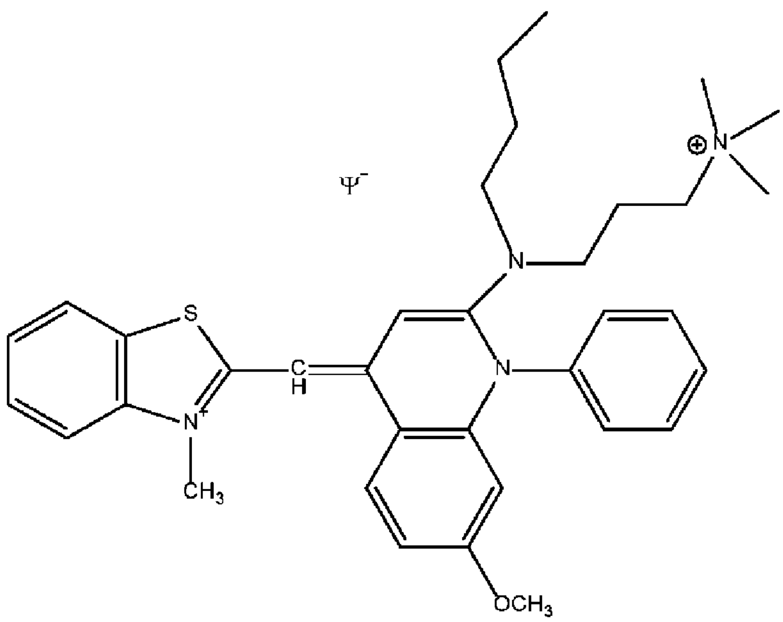

,

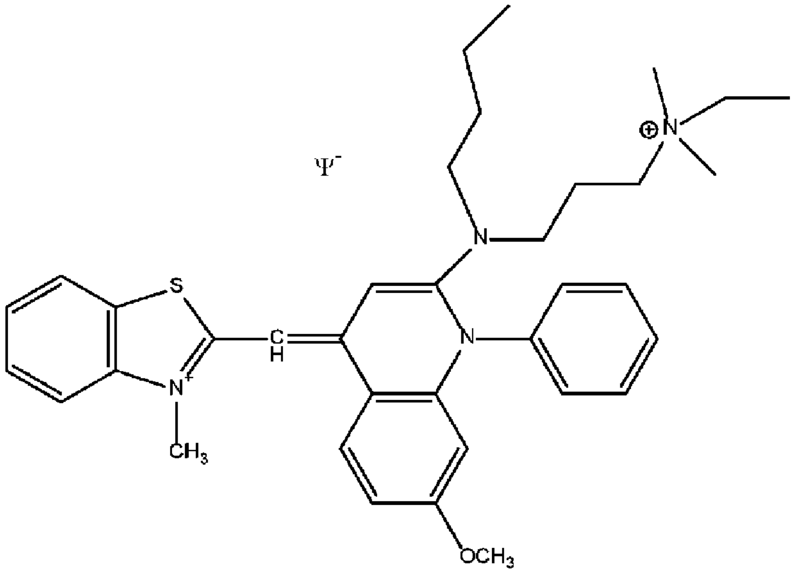

,

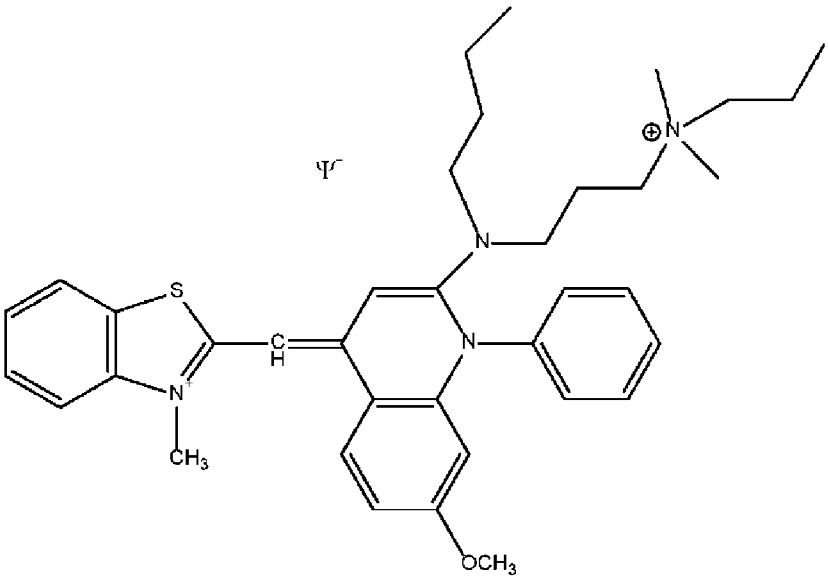

,

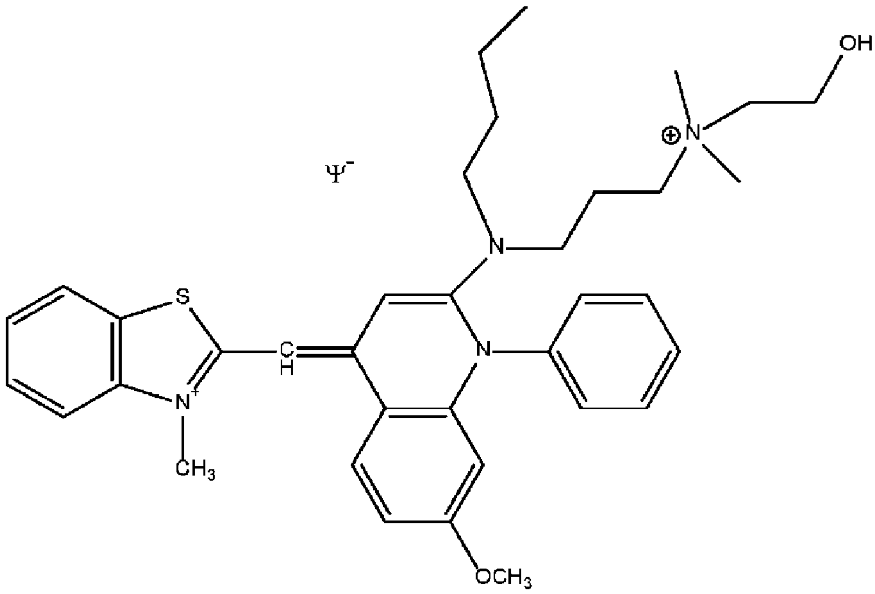

,

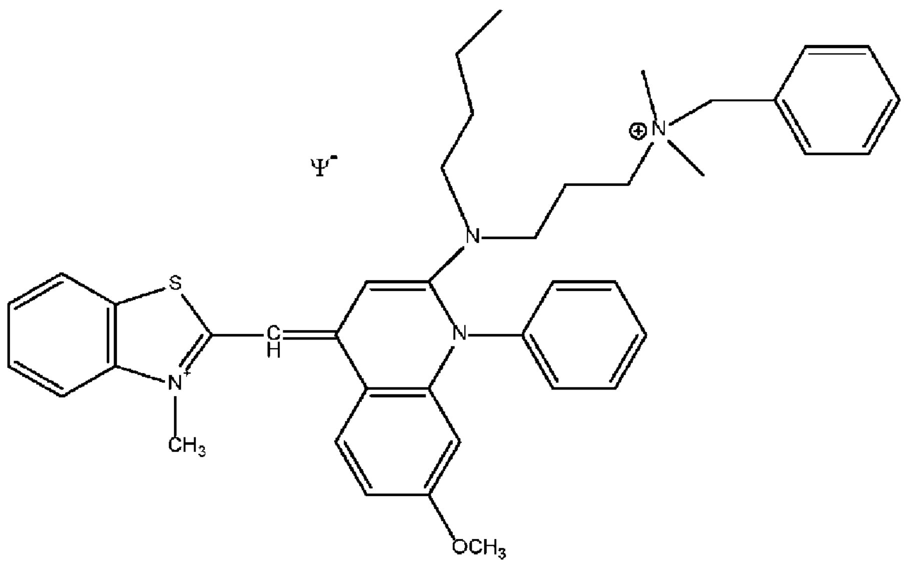

,

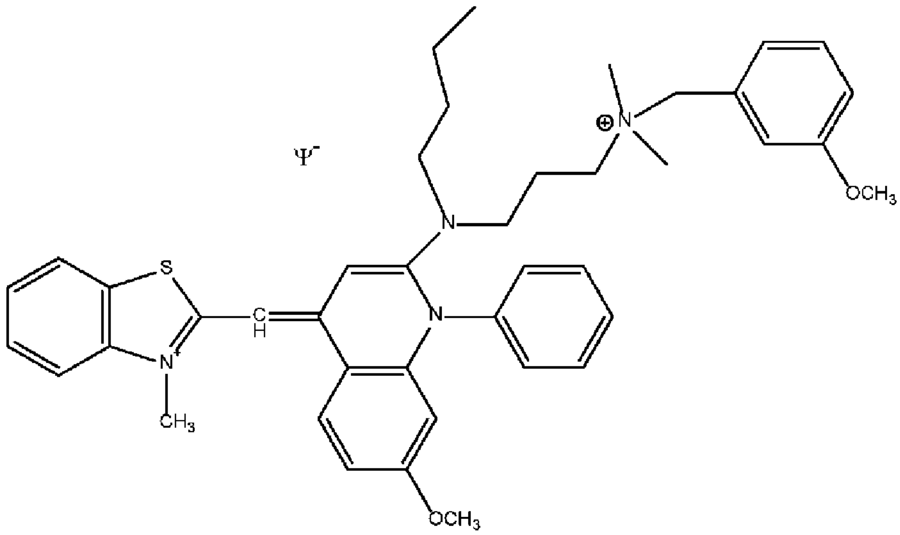

,

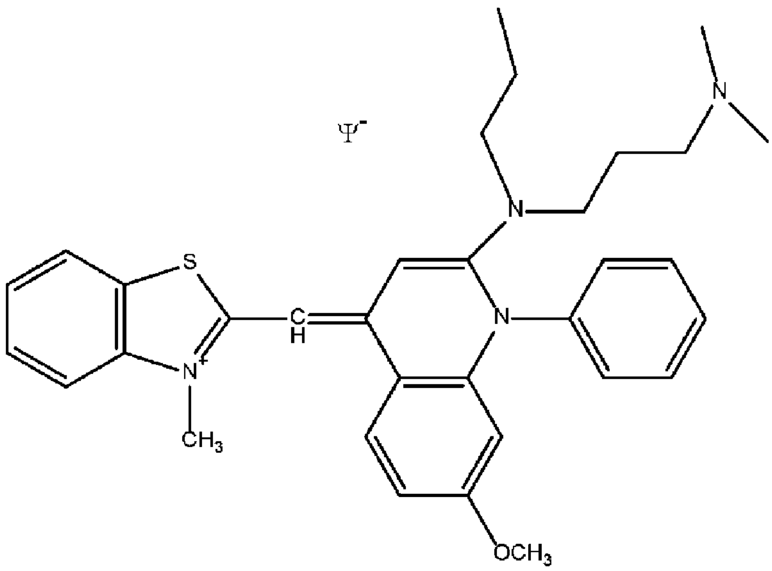

,

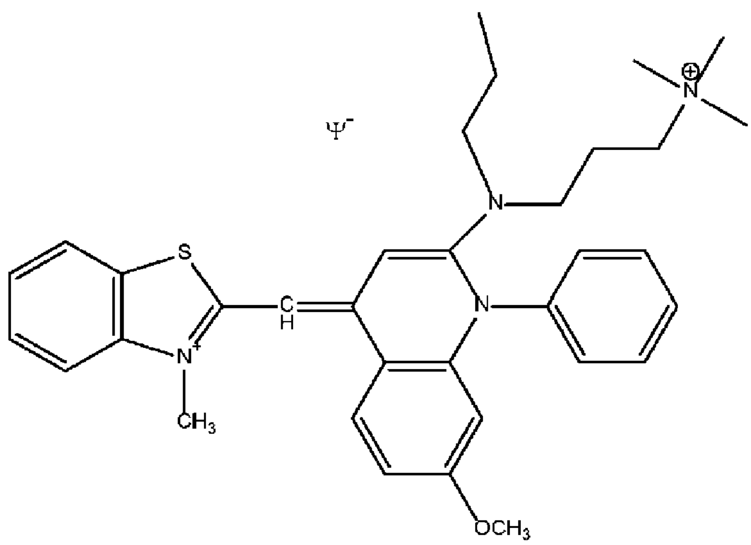

,

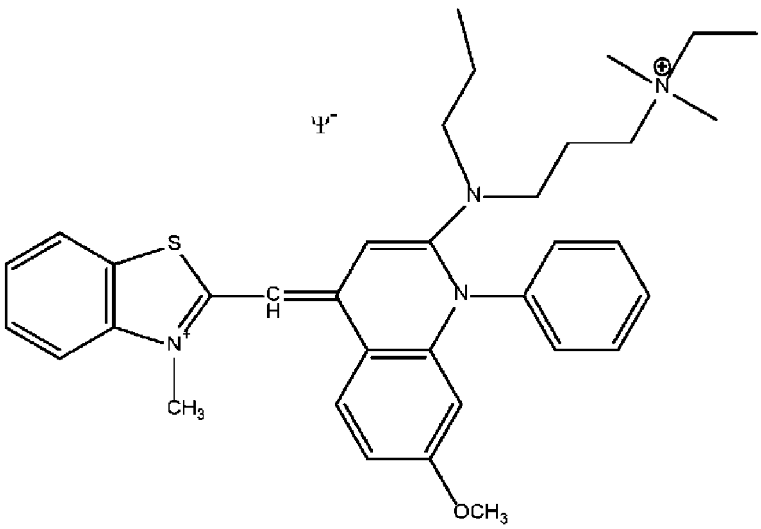

,

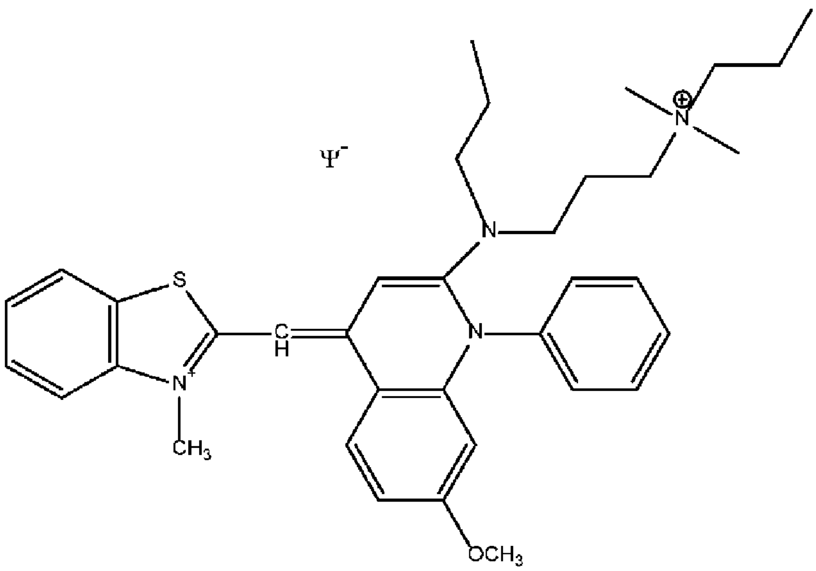

,

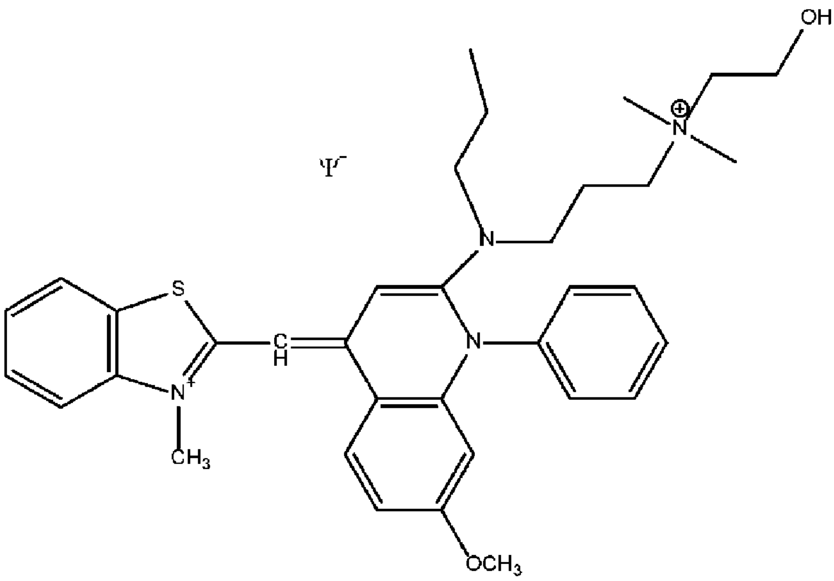

,

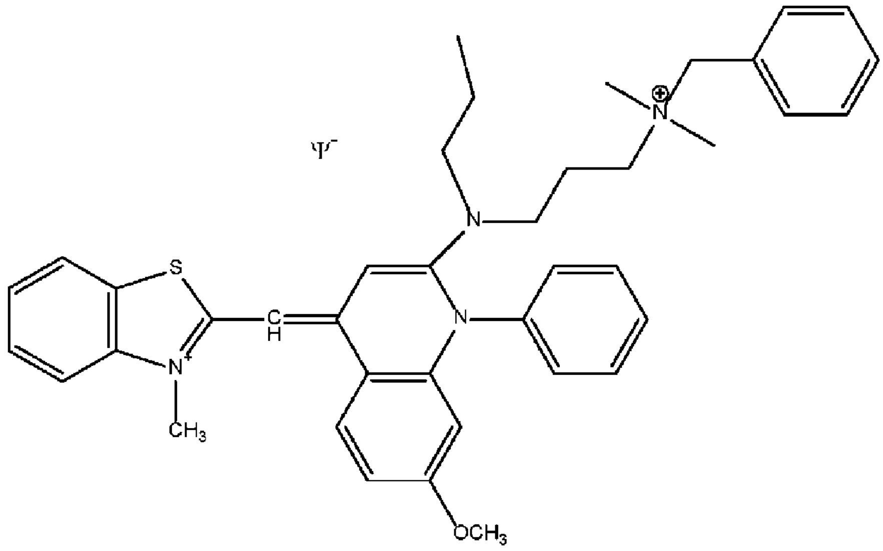

,

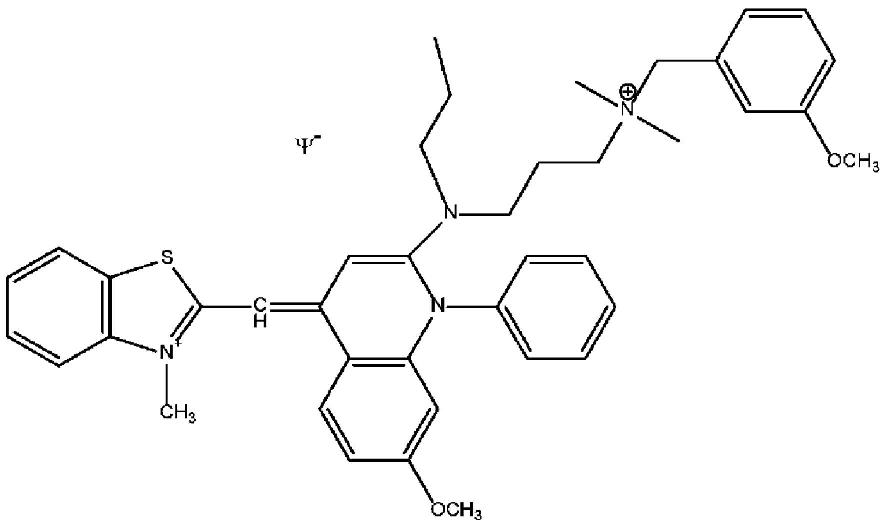

,

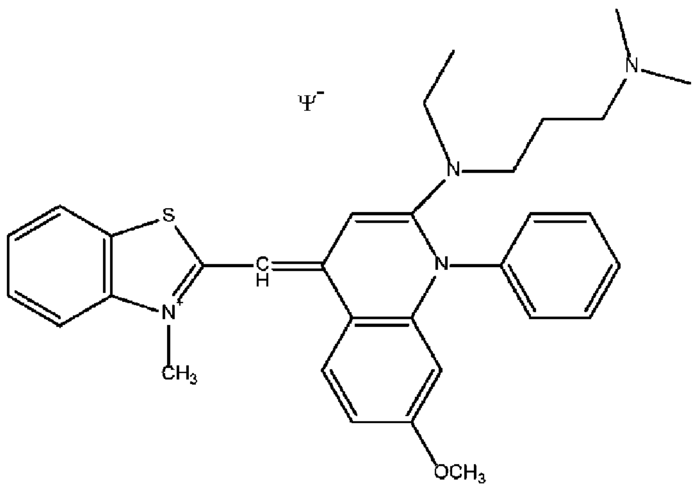

,

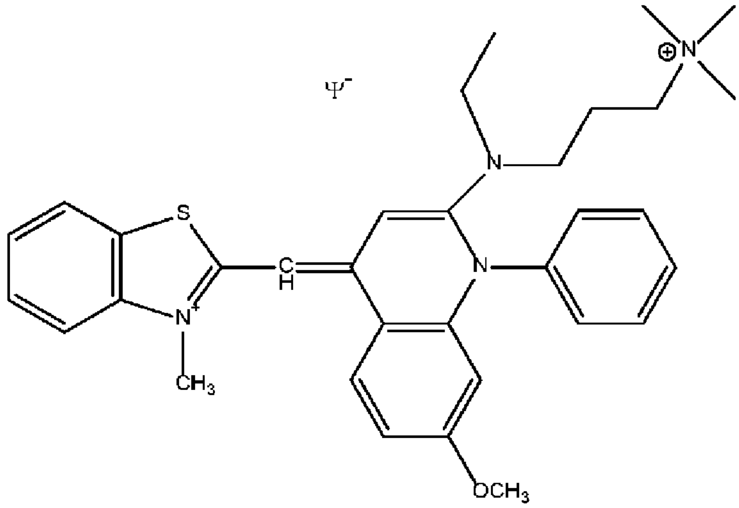

,

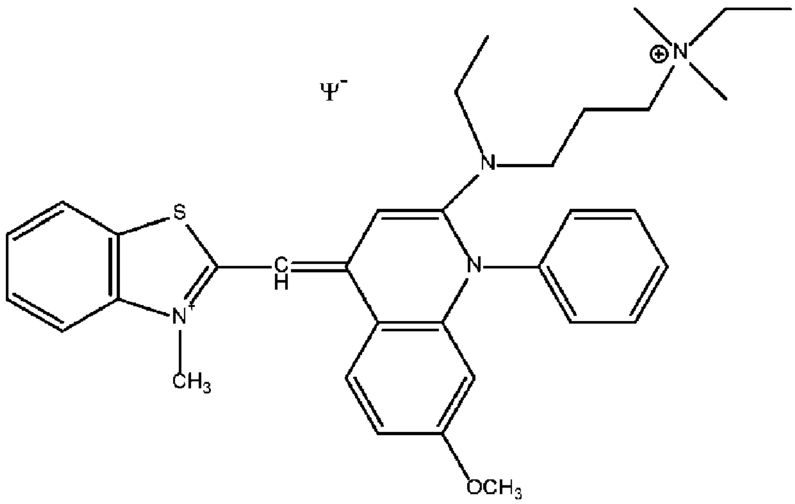

,

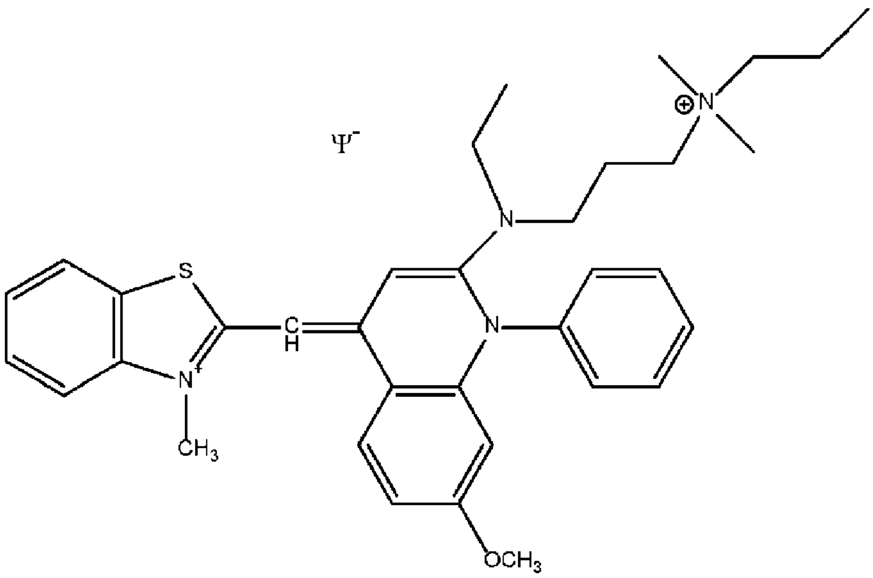

,

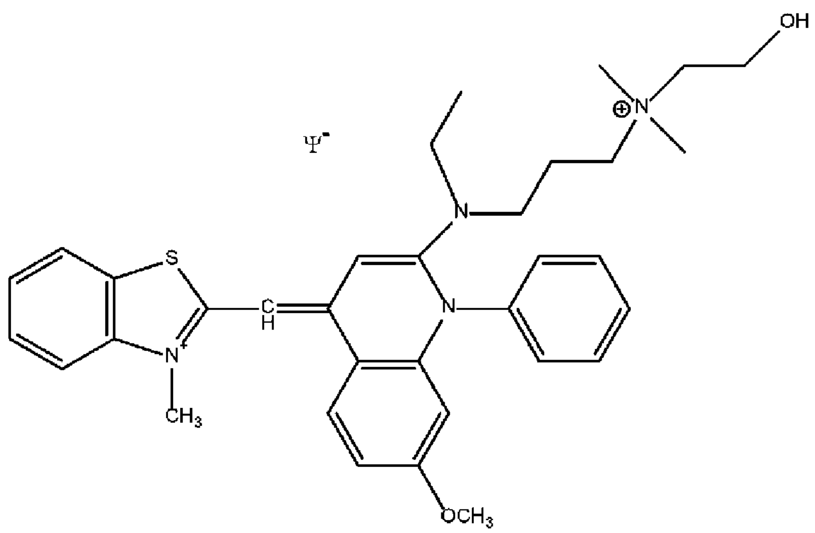

,

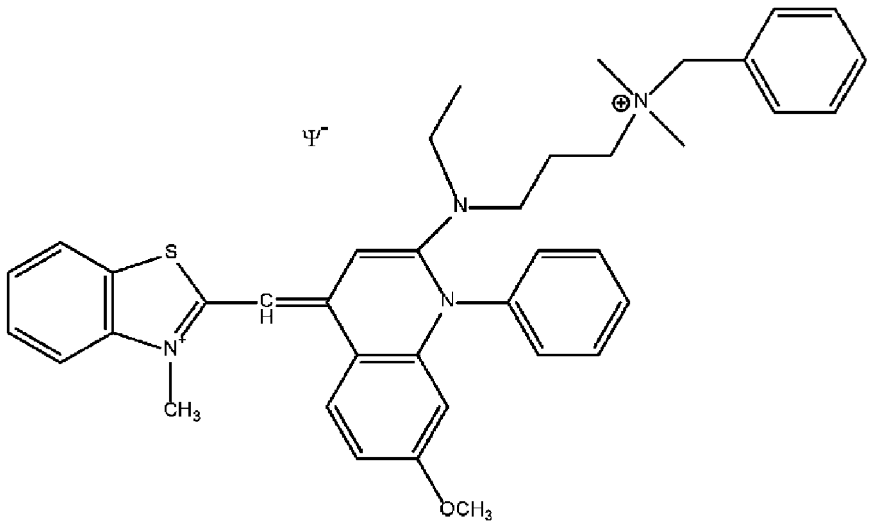

,

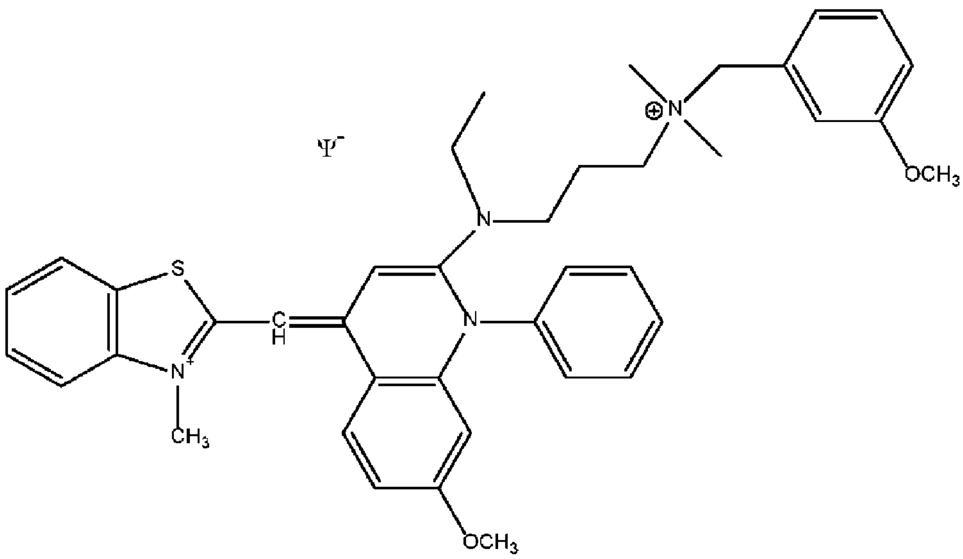

,

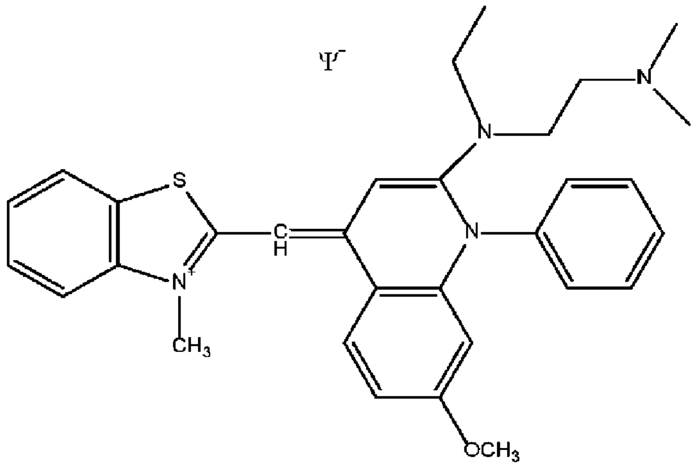

,

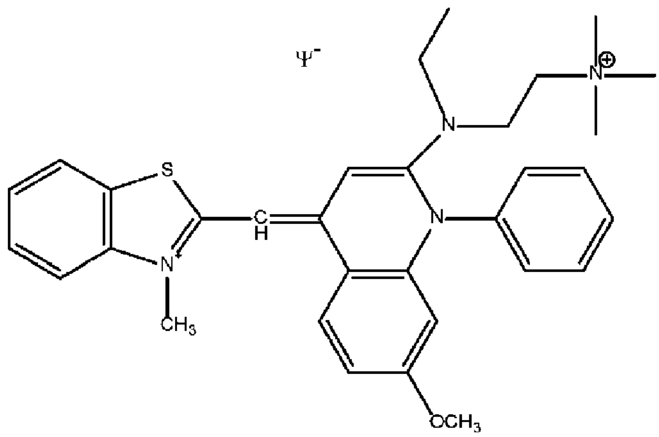

,

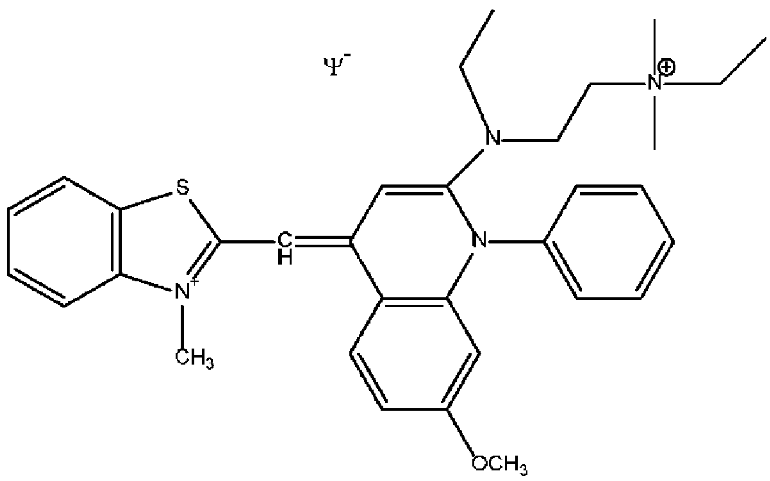

,

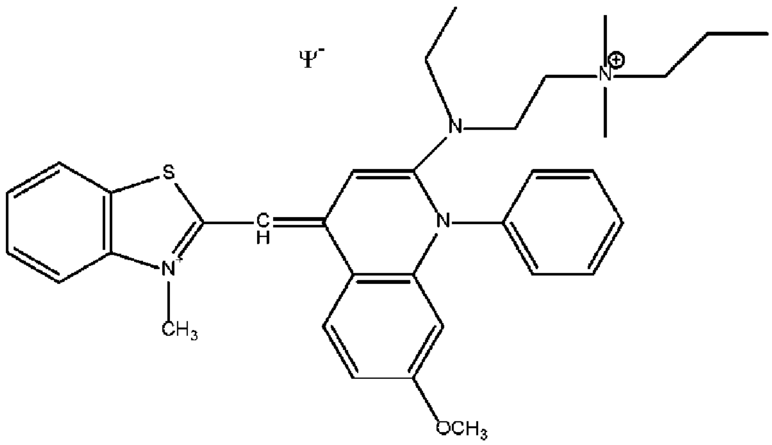

,

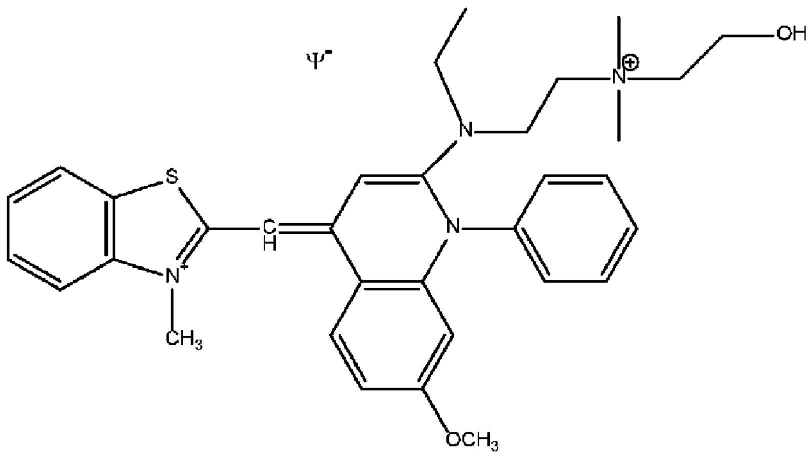

et

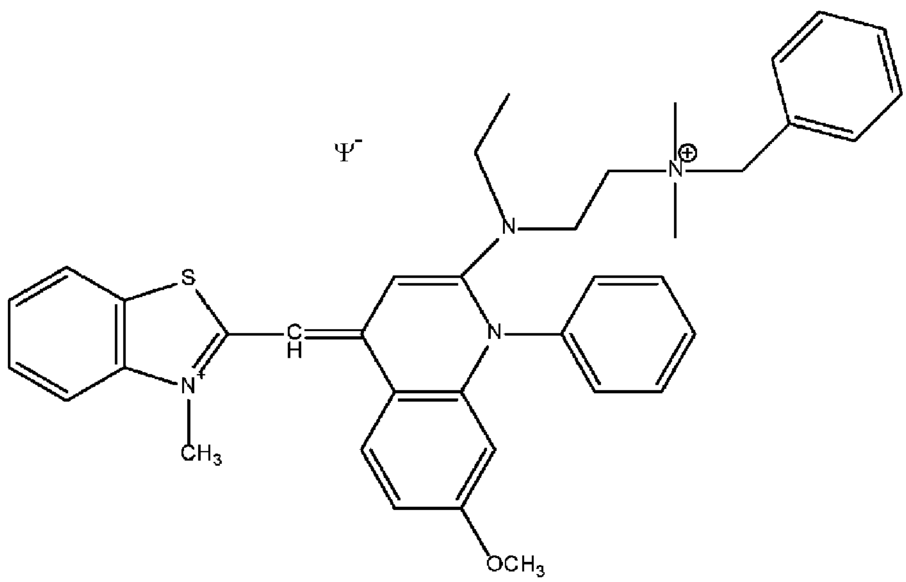

.

, dans lequel Ψ est un contre-ion biologiquement compatible

et dans lequel le second colorant de liaison d'acide nucléique est choisi parmi :

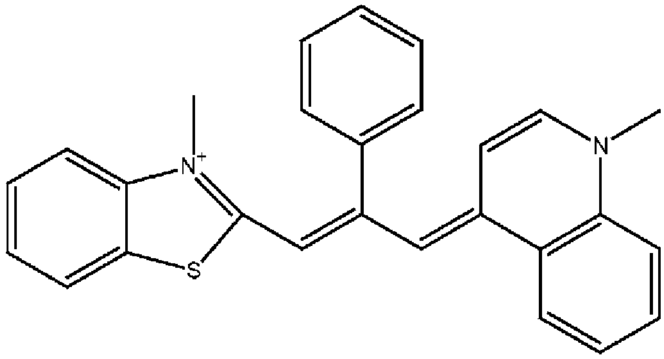

,

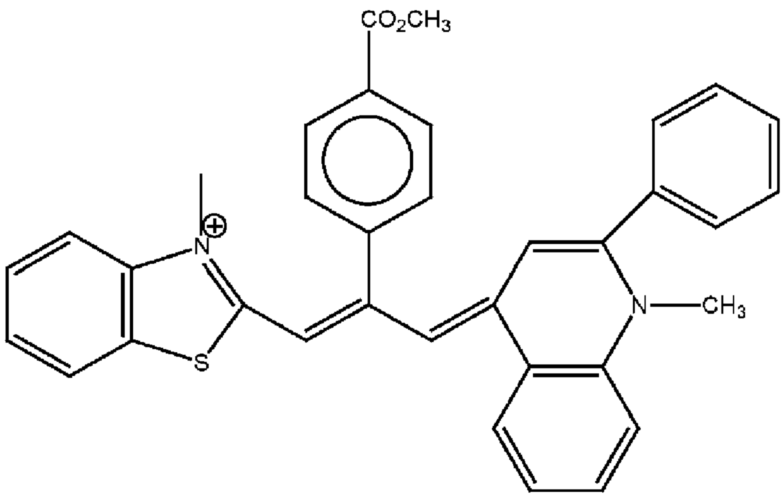

,

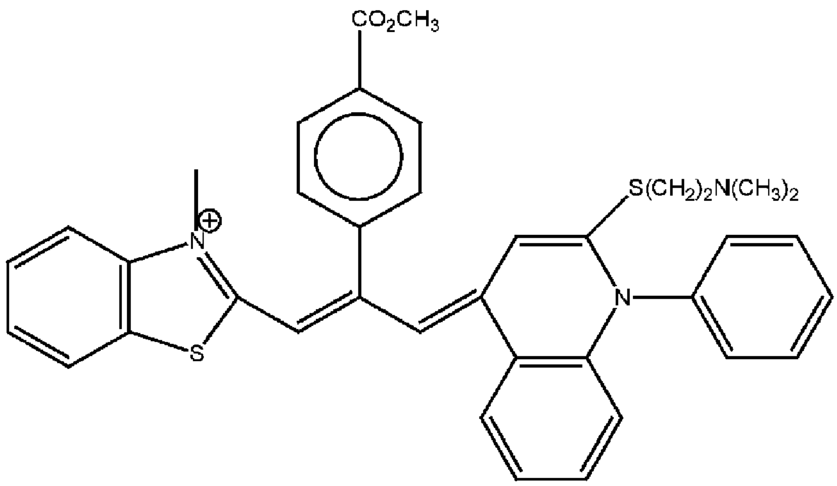

,

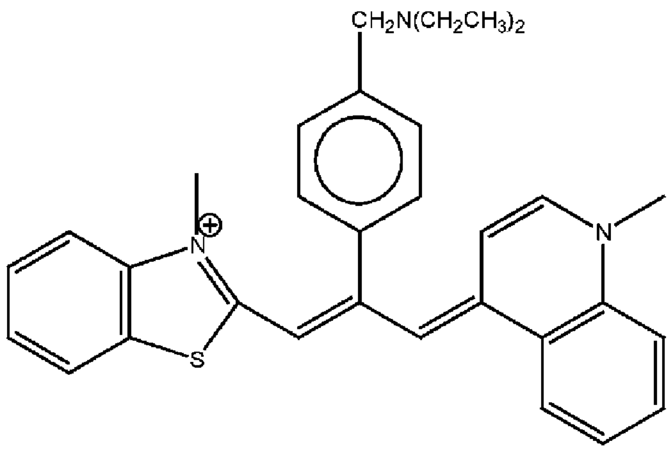

,

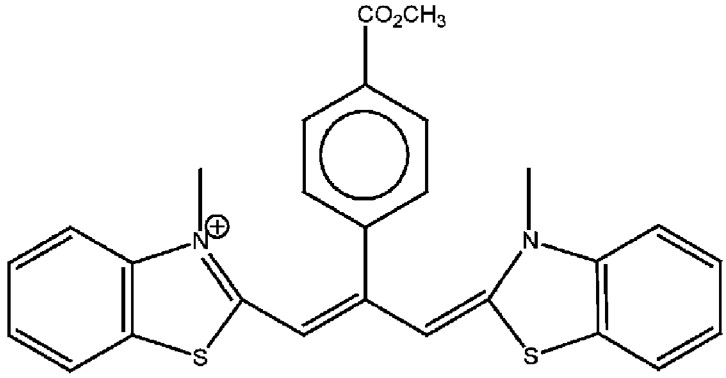

,

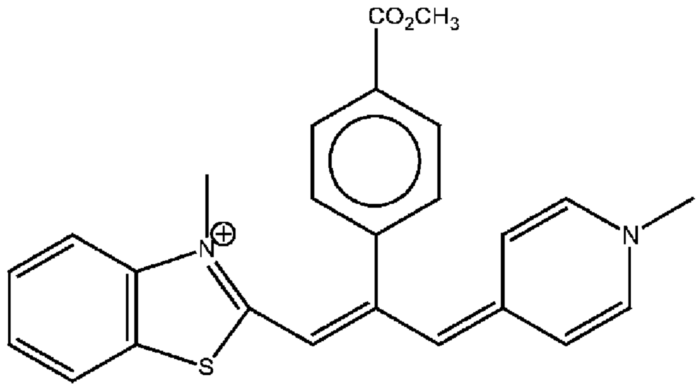

,

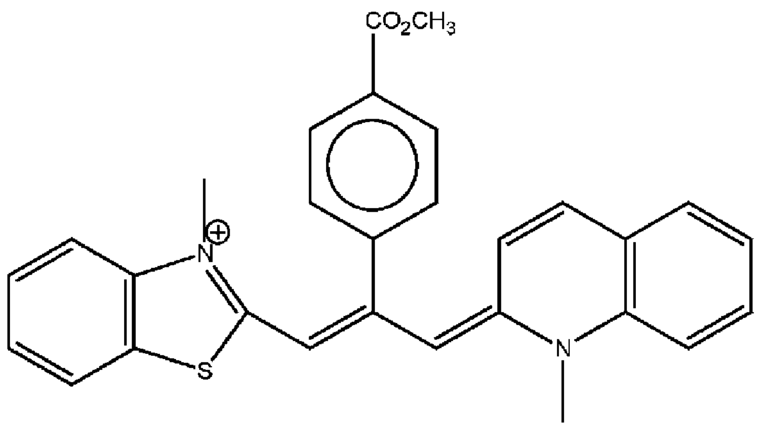

,

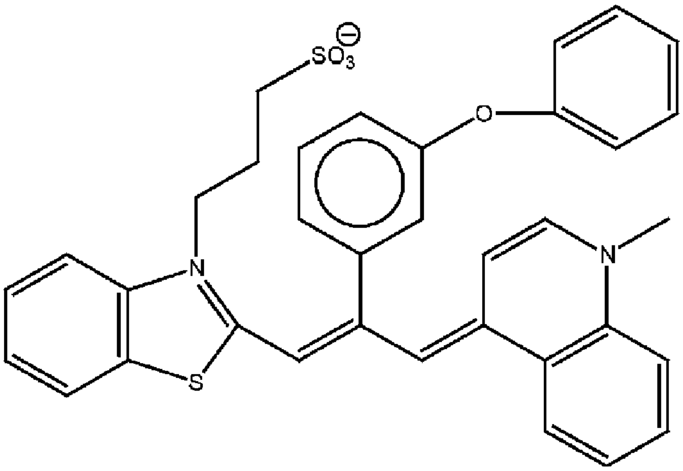

,

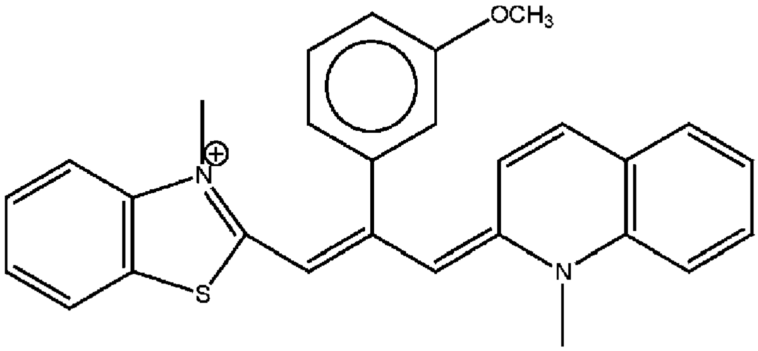

,

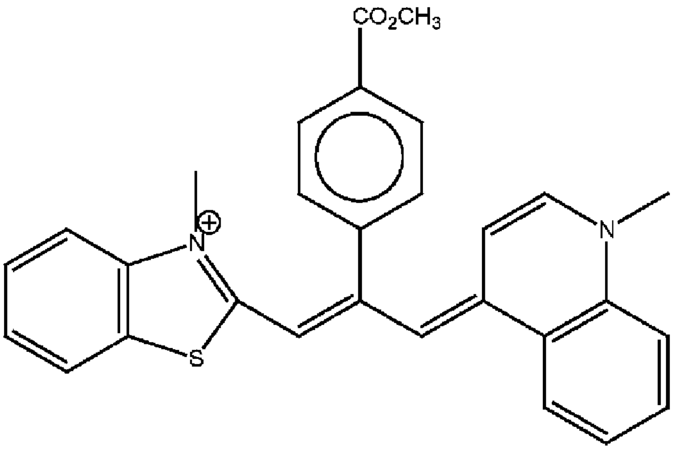

,

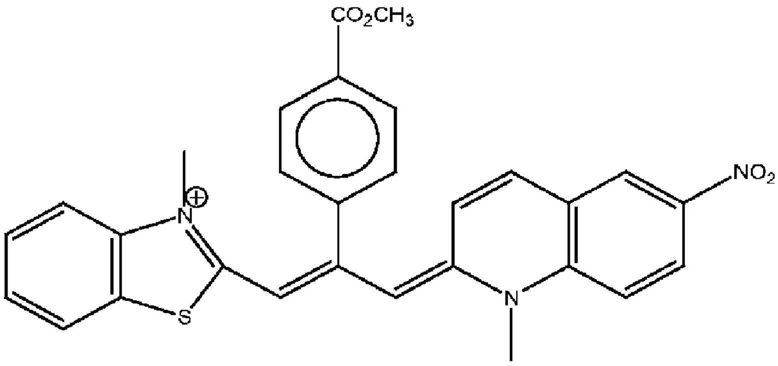

,

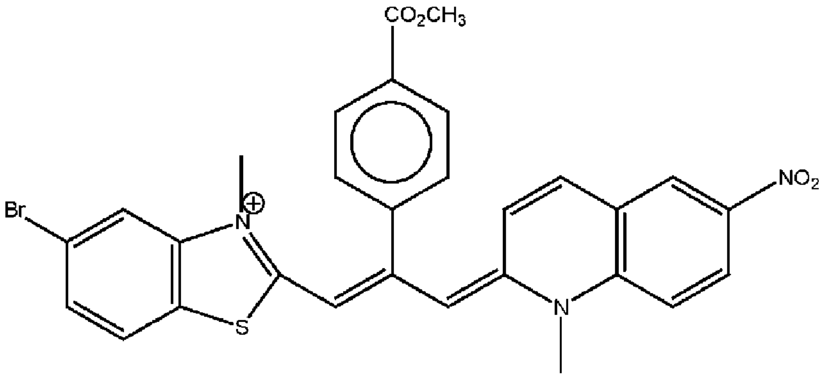

,

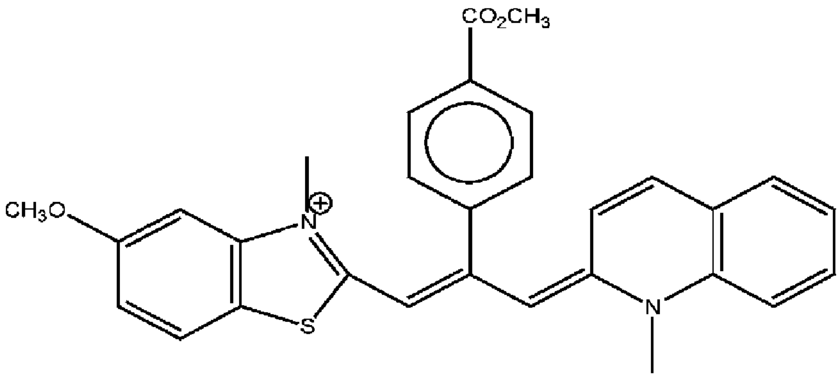

,

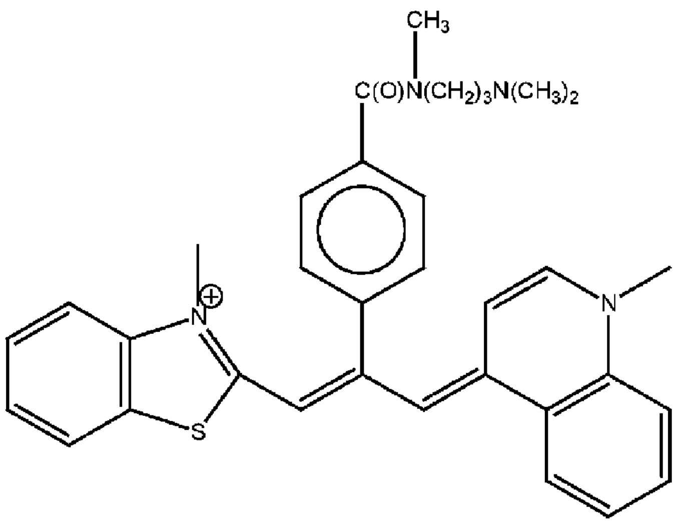

,

et

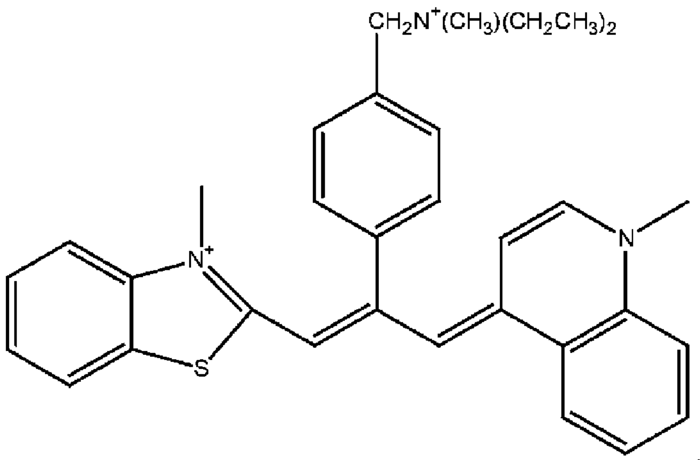

2. Procédé de détermination de l'étendue de dégradation d'ARN dans un échantillon, exprimée en tant que valeur de qualité d'ARN, le procédé comprenant :

a) la combinaison d'un échantillon soupçonné de contenir de l'ARN avec un premier colorant de liaison d'acide nucléique qui se lie sélectivement à un petit ARN comprenant 200 nucléotides ou moins et un second colorant de liaison d'acide nucléique qui se lie sélectivement à un ARN intact avec une structure secondaire et/ou tertiaire, dans lequel le premier colorant de liaison d'acide nucléique et le second colorant de liaison d'acide nucléique sont distincts de manière détectable ;
b) l'incubation de l'échantillon pendant une période de temps suffisante pour permettre au premier colorant de liaison d'acide nucléique et au second colorant de liaison d'acide nucléique de se combiner avec l'ARN dans l'échantillon pour former un complexe premier colorant de liaison d'acide nucléique-ARN et un complexe second colorant de liaison d'acide nucléique-ARN ;
c) l'éclairage du complexe premier colorant de liaison d'acide nucléique-ARN et du complexe second colorant de liaison d'acide nucléique-ARN avec une longueur d'onde appropriée de lumière pour former un mélange d'échantillon éclairé, dans lequel le complexe premier colorant de liaison d'acide nucléique-ARN émet un premier signal fluorescent et le complexe second colorant de liaison d'acide nucléique-ARN émet un second signal fluorescent ;
d) la détection du premier signal fluorescent et du second signal fluorescent ; et
e) le calcul d'une relation de l'intensité du premier signal fluorescent à l'intensité du second signal fluorescent, permettant ainsi d'obtenir la valeur de qualité d'ARN, dans lequel la valeur de qualité d'ARN représente l'étendue de dégradation d'ARN dans l'échantillon,

dans lequel les premier et second colorants de liaison d'acide nucléique sont tels que définis dans la revendication 1.

3. Procédé selon **la revendication 1 ou 2,** dans lequel l'ARN intact avec une structure secondaire et/ou tertiaire est un ARNr.

4. Procédé selon **la revendication 1 ou 2,** dans lequel le premier colorant de liaison d'acide nucléique et le second colorant de liaison d'acide nucléique sont ajoutés à l'échantillon simultanément ou dans lequel le premier colorant de liaison d'acide nucléique et le second colorant de liaison d'acide nucléique sont ajoutés à l'échantillon séquentiellement.

5. Procédé selon **la revendication 1 ou 2,** dans lequel l'ARN intact avec une structure secondaire et/ou tertiaire est ARNt.

6. Procédé selon **la revendication 1 ou 2,** dans lequel l'ARN intact avec une structure secondaire et/ou tertiaire est un ARNm.

7. Procédé selon **l'une quelconque des revendications précédentes,** dans lequel le second colorant de liaison d'acide nucléique a un rapport d'amélioration de fluorescence ARN/ADN ≥ 7, ≥ 10 ou ≥ 20.

8. Procédé selon **l'une quelconque des revendications précédentes,** dans lequel le premier colorant de liaison d'acide nucléique a une amélioration de fluorescence d'ARN simple brin ≥ 30 %.

9. Procédé selon **l'une quelconque des revendications précédentes,** dans lequel l'étape de détection est effectuée par fluorométrie.

10. Procédé selon **la revendication 1**, comprenant en outre
la préparation :

d'une première solution étalon en combinant le premier colorant de liaison d'acide nucléique et le second colorant de liaison d'acide nucléique avec un blanc de solution tampon ;
d'une deuxième solution étalon en combinant le premier colorant de liaison d'acide nucléique et le second colorant de liaison d'acide nucléique avec un étalon de petit ARN ; et
une troisième solution étalon en combinant le premier colorant de liaison d'acide nucléique et le second colorant de liaison d'acide nucléique avec un ARN intact avec un étalon de structure secondaire et/ou tertiaire ;
l'incubation de la première solution étalon, de la deuxième solution étalon et de la troisième solution étalon pendant une durée suffisante pour que le premier colorant de liaison d'acide nucléique et le second colorant de liaison d'acide nucléique se combinent avec l'ARN dans l'étalon, s'il est présent, pour former un premier complexe étalon-colorant, un deuxième complexe étalon-colorant et un troisième complexe étalon-colorant ;
l'éclairage du premier complexe étalon-colorant, du deuxième complexe étalon-colorant et du troisième complexe étalon-colorant avec une longueur d'onde appropriée de lumière pour former un premier mélange étalon éclairé, un deuxième mélange étalon éclairé et un troisième mélange étalon éclairé ; et
la détection d'un signal fluorescent provenant de chacun du premier mélange étalon éclairé, du deuxième mélange étalon éclairé et du troisième mélange étalon éclairé, et comprenant facultativement en outre la détermination de l'étendue de dégradation de l'ARN présent dans le mélange d'échantillon éclairé sur la base de la comparaison du signal fluorescent détectable dans le mélange d'échantillon éclairé avec un rapport du signal fluorescent de la deuxième solution étalon et du signal fluorescent de la troisième solution étalon.

11. Trousse comprenant :

une solution de colorant comprenant un premier colorant de liaison d'acide nucléique qui se lie sélectivement à un petit ARN comprenant 200 nucléotides ou moins et un second colorant de liaison d'acide nucléique qui se lie sélectivement à un ARN intact avec une structure secondaire et/ou tertiaire selon la revendication 1 ; et
des instructions pour une utilisation conformément aux procédés selon **l'une quelconque des revendications précédentes**
dans laquelle le premier colorant de liaison d'acide nucléique et le second colorant de liaison d'acide nucléique sont distincts de manière détectable et comprenant facultativement en outre :

une première solution étalon comprenant un tampon et aucun acide nucléique ;
une deuxième solution étalon comprenant un étalon de petit ARN, dans laquelle l'étalon de petit ARN consiste en moins de 200 nt ; et
une troisième solution étalon comprenant un ARN intact avec un étalon de structure secondaire et/ou tertiaire.

12. Solution de coloration de qualité d'ARN comprenant :

un premier colorant de liaison d'acide nucléique qui se lie préférentiellement à un petit ARN comprenant 200 nucléotides ou moins ;
un second colorant de liaison d'acide nucléique qui se lie préférentiellement à un ARN intact avec une structure secondaire et/ou tertiaire ;
un tampon ; et
un solvant organique,
dans laquelle le premier colorant de liaison d'acide nucléique et le second colorant de liaison d'acide nucléique sont distincts de manière détectable, et
dans laquelle le premier colorant de liaison d'acide nucléique est

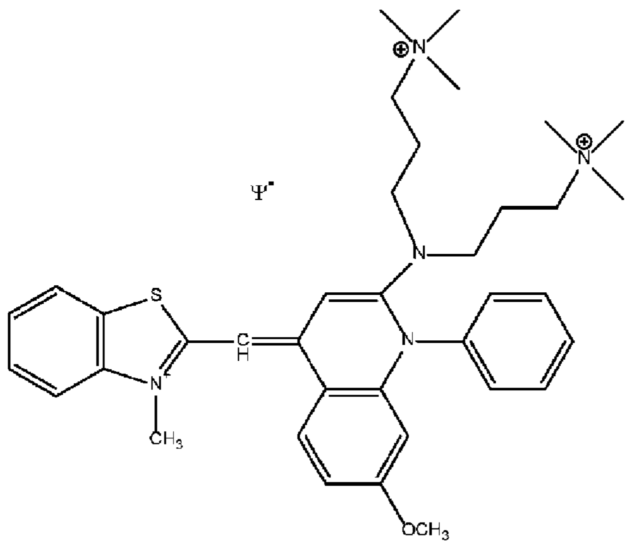

le Composé **16**,

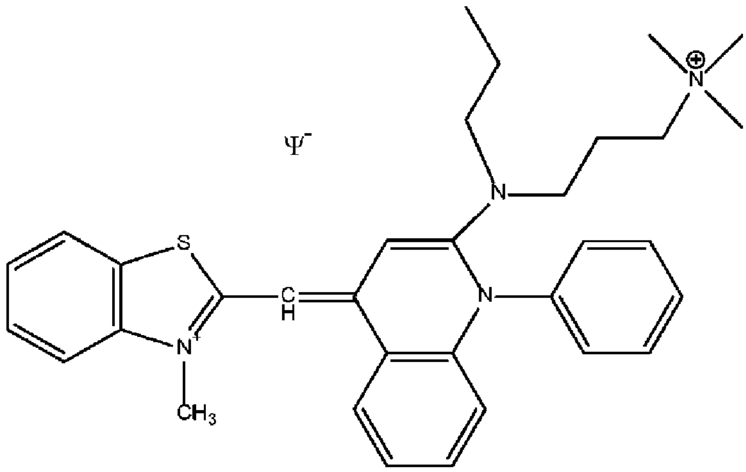

le Composé **17**,

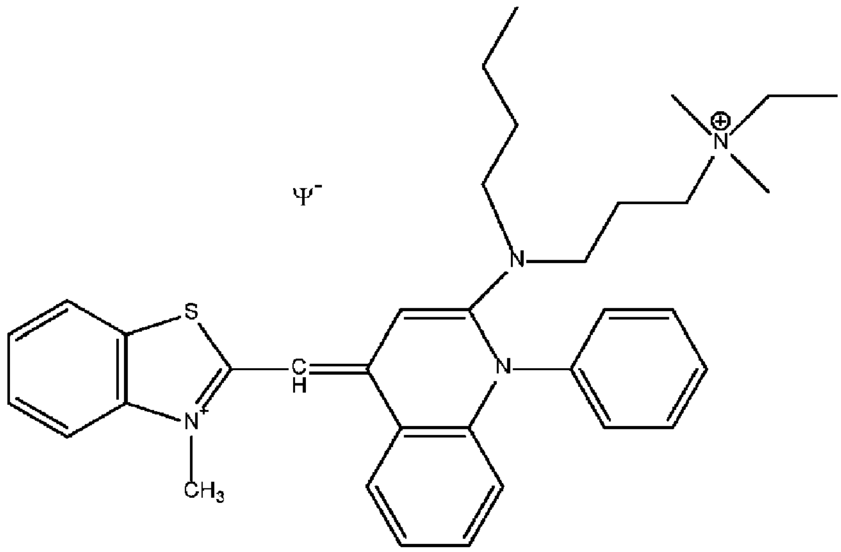

le Composé **23**,

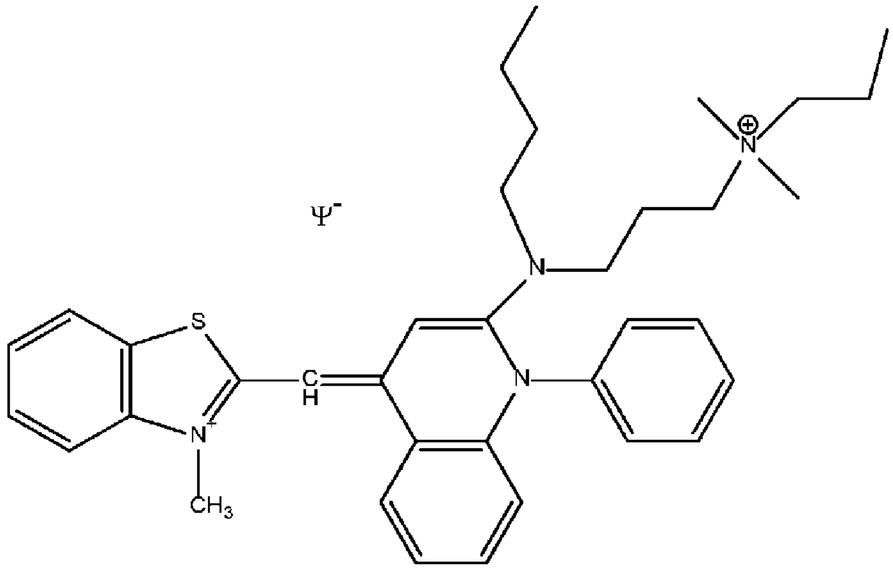

le Composé **24**,

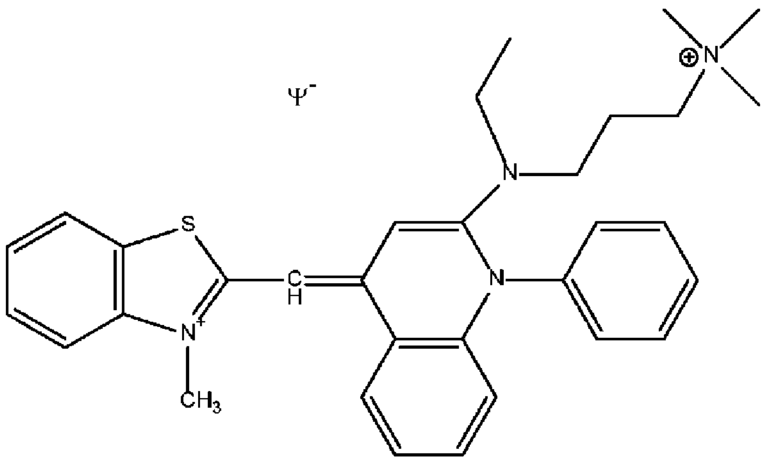

le Composé **29**,

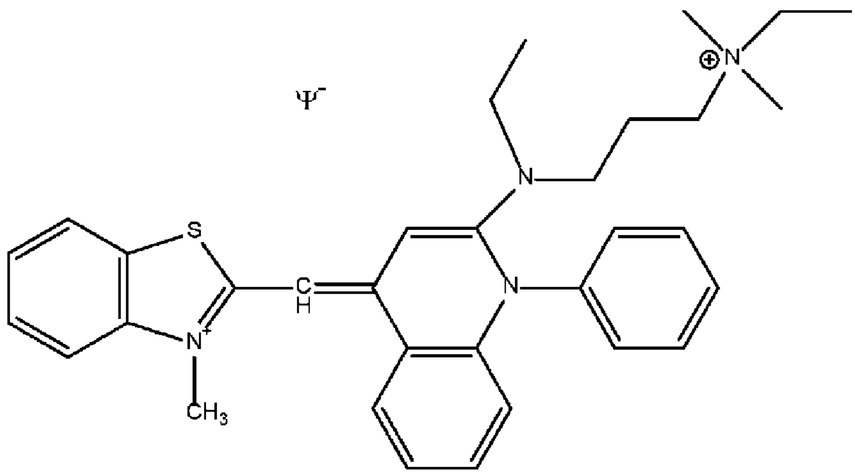

le Composé **30**,

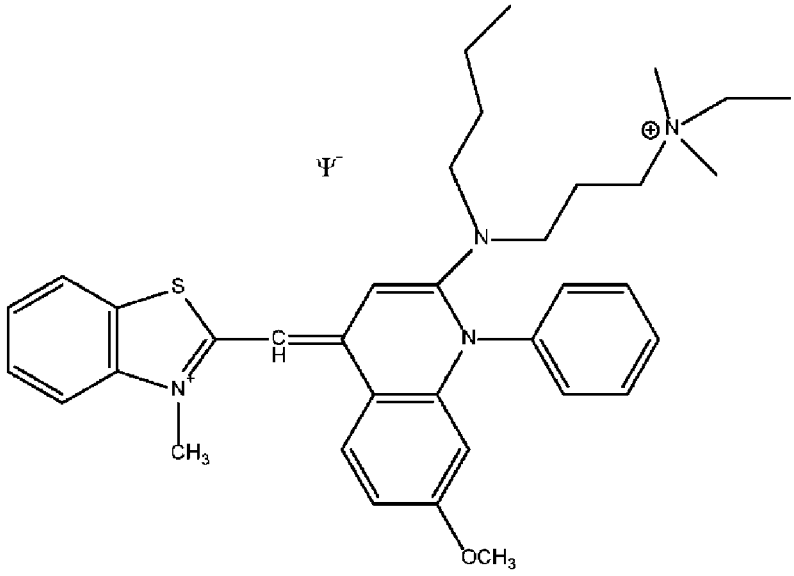

le Composé **45**,

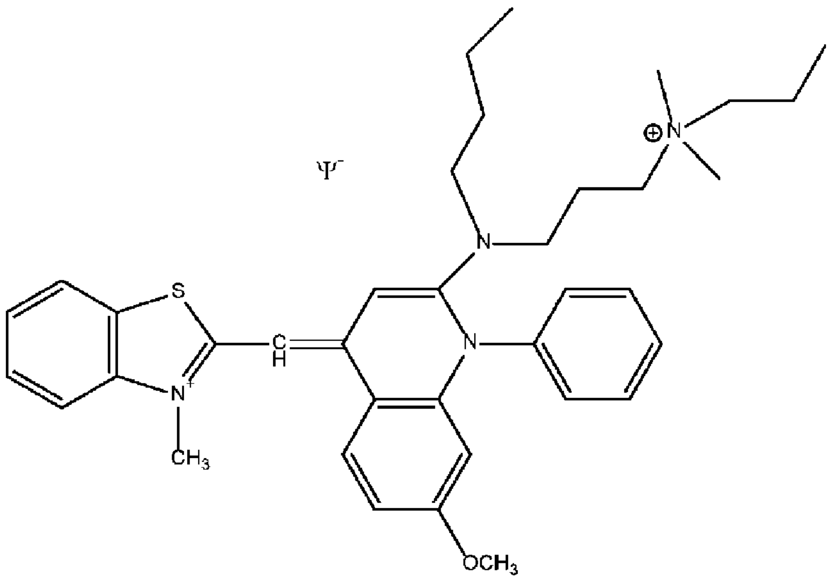

le Composé **46**,

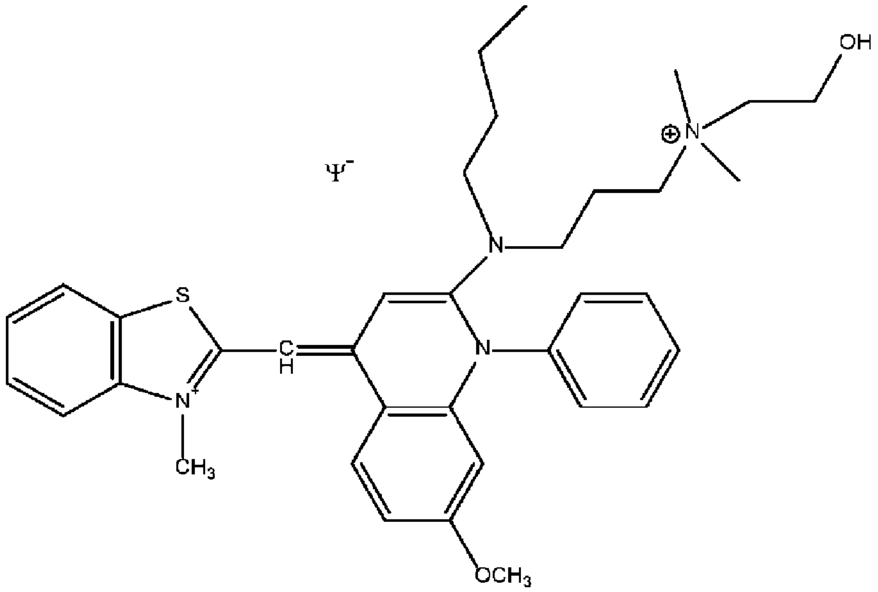

le Composé **47**,

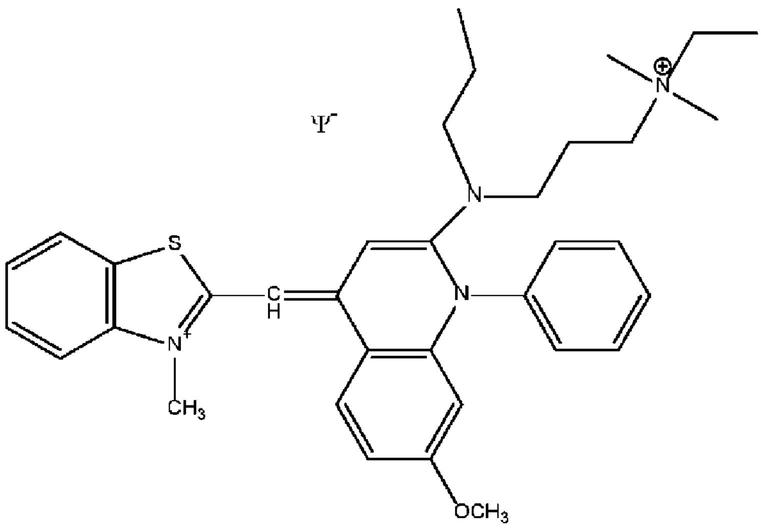

le Composé **52**,

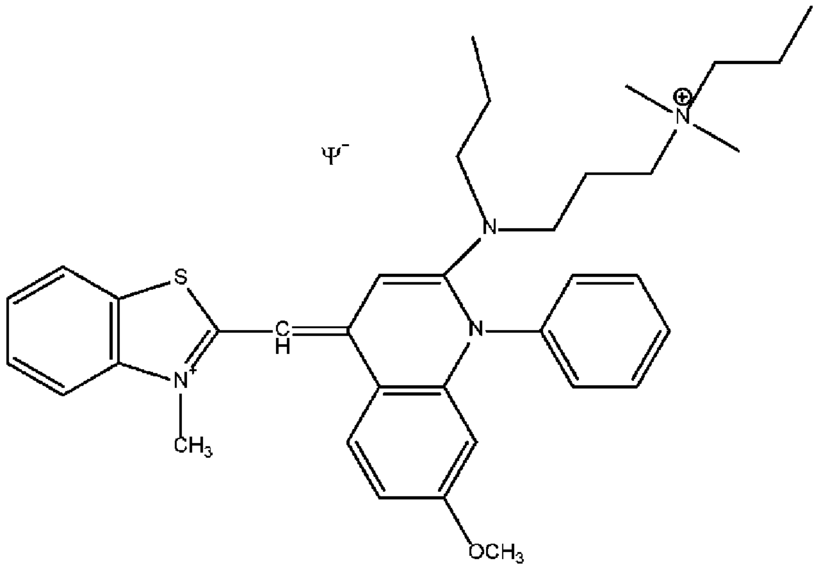

le Composé **53**,

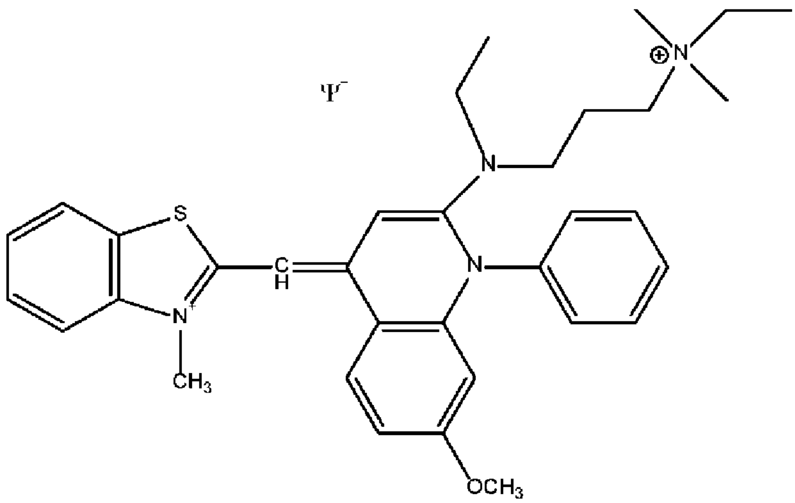

le Composé **59**,

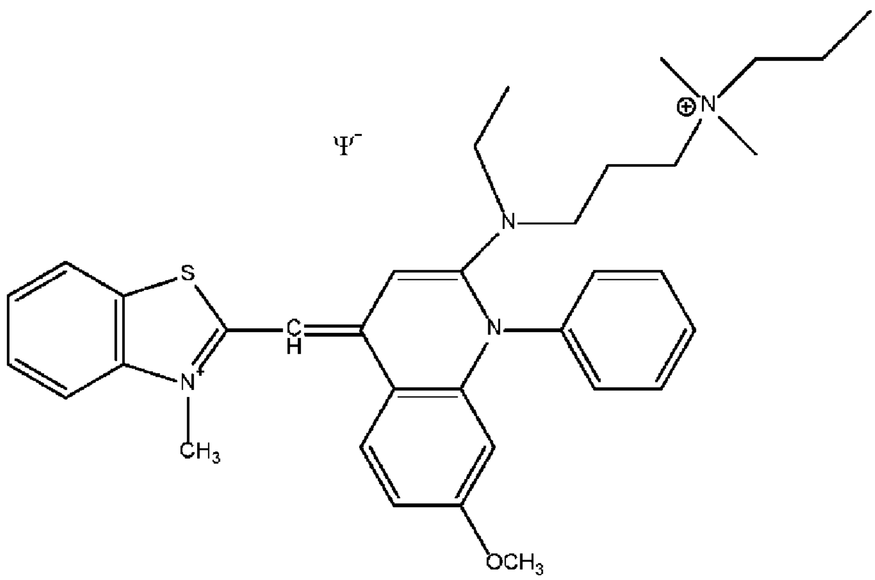

le Composé **60**

ou

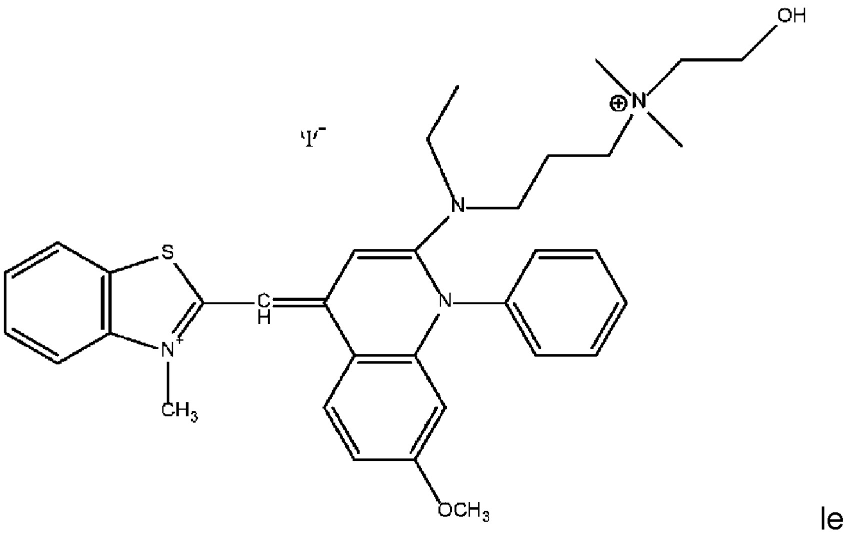

le Composé **61**,

dans laquelle Ψ est un contre-ion biologiquement compatible ; et le second colorant de liaison d'acide nucléique est

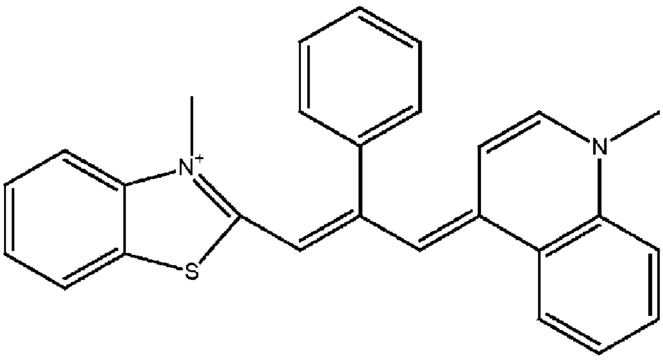

le Composé **1**,

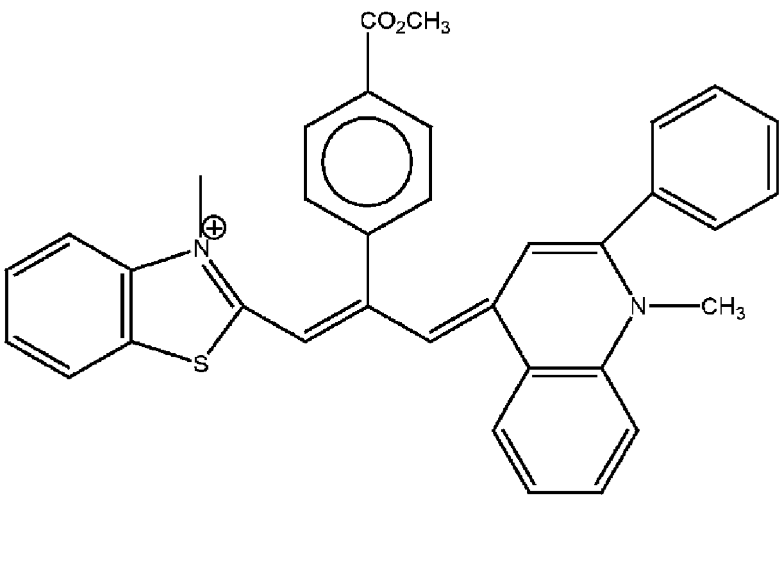

le Composé **2**,

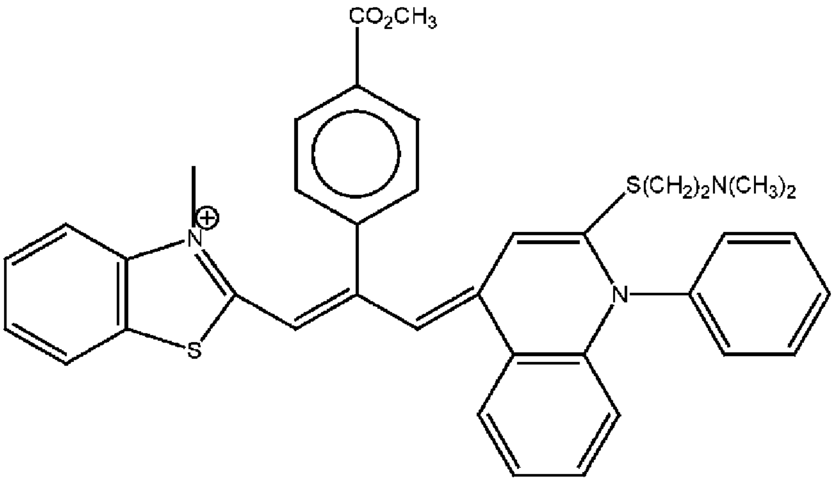

le Composé **3**,

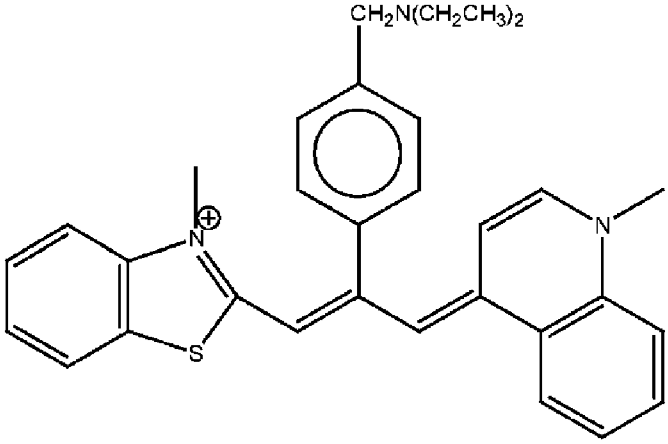

le Composé **4**,

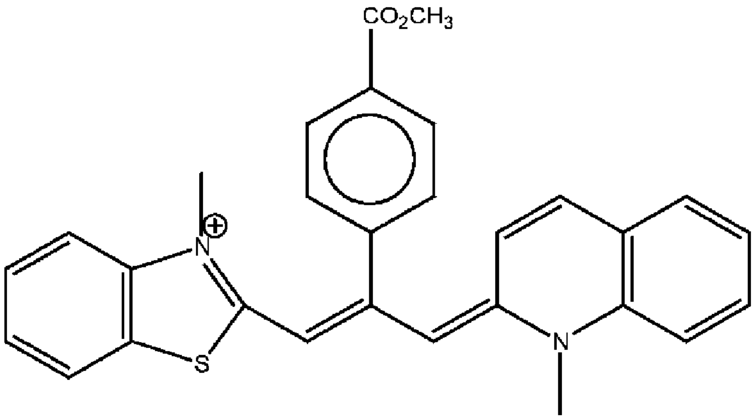

le Composé **7**,

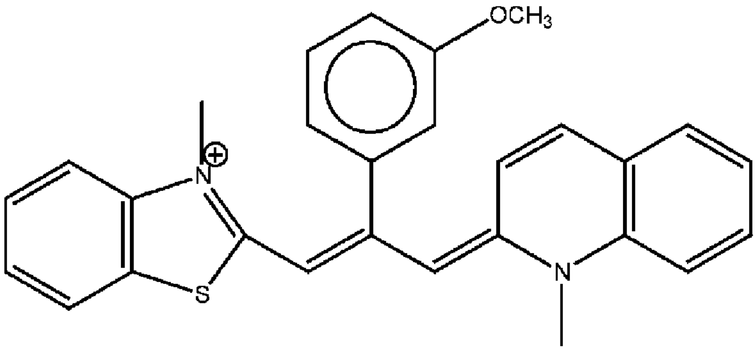

le Composé **9**,

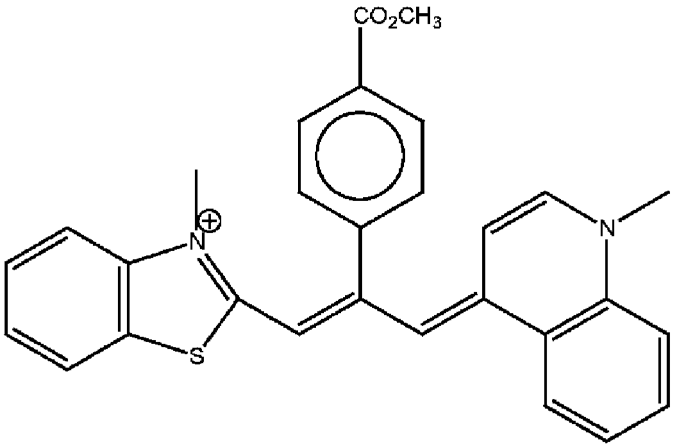

le Composé **10**,

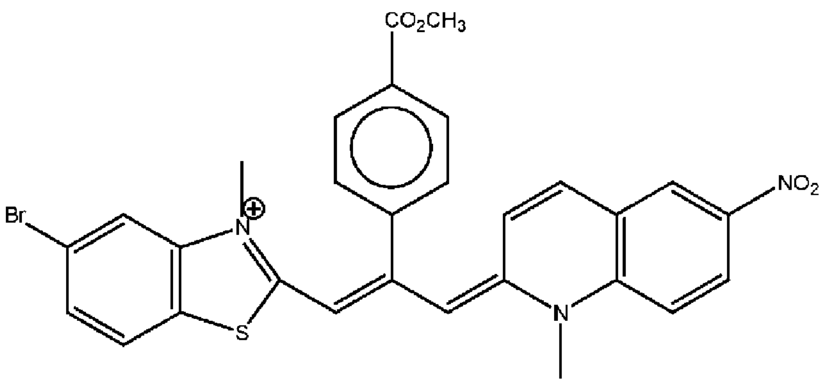

le Composé **12**,

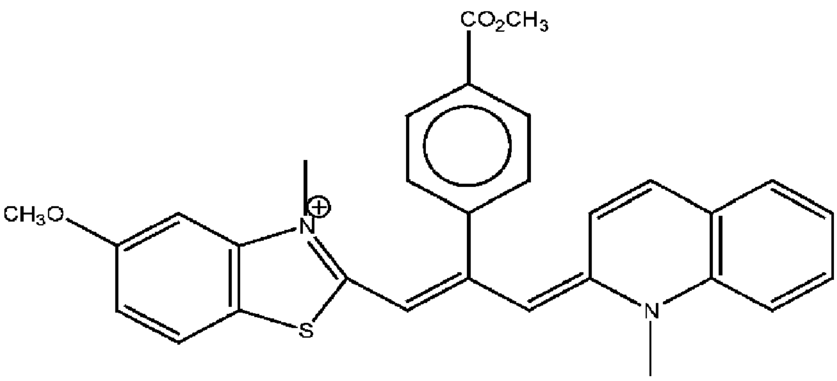

le Composé **13**,

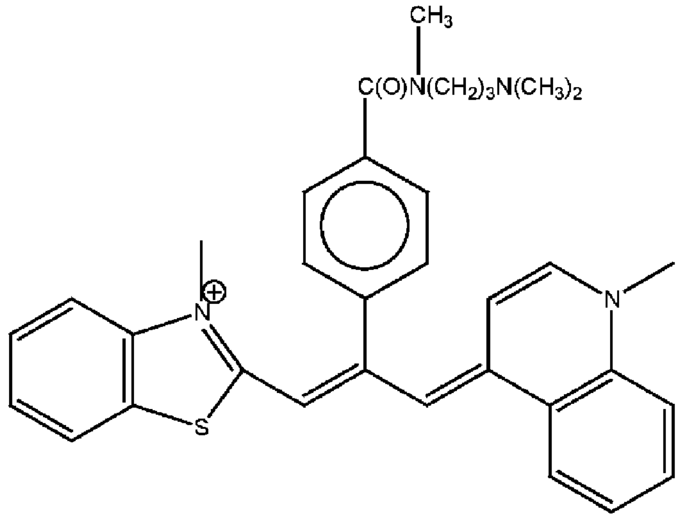

le Composé **14**

ou

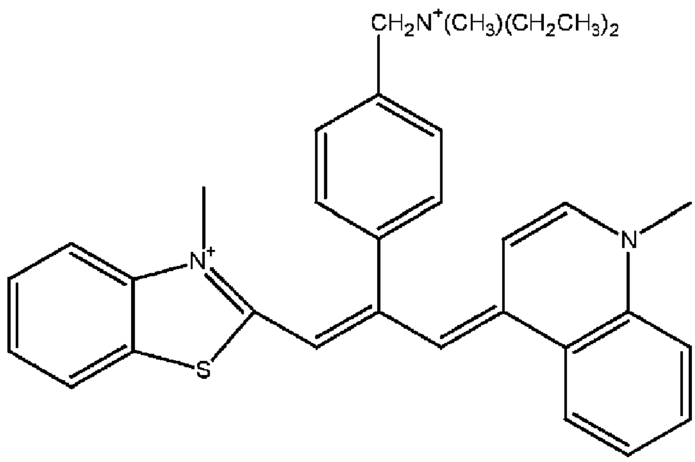

le Composé **15**.

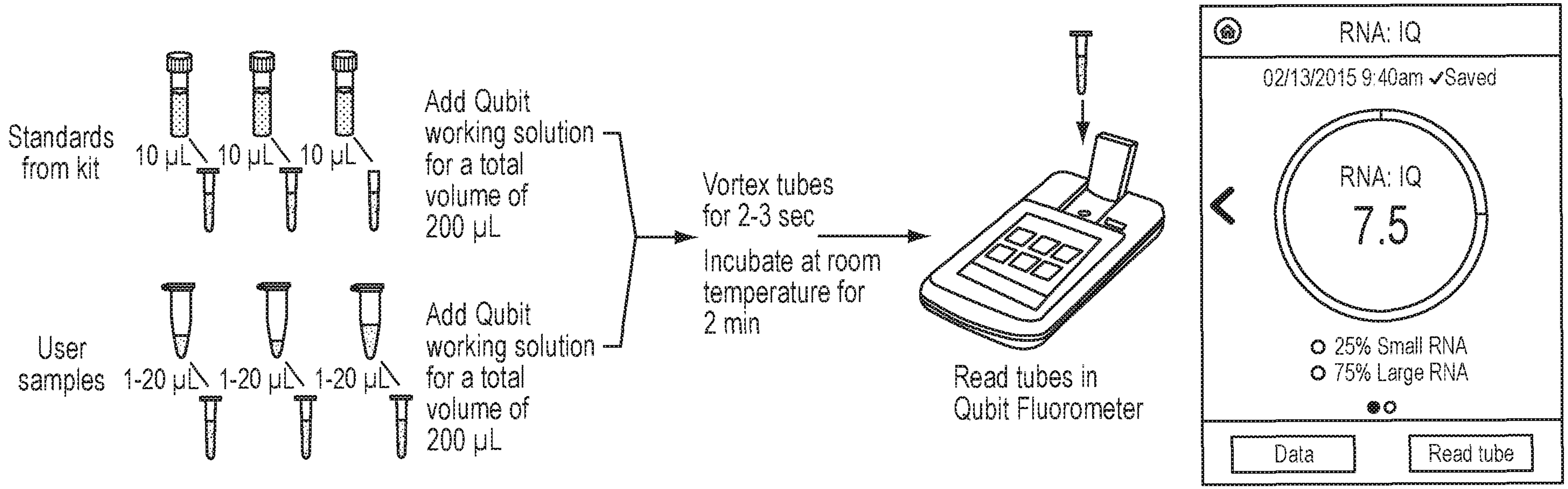
Standards
from kit
10 µL
10 µL
10 µL
User
samples
1-20 µL
1-20 µL
1-20 µL
Add Qubit
working solution
for a total
volume of
200 µL
Add Qubit
working solution
for a total
volume of
200 µL
Vortex tubes
for 2-3 sec
Incubate at room
temperature for
2 min
Read tubes in
Qubit Fluorometer
RNA: IQ
02/13/2015 9:40am ✓Saved
RNA: IQ
7.5
25% Small RNA
75% Large RNA
Data
Read tube

FIG. 1

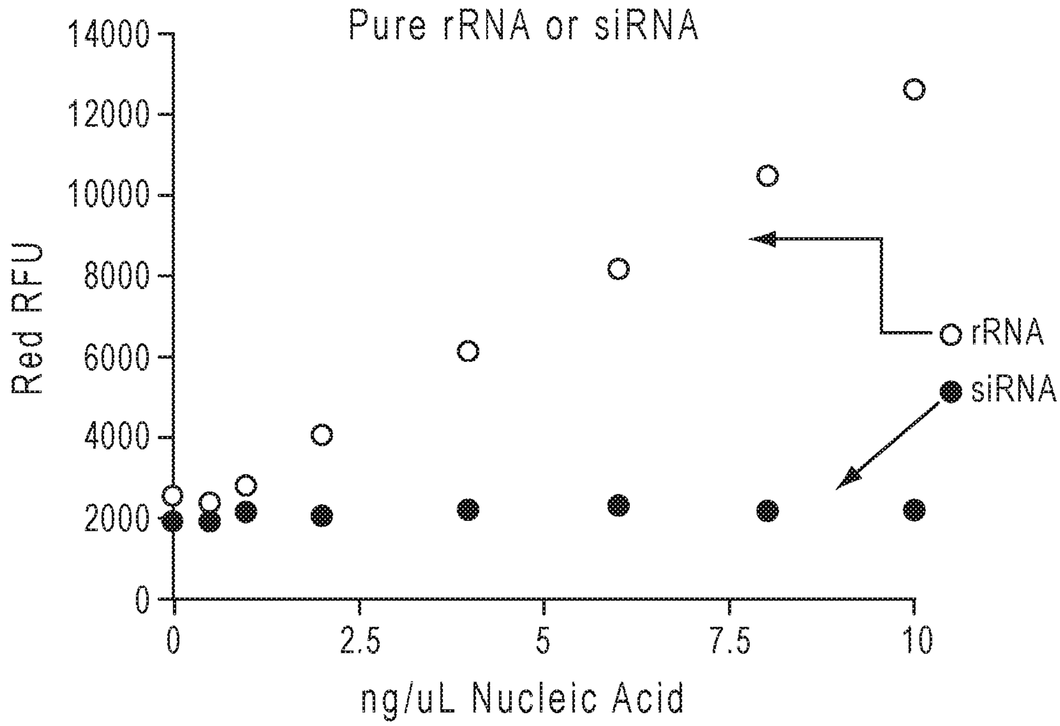
Pure rRNA or siRNA
Red RFU
14000
12000
10000
8000
6000
4000
2000
0
0
2.5
5
7.5
10
ng/uL Nucleic Acid
rRNA
siRNA

FIG. 2A

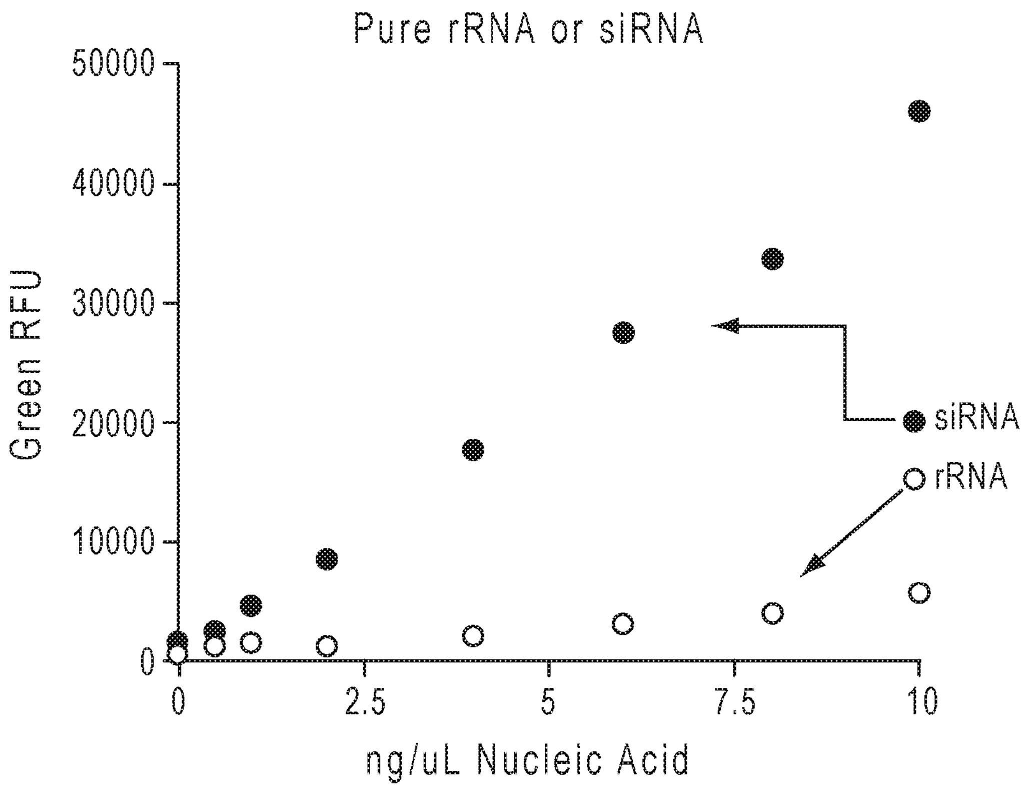
Pure rRNA or siRNA
Green RFU
50000
40000
30000
20000
10000
0
0
2.5
5
7.5
10
ng/uL Nucleic Acid
siRNA
rRNA

FIG. 2B

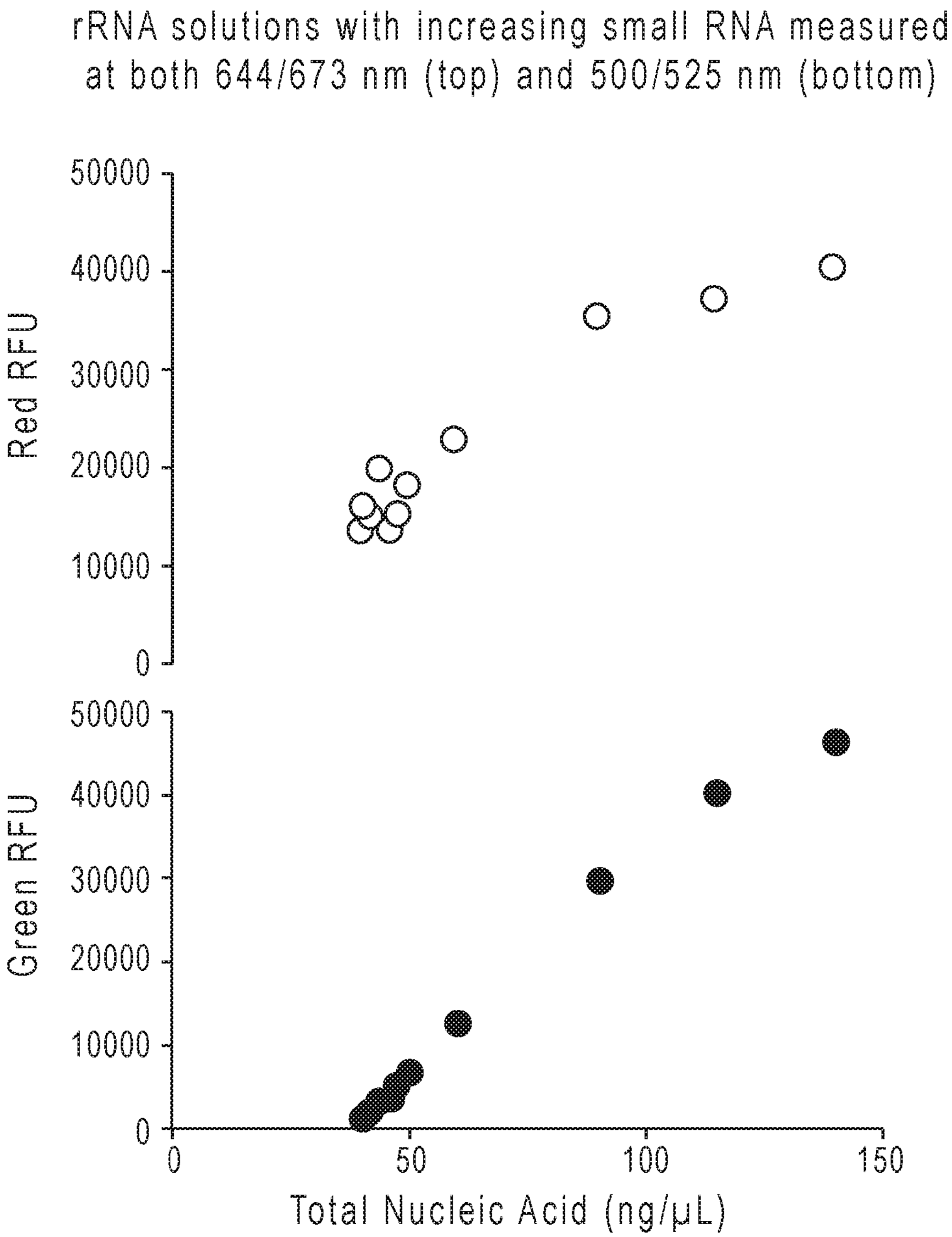
rRNA solutions with increasing small RNA measured
at both 644/673 nm (top) and 500/525 nm (bottom)
Red RFU
50000
40000
30000
20000
10000
0
Green RFU
50000
40000
30000
20000
10000
0
0
50
100
150
Total Nucleic Acid (ng/μL)

FIG. 3A

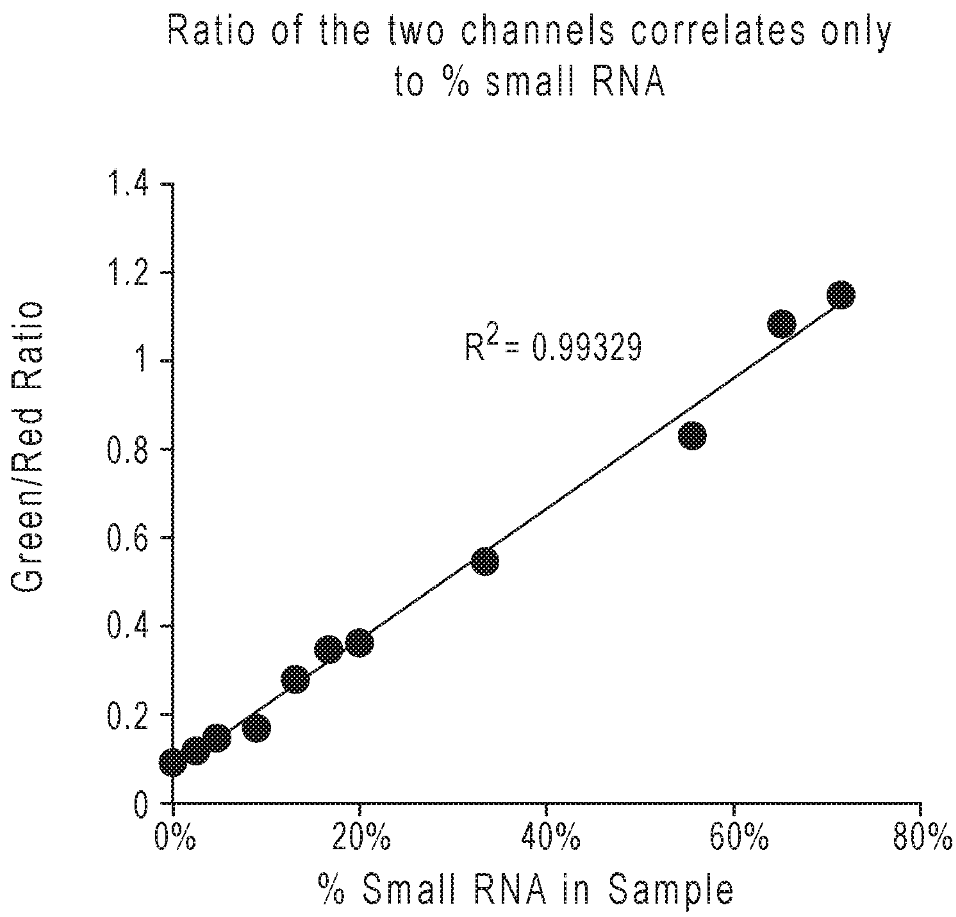
Ratio of the two channels correlates only
to % small RNA
Green/Red Ratio
1.4
1.2
1
0.8
0.6
0.4
0.2
0
R2= 0.99329
0%
20%
40%
60%
80%
% Small RNA in Sample

FIG. 3B

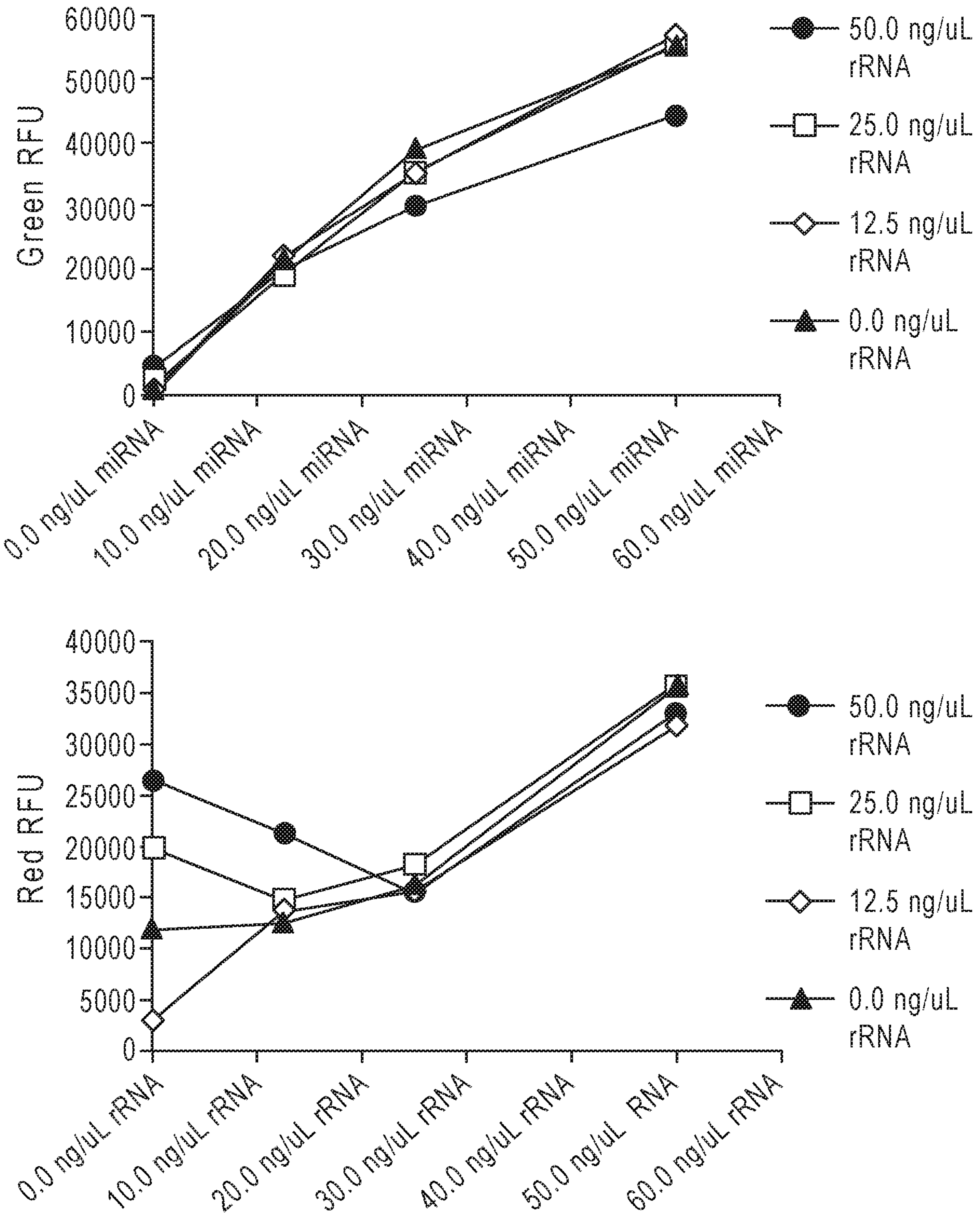
60000
50000
40000
30000
20000
10000
0
Green RFU
0.0 ng/uL miRNA
10.0 ng/uL miRNA
20.0 ng/uL miRNA
30.0 ng/uL miRNA
40.0 ng/uL miRNA
50.0 ng/uL miRNA
60.0 ng/uL miRNA
50.0 ng/uL rRNA
25.0 ng/uL rRNA
12.5 ng/uL rRNA
0.0 ng/uL rRNA
40000
35000
30000
25000
20000
15000
10000
5000
0
Red RFU
0.0 ng/uL rRNA
10.0 ng/uL rRNA
20.0 ng/uL rRNA
30.0 ng/uL rRNA
40.0 ng/uL rRNA
50.0 ng/uL RNA
60.0 ng/uL rRNA
50.0 ng/uL rRNA
25.0 ng/uL rRNA
12.5 ng/uL rRNA
0.0 ng/uL rRNA

FIG. 4A

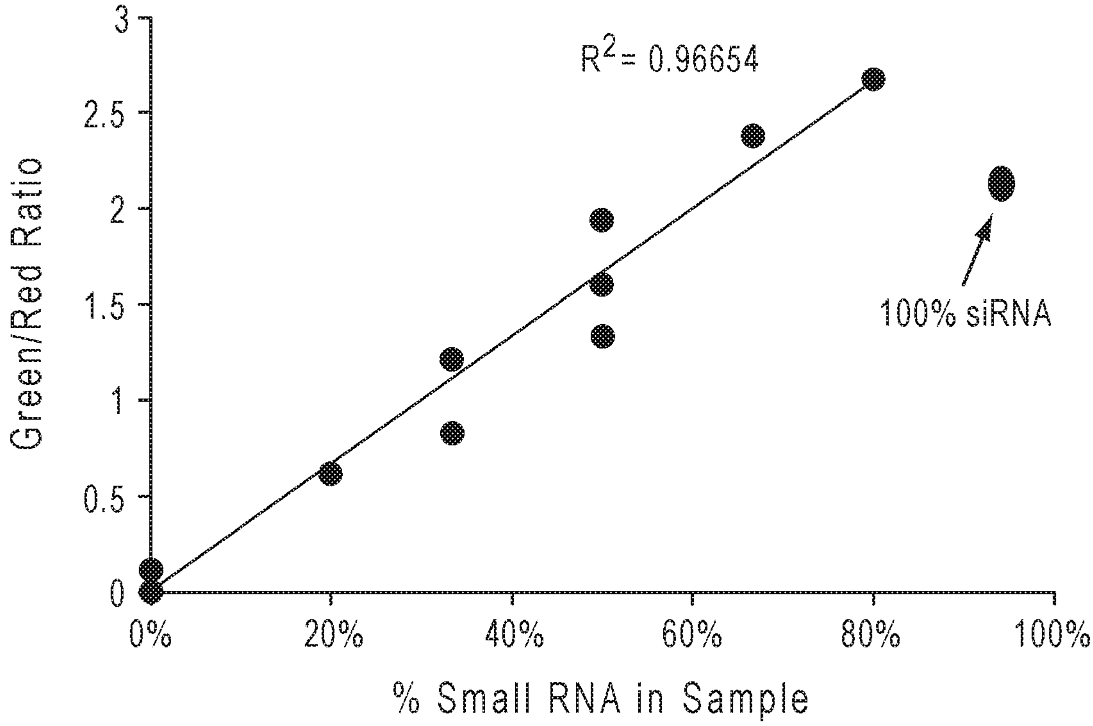
3
2.5
2
1.5
1
0.5
0
Green/Red Ratio
$R^2$= 0.96654
100% siRNA
0%
20%
40%
60%
80%
100%
% Small RNA in Sample

FIG. 4B

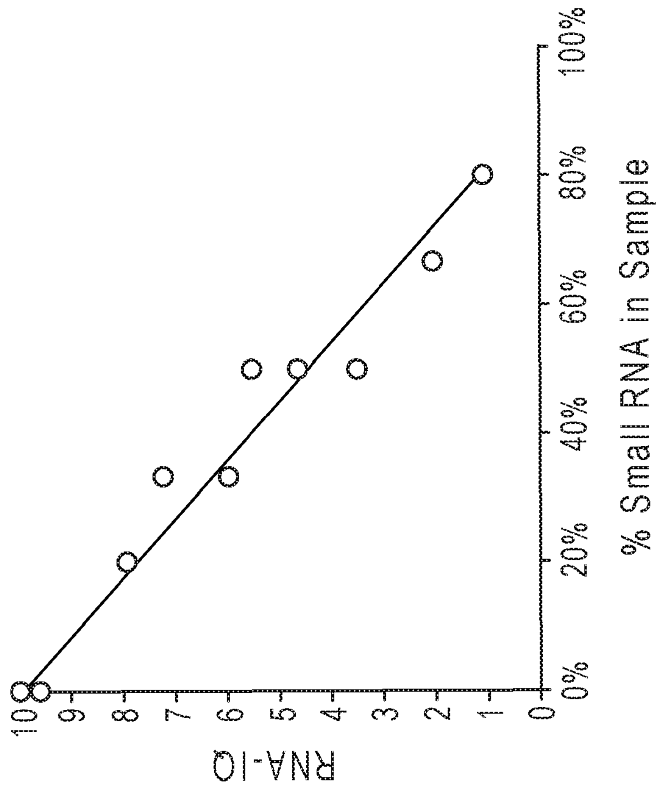
RNA-IQ
10
9
8
7
6
5
4
3
2
1
0
0%
20%
40%
60%
80%
100%
% Small RNA in Sample

FIG. 5B

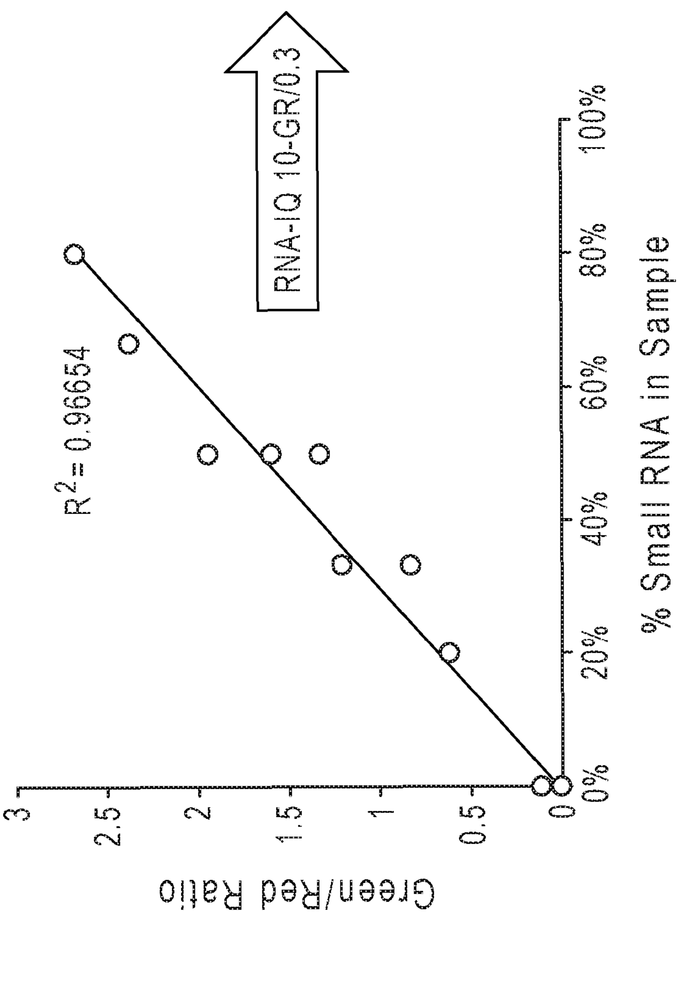
RNA-IQ 10-GR/0.3
$R^2$= 0.96654
Green/Red Ratio
3
2.5
2
1.5
1
0.5
0
0%
20%
40%
60%
80%
100%
% Small RNA in Sample

FIG. 5A

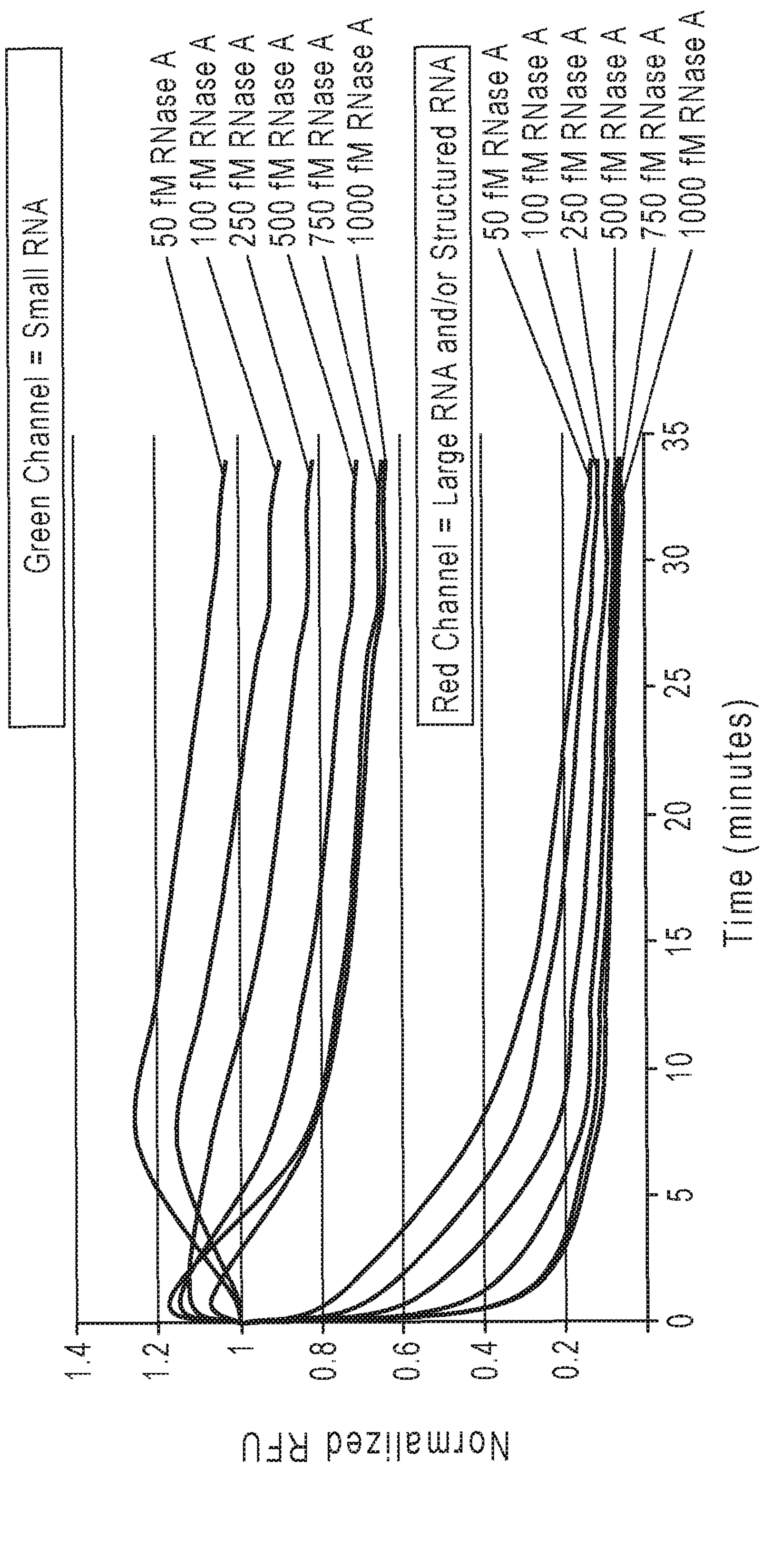
Green Channel = Small RNA
50 fM RNase A
100 fM RNase A
250 fM RNase A
500 fM RNase A
750 fM RNase A
1000 fM RNase A
Red Channel = Large RNA and/or Structured RNA
50 fM RNase A
100 fM RNase A
250 fM RNase A
500 fM RNase A
750 fM RNase A
1000 fM RNase A
Normalized RFU
1.4
1.2
1
0.8
0.6
0.4
0.2
0
5
10
15
20
25
30
35
Time (minutes)

FIG. 6

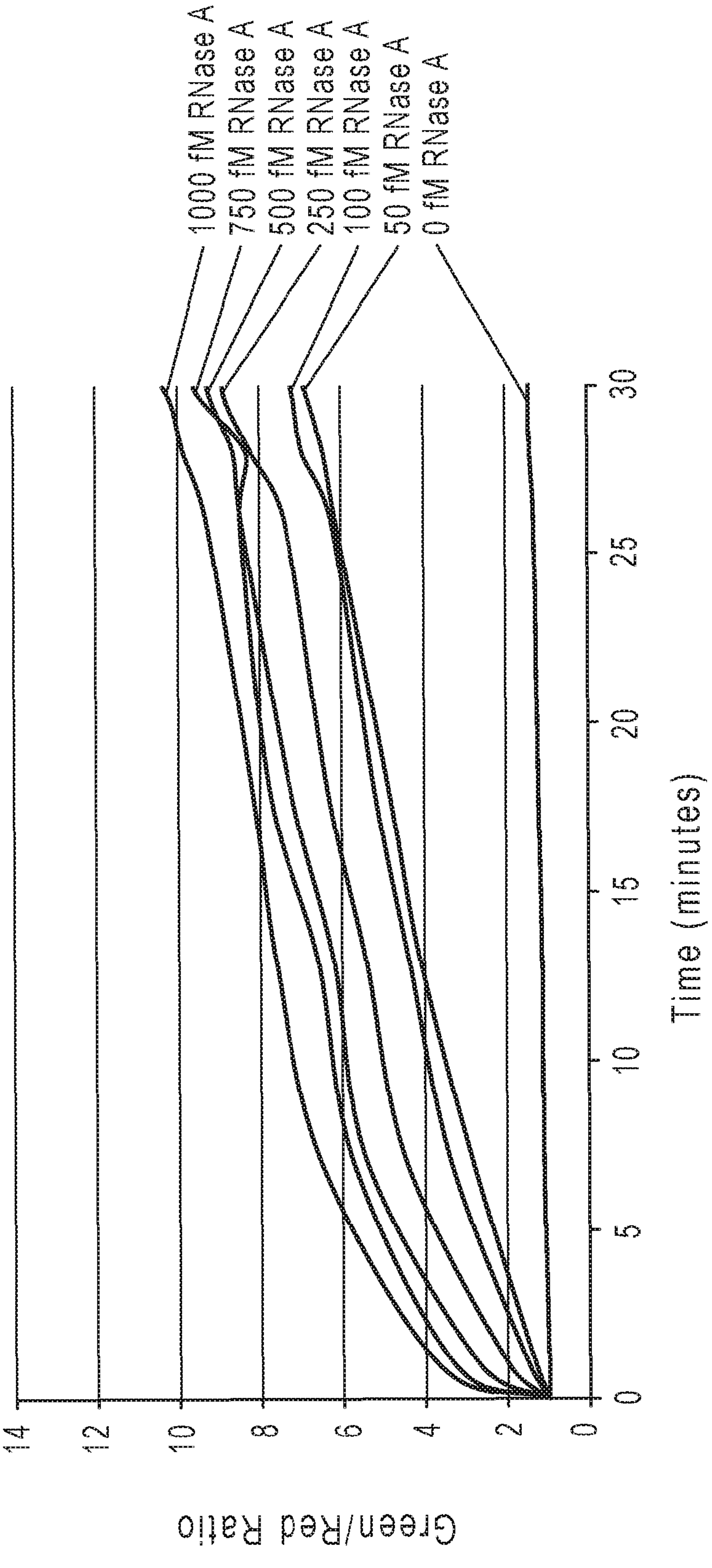
1000 fM RNase A
750 fM RNase A
500 fM RNase A
250 fM RNase A
100 fM RNase A
50 fM RNase A
0 fM RNase A
Green/Red Ratio
0
2
4
6
8
10
12
14
Time (minutes)
0
5
10
15
20
25
30

FIG. 7

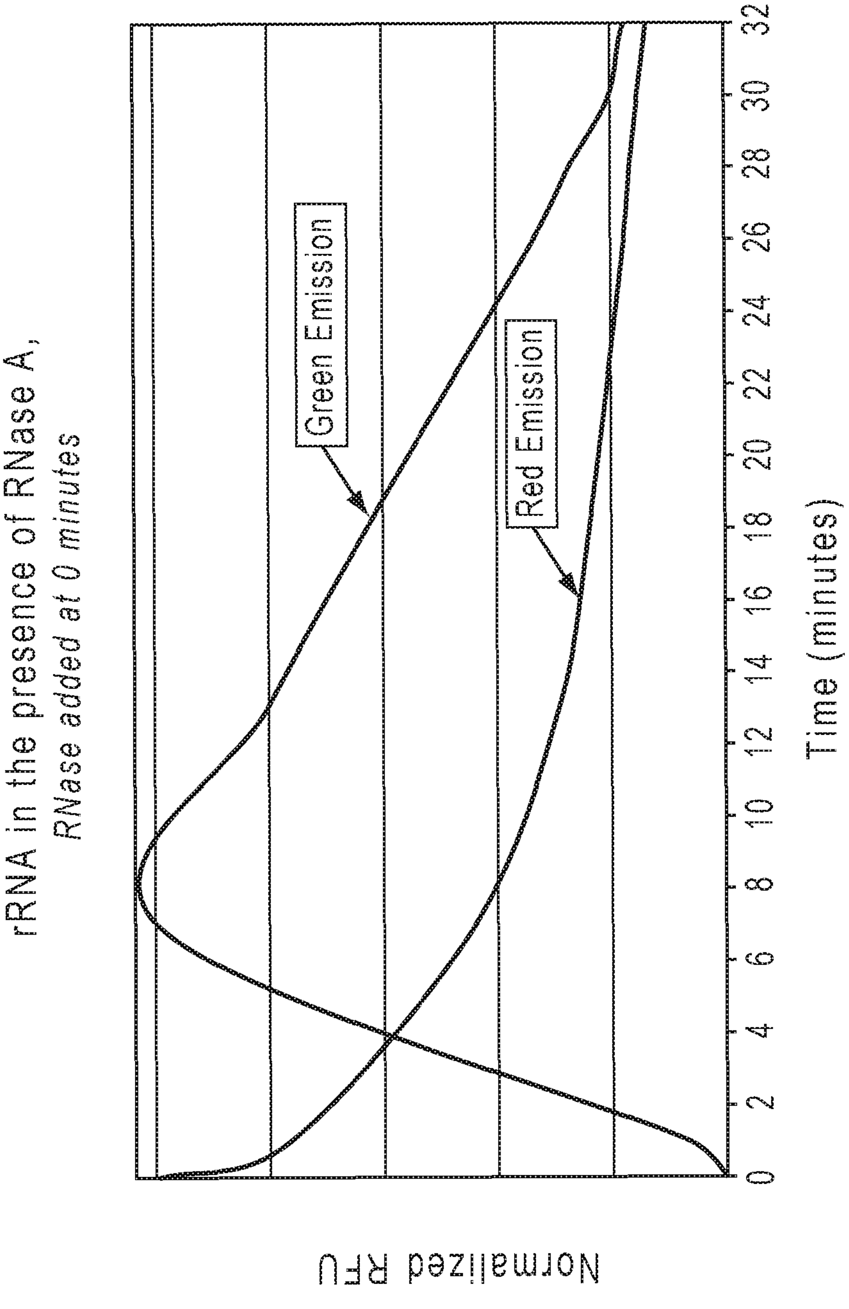
rRNA in the presence of RNase A,
RNase added at 0 minutes
Green Emission
Red Emission
Normalized RFU
0
2
4
6
8
10
12
14
16
18
20
22
24
26
28
30
32
Time (minutes)

FIG. 8

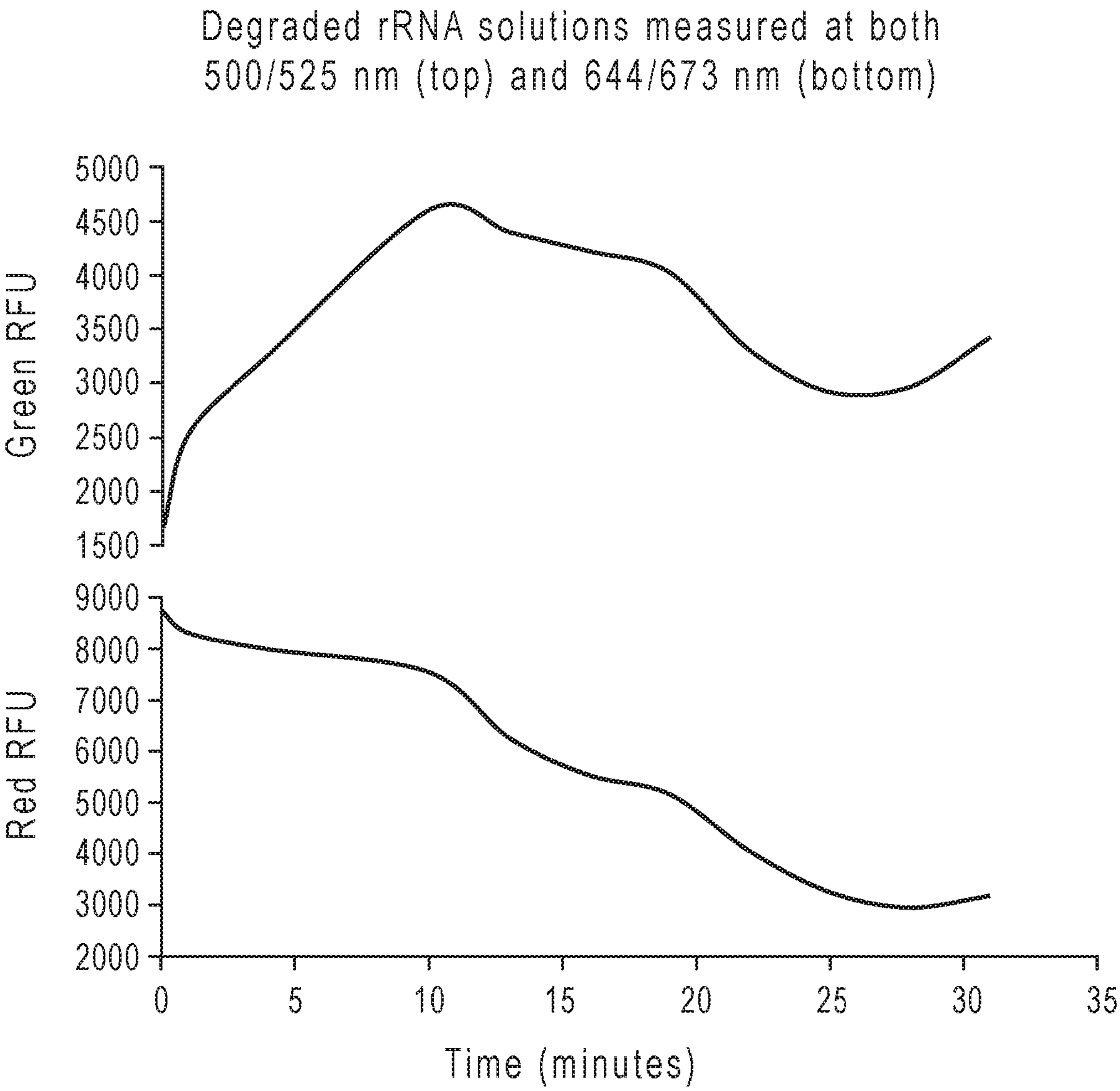
Degraded rRNA solutions measured at both
500/525 nm (top) and 644/673 nm (bottom)
Green RFU
5000
4500
4000
3500
3000
2500
2000
1500
Red RFU
9000
8000
7000
6000
5000
4000
3000
2000
0
5
10
15
20
25
30
35
Time (minutes)

FIG. 9A

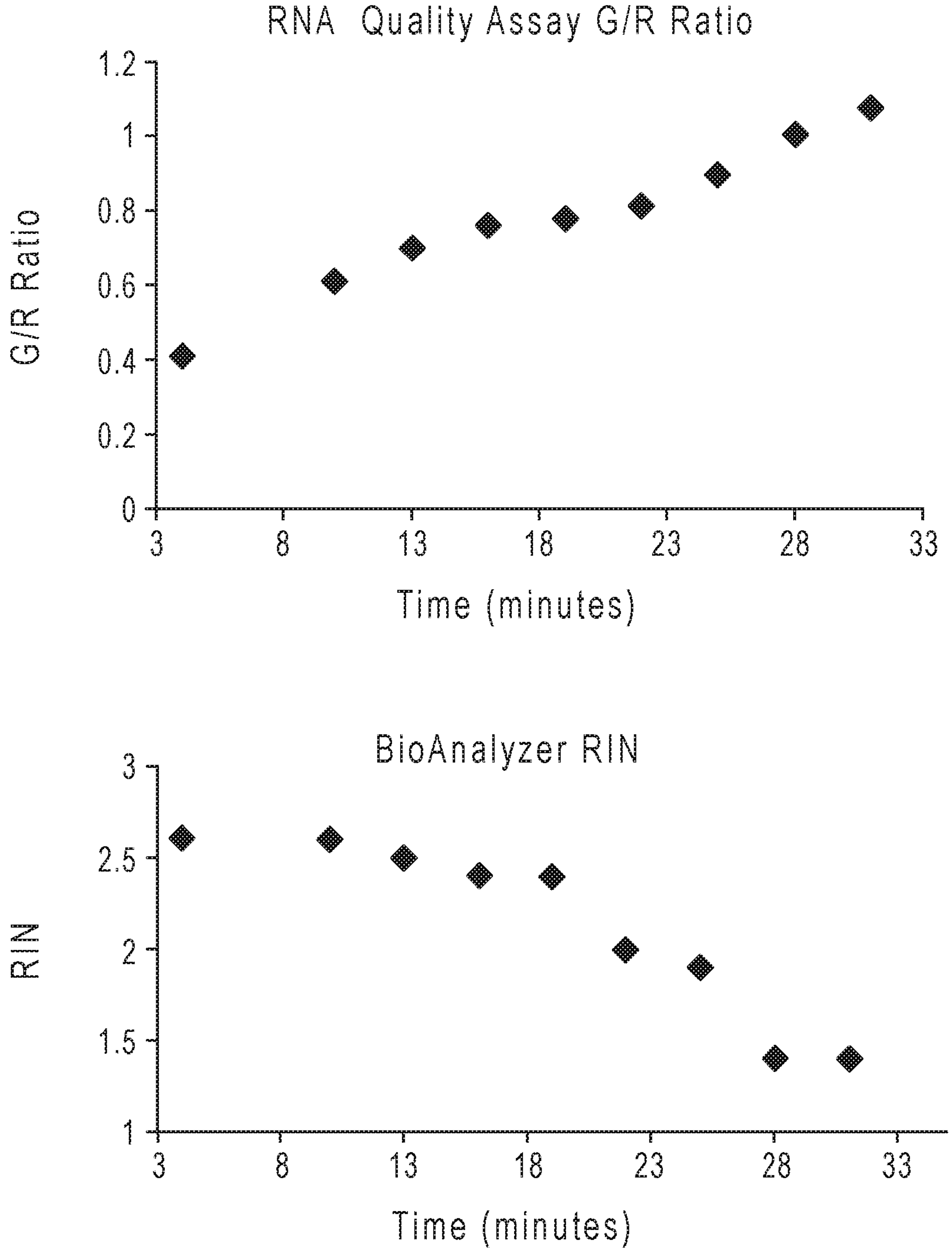
RNA Quality Assay G/R Ratio
G/R Ratio
1.2
1
0.8
0.6
0.4
0.2
0
3
8
13
18
23
28
33
Time (minutes)
BioAnalyzer RIN
RIN
3
2.5
2
1.5
1
3
8
13
18
23
28
33
Time (minutes)

FIG. 9B

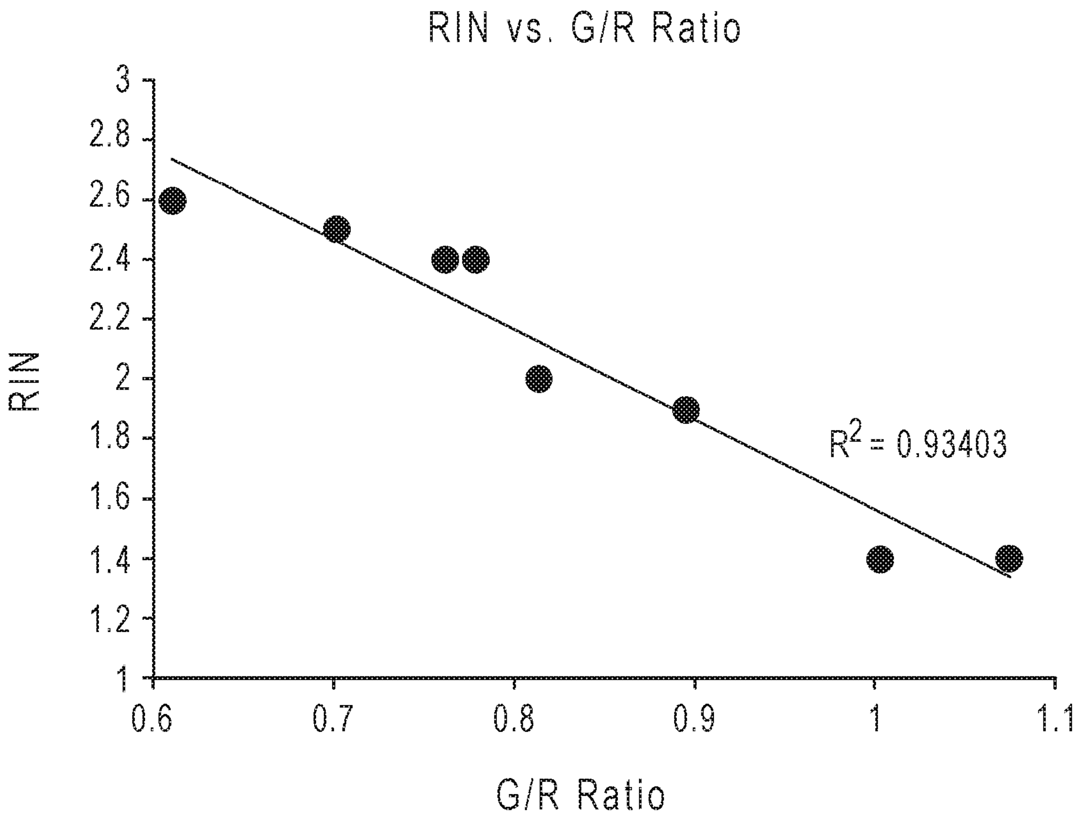

RIN vs. G/R Ratio
RIN
3
2.8
2.6
2.4
2.2
2
1.8
1.6
1.4
1.2
1
0.6
0.7
0.8
0.9
1
1.1
G/R Ratio
$R^2$ = 0.93403


FIG. 9C

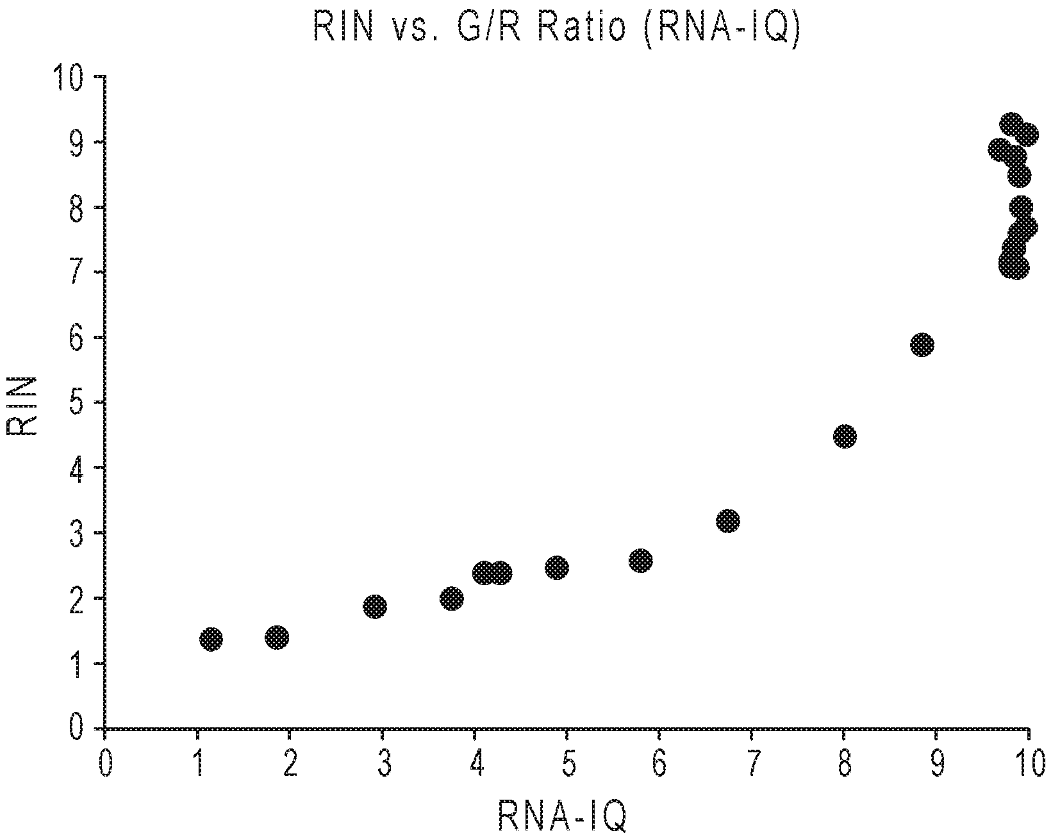


FIG. 9D

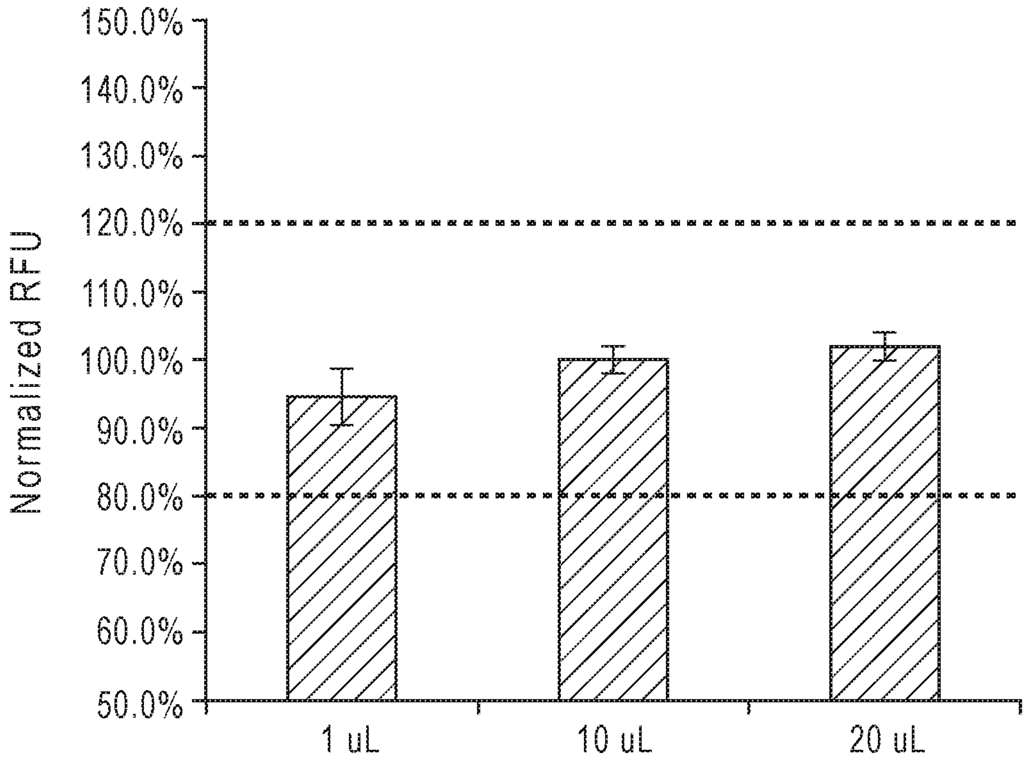
150.0%
140.0%
130.0%
120.0%
110.0%
100.0%
90.0%
80.0%
70.0%
60.0%
50.0%
Normalized RFU
1 uL
10 uL
20 uL

FIG. 10A

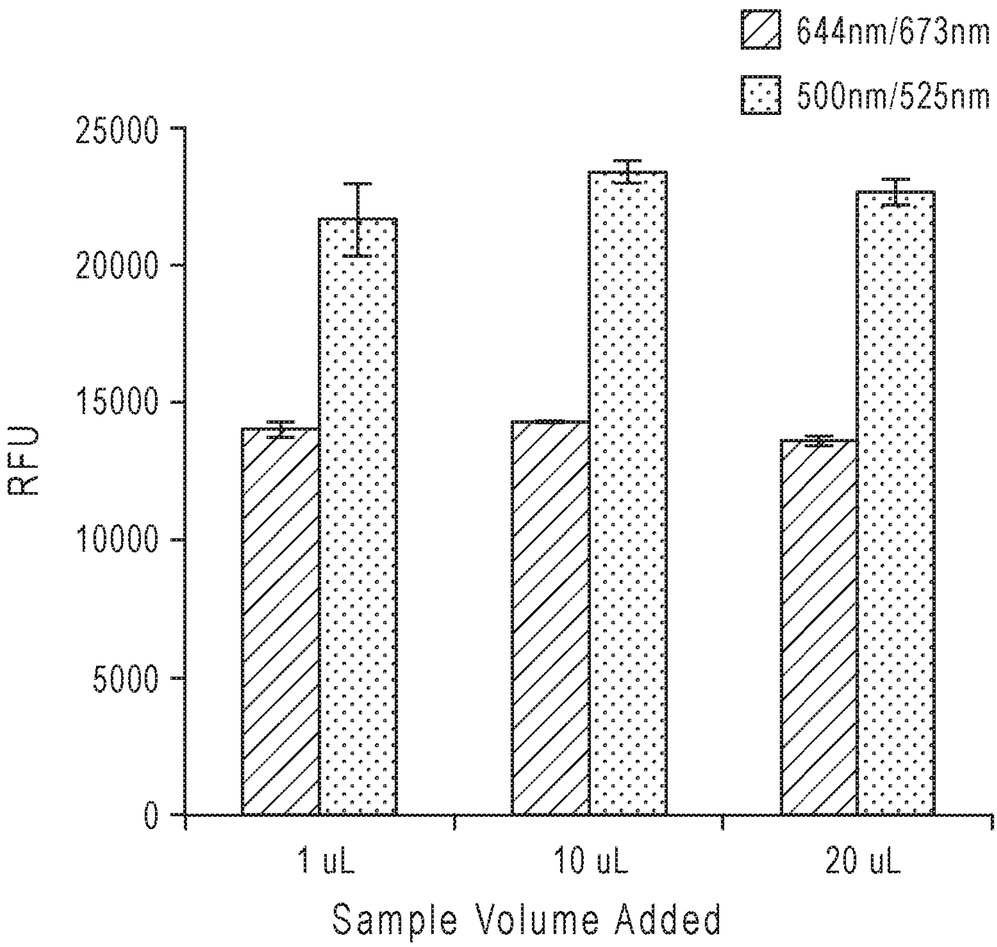
644nm/673nm
500nm/525nm
25000
20000
15000
10000
5000
0
RFU
1 uL
10 uL
20 uL
Sample Volume Added

FIG. 10B

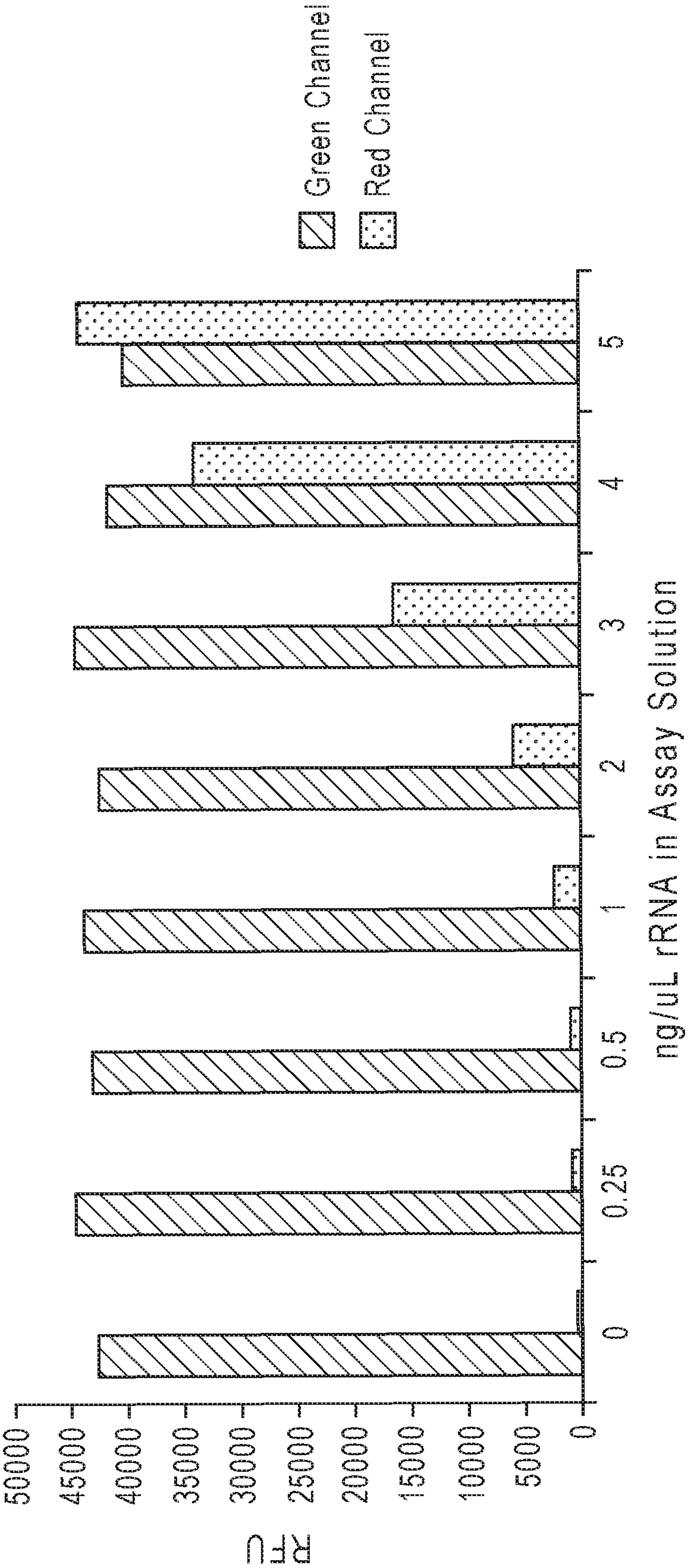
Green Channel
Red Channel
RFU
50000
45000
40000
35000
30000
25000
20000
15000
10000
5000
0
0
0.25
0.5
1
2
3
4
5
ng/uL rRNA in Assay Solution

FIG. 11A

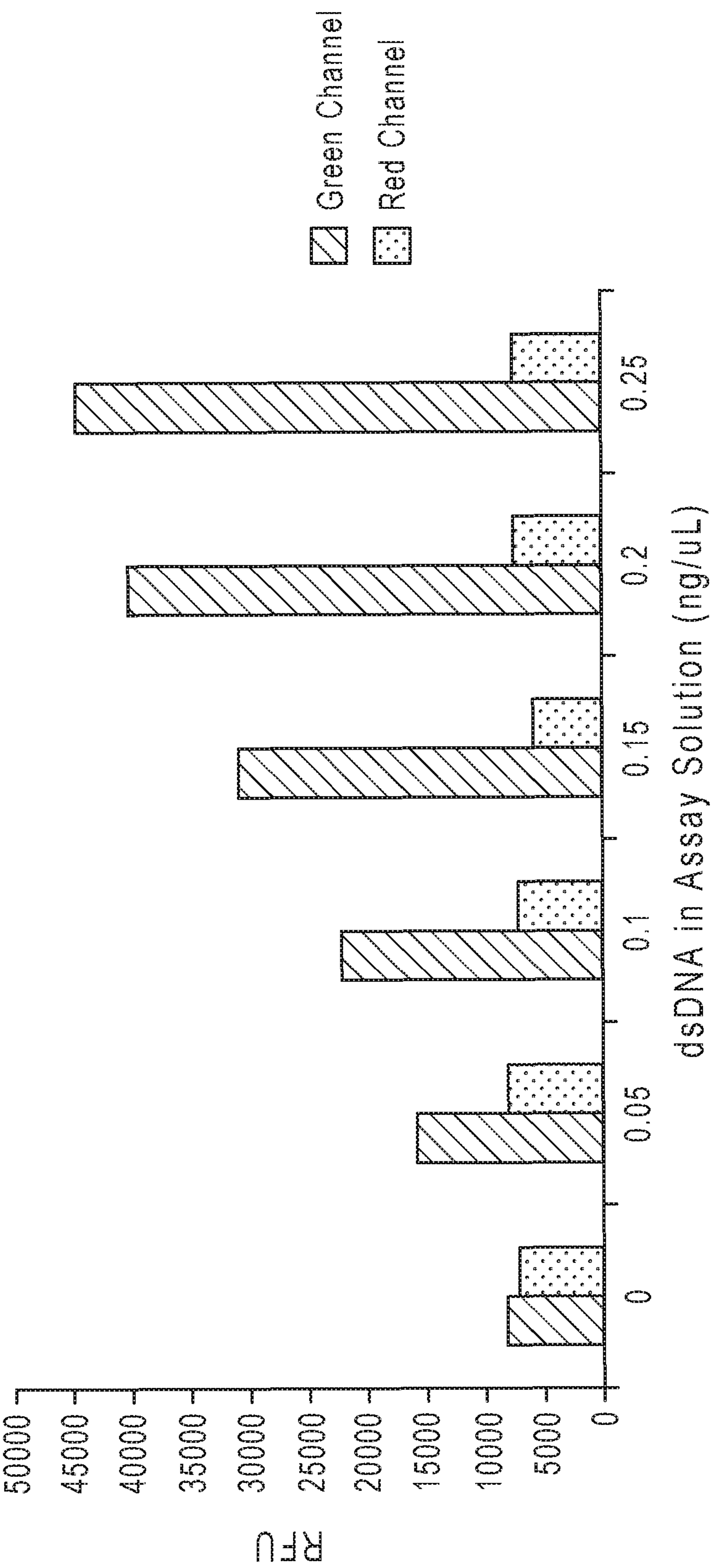
Green Channel
Red Channel
RFU
50000
45000
40000
35000
30000
25000
20000
15000
10000
5000
0
0
0.05
0.1
0.15
0.2
0.25
dsDNA in Assay Solution (ng/uL)

FIG. 11B

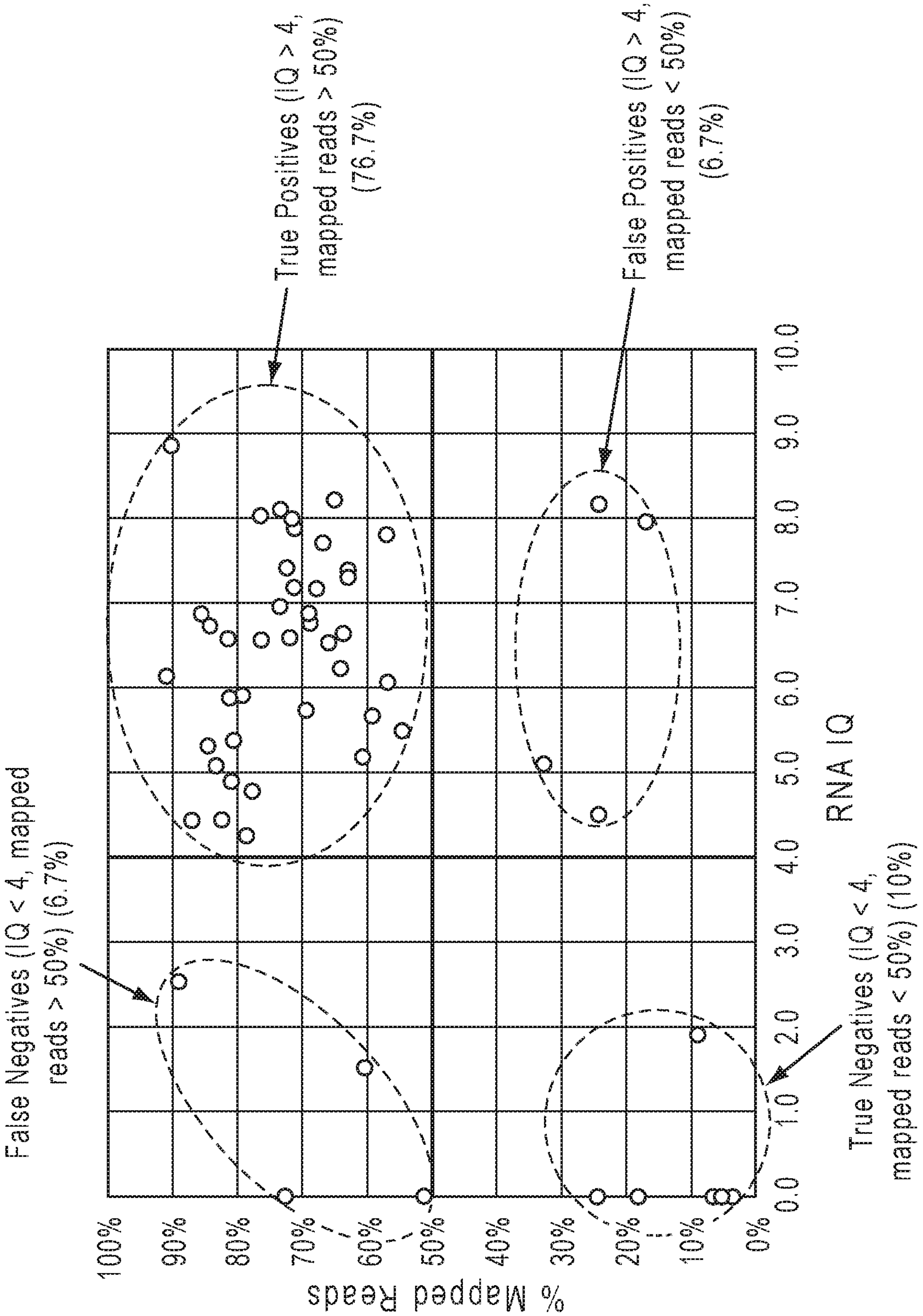
False Negatives (IQ < 4, mapped reads > 50%) (6.7%)
True Positives (IQ > 4, mapped reads > 50%) (76.7%)
False Positives (IQ > 4, mapped reads < 50%) (6.7%)
True Negatives (IQ < 4, mapped reads < 50%) (10%)
% Mapped Reads
100%
90%
80%
70%
60%
50%
40%
30%
20%
10%
0%
0.0
1.0
2.0
3.0
4.0
5.0
6.0
7.0
8.0
9.0
10.0
RNA IQ

FIG. 12

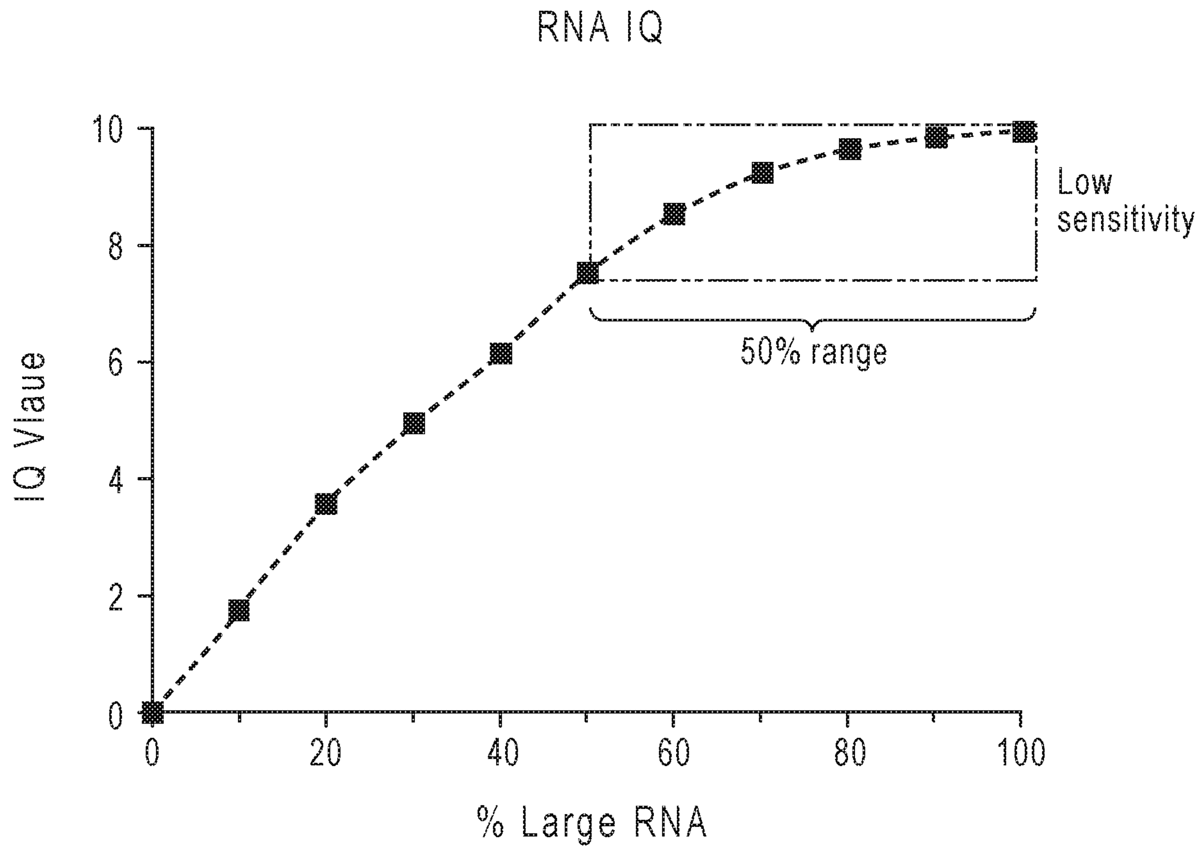
RNA IQ
10
8
6
4
2
0
IQ Viaue
0
20
40
60
80
100
% Large RNA
Low
sensitivity
50% range

FIG. 13

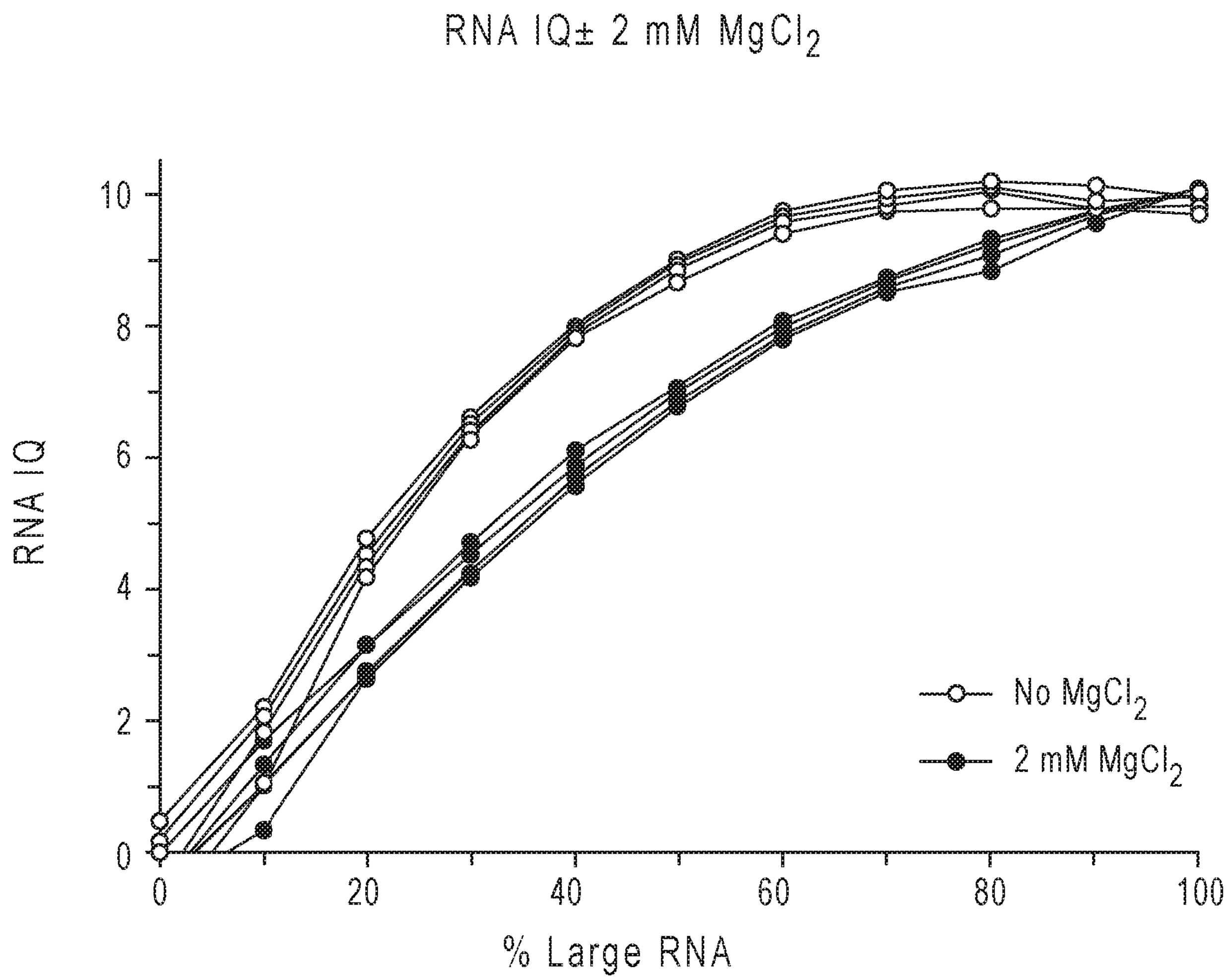
RNA IQ± 2 mM $MgCl_2$
RNA IQ
0
2
4
6
8
10
0
20
40
60
80
100
% Large RNA
No $MgCl_2$
2 mM $MgCl_2$

FIG. 14

FIG. 15A

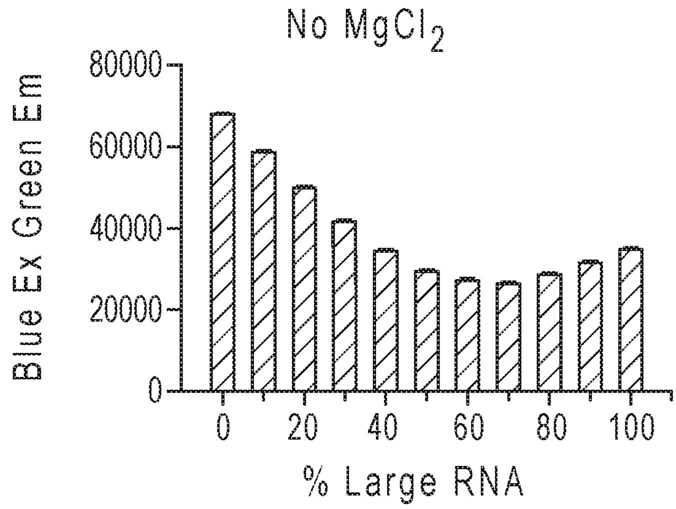


FIG. 15B

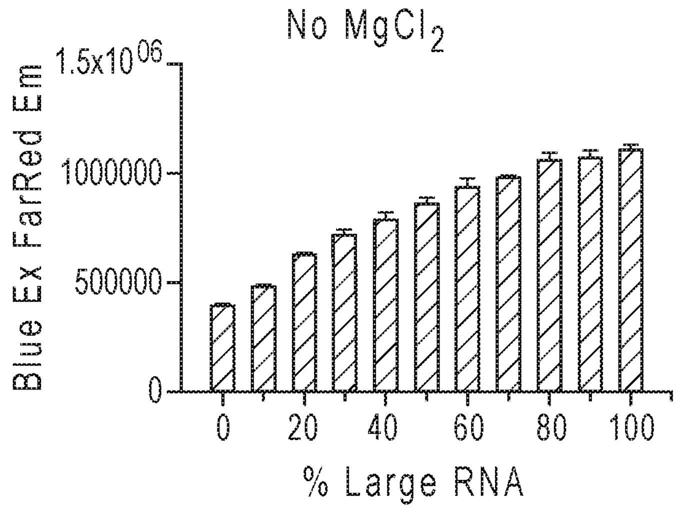


FIG. 15C

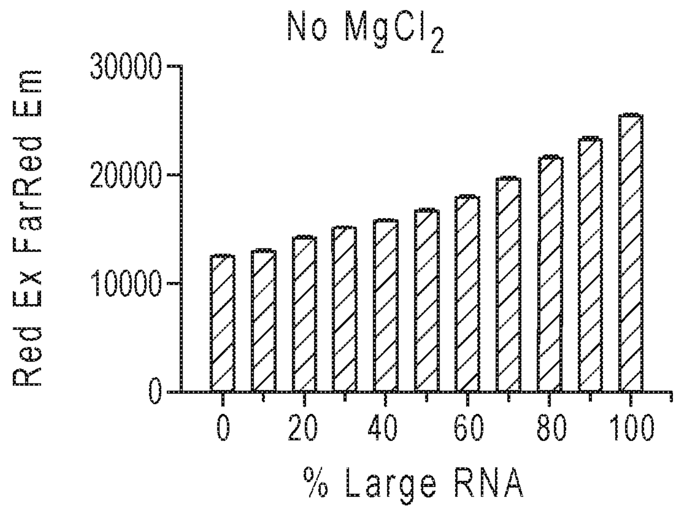

FIG. 15D

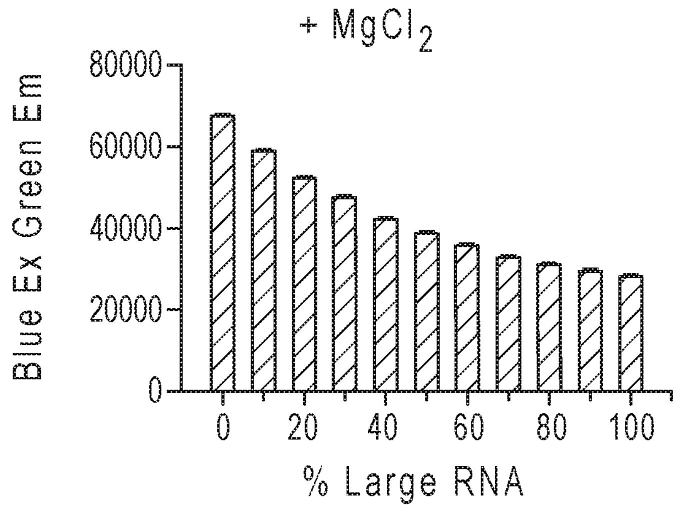


FIG. 15E

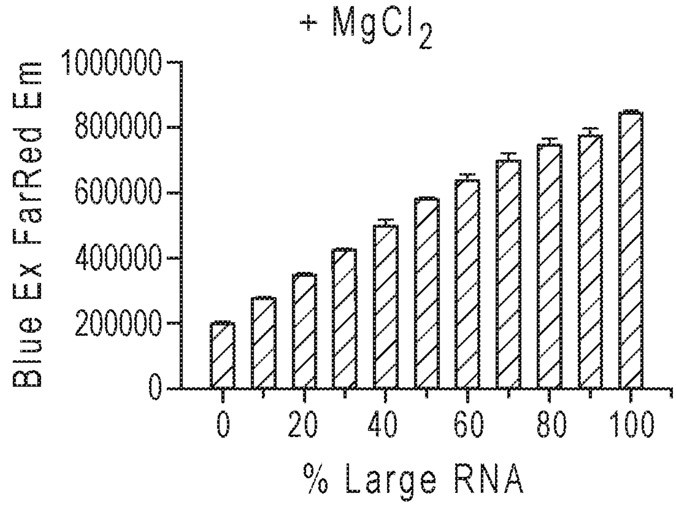


FIG. 15F

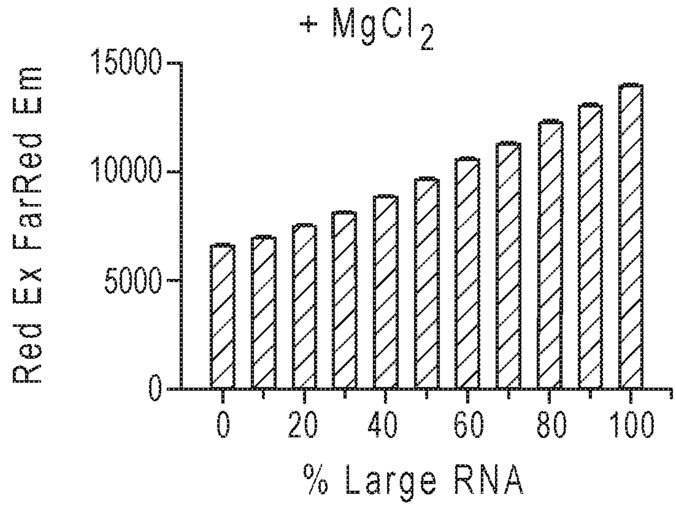

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62568896
- US 8346486 B
- US 4957870 A
- US 4883867 A
- US 5436134 A
- US 5658751 A
- US 5534416 A
- US 5863753 A
- WO 2014089421 A
- US 7776529 B
- US 8470529 B
- US 9040561 B
- US 9403985 B
- US 8623282 B

### Non-patent literature cited in the description

- **TZU-HSUEH YANG et al.** Determination of RNA degradation by capillary electrophoresis with cyan lightemitted diode-induced fluorescence. *JOURNAL OF CHROMATOGRAPHY A,* 21 March 2012, vol. 1239, 78-84